# EUROPEAN PATENT APPLICATION

(11) **EP 4 574 823 A1**
(43) Date of publication of application: **25.06.2025**
(21) Application number: 23855176.6
(22) Date of filing: 17.08.2023
(51) Int. Cl.: C07D 473/34, A61K 31/52, A61P 35/00, A61P 31/18

(54) **COMPOUNDS FOR INHIBITING OR DECOMPOSING CDK2 AND/OR CDK9, AND MEDICINAL USES THEREOF**

(30) Priority: 17.08.2022 KR 20220102979
(71) Applicant: Korea Research Institute of Chemical Technology, Daejeon 34114 (KR); Korea Research Institute of Bioscience and Biotechnology, Daejeon 34141 (KR)
(72) Inventor: HWANG, Jong Yeon, Daejeon 34114 (KR); HA, Jae Du, Daejeon 34114 (KR); CHO, Sung Yun, Daejeon 34114 (KR); KIM, Pilho, Daejeon 34114 (KR); PARK, Chi Hoon, Daejeon 34114 (KR); KIM, Hyun Jin, Daejeon 34114 (KR); KIM, Jeong Hoon, Daejeon 34141 (KR); CHO, Jin Hwa, Daejeon 34141 (KR); PARK, Byoung Chul, Daejeon 34141 (KR); PARK, Sung Goo, Daejeon 34141 (KR); KIM, Jung-Ae, Daejeon 34141 (KR); CHOI, Ji Hoon, Yongin-si, Gyeonggi-do 17106 (KR); LEE, Hye Yeon, Yongin-si, Gyeonggi-do 17072 (KR); LEE, Yujin, Gimcheon-si, Gyeongsangbuk-do 39589 (KR)
(74) Representative: Dolphin, Kirsty Mairi
(86) International application number: PCT/KR2023/012222
(87) International publication number: WO 2024/039210

(57) **Abstract**

The present invention provides a compound of a certain chemical structure or a pharmaceutically acceptable salt thereof, exhibiting activity of inhibiting or degrading CDK2 and/or CDK9, and a method for preparing thereof. The present invention provides a composition comprising the compound or a pharmaceutically acceptable salt thereof. The present invention provides use of the compound, a salt thereof, or a composition comprising the same in inhibiting or degrading CDK2 and/or CDK9 and medicinal use of the same in preventing or treating a disease associated therewith. The present invention further provides method for preventing or treating a disease associated with CDK2 and/or CDK9, comprising administering the compound, a salt thereof, or a composition comprising the same to a subject in need of treatment.

## Description

### [Technical Field]

The present invention relates to a group of compounds having activity of inhibiting or degrading cyclin-dependent kinase 2 (CDK2) and/or cyclin-dependent kinase 9 (CDK9), and a method for preparing thereof. The present invention also relates to a pharmaceutical composition comprising these compounds. The present invention relates to a useful method for treating a disease associated with CDK2 or CDK9 using these compounds. In other words, the present invention relates to medicinal use of the compounds according to the present invention in treating or preventing a disease associated with CDK2 or CDK9.

### [Background Art]

Cyclin-dependent kinase (CDK) is a serine/threonine protein kinase that is activated through binding with cyclins. CDK has multiple isoforms, with 21 CDK isoforms being expressed in humans. Each CDK performs distinct roles within cells; however, in terms of functions, the CDKs can be broadly categorized into those involved in cell cycle and those involved in transcription regulation.

CDK2 is one of the CDKs that regulate the cell cycle and is activated through binding with cyclin E or cyclin A. The CDK2-cyclin E complex regulates the entry of cells into the S-phase, while the CDK2-cyclin A complex plays a role in regulating DNA synthesis during the S-phase. The activation of uncontrolled CDK2 induces cells to enter the S-phase even when there is a lack of growth factors, leading to chromosomal instability and increased cell proliferation, thereby resulting in the development of cancer. In fact, CDK2 has been reported to be overexpressed in various types of cancer (prostate cancer, B cell lymphoma, glioblastoma; Cell Cycle, 2007, 6: 2982-2989, Future Oncol., 2018, 14: 709-718, Transl. Oncol., 2016, 9: 548-556) and cyclin E1 (ovarian cancer, TNBC, hepatocellular carcinoma; Oncotarget, 2015, 6: 20801-20812, Clin. Cancer Res., 2017, 23: 1862-1874, Oncol. Rep., 2015, 33: 990-996, Gynecol. Oncol., 2018, 151: 327-336, Oncotarget, 2017, 8: 14897-14911). These research findings suggest that therapeutic effects may be expected in cancer types with CDK2 overexpression through selective degradation of CDK2. Furthermore, International Patent Publication No. WO 2006/040036 discloses that CDK2 inhibitors are useful for the treatment of breast cancer, colon cancer, lung cancer, and prostate cancer.

CDK9 is one of the CDKs that regulate transcription, and CDK9 binds to cyclin T1, cyclin T2, or cyclin K to form a positive transcription elongation factor (P-TEFb) complex. The P-TEFb complex phosphorylates RNA polymerase II, promoting transcription elongation. CDK9 regulates the expression of the transcription factor Myc (Mol. Cancer Ther., 2019, 18: 1520-32) and the anti-apoptotic protein MCL-1 in cancer (Am. J. Manag. Care, 2018, 24: S356-S365). Therefore, the inhibition of CDK9 was found to suppress cell proliferation in various cancers, including prostate cancer, pancreatic cancer, breast cancer, multiple myeloma, and chronic lymphocytic leukemia (CLL) (Endocrine-Related Cancer, 2016, 23, T211-T226). In addition, since human immunodeficiency virus (HIV) utilizes P-TEFb of the host for virus replication, CDK9 is being used as a candidate target protein for inhibiting HIV replication (Cancer Biol. Ther., 2002, 1: 342-347). Furthermore, International Patent Publication WO 2017/001354 discloses that CDK9 inhibitors may be useful in treating cancer such as acute myeloid leukemia, multiple myeloma, chronic lymphocytic leukemia, diffuse large B-cell lymphoma, Burkitt lymphoma, follicular lymphoma, breast cancer, lung cancer, neuroblastoma, and colorectal cancer, as well as other diseases including cardiovascular diseases, viral diseases, inflammation, and pain.

Meanwhile, Proteolysis Targeting Chimera (PROTAC) technology, which degrades target proteins, has recently been actively utilized in drug development (PNAS, 2001, 98(15): 8554-8559, Cell, 2020, 2: 1-3). PROTAC is a novel therapeutic agent that is manufactured by linking inhibitor or binder compounds that specifically bind to certain proteins and ligand compounds that specifically bind to E3 ubiquitin ligase with various types of linkers. Since PROTAC compounds selectively degrade target proteins that cause diseases, the potential for drug resistance is low, and without high binding affinities to the target proteins, sufficient therapeutic effects are achieved through protein degradation. In addition, they can be applied to undruggable targets, which were difficult to inhibit with conventional drugs. Therefore, PROTAC compounds are gaining attention as a novel therapeutic technology.

However, not every inhibitor (i.e., binding factor) becomes an effective PROTAC compound by binding to an E3 ubiquitin ligase ligand. The effectiveness of the PROTAC compound varies depending on the moieties that are bound, such as the inhibitor, E3 ubiquitin ligase ligand, and linker.

### [Disclosure]

### [Technical Problem]

One object of the present invention is to provide a compound that exhibits activity of inhibiting or degrading CDK2 and/or CDK9, or a pharmaceutically acceptable salt thereof.

Another object of the present invention is to provide a method for preparing the compound or a pharmaceutically acceptable salt thereof.

Still another object of the present invention is to provide a composition comprising the compound or a pharmaceutically acceptable salt thereof, and a pharmaceutically acceptable carrier.

Still another object of the present invention is to provide a pharmaceutical composition for inhibiting or degrading CDK2, CDK9, or both, comprising the compound or a pharmaceutically acceptable salt thereof as an active ingredient.

Still another object of the present invention is to provide a pharmaceutical composition for preventing or treating cancer or HIV infection, comprising the compound or a pharmaceutically acceptable salt thereof as an active ingredient.

Still another object of the present invention is to provide a method for preventing or treating cancer or HIV infection, comprising administering the pharmaceutical composition to a subject in need thereof.

### [Advantageous Effects]

The present invention provides a compound that can exhibit various pharmacological activities by inhibiting or degrading CDK2 and/or CDK9 or a pharmaceutically acceptable salt thereof, a pharmaceutical composition comprising the same as an active ingredient, a medicinal use thereof (particularly for treating or preventing cancer or HIV infection), and a therapeutic method comprising administering the same to a subject in need of treatment or prevention. The compound according to the present invention or a pharmaceutically acceptable salt thereof is superior in various aspects, including safety and stability, and has high selectivity in the aspect of CDK2 and/or CDK9 activity inhibition or degradation, thereby providing excellent medicinal effects.

### [Brief Description of the Drawings]

FIG. 1 is a diagram illustrating the evaluation results of CDK protein degradation activity for certain compounds according to the present invention.
FIG. 2 is a diagram illustrating the evaluation results of cytotoxicity of certain compounds according to the present invention.
FIGS. 3 to 13 are diagrams summarizing the analysis results of 479 compounds synthesized according to the present invention, obtained using a mass spectrometer and/or NMR spectrometer.

### [Detailed Description of Preferred Embodiments]

Each description and embodiment described herein may be applied to other descriptions and embodiments, respectively. That is, all combinations of various elements described herein fall within the scope of the present invention. Further, the scope of the present invention is not limited by the specific description described below.

Furthermore, a person of ordinary skill in the art may recognize or identify numerous equivalents to the specific embodiments of the present invention described herein by using routine experimentation. Moreover, such equivalents are intended to be included within the present invention.

In addition, throughout the specification of the present invention, when it is stated that a part "comprises" a certain component, it means that the part may further include other components, rather than excluding all other components, unless explicitly stated otherwise.

The following description is illustrative only and is not intended to limit the present invention, application, or uses.

Hereinafter, the present invention will be described in more detail.

The first aspect of the present invention provides a compound of the following Formula 1 or a pharmaceutically acceptable salt thereof to achieve the above-described objects: 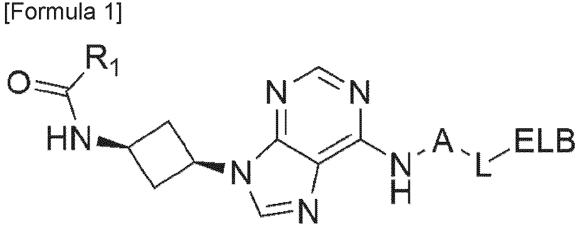 wherein in Formula 1 above:
R₁ is C₁₋₁₀ alkyl, aryl,-C₁₋₃ alkyl-C₆₋₁₀ aryl, 5-10 membered heteroaryl, -C₁₋₃ alkyl-5-10 membered heteroaryl, C₃₋₁₀ cycloalkyl, -C₁₋₃ alkyl-C₃₋₁₀ cycloalkyl, 3-10 membered heterocycloalkyl, or -C₁₋₃ alkyl-3-10 membered heterocycloalkyl, wherein the aryl, heteroaryl, cycloalkyl, or heterocycloalkyl is unsubstituted or substituted with one or more selected from the group consisting of halogen, cyano, tert-butoxycarbonyl(-Boc), amino, nitro, hydroxy, C₁₋₆ alkyl, halo-C₁₋₆ alkyl, -ORₐ, -C(=O)Rₐ, -C(=O)ORₐ, - C(O)NRₐR_{b}, -SO₂Rₐ, -S(O)Rₐ, -SO(N)Rₐ, C₁₋₂ alkyl-C₆₋₁₀ aryl, and C₆₋₁₀ aryl, wherein Rₐ and R_{b} are identical or different and are independently hydrogen, halogen, amino, C₁₋₆ alkyl, halo-C₁₋₆ alkyl, C₆₋₁₀ aryl, 5-10 membered heteroaryl, C₃₋₁₀ cycloalkyl, or 3-10 membered heterocycloalkyl;
A is a moiety functioning as an exit vector;
L is a linker; and
ELB is an E3 ubiquitin ligase binder.

For example, in Formula 1 above, ELB may have a structure represented by the following Formula 2: 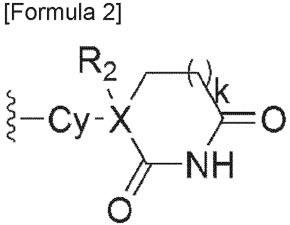 wherein in Formula 2 above:
Cy is C₆₋₁₀ aryl, 5-10 membered heteroaryl, or 5-10 membered heterocycloalkyl;
k is an integer selected from 0 to 2;
X is N or C;
R₂ is absent, hydrogen, deuterium (D), or C₁₋C₃ alkyl; and
the aryl, heteroaryl, or heterocycloalkyl is unsubstituted or substituted with one or more selected from the group consisting of oxo, halogen, nitro, amino, hydroxy, carboxy, C₁₋₆ alkyl, and C₁₋₆ alkoxy.

Specifically, Formula 2 above may have any one of the following structures, but the structure is not limited thereto: 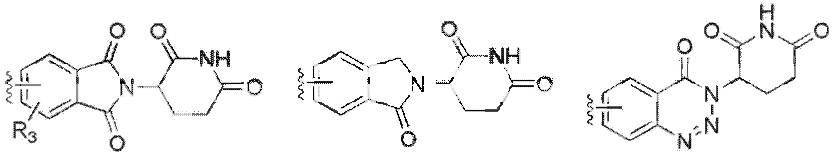 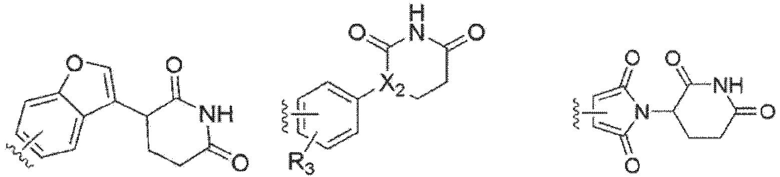 (R₃ is hydrogen or halogen, and X₂ is CH or N).

More specifically, Formula 2 above may have any one of the following structures, but the structure is not limited thereto.

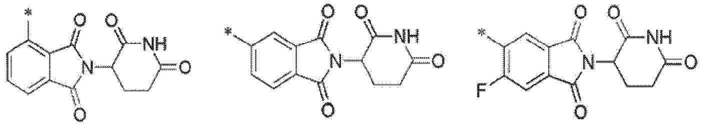

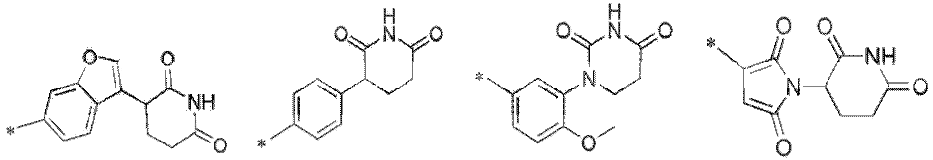

For example, R₁ in Formula 1 above is C₁₋₆ alkyl, -C₁₋₃ alkyl-phenyl, phenyl, -C₁-₃ alkyl-pyridyl, or pyridyl, in which the phenyl or pyridyl is unsubstituted or substituted with one or more substituents selected from the group consisting of C₁₋₃ alkyl, halogen, cyano, and halo-C₁₋₃ alkyl, but is not limited thereto.

Specifically, R₁ may have any one of the following structures, but the structure is not limited thereto:

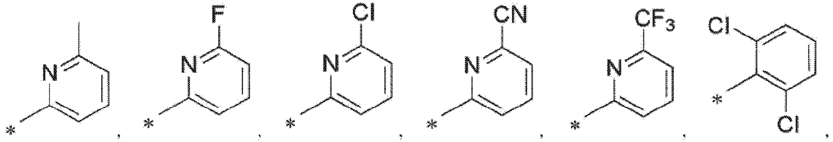

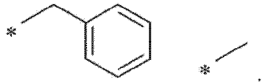

For example, A in Formula 1 above may have the structure of the following Formula 3, but the structure is not limited thereto. 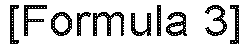 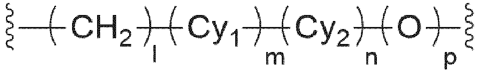 wherein in Formula 3 above:
Cy₁ is C₆₋₁₀ aryl or 5-10 membered heteroaryl, which is unsubstituted or substituted with C₁₋₃ alkyl;
Cy₂ is 3-10 membered heterocycloalkyl; and
I is an integer selected from 0 to 4, and m, n, and p are independently 0 or 1, but the case in which all of I, m, n, and p are 0 is not included.

Specifically, A may have any one of the following structures, but the structure is not limited thereto:

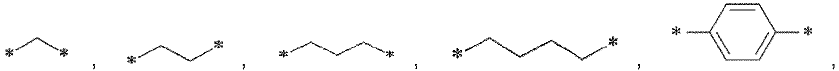

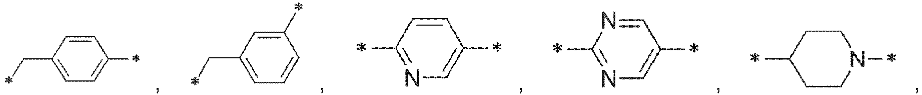

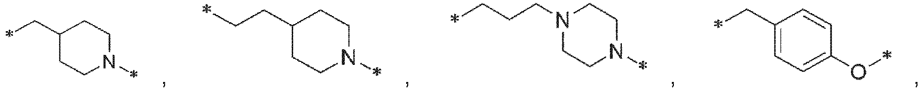

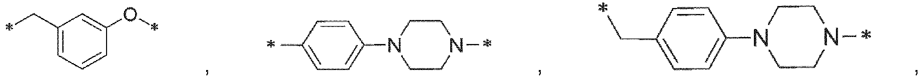

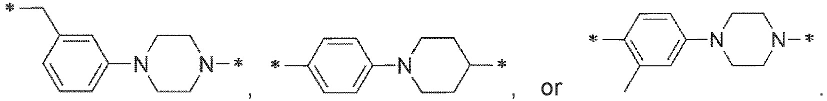

For example, in Formula 1 above, the linker may be represented by -L1-L2-.

In particular, L2 may be a direct linker, 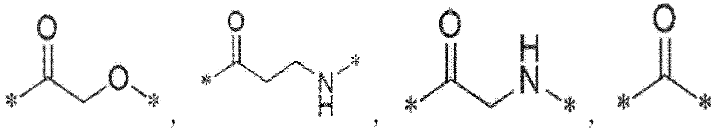 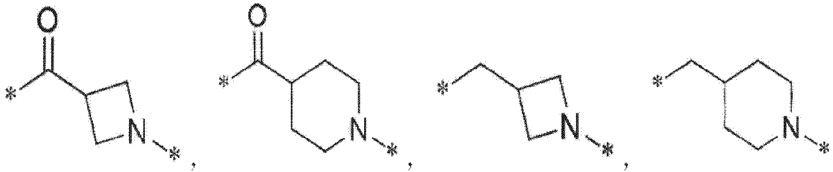 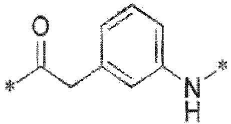 and L1 may be 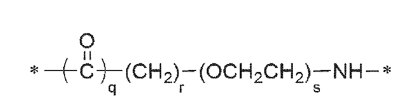 or

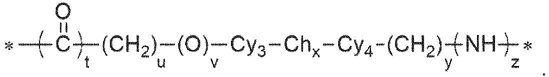

In the above, q, t, v, and y may each be independently 0 or 1; r, s, u, and x may each be independently an integer selected from 0 to 6; and Cy₃ and Cy₄ may each be independently absent, 3-10 membered heterocycloalkyl, or C₃₋₁₀ cycloalkyl. However, they are not limited thereto.

Specifically, L1 may have any one of the following structures, but the structure is not limited thereto:

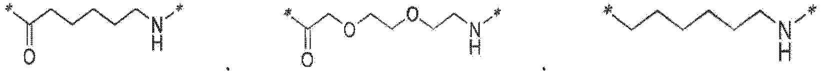

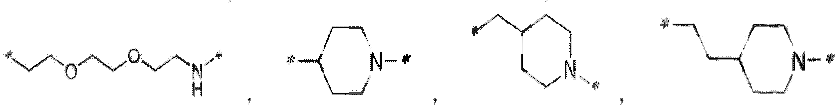

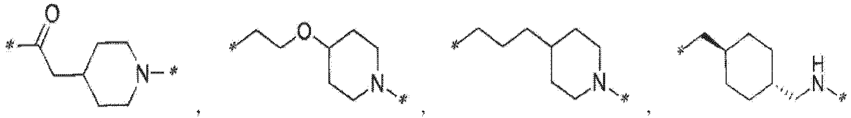

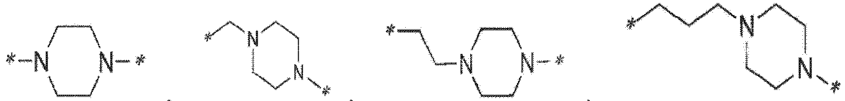

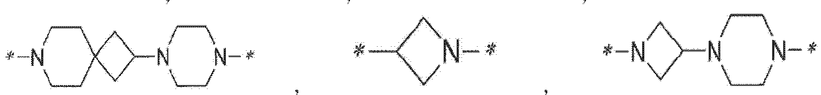

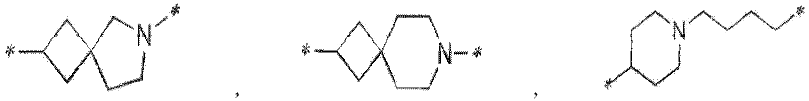

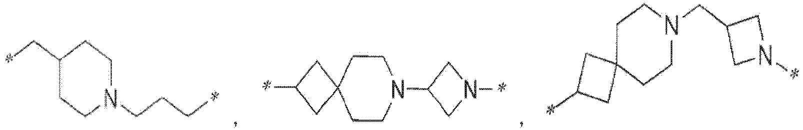

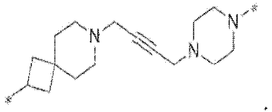

In one embodiment of the present invention, the ELB moiety of Formula 1 above refers to an E3 ligase ligand and functions to degrade or inhibit CDK2 and/or CDK9 through the following steps: binding a CDK2 and/or CDK9 binder moiety of Formula 1 according to the present invention to CDK2 and/or CDK9 (i.e., the target protein) and then binding the CDK2 and/or CDK9 to an E3 ligase (step 1); poly-ubiquitinating the lysine residues of the target protein using ubiquitin from E2 complexes formed with E3 ligases (step 2); and degrading the target protein via a proteasome (step 3). The degrader may be recycled using the same method (step 4).

In one embodiment of the present invention, the linker of Formula 1 above refers to a linker connecting the CDK2 and/or CDK9 inhibitor and a E3 ligase ligand compound. These linkers may bind to CDK2 and/or CDK9 inhibitor compounds through alkyl bonds via a chloride, bromide, iodide, or tosylate, or through amide bonds via an acid or amine. Such linkers may include, for example, linkers disclosed in prior patents US20180353501 A1, WO 2019199816 A1, WO 2019023553 A1, US20180125821 A1, US20190192668 A1, WO 2017197056 A1, WO 2019186358 A1, and/or WO 2018089736 A1. The contents disclosed in the above-mentioned prior patent application publications are incorporated herein by reference in their entirety.

In one typical embodiment, the linker has a chain of 2 to 14, 15, 16, 17, 18, or 20 or more carbon atoms, and one or more carbon atoms may be replaced by heteroatoms such as O, N, S or P. In a certain embodiment, the chain has 2, 3, 4, 5, 6, 7, 8, 9, 10, 11, 12, 13, 14, 15, 16, 17, 18, 19, or 20 contiguous atoms in the chain. For example, the chain may comprise one or more ethylene glycol units (for example, 2, 3, 4, 5, 6, 7, 8, 9, 10, 11, or 12 ethylene glycol units), which may be continuous, partially continuous, or discontinuous. In a certain embodiment, the chain includes at least 1, 2, 3, 4, 5, 6, 7, or 8 continuous chains, which may have branches that are independently alkyl, heteroalkyl, aryl, heteroaryl, alkenyl or alkynyl, cycloalkyl, or heterocycloalkyl.

In another embodiment, the linker may comprise one or more ethylene glycol, propylene glycol, lactic acid and/or glycolic acid, or consist of these units. Typically, propylene glycol increases hydrophobicity, while ethylene glycol increases hydrophilicity. Lactic acid segments exhibit longer half-lives than glycolic acid segments. In addition to ethylene glycol and propylene glycol, blocked and random lactic acid-glycolic acid moieties are known in the art to be pharmaceutically acceptable and can be modified or arranged to obtain a desired half-life and hydrophilicity. In a certain aspect, these units may be flanked or interspersed with other moieties, such as alkyl, heteroalkyl, aryl, heteroaryl, cycloalkyl, and heterocycloalkyl, as needed to achieve appropriate drug properties.

Non-limiting examples of the compounds of Formula 1 according to the present invention are disclosed by name and chemical structural formula in the following Tables 1 to 14.

**[Table 1]**

| Compound No. | Compound Name |
|---|---|
| 1 | N-((1s,3s)-3-(6-((1-(6-(2-((2-(2,6-dioxopiperidin-3-yl)-1,3-dioxoisoindolin-5-yl)amino)acetamido)hexanoyl)piperidin-4-yl)amino)-9H-purin-9-yl)cyclobutyl)-6-methylpicolinamide |
| 2 | N-((1s,3s)-3-(6-((1-(6-(2-((2-(2,6-dioxopiperidin-3-yl)-1,3-dioxoisoindolin-4-yl)amino)acetamido)hexanoyl)piperidin-4-yl)amino)-9H-purin-9-yl)cyclobutyl)-6-methylpicolinamide |
| 3 | N-((1s,3s)-3-(6-((1-(6-(2-((2-(2,6-dioxopiperidin-3-yl)-1,3-dioxoisoindolin-5-yl)oxy)acetamido)hexanoyl)piperidin-4-yl)amino)-9H-purin-9-yl)cyclobutyl)-6-methylpicolinamide |
| 4 | N-((1s,3s)-3-(6-((1-(6-(2-((2-(2,6-dioxopiperidin-3-yl)-1,3-dioxoisoindolin-4-yl)oxy)acetamido)hexanoyl)piperidin-4-yl)amino)-9H-purin-9-yl)cyclobutyl)-6-methylpicolinamide |
| 5 | N-((1s,3s)-3-(6-((1-(6-((2-(2,6-dioxopiperidin-3-yl)-1,3-dioxoisoindolin-5-yl)amino)hexanoyl)piperidin-4-yl)amino)-9H-purin-9-yl)cyclobutyl)-6-methylpicolinamide |
| 6 | N-((1s,3s)-3-(6-((1-(6-((2-(2,6-dioxopiperidin-3-yl)-1,3-dioxoisoindolin-4-yl)amino)hexanoyl)piperidin-4-yl)amino)-9H-purin-9-yl)cyclobutyl)-6-methylpicolinamide |
| 7 | N-((1s,3s)-3-(6-((1-(2-(2-(2-(2-((2-(2,6-dioxopiperidin-3-yl)-1,3-dioxoisoindolin-5-yl)oxy)acetamido)ethoxy)ethoxy)acetyl)piperidin-4-yl)amino)-9H-purin-9-yl)cyclobutyl)-6-methylpicolinamide |
| 8 | N-((1s,3s)-3-(6-((1-(2-(2-(2-(2-((2-(2,6-dioxopiperidin-3-yl)-1,3-dioxoisoindolin-4-yl)oxy)acetamido)ethoxy)ethoxy)acetyl)piperidin-4-yl)amino)-9H-purin-9-yl)cyclobutyl)-6-methylpicolinamide |
| 9 | N-((1s,3s)-3-(6-((1-(2-(2-(2-(2-((2-(2,6-dioxopiperidin-3-yl)-1,3-dioxoisoindolin-5-yl)amino)acetamido)ethoxy)ethoxy)acetyl)piperidin-4-yl)amino)-9H-purin-9-yl)cyclobutyl)-6-methylpicolinamide |
| 10 | N-((1s,3s)-3-(6-((1-(2-(2-(2-(2-((2-(2,6-dioxopiperidin-3-yl)-1,3-dioxoisoindolin-4-yl)amino)acetamido)ethoxy)ethoxy)acetyl)piperidin-4-yl)amino)-9H-purin-9-yl)cyclobutyl)-6-methylpicolinamide |
| 11 | N-((1s,3s)-3-(6-((1-(2-(2-(2-((2-(2,6-dioxopiperidin-3-yl)-1,3-dioxoisoindolin-5-yl)amino)ethoxy)ethoxy)acetyl)piperidin-4-yl)amino)-9H-purin-9-yl)cyclobutyl)-6-methylpicolinamide |
| 12 | N-((1s,3s)-3-(6-((1-(2-(2-(2-((2-(2,6-dioxopiperidin-3-yl)-1,3-dioxoisoindolin-4-yl)amino)ethoxy)ethoxy)acetyl)piperidin-4-yl)amino)-9H-purin-9-yl)cyclobutyl)-6-methylpicolinamide |
| 13 | N-((1s,3s)-3-(6-((1-(6-(2-((2-(2,6-dioxopiperidin-3-yl)-1,3-dioxoisoindolin-5-yl)amino)acetamido)hexyl)piperidin-4-yl)amino)-9H-purin-9-yl)cyclobutyl)-6-methylpicolinamide |
| 14 | N-((1s,3s)-3-(6-((1-(6-(2-((2-(2,6-dioxopiperidin-3-yl)-1,3-dioxoisoindolin-4-yl)amino)acetamido)hexyl)piperidin-4-yl)amino)-9H-purin-9-yl)cyclobutyl)-6-methylpicolinamide |
| 15 | N-((1s,3s)-3-(6-((1-(6-(2-((2-(2,6-dioxopiperidin-3-yl)-1,3-dioxoisoindolin-5-yl)oxy)acetamido)hexyl)piperidin-4-yl)amino)-9H-purin-9-yl)cyclobutyl)-6-methylpicolinamide |
| 16 | N-((1s,3s)-3-(6-((1-(6-(2-((2-(2,6-dioxopiperidin-3-yl)-1,3-dioxoisoindolin-4-yl)oxy)acetamido)hexyl)piperidin-4-yl)amino)-9H-purin-9-yl)cyclobutyl)-6-methylpicolinamide |
| 17 | N-((1s,3s)-3-(6-((1-(6-((2-(2,6-dioxopiperidin-3-yl)-1,3-dioxoisoindolin-5-yl)amino)hexyl)piperidin-4-yl)amino)-9H-purin-9-yl)cyclobutyl)-6-methylpicolinamide |
| 18 | N-((1s,3s)-3-(6-((1-(6-((2-(2,6-dioxopiperidin-3-yl)-1,3-dioxoisoindolin-4-yl)amino)hexyl)piperidin-4-yl)amino)-9H-purin-9-yl)cyclobutyl)-6-methylpicolinamide |
| 19 | N-((1s,3s)-3-(6-((1-(2-(2-(2-(2-((2-(2,6-dioxopiperidin-3-yl)-1,3-dioxoisoindolin-5-yl)amino)acetamido)ethoxy)ethoxy)ethyl)piperidin-4-yl)amino)-9H-purin-9-yl)cyclobutyl)-6-methylpicolinamide |
| 20 | N-((1s,3s)-3-(6-((1-(2-(2-(2-(2-((2-(2,6-dioxopiperidin-3-yl)-1,3-dioxoisoindolin-4-yl)amino)acetamido)ethoxy)ethoxy)ethyl)piperidin-4-yl)amino)-9H-purin-9-yl)cyclobutyl)-6-methylpicolinamide |
| 21 | N-((1s,3s)-3-(6-((1-(2-(2-(2-(2-((2-(2,6-dioxopiperidin-3-yl)-1,3-dioxoisoindolin-5-yl)oxy)acetamido)ethoxy)ethoxy)ethyl)piperidin-4-yl)amino)-9H-purin-9-yl)cyclobutyl)-6-methylpicolinamide |
| 22 | N-((1s,3s)-3-(6-((1-(2-(2-(2-(2-((2-(2,6-dioxopiperidin-3-yl)-1,3-dioxoisoindolin-4-yl)oxy)acetamido)ethoxy)ethoxy)ethyl)piperidin-4-yl)amino)-9H-purin-9-yl)cyclobutyl)-6-methylpicolinamide |
| 23 | N-((1s,3s)-3-(6-((1-(2-(2-(2-((2-(2,6-dioxopiperidin-3-yl)-1,3-dioxoisoindolin-5-yl)amino)ethoxy)ethoxy)ethyl)piperidin-4-yl)amino)-9H-purin-9-yl)cyclobutyl)-6-methylpicolinamide |
| 24 | N-((1s,3s)-3-(6-((1-(2-(2-(2-((2-(2,6-dioxopiperidin-3-yl)-1,3-dioxoisoindolin-4-yl)amino)ethoxy)ethoxy)ethyl)piperidin-4-yl)amino)-9H-purin-9-yl)cyclobutyl)-6-methylpicolinamide |
| 25 | N-((1s,3s)-3-(6-((1-((1-((2-(2,6-dioxopiperidin-3-yl)-1,3-dioxoisoindolin-5-yl)glycyl)piperidin-4-yl)methyl)piperidin-4-yl)amino)-9H-purin-9-yl)cyclobutyl)-6-methylpicolinamide |
| 26 | N-((1s,3s)-3-(6-((1-((1-((2-(2,6-dioxopiperidin-3-yl)-1,3-dioxoisoindolin-4-yl)glycyl)piperidin-4-yl)methyl)piperidin-4-yl)amino)-9H-purin-9-yl)cyclobutyl)-6-methylpicolinamide |
| 27 | N-((1s,3s)-3-(6-((1-((1-(2-((2-(2,6-dioxopiperidin-3-yl)-1,3-dioxoisoindolin-5-yl)oxy)acetyl)piperidin-4-yl)methyl)piperidin-4-yl)amino)-9H-purin-9-yl)cyclobutyl)-6-methylpicolinamide |
| 28 | N-((1s,3s)-3-(6-((1-((1-(2-((2-(2,6-dioxopiperidin-3-yl)-1,3-dioxoisoindolin-4-yl)oxy)acetyl)piperidin-4-yl)methyl)piperidin-4-yl)amino)-9H-purin-9-yl)cyclobutyl)-6-methylpicolinamide |
| 29 | N-((1s,3s)-3-(6-((1-((1-(2-(2,6-dioxopiperidin-3-yl)-1,3-dioxoisoindolin-5-yl)piperidin-4-yl)methyl)piperidin-4-yl)amino)-9H-purin-9-yl)cyclobutyl)-6-methylpicolinamide |
| 30 | N-((1s,3s)-3-(6-((1-((1-(2-(2,6-dioxopiperidin-3-yl)-1,3-dioxoisoindolin-4-yl)piperidin-4-yl)methyl)piperidin-4-yl)amino)-9H-purin-9-yl)cyclobutyl)-6-methylpicolinamide |
| 31 | N-((1s,3s)-3-(6-((1-(2-(1-(2-((2-(2,6-dioxopiperidin-3-yl)-1,3-dioxoisoindolin-5-yl)oxy)acetyl)piperidin-4-yl)ethyl)piperidin-4-yl)amino)-9H-purin-9-yl)cyclobutyl)-6-methylpicolinamide |
| 32 | N-((1s,3s)-3-(6-((1-(2-(1-(2-((2-(2,6-dioxopiperidin-3-yl)-1,3-dioxoisoindolin-4-yl)oxy)acetyl)piperidin-4-yl)ethyl)piperidin-4-yl)amino)-9H-purin-9-yl)cyclobutyl)-6-methylpicolinamide |
| 33 | N-((1s,3s)-3-(6-((1-(2-(1-((2-(2,6-dioxopiperidin-3-yl)-1,3-dioxoisoindolin-5-yl)glycyl)piperidin-4-yl)ethyl)piperidin-4-yl)amino)-9H-purin-9-yl)cyclobutyl)-6-methylpicolinamide |
| 34 | N-((1s,3s)-3-(6-((1-(2-(1-((2-(2,6-dioxopiperidin-3-yl)-1,3-dioxoisoindolin-4-yl)glycyl)piperidin-4-yl)ethyl)piperidin-4-yl)amino)-9H-purin-9-yl)cyclobutyl)-6-methylpicolinamide |
| 35 | N-((1s,3s)-3-(6-((1-(2-(1-(2-(2,6-dioxopiperidin-3-yl)-1,3-dioxoisoindolin-5-yl)piperidin-4-yl)ethyl)piperidin-4-yl)amino)-9H-purin-9-yl)cyclobutyl)-6-methylpicolinamide |
| 36 | N-((1s,3s)-3-(6-((1-(2-(1-(2-(2,4-dioxocyclohexyl)-1,3-dioxoisoindolin-4-yl)piperidin-4-yl)ethyl)piperidin-4-yl)amino)-9H-purin-9-yl)cyclobutyl)-6-methylpicolinamide |
| 37 | N-((1s,3s)-3-(6-((1-(3-(4-((2-(2,6-dioxopiperidin-3-yl)-1,3-dioxoisoindolin-5-yl)glycyl)piperazin-1-yl)propyl)piperidin-4-yl)amino)-9H-purin-9-yl)cyclobutyl)-6-methylpicolinamide |
| 38 | N-((1s,3s)-3-(6-((1-(3-(4-((2-(2,6-dioxopiperidin-3-yl)-1,3-dioxoisoindolin-4-yl)glycyl)piperazin-1-yl)propyl)piperidin-4-yl)amino)-9H-purin-9-yl)cyclobutyl)-6-methylpicolinamide |
| 39 | N-((1s,3s)-3-(6-((1-(3-(4-(2-((2-(2,6-dioxopiperidin-3-yl)-1,3-dioxoisoindolin-5-yl)oxy)acetyl)piperazin-1-yl)propyl)piperidin-4-yl)amino)-9H-purin-9-yl)cyclobutyl)-6-methylpicolinamide |
| 40 | N-((1s,3s)-3-(6-((1-(3-(4-(2-((2-(2,6-dioxopiperidin-3-yl)-1,3-dioxoisoindolin-4-yl)oxy)acetyl)piperazin-1-yl)propyl)piperidin-4-yl)amino)-9H-purin-9-yl)cyclobutyl)-6-methylpicolinamide |
| 41 | N-((1s,3s)-3-(6-((1-(3-(4-(2-(2,6-dioxopiperidin-3-yl)-1,3-dioxoisoindolin-5-yl)piperazin-1-yl)propyl)piperidin-4-yl)amino)-9H-purin-9-yl)cyclobutyl)-6-methylpicolinamide |
| 42 | N-((1s,3s)-3-(6-((1-(3-(4-(2-(2,6-dioxopiperidin-3-yl)-1,3-dioxoisoindolin-4-yl)piperazin-1-yl)propyl)piperidin-4-yl)amino)-9H-purin-9-yl)cyclobutyl)-6-methylpicolinamide |
| 43 | N-((1s,3s)-3-(6-((1-(2-((1-(2-((2-(2,6-dioxopiperidin-3-yl)-1,3-dioxoisoindolin-5-yl)oxy)acetyl)piperidin-4-yl)oxy)ethyl)piperidin-4-yl)amino)-9H-purin-9-yl)cyclobutyl)-6-methylpicolinamide |
| 44 | N-((1s,3s)-3-(6-((1-(2-((1-(2-((2-(2,6-dioxopiperidin-3-yl)-1,3-dioxoisoindolin-4-yl)oxy)acetyl)piperidin-4-yl)oxy)ethyl)piperidin-4-yl)amino)-9H-purin-9-yl)cyclobutyl)-6-methylpicolinamide |
| 45 | N-((1s,3s)-3-(6-((1-(2-((1-((2-(2,6-dioxopiperidin-3-yl)-1,3-dioxoisoindolin-5-yl)glycyl)piperidin-4-yl)oxy)ethyl)piperidin-4-yl)amino)-9H-purin-9-yl)cyclobutyl)-6-methylpicolinamide |
| 46 | N-((1s,3s)-3-(6-((1-(2-((1-((2-(2,6-dioxopiperidin-3-yl)-1,3-dioxoisoindolin-4-yl)glycyl)piperidin-4-yl)oxy)ethyl)piperidin-4-yl)amino)-9H-purin-9-yl)cyclobutyl)-6-methylpicolinamide |
| 47 | N-((1s,3s)-3-(6-((1-(2-((1-(2-(2,6-dioxopiperidin-3-yl)-1,3-dioxoisoindolin-5-yl)piperidin-4-yl)oxy)ethyl)piperidin-4-yl)amino)-9H-purin-9-yl)cyclobutyl)-6-methylpicolinamide |
| 48 | N-((1s,3s)-3-(6-((1-(2-((1-(2-(2,6-dioxopiperidin-3-yl)-1,3-dioxoisoindolin-4-yl)piperidin-4-yl)oxy)ethyl)piperidin-4-yl)amino)-9H-purin-9-yl)cyclobutyl)-6-methylpicolinamide |
| 49 | N-((1s,3s)-3-(6-((1-(2-(1-(2-((2-(2,6-dioxopiperidin-3-yl)-1,3-dioxoisoindolin-5-yl)oxy)acetyl)piperidin-4-yl)acetyl)piperidin-4-yl)amino)-9H-purin-9-yl)cyclobutyl)-6-methylpicolinamide |
| 50 | N-((1s,3s)-3-(6-((1-(2-(1-(2-((2-(2,6-dioxopiperidin-3-yl)-1,3-dioxoisoindolin-4-yl)oxy)acetyl)piperidin-4-yl)acetyl)piperidin-4-yl)amino)-9H-purin-9-yl)cyclobutyl)-6-methylpicolinamide |
| 51 | N-((1s,3s)-3-(6-((1-(2-(1-((2-(2,6-dioxopiperidin-3-yl)-1,3-dioxoisoindolin-5-yl)glycyl)piperidin-4-yl)acetyl)piperidin-4-yl)amino)-9H-purin-9-yl)cyclobutyl)-6-methylpicolinamide |
| 52 | N-((1s,3s)-3-(6-((1-(2-(1-((2-(2,6-dioxopiperidin-3-yl)-1,3-dioxoisoindolin-4-yl)glycyl)piperidin-4-yl)acetyl)piperidin-4-yl)amino)-9H-purin-9-yl)cyclobutyl)-6-methylpicolinamide |
| 53 | N-((1s,3s)-3-(6-((1-(2-(1-(2-(2,6-dioxopiperidin-3-yl)-1,3-dioxoisoindolin-5-yl)piperidin-4-yl)acetyl)piperidin-4-yl)amino)-9H-purin-9-yl)cyclobutyl)-6-methylpicolinamide |
| 54 | N-((1s,3s)-3-(6-((1-(2-(1-(2-(2,6-dioxopiperidin-3-yl)-1,3-dioxoisoindolin-4-yl)piperidin-4-yl)acetyl)piperidin-4-yl)amino)-9H-purin-9-yl)cyclobutyl)-6-methylpicolinamide |
| 55 | N-((1s,3s)-3-(6-(((1-((2-(2,6-dioxopiperidin-3-yl)-1,3-dioxoisoindolin-5-yl)glycyl)piperidin-4-yl)methyl)amino)-9H-purin-9-yl)cyclobutyl)-6-methylpicolinamide |
| 56 | N-((1s,3s)-3-(6-(((1-((2-(2,6-dioxopiperidin-3-yl)-1,3-dioxoisoindolin-4-yl)glycyl)piperidin-4-yl)methyl)amino)-9H-purin-9-yl)cyclobutyl)-6-methylpicolinamide |
| 57 | N-((1s,3s)-3-(6-(((1-(2-((2-(2,6-dioxopiperidin-3-yl)-1,3-dioxoisoindolin-5-yl)oxy)acetyl)piperidin-4-yl)methyl)amino)-9H-purin-9-yl)cyclobutyl)-6-methylpicolinamide |
| 58 | N-((1s,3s)-3-(6-(((1-(2-((2-(2,6-dioxopiperidin-3-yl)-1,3-dioxoisoindolin-4-yl)oxy)acetyl)piperidin-4-yl)methyl)amino)-9H-purin-9-yl)cyclobutyl)-6-methylpicolinamide |
| 59 | N-((1s,3s)-3-(6-(((1-(2-(2,6-dioxopiperidin-3-yl)-1,3-dioxoisoindolin-5-yl)piperidin-4-yl)methyl)amino)-9H-purin-9-yl)cyclobutyl)-6-methylpicolinamide |
| 60 | N-((1s,3s)-3-(6-(((1-(2-(2,6-dioxopiperidin-3-yl)-1,3-dioxoisoindolin-4-yl)piperidin-4-yl)methyl)amino)-9H-purin-9-yl)cyclobutyl)-6-methylpicolinamide |
| 61 | N-((1r,3r)-3-(6-(((1-(6-(2-((2-(2,6-dioxopiperidin-3-yl)-1,3-dioxoisoindolin-5-yl)amino)acetamido)hexanoyl)piperidin-4-yl)methyl)amino)-9H-purin-9-yl)cyclobutyl)-6-methylpicolinamide |
| 62 | N-((1r,3r)-3-(6-(((1-(6-(2-((2-(2,6-dioxopiperidin-3-yl)-1,3-dioxoisoindolin-4-yl)amino)acetamido)hexanoyl)piperidin-4-yl)methyl)amino)-9H-purin-9-yl)cyclobutyl)-6-methylpicolinamide |
| 63 | N-((1r,3r)-3-(6-(((1-(6-(2-((2-(2,6-dioxopiperidin-3-yl)-1,3-dioxoisoindolin-5-yl)oxy)acetamido)hexanoyl)piperidin-4-yl)methyl)amino)-9H-purin-9-yl)cyclobutyl)-6-methylpicolinamide |
| 64 | N-((1r,3r)-3-(6-(((1-(6-(2-((2-(2,6-dioxopiperidin-3-yl)-1,3-dioxoisoindolin-4-yl)oxy)acetamido)hexanoyl)piperidin-4-yl)methyl)amino)-9H-purin-9-yl)cyclobutyl)-6-methylpicolinamide |
| 65 | N-((1r,3r)-3-(6-(((1-(6-((2-(2,6-dioxopiperidin-3-yl)-1,3-dioxoisoindolin-5-yl)amino)hexanoyl)piperidin-4-yl)methyl)amino)-9H-purin-9-yl)cyclobutyl)-6-methylpicolinamide |
| 66 | N-((1r,3r)-3-(6-(((1-(6-((2-(2,6-dioxopiperidin-3-yl)-1,3-dioxoisoindolin-4-yl)amino)hexanoyl)piperidin-4-yl)methyl)amino)-9H-purin-9-yl)cyclobutyl)-6-methylpicolinamide |
| 67 | N-((1s,3s)-3-(6-(((1-(2-(2-(2-(2-((2-(2,6-dioxopiperidin-3-yl)-1,3-dioxoisoindolin-5-yl)oxy)acetamido)ethoxy)ethoxy)acetyl)piperidin-4-yl)methyl)amino)-9H-purin-9-yl)cyclobutyl)-6-methylpicolinamide |
| 68 | N-((1s,3s)-3-(6-(((1-(2-(2-(2-(2-((2-(2,6-dioxopiperidin-3-yl)-1,3-dioxoisoindolin-4-yl)oxy)acetamido)ethoxy)ethoxy)acetyl)piperidin-4-yl)methyl)amino)-9H-purin-9-yl)cyclobutyl)-6-methylpicolinamide |
| 69 | N-((1s,3s)-3-(6-(((1-(2-(2-(2-(2-((2-(2,6-dioxopiperidin-3-yl)-1,3-dioxoisoindolin-5-yl)amino)acetamido)ethoxy)ethoxy)acetyl)piperidin-4-yl)methyl)amino)-9H-purin-9-yl)cyclobutyl)-6-methylpicolinamide |
| 70 | N-((1s,3s)-3-(6-(((1-(2-(2-(2-(2-((2-(2,6-dioxopiperidin-3-yl)-1,3-dioxoisoindolin-4-yl)amino)acetamido)ethoxy)ethoxy)acetyl)piperidin-4-yl)methyl)amino)-9H-purin-9-yl)cyclobutyl)-6-methylpicolinamide |
| 71 | N-((1s,3s)-3-(6-(((1-(2-(2-(2-((2-(2,6-dioxopiperidin-3-yl)-1,3-dioxoisoindolin-4-yl)amino)ethoxy)ethoxy)acetyl)piperidin-4-yl)methyl)amino)-9H-purin-9-yl)cyclobutyl)-6-methylpicolinamide |
| 72 | N-((1r,3r)-3-(6-(((1-((1-(2-((2-(2,6-dioxopiperidin-3-yl)-1,3-dioxoisoindolin-5-yl)oxy)acetyl)piperidin-4-yl)methyl)piperidin-4-yl)methyl)amino)-9H-purin-9-yl)cyclobutyl)-6-methylpicolinamide |
| 73 | N-((1r,3r)-3-(6-(((1-((1-(2-((2-(2,6-dioxopiperidin-3-yl)-1,3-dioxoisoindolin-4-yl)oxy)acetyl)piperidin-4-yl)methyl)piperidin-4-yl)methyl)amino)-9H-purin-9-yl)cyclobutyl)-6-methylpicolinamide |
| 74 | N-((1r,3r)-3-(6-(((1-((1-((2-(2,6-dioxopiperidin-3-yl)-1,3-dioxoisoindolin-5-yl)glycyl)piperidin-4-yl)methyl)piperidin-4-yl)methyl)amino)-9H-purin-9-yl)cyclobutyl)-6-methylpicolinamide |
| 75 | N-((1r,3r)-3-(6-(((1-((1-((2-(2,6-dioxopiperidin-3-yl)-1,3-dioxoisoindolin-4-yl)glycyl)piperidin-4-yl)methyl)piperidin-4-yl)methyl)amino)-9H-purin-9-yl)cyclobutyl)-6-methylpicolinamide |
| 76 | N-((1r,3r)-3-(6-(((1-((1-(2-(2,6-dioxopiperidin-3-yl)-1,3-dioxoisoindolin-5-yl)piperidin-4-yl)methyl)piperidin-4-yl)methyl)amino)-9H-purin-9-yl)cyclobutyl)-6-methylpicolinamide |
| 77 | N-((1r,3r)-3-(6-(((1-((1-(2-(2,6-dioxopiperidin-3-yl)-1,3-dioxoisoindolin-4-yl)piperidin-4-yl)methyl)piperidin-4-yl)methyl)amino)-9H-purin-9-yl)cyclobutyl)-6-methylpicolinamide |
| 78 | N-((1r,3r)-3-(6-(((1-(2-(2-(2-(2-((2-(2,6-dioxopiperidin-3-yl)-1,3-dioxoisoindolin-4-yl)oxy)acetamido)ethoxy)ethoxy)ethyl)piperidin-4-yl)methyl)amino)-9H-purin-9-yl)cyclobutyl)-6-methylpicolinamide |
| 79 | N-((1r,3r)-3-(6-(((1-(2-(2-(2-(2-((2-(2,6-dioxopiperidin-3-yl)-1,3-dioxoisoindolin-4-yl)amino)acetamido)ethoxy)ethoxy)ethyl)piperidin-4-yl)methyl)amino)-9H-purin-9-yl)cyclobutyl)-6-methylpicolinamide |
| 80 | N-((1r,3r)-3-(6-(((1-(2-(2-(2-(2-((2-(2,6-dioxopiperidin-3-yl)-1,3-dioxoisoindolin-5-yl)oxy)acetamido)ethoxy)ethoxy)ethyl)piperidin-4-yl)methyl)amino)-9H-purin-9-yl)cyclobutyl)-6-methylpicolinamide |
| 81 | N-((1r,3r)-3-(6-(((1-(2-(2-(2-(2-((2-(2,6-dioxopiperidin-3-yl)-1,3-dioxoisoindolin-5-yl)amino)acetamido)ethoxy)ethoxy)ethyl)piperidin-4-yl)methyl)amino)-9H-purin-9-yl)cyclobutyl)-6-methylpicolinamide |
| 82 | N-((1s,3s)-3-(6-((4-(4-(2-(2,6-dioxopiperidin-3-yl)-1,3-dioxoisoindolin-5-yl)piperazin-1-yl)phenyl)amino)-9H-purin-9-yl)cyclobutyl)-6-methylpicolinamide |
| 83 | N-((1s,3s)-3-(6-((4-(4-(2-(2,6-dioxopiperidin-3-yl)-1,3-dioxoisoindolin-4-yl)piperazin-1-yl)phenyl)amino)-9H-purin-9-yl)cyclobutyl)-6-methylpicolinamide |
| 84 | N-((1s,3s)-3-(6-((4-(4-(2-((2-(2,6-dioxopiperidin-3-yl)-1,3-dioxoisoindolin-5-yl)oxy)acetyl)piperazin-1-yl)phenyl)amino)-9H-purin-9-yl)cyclobutyl)-6-methylpicolinamide |
| 85 | N-((1s,3s)-3-(6-((4-(4-(2-((2-(2,6-dioxopiperidin-3-yl)-1,3-dioxoisoindolin-4-yl)oxy)acetyl)piperazin-1-yl)phenyl)amino)-9H-purin-9-yl)cyclobutyl)-6-methylpicolinamide |
| 86 | N-((1s,3s)-3-(6-((4-(4-((2-(2,6-dioxopiperidin-3-yl)-1,3-dioxoisoindolin-5-yl)glycyl)piperazin-1-yl)phenyl)amino)-9H-purin-9-yl)cyclobutyl)-6-methylpicolinamide |
| 87 | N-((1s,3s)-3-(6-((4-(4-((2-(2,6-dioxopiperidin-3-yl)-1,3-dioxoisoindolin-4-yl)glycyl)piperazin-1-yl)phenyl)amino)-9H-purin-9-yl)cyclobutyl)-6-methylpicolinamide |
| 88 | N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)phenyl)amino)-9H-purin-9-yl)cyclobutyl)-6-methylpicolinamide |
| 89 | N-((1s,3s)-3-(6-((4-(4-((1-(2-(2,6-dioxopiperidin-3-yl)-1,3-dioxoisoindolin-4-yl)piperidin-4-yl)methyl)piperazin-1-yl)phenyl)amino)-9H-purin-9-yl)cyclobutyl)-6-methylpicolinamide |
| 90 | N-((1s,3s)-3-(6-((4-(4-((1-(2-((2-(2,6-dioxopiperidin-3-yl)-1,3-dioxoisoindolin-5-yl)oxy)acetyl)piperidin-4-yl)methyl)piperazin-1-yl)phenyl)amino)-9H-purin-9-yl)cyclobutyl)-6-methylpicolinamide |
| 91 | N-((1s,3s)-3-(6-((4-(4-((1-(2-((2-(2,6-dioxopiperidin-3-yl)-1,3-dioxoisoindolin-4-yl)oxy)acetyl)piperidin-4-yl)methyl)piperazin-1-yl)phenyl)amino)-9H-purin-9-yl)cyclobutyl)-6-methylpicolinamide |
| 92 | N-((1s,3s)-3-(6-((4-(4-((1-((2-(2,6-dioxopiperidin-3-yl)-1,3-dioxoisoindolin-5-yl)glycyl)piperidin-4-yl)methyl)piperazin-1-yl)phenyl)amino)-9H-purin-9-yl)cyclobutyl)-6-methylpicolinamide |
| 93 | N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)phenyl)amino)-9H-purin-9-yl)cyclobutyl)-6-methylpicolinamide |
| 94 | N-((1s,3s)-3-(6-((3-((1-(2-((2-(2,6-dioxopiperidin-3-yl)-1,3-dioxoisoindolin-5-yl)oxy)acetyl)piperidin-4-yl)methoxy)benzyl)amino)-9H-purin-9-yl)cyclobutyl)-6-methylpicolinamide |
| 95 | N-((1s,3s)-3-(6-((3-((1-(2-((2-(2,6-dioxopiperidin-3-yl)-1,3-dioxoisoindolin-4-yl)oxy)acetyl)piperidin-4-yl)methoxy)benzyl)amino)-9H-purin-9-yl)cyclobutyl)-6-methylpicolinamide |
| 96 | N-((1s,3s)-3-(6-((3-((1-((2-(2,6-dioxopiperidin-3-yl)-1,3-dioxoisoindolin-5-yl)glycyl)piperidin-4-yl)methoxy)benzyl)amino)-9H-purin-9-yl)cyclobutyl)-6-methylpicolinamide |
| 97 | N-((1s,3s)-3-(6-((3-((1-((2-(2,6-dioxopiperidin-3-yl)-1,3-dioxoisoindolin-4-yl)glycyl)piperidin-4-yl)methoxy)benzyl)amino)-9H-purin-9-yl)cyclobutyl)-6-methylpicolinamide |
| 98 | N-((1s,3s)-3-(6-((3-((1-(2-(2,6-dioxopiperidin-3-yl)-1,3-dioxoisoindolin-5-yl)piperidin-4-yl)methoxy)benzyl)amino)-9H-purin-9-yl)cyclobutyl)-6-methylpicolinamide |
| 99 | N-((1s,3s)-3-(6-((3-((1-(2-(2,6-dioxopiperidin-3-yl)-1,3-dioxoisoindolin-4-yl)piperidin-4-yl)methoxy)benzyl)amino)-9H-purin-9-yl)cyclobutyl)-6-methylpicolinamide |
| 100 | N-((1s,3s)-3-(6-((3-(3-(4-(2-((2-(2,6-dioxopiperidin-3-yl)-1,3-dioxoisoindolin-5-yl)oxy)acetyl)piperazin-1-yl)propoxy)benzyl)amino)-9H-purin-9-yl)cyclobutyl)-6-methylpicolinamide |
| 101 | N-((1s,3s)-3-(6-((3-(3-(4-(2-((2-(2,6-dioxopiperidin-3-yl)-1,3-dioxoisoindolin-4-yl)oxy)acetyl)piperazin-1-yl)propoxy)benzyl)amino)-9H-purin-9-yl)cyclobutyl)-6-methylpicolinamide |
| 102 | N-((1s,3s)-3-(6-((3-(3-(4-((2-(2,6-dioxopiperidin-3-yl)-1,3-dioxoisoindolin-5-yl)glycyl)piperazin-1-yl)propoxy)benzyl)amino)-9H-purin-9-yl)cyclobutyl)-6-methylpicolinamide |
| 103 | N-((1s,3s)-3-(6-((3-(3-(4-((2-(2,6-dioxopiperidin-3-yl)-1,3-dioxoisoindolin-4-yl)glycyl)piperazin-1-yl)propoxy)benzyl)amino)-9H-purin-9-yl)cyclobutyl)-6-methylpicolinamide |
| 104 | N-((1s,3s)-3-(6-((3-(3-(4-(2-(2,6-dioxopiperidin-3-yl)-1,3-dioxoisoindolin-5-yl)piperazin-1-yl)propoxy)benzyl)amino)-9H-purin-9-yl)cyclobutyl)-6-methylpicolinamide |
| 105 | N-((1s,3s)-3-(6-((3-(3-(4-(2-(2,6-dioxopiperidin-3-yl)-1,3-dioxoisoindolin-4-yl)piperazin-1-yl)propoxy)benzyl)amino)-9H-purin-9-yl)cyclobutyl)-6-methylpicolinamide |
| 106 | N-((1s,3s)-3-(6-((4-((1-(2-((2-(2,6-dioxopiperidin-3-yl)-1,3-dioxoisoindolin-5-yl)oxy)acetyl)piperidin-4-yl)methoxy)benzyl)amino)-9H-purin-9-yl)cyclobutyl)-6-methylpicolinamide |
| 107 | N-((1s,3s)-3-(6-((4-((1-(2-((2-(2,6-dioxopiperidin-3-yl)-1,3-dioxoisoindolin-4-yl)oxy)acetyl)piperidin-4-yl)methoxy)benzyl)amino)-9H-purin-9-yl)cyclobutyl)-6-methylpicolinamide |
| 108 | N-((1s,3s)-3-(6-((4-((1-((2-(2,6-dioxopiperidin-3-yl)-1,3-dioxoisoindolin-5-yl)glycyl)piperidin-4-yl)methoxy)benzyl)amino)-9H-purin-9-yl)cyclobutyl)-6-methylpicolinamide |
| 109 | N-((1s,3s)-3-(6-((4-((1-((2-(2,6-dioxopiperidin-3-yl)-1,3-dioxoisoindolin-4-yl)glycyl)piperidin-4-yl)methoxy)benzyl)amino)-9H-purin-9-yl)cyclobutyl)-6-methylpicolinamide |
| 110 | N-((1s,3s)-3-(6-((4-((1-(2-(2,6-dioxopiperidin-3-yl)-1,3-dioxoisoindolin-5-yl)piperidin-4-yl)methoxy)benzyl)amino)-9H-purin-9-yl)cyclobutyl)-6-methylpicolinamide |
| 111 | N-((1s,3s)-3-(6-((4-((1-(2-(2,6-dioxopiperidin-3-yl)-1,3-dioxoisoindolin-4-yl)piperidin-4-yl)methoxy)benzyl)amino)-9H-purin-9-yl)cyclobutyl)-6-methylpicolinamide |
| 112 | N-((1s,3s)-3-(6-((4-(3-(4-(2-((2-(2,6-dioxopiperidin-3-yl)-1,3-dioxoisoindolin-5-yl)oxy)acetyl)piperazin-1-yl)propoxy)benzyl)amino)-9H-purin-9-yl)cyclobutyl)-6-methylpicolinamide |
| 113 | N-((1s,3s)-3-(6-((4-(3-(4-(2-((2-(2,6-dioxopiperidin-3-yl)-1,3-dioxoisoindolin-4-yl)oxy)acetyl)piperazin-1-yl)propoxy)benzyl)amino)-9H-purin-9-yl)cyclobutyl)-6-methylpicolinamide |
| 114 | N-((1s,3s)-3-(6-((4-(3-(4-((2-(2,6-dioxopiperidin-3-yl)-1,3-dioxoisoindolin-5-yl)glycyl)piperazin-1-yl)propoxy)benzyl)amino)-9H-purin-9-yl)cyclobutyl)-6-methylpicolinamide |
| 115 | N-((1s,3s)-3-(6-((4-(3-(4-((2-(2,6-dioxopiperidin-3-yl)-1,3-dioxoisoindolin-4-yl)glycyl)piperazin-1-yl)propoxy)benzyl)amino)-9H-purin-9-yl)cyclobutyl)-6-methylpicolinamide |
| 116 | N-((1s,3s)-3-(6-((4-(3-(4-(2-(2,6-dioxopiperidin-3-yl)-1,3-dioxoisoindolin-5-yl)piperazin-1-yl)propoxy)benzyl)amino)-9H-purin-9-yl)cyclobutyl)-6-methylpicolinamide |
| 117 | N-((1s,3s)-3-(6-((4-(3-(4-(2-(2,6-dioxopiperidin-3-yl)-1,3-dioxoisoindolin-4-yl)piperazin-1-yl)propoxy)benzyl)amino)-9H-purin-9-yl)cyclobutyl)-6-methylpicolinamide |
| 118 | N-((1s,3s)-3-(6-((2-(1-(2-((2-(2,6-dioxopiperidin-3-yl)-1,3-dioxoisoindolin-4-yl)oxy)acetyl)piperidin-4-yl)ethyl)amino)-9H-purin-9-yl)cyclobutyl)-6-methylpicolinamide |
| 119 | N-((1s,3s)-3-(6-((2-(1-((2-(2,6-dioxopiperidin-3-yl)-1,3-dioxoisoindolin-4-yl)glycyl)piperidin-4-yl)ethyl)amino)-9H-purin-9-yl)cyclobutyl)-6-methylpicolinamide |
| 120 | N-((1s,3s)-3-(6-((2-(1-(2-((2-(2,6-dioxopiperidin-3-yl)-1,3-dioxoisoindolin-5-yl)oxy)acetyl)piperidin-4-yl)ethyl)amino)-9H-purin-9-yl)cyclobutyl)-6-methylpicolinamide |
| 121 | N-((1s,3s)-3-(6-((2-(1-((2-(2,6-dioxopiperidin-3-yl)-1,3-dioxoisoindolin-5-yl)glycyl)piperidin-4-yl)ethyl)amino)-9H-purin-9-yl)cyclobutyl)-6-methylpicolinamide |
| 122 | N-((1s,3s)-3-(6-((2-(1-((1-(2-((2-(2,6-dioxopiperidin-3-yl)-1,3-dioxoisoindolin-4-yl)oxy)acetyl)piperidin-4-yl)methyl)piperidin-4-yl)ethyl)amino)-9H-purin-9-yl)cyclobutyl)-6-methylpicolinamide |
| 123 | N-((1s,3s)-3-(6-((2-(1-((1-((2-(2,6-dioxopiperidin-3-yl)-1,3-dioxoisoindolin-4-yl)glycyl)piperidin-4-yl)methyl)piperidin-4-yl)ethyl)amino)-9H-purin-9-yl)cyclobutyl)-6-methylpicolinamide |
| 124 | N-((1s,3s)-3-(6-((2-(1-((1-(2-((2-(2,6-dioxopiperidin-3-yl)-1,3-dioxoisoindolin-5-yl)oxy)acetyl)piperidin-4-yl)methyl)piperidin-4-yl)ethyl)amino)-9H-purin-9-yl)cyclobutyl)-6-methylpicolinamide |
| 125 | N-((1s,3s)-3-(6-((2-(1-((1-((2-(2,6-dioxopiperidin-3-yl)-1,3-dioxoisoindolin-5-yl)glycyl)piperidin-4-yl)methyl)piperidin-4-yl)ethyl)amino)-9H-purin-9-yl)cyclobutyl)-6-methylpicolinamide |
| 126 | N-((1s,3s)-3-(6-((3-(4-(2-(2,6-dioxopiperidin-3-yl)-1,3-dioxoisoindolin-4-yl)piperazin-1-yl)benzyl)amino)-9H-purin-9-yl)cyclobutyl)-6-methylpicolinamide |
| 127 | N-((1s,3s)-3-(6-((3-(4-(2-(2,6-dioxopiperidin-3-yl)-1,3-dioxoisoindolin-5-yl)piperazin-1-yl)benzyl)amino)-9H-purin-9-yl)cyclobutyl)-6-methylpicolinamide |
| 128 | N-((1s,3s)-3-(6-((3-(4-(2-((2-(2,6-dioxopiperidin-3-yl)-1,3-dioxoisoindolin-4-yl)oxy)acetyl)piperazin-1-yl)benzyl)amino)-9H-purin-9-yl)cyclobutyl)-6-methylpicolinamide |
| 129 | N-((1s,3s)-3-(6-((3-(4-(2-((2-(2,6-dioxopiperidin-3-yl)-1,3-dioxoisoindolin-5-yl)oxy)acetyl)piperazin-1-yl)benzyl)amino)-9H-purin-9-yl)cyclobutyl)-6-methylpicolinamide |
| 130 | N-((1s,3s)-3-(6-((3-(4-((2-(2,6-dioxopiperidin-3-yl)-1,3-dioxoisoindolin-4-yl)glycyl)piperazin-1-yl)benzyl)amino)-9H-purin-9-yl)cyclobutyl)-6-methylpicolinamide |
| 131 | N-((1s,3s)-3-(6-((3-(4-((2-(2,6-dioxopiperidin-3-yl)-1,3-dioxoisoindolin-5-yl)glycyl)piperazin-1-yl)benzyl)amino)-9H-purin-9-yl)cyclobutyl)-6-methylpicolinamide |
| 132 | N-((1s,3s)-3-(6-((3-(4-((1-(2-((2-(2,6-dioxopiperidin-3-yl)-1,3-dioxoisoindolin-4-yl)oxy)acetyl)piperidin-4-yl)methyl)piperazin-1-yl)benzyl)amino)-9H-purin-9-yl)cyclobutyl)-6-methylpicolinamide |
| 133 | N-((1s,3s)-3-(6-((3-(4-((1-(2-((2-(2,6-dioxopiperidin-3-yl)-1,3-dioxoisoindolin-5-yl)oxy)acetyl)piperidin-4-yl)methyl)piperazin-1-yl)benzyl)amino)-9H-purin-9-yl)cyclobutyl)-6-methylpicolinamide |
| 134 | N-((1s,3s)-3-(6-((3-(4-((1-((2-(2,6-dioxopiperidin-3-yl)-1,3-dioxoisoindolin-4-yl)glycyl)piperidin-4-yl)methyl)piperazin-1-yl)benzyl)amino)-9H-purin-9-yl)cyclobutyl)-6-methylpicolinamide |
| 135 | N-((1s,3s)-3-(6-((3-(4-((1-((2-(2,6-dioxopiperidin-3-yl)-1,3-dioxoisoindolin-5-yl)glycyl)piperidin-4-yl)methyl)piperazin-1-yl)benzyl)amino)-9H-purin-9-yl)cyclobutyl)-6-methylpicolinamide |
| 136 | N-((1s,3s)-3-(6-((3-(4-((1-(2-(2,6-dioxopiperidin-3-yl)-1,3-dioxoisoindolin-4-yl)piperidin-4-yl)methyl)piperazin-1-yl)benzyl)amino)-9H-purin-9-yl)cyclobutyl)-6-methylpicolinamide |
| 137 | N-((1s,3s)-3-(6-((3-(4-((1-(2-(2,6-dioxopiperidin-3-yl)-1,3-dioxoisoindolin-5-yl)piperidin-4-yl)methyl)piperazin-1-yl)benzyl)amino)-9H-purin-9-yl)cyclobutyl)-6-methylpicolinamide |
| 138 | N-((1r,3r)-3-(6-((3-(4-(2-((2-(2,6-dioxopiperidin-3-yl)-1,3-dioxoisoindolin-4-yl)oxy)acetyl)piperazin-1-yl)propyl)amino)-9H-purin-9-yl)cyclobutyl)-6-methylpicolinamide |
| 139 | N-((1r,3r)-3-(6-((3-(4-((2-(2,6-dioxopiperidin-3-yl)-1,3-dioxoisoindolin-4-yl)glycyl)piperazin-1-yl)propyl)amino)-9H-purin-9-yl)cyclobutyl)-6-methylpicolinamide |
| 140 | N-((1r,3r)-3-(6-((3-(4-(2-((2-(2,6-dioxopiperidin-3-yl)-1,3-dioxoisoindolin-5-yl)oxy)acetyl)piperazin-1-yl)propyl)amino)-9H-purin-9-yl)cyclobutyl)-6-methylpicolinamide |
| 141 | N-((1r,3r)-3-(6-((3-(4-((2-(2,6-dioxopiperidin-3-yl)-1,3-dioxoisoindolin-5-yl)glycyl)piperazin-1-yl)propyl)amino)-9H-purin-9-yl)cyclobutyl)-6-methylpicolinamide |
| 142 | N-((1r,3r)-3-(6-((3-(4-((1-(2-((2-(2,6-dioxopiperidin-3-yl)-1,3-dioxoisoindolin-4-yl)oxy)acetyl)piperidin-4-yl)methyl)piperazin-1-yl)propyl)amino)-9H-purin-9-yl)cyclobutyl)-6-methylpicolinamide |
| 143 | N-((1r,3r)-3-(6-((3-(4-((1-((2-(2,6-dioxopiperidin-3-yl)-1,3-dioxoisoindolin-4-yl)glycyl)piperidin-4-yl)methyl)piperazin-1-yl)propyl)amino)-9H-purin-9-yl)cyclobutyl)-6-methylpicolinamide |
| 144 | N-((1r,3r)-3-(6-((3-(4-((1-(2-((2-(2,6-dioxopiperidin-3-yl)-1,3-dioxoisoindolin-5-yl)oxy)acetyl)piperidin-4-yl)methyl)piperazin-1-yl)propyl)amino)-9H-purin-9-yl)cyclobutyl)-6-methylpicolinamide |
| 145 | N-((1r,3r)-3-(6-((3-(4-((1-((2-(2,6-dioxopiperidin-3-yl)-1,3-dioxoisoindolin-5-yl)glycyl)piperidin-4-yl)methyl)piperazin-1-yl)propyl)amino)-9H-purin-9-yl)cyclobutyl)-6-methylpicolinamide |
| 146 | N-((1s,3s)-3-(6-((4-(4-(2-(2,6-dioxopiperidin-3-yl)-1,3-dioxoisoindolin-4-yl)piperazin-1-yl)benzyl)amino)-9H-purin-9-yl)cyclobutyl)-6-methylpicolinamide |
| 147 | N-((1s,3s)-3-(6-((4-(4-(2-(2,6-dioxopiperidin-3-yl)-1,3-dioxoisoindolin-5-yl)piperazin-1-yl)benzyl)amino)-9H-purin-9-yl)cyclobutyl)-6-methylpicolinamide |
| 148 | N-((1s,3s)-3-(6-((4-(4-((2-(2,6-dioxopiperidin-3-yl)-1,3-dioxoisoindolin-4-yl)glycyl)piperazin-1-yl)benzyl)amino)-9H-purin-9-yl)cyclobutyl)-6-methylpicolinamide |
| 149 | N-((1s,3s)-3-(6-((4-(4-((2-(2,6-dioxopiperidin-3-yl)-1,3-dioxoisoindolin-5-yl)glycyl)piperazin-1-yl)benzyl)amino)-9H-purin-9-yl)cyclobutyl)-6-methylpicolinamide |
| 150 | N-((1s,3s)-3-(6-((4-(4-((1-(2-(2,6-dioxopiperidin-3-yl)-1,3-dioxoisoindolin-4-yl)piperidin-4-yl)methyl)piperazin-1-yl)benzyl)amino)-9H-purin-9-yl)cyclobutyl)-6-methylpicolinamide |
| 151 | N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)benzyl)amino)-9H-purin-9-yl)cyclobutyl)-6-methylpicolinamide |
| 152 | N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)benzyl)amino)-9H-purin-9-yl)cyclobutyl)-6-methylpicolinamide |
| 153 | N-((1s,3s)-3-(6-((4-(4-((1-((2-(2,6-dioxopiperidin-3-yl)-1,3-dioxoisoindolin-5-yl)glycyl)piperidin-4-yl)methyl)piperazin-1-yl)benzyl)amino)-9H-purin-9-yl)cyclobutyl)-6-methylpicolinamide |
| 154 | N-((1s,3s)-3-(6-(((1-((1-(2-((2-(2,6-dioxopiperidin-3-yl)-1,3-dioxoisoindolin-5-yl)oxy)acetyl)piperidin-4-yl)methyl)piperidin-4-yl)methyl)amino)-9H-purin-9-yl)cyclobutyl)acetamide |
| 155 | N-((1s,3s)-3-(6-(((1-((1-(2-((2-(2,6-dioxopiperidin-3-yl)-1,3-dioxoisoindolin-4-yl)oxy)acetyl)piperidin-4-yl)methyl)piperidin-4-yl)methyl)amino)-9H-purin-9-yl)cyclobutyl)acetamide |
| 156 | N-((1s,3s)-3-(6-(((1-((1-((2-(2,6-dioxopiperidin-3-yl)-1,3-dioxoisoindolin-5-yl)glycyl)piperidin-4-yl)methyl)piperidin-4-yl)methyl)amino)-9H-purin-9-yl)cyclobutyl)acetamide |
| 157 | N-((1s,3s)-3-(6-(((1-((1-((2-(2,6-dioxopiperidin-3-yl)-1,3-dioxoisoindolin-4-yl)glycyl)piperidin-4-yl)methyl)piperidin-4-yl)methyl)amino)-9H-purin-9-yl)cyclobutyl)acetamide |
| 158 | N-((1s,3s)-3-(6-(((1-((1-(2-(2,6-dioxopiperidin-3-yl)-1,3-dioxoisoindolin-5-yl)piperidin-4-yl)methyl)piperidin-4-yl)methyl)amino)-9H-purin-9-yl)cyclobutyl)acetamide |
| 159 | N-((1s,3s)-3-(6-(((1-((1-(2-(2,6-dioxopiperidin-3-yl)-1,3-dioxoisoindolin-4-yl)piperidin-4-yl)methyl)piperidin-4-yl)methyl)amino)-9H-purin-9-yl)cyclobutyl)acetamide |
| 160 | N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)benzyl)amino)-9H-purin-9-yl)cyclobutyl)acetamide |
| 161 | N-((1s,3s)-3-(6-((4-(4-((1-((2-(2,6-dioxopiperidin-3-yl)-1,3-dioxoisoindolin-5-yl)glycyl)piperidin-4-yl)methyl)piperazin-1-yl)benzyl)amino)-9H-purin-9-yl)cyclobutyl)acetamide |
| 162 | 2,6-dichloro-N-((1s,3s)-3-(6-((4-(4-((1-(2-((2-(2,6-dioxopiperidin-3-yl)-1,3-dioxoisoindolin-5-yl)oxy)acetyl)piperidin-4-yl)methyl)piperazin-1-yl)phenyl)amino)-9H-purin-9-yl)cyclobutyl)benzamide |
| 163 | 2,6-dichloro-N-((1s,3s)-3-(6-((4-(4-((1-(2-((2-(2,6-dioxopiperidin-3-yl)-1,3-dioxoisoindolin-4-yl)oxy)acetyl)piperidin-4-yl)methyl)piperazin-1-yl)phenyl)amino)-9H-purin-9-yl)cyclobutyl)benzamide |
| 164 | 2,6-dichloro-N-((1s,3s)-3-(6-((4-(4-((1-((2-(2,6-dioxopiperidin-3-yl)-1,3-dioxoisoindolin-5-yl)glycyl)piperidin-4-yl)methyl)piperazin-1-yl)phenyl)amino)-9H-purin-9-yl)cyclobutyl)benzamide |
| 165 | 2,6-dichloro-N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)phenyl)amino)-9H-purin-9-yl)cyclobutyl)benzamide |
| 166 | 2,6-dichloro-N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)phenyl)amino)-9H-purin-9-yl)cyclobutyl)benzamide |
| 167 | 2,6-dichloro-N-((1s,3s)-3-(6-((4-(4-((1-(2-(2,6-dioxopiperidin-3-yl)-1,3-dioxoisoindolin-4-yl)piperidin-4-yl)methyl)piperazin-1-yl)phenyl)amino)-9H-purin-9-yl)cyclobutyl)benzamide |
| 168 | 2,6-dichloro-N-((1s,3s)-3-(6-(((1-((1-(2-((2-(2,6-dioxopiperidin-3-yl)-1,3-dioxoisoindolin-4-yl)oxy)acetyl)piperidin-4-yl)methyl)piperidin-4-yl)methyl)amino)-9H-purin-9-yl)cyclobutyl)benzamide |
| 169 | 2,6-dichloro-N-((1s,3s)-3-(6-(((1-((1-(2-((2-(2,6-dioxopiperidin-3-yl)-1,3-dioxoisoindolin-5-yl)oxy)acetyl)piperidin-4-yl)methyl)piperidin-4-yl)methyl)amino)-9H-purin-9-yl)cyclobutyl)benzamide |
| 170 | 2,6-dichloro-N-((1s,3s)-3-(6-(((1-((1-((2-(2,6-dioxopiperidin-3-yl)-1,3-dioxoisoindolin-4-yl)glycyl)piperidin-4-yl)methyl)piperidin-4-yl)methyl)amino)-9H-purin-9-yl)cyclobutyl)benzamide |
| 171 | 2,6-dichloro-N-((1s,3s)-3-(6-(((1-((1-((2-(2,6-dioxopiperidin-3-yl)-1,3-dioxoisoindolin-5-yl)glycyl)piperidin-4-yl)methyl)piperidin-4-yl)methyl)amino)-9H-purin-9-yl)cyclobutyl)benzamide |
| 172 | 2,6-dichloro-N-((1s,3s)-3-(6-(((1-((1-(2-(2,6-dioxopiperidin-3-yl)-1,3-dioxoisoindolin-4-yl)piperidin-4-yl)methyl)piperidin-4-yl)methyl)amino)-9H-purin-9-yl)cyclobutyl)benzamide |
| 173 | 2,6-dichloro-N-((1s,3s)-3-(6-(((1-((1-(2-(2,6-dioxopiperidin-3-yl)-1,3-dioxoisoindolin-5-yl)piperidin-4-yl)methyl)piperidin-4-yl)methyl)amino)-9H-purin-9-yl)cyclobutyl)benzamide |
| 174 | N-((1s,3s)-3-(6-((4-(4-((1-(2-((2-(2,6-dioxopiperidin-3-yl)-1,3-dioxoisoindolin-4-yl)oxy)acetyl)piperidin-4-yl)methyl)piperazin-1-yl)phenyl)amino)-9H-purin-9-yl)cyclobutyl)-2-phenylacetamide |
| 175 | N-((1s,3s)-3-(6-((4-(4-((1-(2-((2-(2,6-dioxopiperidin-3-yl)-1,3-dioxoisoindolin-5-yl)oxy)acetyl)piperidin-4-yl)methyl)piperazin-1-yl)phenyl)amino)-9H-purin-9-yl)cyclobutyl)-2-phenylacetamide |
| 176 | N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)phenyl)amino)-9H-purin-9-yl)cyclobutyl)-2-phenylacetamide |
| 177 | N-((1s,3s)-3-(6-((4-(4-((1-((2-(2,6-dioxopiperidin-3-yl)-1,3-dioxoisoindolin-5-yl)glycyl)piperidin-4-yl)methyl)piperazin-1-yl)phenyl)amino)-9H-purin-9-yl)cyclobutyl)-2-phenylacetamide |
| 178 | N-((1s,3s)-3-(6-((4-(4-((1-(2-(2,6-dioxopiperidin-3-yl)-1,3-dioxoisoindolin-4-yl)piperidin-4-yl)methyl)piperazin-1-yl)phenyl)amino)-9H-purin-9-yl)cyclobutyl)-2-phenylacetamide |
| 179 | N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)phenyl)amino)-9H-purin-9-yl)cyclobutyl)-2-phenylacetamide |
| 180 | N-((1s,3s)-3-(6-(((1-((1-(2-(2,6-dioxopiperidin-3-yl)-1,3-dioxoisoindolin-4-yl)piperidin-4-yl)methyl)piperidin-4-yl)methyl)amino)-9H-purin-9-yl)cyclobutyl)-2-phenylacetamide |
| 181 | N-((1s,3s)-3-(6-(((1-((1-(2-(2,6-dioxopiperidin-3-yl)-1,3-dioxoisoindolin-5-yl)piperidin-4-yl)methyl)piperidin-4-yl)methyl)amino)-9H-purin-9-yl)cyclobutyl)-2-phenylacetamide |
| 182 | N-((1s,3s)-3-(6-(((1-((1-(2-((2-(2,6-dioxopiperidin-3-yl)-1,3-dioxoisoindolin-4-yl)oxy)acetyl)piperidin-4-yl)methyl)piperidin-4-yl)methyl)amino)-9H-purin-9-yl)cyclobutyl)-2-phenylacetamide |
| 183 | N-((1s,3s)-3-(6-(((1-((1-(2-((2-(2,6-dioxopiperidin-3-yl)-1,3-dioxoisoindolin-5-yl)oxy)acetyl)piperidin-4-yl)methyl)piperidin-4-yl)methyl)amino)-9H-purin-9-yl)cyclobutyl)-2-phenylacetamide |
| 184 | N-((1s,3s)-3-(6-(((1-((1-((2-(2,6-dioxopiperidin-3-yl)-1,3-dioxoisoindolin-4-yl)glycyl)piperidin-4-yl)methyl)piperidin-4-yl)methyl)amino)-9H-purin-9-yl)cyclobutyl)-2-phenylacetamide |
| 185 | N-((1s,3s)-3-(6-(((1-((1-((2-(2,6-dioxopiperidin-3-yl)-1,3-dioxoisoindolin-5-yl)glycyl)piperidin-4-yl)methyl)piperidin-4-yl)methyl)amino)-9H-purin-9-yl)cyclobutyl)-2-phenylacetamide |
| 186 | N-((1s,3s)-3-(6-((4-(4-((1-(3-(2,4-dioxotetrahydropyrimidin-1(2H)-yl)-4-methoxybenzoyl)piperidin-4-yl)methyl)piperazin-1-yl)phenyl)amino)-9H-purin-9-yl)cyclobutyl)-2-phenylacetamide |
| 187 | N-((1s,3s)-3-(6-((4-(4-((1-(3-(2,4-dioxotetrahydropyrimidin-1(2H)-yl)benzofuran-6-carbonyl)piperidin-4-yl)methyl)piperazin-1-yl)phenyl)amino)-9H-purin-9-yl)cyclobutyl)-2-phenylacetamide |
| 188 | N-((1s,3s)-3-(6-((4-(4-((1-(2-(3-((1-(2,6-dioxopiperidin-3-yl)-2,5-dioxo-2,5-dihydro-1H-pyrrol-3-yl)amino)phenyl)acetyl)piperidin-4-yl)methyl)piperazin-1-yl)phenyl)amino)-9H-purin-9-yl)cyclobutyl)-2-phenylacetamide |
| 189 | N-((1s,3s)-3-(6-((4-(4-((1-(1-(1-(2,6-dioxopiperidin-3-yl)-2,5-dioxo-2,5-dihydro-1H-pyrrol-3-yl)piperidin-4-carbonyl)piperidin-4-yl)methyl)piperazin-1-yl)phenyl)amino)-9H-purin-9-yl)cyclobutyl)-2-phenylacetamide |
| 190 | N-((1s,3s)-3-(6-((4-(4-(7-(3-(2,4-dioxotetrahydropyrimidin-1 (2H)-yl)-4-methoxybenzoyl)-7-azaspiro[3.5]nonan-2-yl)piperazin-1-yl)phenyl)amino)-9H-purin-9-yl)cyclobutyl)-2-phenylacetamide |
| 191 | N-((1s,3s)-3-(6-((4-(4-(7-(3-(2,4-dioxotetrahydropyrimidin-1 (2H)-yl)benzofuran-6-carbonyl)-7-azaspiro[3.5]nonan-2-yl)piperazin-1-yl)phenyl)amino)-9H-purin-9-yl)cyclobutyl)-2-phenylacetamide |
| 192 | N-((1s,3s)-3-(6-((4-(4-(7-(2-(3-((1-(2,6-dioxopiperidin-3-yl)-2,5-dioxo-2,5-dihydro-1H-pyrrol-3-yl)amino)phenyl)acetyl)-7-azaspiro[3.5]nonan-2-yl)piperazin-1-yl)phenyl)amino)-9H-purin-9-yl)cyclobutyl)-2-phenylacetamide |
| 193 | N-((1s,3s)-3-(6-((4-(4-(7-(1-(1-(2,6-dioxopiperidin-3-yl)-2,5-dioxo-2,5-dihydro-1H-pyrrol-3-yl)piperidin-4-carbonyl)-7-azaspiro[3.5]nonan-2-yl)piperazin-1-yl)phenyl)amino)-9H-purin-9-yl)cyclobutyl)-2-phenylacetamide |
| 194 | N-((1s,3s)-3-(6-((4-(4-(2-(1-(3-(2,4-dioxotetrahydropyrimidin-1(2H)-yl)-4-methoxybenzoyl)piperidin-4-yl)acetyl)piperazin-1-yl)phenyl)amino)-9H-purin-9-yl)cyclobutyl)-2-phenylacetamide |
| 195 | N-((1s,3s)-3-(6-((4-(4-(2-(1-(3-(2,4-dioxotetrahydropyrimidin-1 (2H)-yl)benzofuran-6-carbonyl)piperidin-4-yl)acetyl)piperazin-1-yl)phenyl)amino)-9H-purin-9-yl)cyclobutyl)-2-phenylacetamide |
| 196 | N-((1s,3s)-3-(6-((4-(4-(2-(1-(2-(3-((1-(2,6-dioxopiperidin-3-yl)-2,5-dioxo-2,5-dihydro-1H-pyrrol-3-yl)amino)phenyl)acetyl)piperidin-4-yl)acetyl)piperazin-1-yl)phenyl)amino)-9H-purin-9-yl)cyclobutyl)-2-phenylacetamide |
| 197 | N-((1s,3s)-3-(6-((4-(4-(2-(1-(1-(1-(2,6-dioxopiperidin-3-yl)-2,5-dioxo-2,5-dihydro-1H-pyrrol-3-yl)piperidin-4-carbonyl)piperidin-4-yl)acetyl)piperazin-1-yl)phenyl)amino)-9H-purin-9-yl)cyclobutyl)-2-phenylacetamide |
| 198 | 3-(2,4-dioxotetrahydropyrimidin-1(2H)-yl)-4-methoxy-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)benzamide |
| 199 | 3-(2,4-dioxotetrahydropyrimidin-1(2H)-yl)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)benzofuran-6-carboxamide |
| 200 | 2-(3-((1-(2,6-dioxopiperidin-3-yl)-2,5-dioxo-2,5-dihydro-1H-pyrrol-3-yl)amino)phenyl)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)acetamide |
| 201 | 1-(1-(2,6-dioxopiperidin-3-yl)-2,5-dioxo-2,5-dihydro-1H-pyrrol-3-yl)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)piperidin-4-carboxamide |
| 202 | N-((1s,3s)-3-(6-((4-(4-((1-(2-(4-(2,6-dioxopiperidin-3-yl)phenoxy)acetyl)piperidin-4-yl)methyl)piperazin-1-yl)phenyl)amino)-9H-purin-9-yl)cyclobutyl)-2-phenylacetamide |
| 203 | N-((1s,3s)-3-(6-((4-(4-(2-(1-(2-(4-(2,6-dioxopiperidin-3-yl)phenoxy)acetyl)piperidin-4-yl)acetyl)piperazin-1-yl)phenyl)amino)-9H-purin-9-yl)cyclobutyl)-2-phenylacetamide |
| 204 | N-((1s,3s)-3-(6-((4-(4-(7-((2-(2,6-dioxopiperidin-3-yl)-1,3-dioxoisoindolin-4-yl)glycyl)-7-azaspiro[3.5]nonan-2-yl)piperazin-1-yl)phenyl)amino)-9H-purin-9-yl)cyclobutyl)-2-phenylacetamide |
| 205 | N-((1s,3s)-3-(6-((4-(4-(7-(2-(4-(2,6-dioxopiperidin-3-yl)phenoxy)acetyl)-7-azaspiro[3.5]nonan-2-yl)piperazin-1-yl)phenyl)amino)-9H-purin-9-yl)cyclobutyl)-2-phenylacetamide |
| 206 | N-((1s,3s)-3-(6-((4-(4-(7-(2-((2-(2,6-dioxopiperidin-3-yl)-1,3-dioxoisoindolin-4-yl)oxy)acetyl)-7-azaspiro[3.5]nonan-2-yl)piperazin-1-yl)phenyl)amino)-9H-purin-9-yl)cyclobutyl)-2-phenylacetamide |
| 207 | N-((1s,3s)-3-(6-((4-(4-(7-((2-(2,6-dioxopiperidin-3-yl)-1,3-dioxoisoindolin-5-yl)glycyl)-7-azaspiro[3.5]nonan-2-yl)piperazin-1-yl)phenyl)amino)-9H-purin-9-yl)cyclobutyl)-2-phenylacetamide |
| 208 | N-((1s,3s)-3-(6-((4-(4-(7-(2-((2-(2,6-dioxopiperidin-3-yl)-1,3-dioxoisoindolin-5-yl)oxy)acetyl)-7-azaspiro[3.5]nonan-2-yl)piperazin-1-yl)phenyl)amino)-9H-purin-9-yl)cyclobutyl)-2-phenylacetamide |
| 209 | N-((1s,3s)-3-(6-((4-(4-(7-(2-(2,6-dioxopiperidin-3-yl)-1,3-dioxoisoindolin-4-yl)-7-azaspiro[3.5]nonan-2-yl)piperazin-1-yl)phenyl)amino)-9H-purin-9-yl)cyclobutyl)-2-phenylacetamide |
| 210 | N-((1s,3s)-3-(6-((4-(4-(7-(2-(2,6-dioxopiperidin-3-yl)-1,3-dioxoisoindolin-5-yl)-7-azaspiro[3.5]nonan-2-yl)piperazin-1-yl)phenyl)amino)-9H-purin-9-yl)cyclobutyl)-2-phenylacetamide |
| 211 | N-((1s,3s)-3-(6-((4-(4-(2-(1-((2-(2,6-dioxopiperidin-3-yl)-1,3-dioxoisoindolin-4-yl)glycyl)piperidin-4-yl)acetyl)piperazin-1-yl)phenyl)amino)-9H-purin-9-yl)cyclobutyl)-2-phenylacetamide |
| 212 | N-((1s,3s)-3-(6-((4-(4-(2-(1-(2-((2-(2,6-dioxopiperidin-3-yl)-1,3-dioxoisoindolin-4-yl)oxy)acetyl)piperidin-4-yl)acetyl)piperazin-1-yl)phenyl)amino)-9H-purin-9-yl)cyclobutyl)-2-phenylacetamide |
| 213 | N-((1s,3s)-3-(6-((4-(4-(2-(1-((2-(2,6-dioxopiperidin-3-yl)-1,3-dioxoisoindolin-5-yl)glycyl)piperidin-4-yl)acetyl)piperazin-1-yl)phenyl)amino)-9H-purin-9-yl)cyclobutyl)-2-phenylacetamide |
| 214 | N-((1s,3s)-3-(6-((4-(4-(2-(1-(2-((2-(2,6-dioxopiperidin-3-yl)-1,3-dioxoisoindolin-5-yl)oxy)acetyl)piperidin-4-yl)acetyl)piperazin-1-yl)phenyl)amino)-9H-purin-9-yl)cyclobutyl)-2-phenylacetamide |
| 215 | N-((1s,3s)-3-(6-((4-(4-(2-(1-(2-(2,6-dioxopiperidin-3-yl)-1,3-dioxoisoindolin-4-yl)piperidin-4-yl)acetyl)piperazin-1-yl)phenyl)amino)-9H-purin-9-yl)cyclobutyl)-2-phenylacetamide |
| 216 | N-((1s,3s)-3-(6-((4-(4-(2-(1-(2-(2,6-dioxopiperidin-3-yl)-1,3-dioxoisoindolin-5-yl)piperidin-4-yl)acetyl)piperazin-1-yl)phenyl)amino)-9H-purin-9-yl)cyclobutyl)-2-phenylacetamide |
| 217 | 2-(4-(2,6-dioxopiperidin-3-yl)phenoxy)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)acetamide |
| 218 | 2-((2-(2,6-dioxopiperidin-3-yl)-1,3-dioxoisoindolin-4-yl)amino)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)acetamide |
| 219 | 2-((2-(2,6-dioxopiperidin-3-yl)-1,3-dioxoisoindolin-4-yl)oxy)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)acetamide |
| 220 | 2-((2-(2,6-dioxopiperidin-3-yl)-1,3-dioxoisoindolin-5-yl)amino)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)acetamide |
| 221 | 2-((2-(2,6-dioxopiperidin-3-yl)-1,3-dioxoisoindolin-5-yl)oxy)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)acetamide |
| 222 | N-((1s,3s)-3-(6-((4-(4-(((1r,4r)-4-(((2-(2,6-dioxopiperidin-3-yl)-1,3-dioxoisoindolin-4-yl)amino)methyl)cyclohexyl)methyl)piperazin-1-yl)phenyl)amino)-9H-purin-9-yl)cyclobutyl)-2-phenylacetamide |
| 223 | 2-(3-(2,6-dioxopiperidin-3-yl)phenoxy)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)acetamide |
| 224 | N-((1s,3s)-3-(6-((3-(4-((1-(2-(2,6-dioxopiperidin-3-yl)-1,3-dioxoisoindolin-4-yl)piperidin-4-yl)methyl)piperazin-1-yl)propyl)amino)-9H-purin-9-yl)cyclobutyl)-6-methylpicolinamide |
| 225 | N-((1s,3s)-3-(6-((4-(4-((1-(2-(3-(2,6-dioxopiperidin-3-yl)phenoxy)acetyl)piperidin-4-yl)methyl)piperazin-1-yl)phenyl)amino)-9H-purin-9-yl)cyclobutyl)-2-phenylacetamide |
| 226 | N-((1s,3s)-3-(6-((4-(4-(7-(2-(3-(2,6-dioxopiperidin-3-yl)phenoxy)acetyl)-7-azaspiro[3.5]nonan-2-yl)piperazin-1-yl)phenyl)amino)-9H-purin-9-yl)cyclobutyl)-2-phenylacetamide |
| 227 | N-((1s,3s)-3-(6-((4-(4-(2-(1-(2-(3-(2,6-dioxopiperidin-3-yl)phenoxy)acetyl)piperidin-4-yl)acetyl)piperazin-1-yl)phenyl)amino)-9H-purin-9-yl)cyclobutyl)-2-phenylacetamide |
| 228 | N-((1s,3s)-3-(6-((3-(4-((1-(3-(2,4-dioxotetrahydropyrimidin-1(2H)-yl)-4-methoxybenzoyl)piperidin-4-yl)methyl)piperazin-1-yl)propyl)amino)-9H-purin-9-yl)cyclobutyl)-6-methylpicolinamide |
| 229 | N-((1s,3s)-3-(6-((3-(4-((1-(2-(4-(2,6-dioxopiperidin-3-yl)phenoxy)acetyl)piperidin-4-yl)methyl)piperazin-1-yl)propyl)amino)-9H-purin-9-yl)cyclobutyl)-6-methylpicolinamide |
| 230 | N-((1s,3s)-3-(6-((3-(4-((1-(2-(3-(2,6-dioxopiperidin-3-yl)phenoxy)acetyl)piperidin-4-yl)methyl)piperazin-1-yl)propyl)amino)-9H-purin-9-yl)cyclobutyl)-6-methylpicolinamide |
| 231 | N-((1s,3s)-3-(6-((4-(4-(3-(4-((2-(2,6-dioxopiperidin-3-yl)-1,3-dioxoisoindolin-4-yl)glycyl)piperazin-1-yl)propyl)piperazin-1-yl)phenyl)amino)-9H-purin-9-yl)cyclobutyl)-2-phenylacetamide |
| 232 | N-((1s,3s)-3-(6-((4-(4-(3-(4-(2-((2-(2,6-dioxopiperidin-3-yl)-1,3-dioxoisoindolin-4-yl)oxy)acetyl)piperazin-1-yl)propyl)piperazin-1-yl)phenyl)amino)-9H-purin-9-yl)cyclobutyl)-2-phenylacetamide |
| 233 | N-((1s,3s)-3-(6-((4-(4-(3-(4-((2-(2,6-dioxopiperidin-3-yl)-1,3-dioxoisoindolin-5-yl)glycyl)piperazin-1-yl)propyl)piperazin-1-yl)phenyl)amino)-9H-purin-9-yl)cyclobutyl)-2-phenylacetamide |
| 234 | N-((1s,3s)-3-(6-((4-(4-(3-(4-(2-((2-(2,6-dioxopiperidin-3-yl)-1,3-dioxoisoindolin-5-yl)oxy)acetyl)piperazin-1-yl)propyl)piperazin-1-yl)phenyl)amino)-9H-purin-9-yl)cyclobutyl)-2-phenylacetamide |
| 235 | N-((1s,3s)-3-(6-((4-(4-(3-(4-(3-(2,4-dioxotetrahydropyrimidin-1(2H)-yl)-4-methoxybenzoyl)piperazin-1-yl)propyl)piperazin-1-yl)phenyl)amino)-9H-purin-9-yl)cyclobutyl)-2-phenylacetamide |
| 236 | N-((1s,3s)-3-(6-((4-(4-(3-(4-(2-(4-(2,6-dioxopiperidin-3-yl)phenoxy)acetyl)piperazin-1-yl)propyl)piperazin-1-yl)phenyl)amino)-9H-purin-9-yl)cyclobutyl)-2-phenylacetamide |
| 237 | N-((1s,3s)-3-(6-((4-(4-(3-(4-(2-(3-(2,6-dioxopiperidin-3-yl)phenoxy)acetyl)piperazin-1-yl)propyl)piperazin-1-yl)phenyl)amino)-9H-purin-9-yl)cyclobutyl)-2-phenylacetamide |
| 238 | N-((1s,3s)-3-(6-((4-(4-(3-(4-(2-(2,6-dioxopiperidin-3-yl)-1,3-dioxoisoindolin-4-yl)piperazin-1-yl)propyl)piperazin-1-yl)phenyl)amino)-9H-purin-9-yl)cyclobutyl)-2-phenylacetamide |
| 239 | N-((1s,3s)-3-(6-((4-(4-(3-(4-(2-(2,6-dioxopiperidin-3-yl)-1,3-dioxoisoindolin-5-yl)piperazin-1-yl)propyl)piperazin-1-yl)phenyl)amino)-9H-purin-9-yl)cyclobutyl)-2-phenylacetamide |
| 240 | N-((1s,3s)-3-(6-((4-(4-(3-(4-(2-(2,6-dioxopiperidin-3-yl)-6-fluoro-1,3-dioxoisoindolin-5-yl)piperazin-1-yl)propyl)piperazin-1-yl)phenyl)amino)-9H-purin-9-yl)cyclobutyl)-2-phenylacetamide |
| 241 | N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)-9H-purin-9-yl)cyclobutyl)-2-phenylacetamide |
| 242 | N-((1s,3s)-3-(6-((4-(4-(2-(1-(2-(2,6-dioxopiperidin-3-yl)-6-fluoro-1,3-dioxoisoindolin-5-yl)piperidin-4-yl)acetyl)piperazin-1-yl)phenyl)amino)-9H-purin-9-yl)cyclobutyl)-2-phenylacetamide |
| 243 | N-((1s,3s)-3-(6-((4-(4-(((1r,4r)-4-(((2-(2,6-dioxopiperidin-3-yl)-6-fluoro-1,3-dioxoisoindolin-5-yl)amino)methyl)cyclohexyl)methyl)piperazin-1-yl)phenyl)amino)-9H-purin-9-yl)cyclobutyl)-2-phenylacetamide |
| 244 | N-((1s,3s)-3-(6-((4-(4-(7-(2-(2,6-dioxopiperidin-3-yl)-6-fluoro-1,3-dioxoisoindolin-5-yl)-7-azaspiro[3.5]nonan-2-yl)piperazin-1-yl)phenyl)amino)-9H-purin-9-yl)cyclobutyl)-2-phenylacetamide |
| 245 | N-((1s,3s)-3-(6-((4-(4-(2-((1-((2-(2,6-dioxopiperidin-3-yl)-1,3-dioxoisoindolin-4-yl)glycyl)piperidin-4-yl)oxy)ethyl)piperazin-1-yl)phenyl)amino)-9H-purin-9-yl)cyclobutyl)-2-phenylacetamide |
| 246 | N-((1s,3s)-3-(6-((4-(4-(2-((1-(2-((2-(2,6-dioxopiperidin-3-yl)-1,3-dioxoisoindolin-4-yl)oxy)acetyl)piperidin-4-yl)oxy)ethyl)piperazin-1-yl)phenyl)amino)-9H-purin-9-yl)cyclobutyl)-2-phenylacetamide |
| 247 | N-((1s,3s)-3-(6-((4-(4-(2-((1-(2-((2-(2,6-dioxopiperidin-3-yl)-1,3-dioxoisoindolin-5-yl)oxy)acetyl)piperidin-4-yl)oxy)ethyl)piperazin-1-yl)phenyl)amino)-9H-purin-9-yl)cyclobutyl)-2-phenylacetamide |
| 248 | N-((1s,3s)-3-(6-((4-(4-(2-((1-((2-(2,6-dioxopiperidin-3-yl)-1,3-dioxoisoindolin-5-yl)glycyl)piperidin-4-yl)oxy)ethyl)piperazin-1-yl)phenyl)amino)-9H-purin-9-yl)cyclobutyl)-2-phenylacetamide |
| 249 | N-((1s,3s)-3-(6-((4-(4-(2-((1-(3-(2,4-dioxotetrahydropyrimidin-1(2H)-yl)-4-methoxybenzoyl)piperidin-4-yl)oxy)ethyl)piperazin-1-yl)phenyl)amino)-9H-purin-9-yl)cyclobutyl)-2-phenylacetamide |
| 250 | N-((1s,3s)-3-(6-((4-(4-(2-((1-(2-(4-(2,6-dioxopiperidin-3-yl)phenoxy)acetyl)piperidin-4-yl)oxy)ethyl)piperazin-1-yl)phenyl)amino)-9H-purin-9-yl)cyclobutyl)-2-phenylacetamide |
| 251 | N-((1s,3s)-3-(6-((4-(4-(2-((1-(2-(3-(2,6-dioxopiperidin-3-yl)phenoxy)acetyl)piperidin-4-yl)oxy)ethyl)piperazin-1-yl)phenyl)amino)-9H-purin-9-yl)cyclobutyl)-2-phenylacetamide |
| 252 | N-((1s,3s)-3-(6-((4-(4-(2-((1-(2-(2,6-dioxopiperidin-3-yl)-1,3-dioxoisoindolin-5-yl)piperidin-4-yl)oxy)ethyl)piperazin-1-yl)phenyl)amino)-9H-purin-9-yl)cyclobutyl)-2-phenylacetamide |
| 253 | N-((1s,3s)-3-(6-((4-(4-(3-(1-((2-(2,6-dioxopiperidin-3-yl)-1,3-dioxoisoindolin-4-yl)glycyl)piperidin-4-yl)propyl)piperazin-1-yl)phenyl)amino)-9H-purin-9-yl)cyclobutyl)-2-phenylacetamide |
| 254 | N-((1s,3s)-3-(6-((4-(4-(3-(1-(2-((2-(2,6-dioxopiperidin-3-yl)-1,3-dioxoisoindolin-4-yl)oxy)acetyl)piperidin-4-yl)propyl)piperazin-1-yl)phenyl)amino)-9H-purin-9-yl)cyclobutyl)-2-phenylacetamide |
| 255 | N-((1s,3s)-3-(6-((4-(4-(3-(1-((2-(2,6-dioxopiperidin-3-yl)-1,3-dioxoisoindolin-5-yl)glycyl)piperidin-4-yl)propyl)piperazin-1-yl)phenyl)amino)-9H-purin-9-yl)cyclobutyl)-2-phenylacetamide |
| 256 | N-((1s,3s)-3-(6-((4-(4-(3-(1-(2-((2-(2,6-dioxopiperidin-3-yl)-1,3-dioxoisoindolin-5-yl)oxy)acetyl)piperidin-4-yl)propyl)piperazin-1-yl)phenyl)amino)-9H-purin-9-yl)cyclobutyl)-2-phenylacetamide |
| 257 | N-((1s,3s)-3-(6-((4-(4-(3-(1-(3-(2,4-dioxotetrahydropyrimidin-1(2H)-yl)-4-methoxybenzoyl)piperidin-4-yl)propyl)piperazin-1-yl)phenyl)amino)-9H-purin-9-yl)cyclobutyl)-2-phenylacetamide |
| 258 | N-((1s,3s)-3-(6-((4-(4-(3-(1-(2-(4-(2,6-dioxopiperidin-3-yl)phenoxy)acetyl)piperidin-4-yl)propyl)piperazin-1-yl)phenyl)amino)-9H-purin-9-yl)cyclobutyl)-2-phenylacetamide |
| 259 | N-((1s,3s)-3-(6-((4-(4-(3-(1-(2-(3-(2,6-dioxopiperidin-3-yl)phenoxy)acetyl)piperidin-4-yl)propyl)piperazin-1-yl)phenyl)amino)-9H-purin-9-yl)cyclobutyl)-2-phenylacetamide |
| 260 | N-((1s,3s)-3-(6-((4-(4-(2-((1-(2-(2,6-dioxopiperidin-3-yl)-1,3-dioxoisoindolin-4-yl)piperidin-4-yl)oxy)ethyl)piperazin-1-yl)phenyl)amino)-9H-purin-9-yl)cyclobutyl)-2-phenylacetamide |
| 261 | N-((1s,3s)-3-(6-((4-(4-(3-(1-(2-(2,6-dioxopiperidin-3-yl)-1,3-dioxoisoindolin-4-yl)piperidin-4-yl)propyl)piperazin-1-yl)phenyl)amino)-9H-purin-9-yl)cyclobutyl)-2-phenylacetamide |
| 262 | N-((1s,3s)-3-(6-((4-(4-(3-(1-(2-(2,6-dioxopiperidin-3-yl)-1,3-dioxoisoindolin-5-yl)piperidin-4-yl)propyl)piperazin-1-yl)phenyl)amino)-9H-purin-9-yl)cyclobutyl)-2-phenylacetamide |
| 263 | N-((1s,3s)-3-(6-((4-(4-(6-((2-(2,6-dioxopiperidin-3-yl)-1,3-dioxoisoindolin-4-yl)glycyl)-6-azaspiro[3.4]octan-2-yl)piperazin-1-yl)phenyl)amino)-9H-purin-9-yl)cyclobutyl)-2-phenylacetamide |
| 264 | N-((1s,3s)-3-(6-((4-(4-(6-(2-((2-(2,6-dioxopiperidin-3-yl)-1,3-dioxoisoindolin-4-yl)oxy)acetyl)-6-azaspiro[3.4]octan-2-yl)piperazin-1-yl)phenyl)amino)-9H-purin-9-yl)cyclobutyl)-2-phenylacetamide |
| 265 | N-((1s,3s)-3-(6-((4-(4-(6-((2-(2,6-dioxopiperidin-3-yl)-1,3-dioxoisoindolin-5-yl)glycyl)-6-azaspiro[3.4]octan-2-yl)piperazin-1-yl)phenyl)amino)-9H-purin-9-yl)cyclobutyl)-2-phenylacetamide |
| 266 | N-((1s,3s)-3-(6-((4-(4-(6-(2-((2-(2,6-dioxopiperidin-3-yl)-1,3-dioxoisoindolin-5-yl)oxy)acetyl)-6-azaspiro[3.4]octan-2-yl)piperazin-1-yl)phenyl)amino)-9H-purin-9-yl)cyclobutyl)-2-phenylacetamide |
| 267 | N-((1s,3s)-3-(6-((4-(4-(6-(3-(2,4-dioxotetrahydropyrimidin-1(2H)-yl)-4-methoxybenzoyl)-6-azaspiro[3.4]octan-2-yl)piperazin-1-yl)phenyl)amino)-9H-purin-9-yl)cyclobutyl)-2-phenylacetamide |
| 268 | N-((1s,3s)-3-(6-((4-(4-(6-(2-(4-(2,6-dioxopiperidin-3-yl)phenoxy)acetyl)-6-azaspiro[3.4]octan-2-yl)piperazin-1-yl)phenyl)amino)-9H-purin-9-yl)cyclobutyl)-2-phenylacetamide |
| 269 | N-((1s,3s)-3-(6-((4-(4-(6-(2-(3-(2,6-dioxopiperidin-3-yl)phenoxy)acetyl)-6-azaspiro[3.4]octan-2-yl)piperazin-1-yl)phenyl)amino)-9H-purin-9-yl)cyclobutyl)-2-phenylacetamide |
| 270 | N-((1s,3s)-3-(6-((4-(4-(7-(1-(2-(2,6-dioxopiperidin-3-yl)-1,3-dioxoisoindolin-4-yl)azetidin-3-yl)-7-azaspiro[3.5]nonan-2-yl)piperazin-1-yl)phenyl)amino)-9H-purin-9-yl)cyclobutyl)-2-phenylacetamide |
| 271 | N-((1s,3s)-3-(6-((4-(4-(7-(1-(2-(2,6-dioxopiperidin-3-yl)-1,3-dioxoisoindolin-5-yl)azetidin-3-yl)-7-azaspiro[3.5]nonan-2-yl)piperazin-1-yl)phenyl)amino)-9H-purin-9-yl)cyclobutyl)-2-phenylacetamide |
| 272 | N-((1s,3s)-3-(6-((4-(4-(7-(1-(3-(2,4-dioxotetrahydropyrimidin-1(2H)-yl)-4-methoxybenzoyl)azetidin-3-yl)-7-azaspiro[3.5]nonan-2-yl)piperazin-1-yl)phenyl)amino)-9H-purin-9-yl)cyclobutyl)-2-phenylacetamide |
| 273 | N-((1s,3s)-3-(6-((4-(4-(7-(1-(2-(4-(2,6-dioxopiperidin-3-yl)phenoxy)acetyl)azetidin-3-yl)-7-azaspiro[3.5]nonan-2-yl)piperazin-1-yl)phenyl)amino)-9H-purin-9-yl)cyclobutyl)-2-phenylacetamide |
| 274 | N-((1s,3s)-3-(6-((4-(4-(7-(1-(2-(3-(2,6-dioxopiperidin-3-yl)phenoxy)acetyl)azetidin-3-yl)-7-azaspiro[3.5]nonan-2-yl)piperazin-1-yl)phenyl)amino)-9H-purin-9-yl)cyclobutyl)-2-phenylacetamide |
| 275 | N-((1s,3s)-3-(6-((4-(4-(7-((1-(2-(4-(2,6-dioxopiperidin-3-yl)phenoxy)acetyl)azetidin-3-yl)methyl)-7-azaspiro[3.5]nonan-2-yl)piperazin-1-yl)phenyl)amino)-9H-purin-9-yl)cyclobutyl)-2-phenylacetamide |
| 276 | N-((1s,3s)-3-(6-((4-(4-(7-((1-(2-(3-(2,6-dioxopiperidin-3-yl)phenoxy)acetyl)azetidin-3-yl)methyl)-7-azaspiro[3.5]nonan-2-yl)piperazin-1-yl)phenyl)amino)-9H-purin-9-yl)cyclobutyl)-2-phenylacetamide |
| 277 | N-((1s,3s)-3-(6-((4-(4-(7-(1-((2-(2,6-dioxopiperidin-3-yl)-1,3-dioxoisoindolin-4-yl)glycyl)azetidin-3-yl)-7-azaspiro[3.5]nonan-2-yl)piperazin-1-yl)phenyl)amino)-9H-purin-9-yl)cyclobutyl)-2-phenylacetamide |
| 278 | N-((1s,3s)-3-(6-((4-(4-(7-(1-((2-(2,6-dioxopiperidin-3-yl)-1,3-dioxoisoindolin-5-yl)glycyl)azetidin-3-yl)-7-azaspiro[3.5]nonan-2-yl)piperazin-1-yl)phenyl)amino)-9H-purin-9-yl)cyclobutyl)-2-phenylacetamide |
| 279 | N-((1s,3s)-3-(6-((4-(4-(7-((1-(2-(2,6-dioxopiperidin-3-yl)-1,3-dioxoisoindolin-4-yl)azetidin-3-yl)methyl)-7-azaspiro[3.5]nonan-2-yl)piperazin-1-yl)phenyl)amino)-9H-purin-9-yl)cyclobutyl)-2-phenylacetamide |
| 280 | N-((1s,3s)-3-(6-((4-(4-(7-((1-(3-(2,4-dioxotetrahydropyrimidin-1(2H)-yl)-4-methoxybenzoyl)azetidin-3-yl)methyl)-7-azaspiro[3.5]nonan-2-yl)piperazin-1-yl)phenyl)amino)-9H-purin-9-yl)cyclobutyl)-2-phenylacetamide |
| 281 | N-((1s,3s)-3-(6-((4-(1-((1-(2-(4-(2,6-dioxopiperidin-3-yl)phenoxy)acetyl)piperidin-4-yl)methyl)piperidin-4-yl)phenyl)amino)-9H-purin-9-yl)cyclobutyl)-2-phenylacetamide |
| 282 | N-((1s,3s)-3-(6-((4-(1-((1-(2-(3-(2,6-dioxopiperidin-3-yl)phenoxy)acetyl)piperidin-4-yl)methyl)piperidin-4-yl)phenyl)amino)-9H-purin-9-yl)cyclobutyl)-2-phenylacetamide |
| 283 | N-((1s,3s)-3-(6-((4-(1-((1-(3-(2,4-dioxotetrahydropyrimidin-1 (2H)-yl)-4-methoxybenzoyl)piperidin-4-yl)methyl)piperidin-4-yl)phenyl)amino)-9H-purin-9-yl)cyclobutyl)-2-phenylacetamide |
| 284 | N-((1s,3s)-3-(6-((4-(1-(7-(2-(4-(2,6-dioxopiperidin-3-yl)phenoxy)acetyl)-7-azaspiro[3.5]nonan-2-yl)piperidin-4-yl)phenyl)amino)-9H-purin-9-yl)cyclobutyl)-2-phenylacetamide |
| 285 | N-((1s,3s)-3-(6-((4-(1-(7-(2-(3-(2,6-dioxopiperidin-3-yl)phenoxy)acetyl)-7-azaspiro[3.5]nonan-2-yl)piperidin-4-yl)phenyl)amino)-9H-purin-9-yl)cyclobutyl)-2-phenylacetamide |
| 286 | N-((1s,3s)-3-(6-((4-(1-(7-(3-(2,4-dioxotetrahydropyrimidin-1(2H)-yl)-4-methoxybenzoyl)-7-azaspiro[3.5]nonan-2-yl)piperidin-4-yl)phenyl)amino)-9H-purin-9-yl)cyclobutyl)-2-phenylacetamide |
| 287 | N-((1s,3s)-3-(6-((4-(1-((1-(2-((2-(2,6-dioxopiperidin-3-yl)-1,3-dioxoisoindolin-4-yl)oxy)acetyl)piperidin-4-yl)methyl)piperidin-4-yl)phenyl)amino)-9H-purin-9-yl)cyclobutyl)-2-phenylacetamide |
| 288 | N-((1s,3s)-3-(6-((4-(1-((1-((2-(2,6-dioxopiperidin-3-yl)-1,3-dioxoisoindolin-4-yl)glycyl)piperidin-4-yl)methyl)piperidin-4-yl)phenyl)amino)-9H-purin-9-yl)cyclobutyl)-2-phenylacetamide |
| 289 | N-((1s,3s)-3-(6-((4-(1-((1-(2-((2-(2,6-dioxopiperidin-3-yl)-1,3-dioxoisoindolin-5-yl)oxy)acetyl)piperidin-4-yl)methyl)piperidin-4-yl)phenyl)amino)-9H-purin-9-yl)cyclobutyl)-2-phenylacetamide |
| 290 | N-((1s,3s)-3-(6-((4-(1-((1-((2-(2,6-dioxopiperidin-3-yl)-1,3-dioxoisoindolin-5-yl)glycyl)piperidin-4-yl)methyl)piperidin-4-yl)phenyl)amino)-9H-purin-9-yl)cyclobutyl)-2-phenylacetamide |
| 291 | N-((1s,3s)-3-(6-((4-(1-((1-(2-(2,6-dioxopiperidin-3-yl)-1,3-dioxoisoindolin-4-yl)piperidin-4-yl)methyl)piperidin-4-yl)phenyl)amino)-9H-purin-9-yl)cyclobutyl)-2-phenylacetamide |
| 292 | N-((1s,3s)-3-(6-((4-(1-((1-(2-(2,6-dioxopiperidin-3-yl)-1,3-dioxoisoindolin-5-yl)piperidin-4-yl)methyl)piperidin-4-yl)phenyl)amino)-9H-purin-9-yl)cyclobutyl)-2-phenylacetamide |
| 293 | N-((1s,3s)-3-(6-((4-(1-(7-(2-((2-(2,6-dioxopiperidin-3-yl)-1,3-dioxoisoindolin-4-yl)oxy)acetyl)-7-azaspiro[3.5]nonan-2-yl)piperidin-4-yl)phenyl)amino)-9H-purin-9-yl)cyclobutyl)-2-phenylacetamide |
| 294 | N-((1s,3s)-3-(6-((4-(1-(7-((2-(2,6-dioxopiperidin-3-yl)-1,3-dioxoisoindolin-4-yl)glycyl)-7-azaspiro[3.5]nonan-2-yl)piperidin-4-yl)phenyl)amino)-9H-purin-9-yl)cyclobutyl)-2-phenylacetamide |
| 295 | N-((1s,3s)-3-(6-((4-(1-(7-(2-((2-(2,6-dioxopiperidin-3-yl)-1,3-dioxoisoindolin-5-yl)oxy)acetyl)-7-azaspiro[3.5]nonan-2-yl)piperidin-4-yl)phenyl)amino)-9H-purin-9-yl)cyclobutyl)-2-phenylacetamide |
| 296 | N-((1s,3s)-3-(6-((4-(1-(7-((2-(2,6-dioxopiperidin-3-yl)-1,3-dioxoisoindolin-5-yl)glycyl)-7-azaspiro[3.5]nonan-2-yl)piperidin-4-yl)phenyl)amino)-9H-purin-9-yl)cyclobutyl)-2-phenylacetamide |
| 297 | N-((1s,3s)-3-(6-((4-(1-(7-(2-(2,6-dioxopiperidin-3-yl)-1,3-dioxoisoindolin-4-yl)-7-azaspiro[3.5]nonan-2-yl)piperidin-4-yl)phenyl)amino)-9H-purin-9-yl)cyclobutyl)-2-phenylacetamide |
| 298 | N-((1s,3s)-3-(6-((4-(1-(7-(2-(2,6-dioxopiperidin-3-yl)-1,3-dioxoisoindolin-5-yl)-7-azaspiro[3.5]nonan-2-yl)piperidin-4-yl)phenyl)amino)-9H-purin-9-yl)cyclobutyl)-2-phenylacetamide |
| 299 | 2,6-dichloro-N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)-9H-purin-9-yl)cyclobutyl)benzamide |
| 300 | 2,6-dichloro-N-((1s,3s)-3-(6-((4-(4-((4-(2-(2,6-dioxopiperidin-3-yl)-1,3-dioxoisoindolin-5-yl)piperazin-1-yl)methyl)piperidin-1-yl)phenyl)amino)-9H-purin-9-yl)cyclobutyl)benzamide |
| 301 | 2,6-dichloro-N-((1s,3s)-3-(6-((4-(4-((4-(2-(2,6-dioxopiperidin-3-yl)-6-fluoro-1,3-dioxoisoindolin-5-yl)piperazin-1-yl)methyl)piperidin-1-yl)phenyl)amino)-9H-purin-9-yl)cyclobutyl)benzamide |
| 302 | N-((1s,3s)-3-(6-((2-(1-((1-(2-(2,6-dioxopiperidin-3-yl)-6-fluoro-1,3-dioxoisoindolin-5-yl)piperidin-4-yl)methyl)piperidin-4-yl)ethyl)amino)-9H-purin-9-yl)cyclobutyl)-6-methylpicolinamide |
| 303 | N-((1s,3s)-3-(6-((2-(1-((1-(3-(2,6-dioxopiperidin-3-yl)-4-oxo-3,4-dihydrobenzo[d][1,2,3]triazin-5-yl)piperidin-4-yl)methyl)piperidin-4-yl)ethyl)amino)-9H-purin-9-yl)cyclobutyl)-6-methylpicolinamide |
| 304 | N-((1s,3s)-3-(6-((2-(1-((1-(3-(2,6-dioxopiperidin-3-yl)-4-oxo-3,4-dihydrobenzo[d][1,2,3]triazin-6-yl)piperidin-4-yl)methyl)piperidin-4-yl)ethyl)amino)-9H-purin-9-yl)cyclobutyl)-6-methylpicolinamide |
| 305 | 2,6-dichloro-N-((1s,3s)-3-(6-((4-(4-((4-(2-(2,6-dioxopiperidin-3-yl)-1,3-dioxoisoindolin-4-yl)piperazin-1-yl)methyl)piperidin-1-yl)phenyl)amino)-9H-purin-9-yl)cyclobutyl)benzamide |
| 306 | N-((1s,3s)-3-(6-((2-(1-((1-(2-(2,6-dioxopiperidin-3-yl)-1,3-dioxoisoindolin-4-yl)piperidin-4-yl)methyl)piperidin-4-yl)ethyl)amino)-9H-purin-9-yl)cyclobutyl)-6-methylpicolinamide |
| 307 | N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)phenyl)amino)-9H-purin-9-yl)cyclobutyl)-2-phenylacetamide |
| 308 | N-((1s,3s)-3-(6-((2-(1-((1-(2-(2,6-dioxopiperidin-3-yl)-1,3-dioxoisoindolin-5-yl)piperidin-4-yl)methyl)piperidin-4-yl)ethyl)amino)-9H-purin-9-yl)cyclobutyl)-6-methylpicolinamide |
| 309 | N-((1s,3s)-3-(6-((2-(1-((1-(1-(2-(2,6-dioxopiperidin-3-yl)-1,3-dioxoisoindolin-5-yl)azetidin-3-carbonyl)piperidin-4-yl)methyl)piperidin-4-yl)ethyl)amino)-9H-purin-9-yl)cyclobutyl)-6-methylpicolinamide |
| 310 | N-((1s,3s)-3-(6-((2-(1-((1-(3-((2-(2,6-dioxopiperidin-3-yl)-1,3-dioxoisoindolin-5-yl)amino)propanoyl)piperidin-4-yl)methyl)piperidin-4-yl)ethyl)amino)-9H-purin-9-yl)cyclobutyl)-6-methylpicolinamide |
| 311 | 2,6-dichloro-N-((1s,3s)-3-(6-((4-(4-((4-((2-(2,6-dioxopiperidin-3-yl)-1,3-dioxoisoindolin-4-yl)glycyl)piperazin-1-yl)methyl)piperidin-1-yl)phenyl)amino)-9H-purin-9-yl)cyclobutyl)benzamide |
| 312 | 2,6-dichloro-N-((1s,3s)-3-(6-((4-(4-((4-((2-(2,6-dioxopiperidin-3-yl)-1,3-dioxoisoindolin-5-yl)glycyl)piperazin-1-yl)methyl)piperidin-1-yl)phenyl)amino)-9H-purin-9-yl)cyclobutyl)benzamide |
| 313 | N-((1s,3s)-3-(6-((4-(4-((1-(3-(2,6-dioxopiperidin-3-yl)-4-oxo-3,4-dihydrobenzo[d][1,2,3]triazin-5-yl)piperidin-4-yl)methyl)piperazin-1-yl)phenyl)amino)-9H-purin-9-yl)cyclobutyl)-2-phenylacetamide |
| 314 | N-((1s,3s)-3-(6-((4-(4-(7-(3-(2,6-dioxopiperidin-3-yl)-4-oxo-3,4-dihydrobenzo[d][1,2,3]triazin-6-yl)-7-azaspiro[3.5]nonan-2-yl)piperazin-1-yl)phenyl)amino)-9H-purin-9-yl)cyclobutyl)-2-phenylacetamide |
| 315 | 2,6-dichloro-N-((1s,3s)-3-(6-((4-(4-(7-(2-(2,6-dioxopiperidin-3-yl)-1,3-dioxoisoindolin-5-yl)-7-azaspiro[3.5]nonan-2-yl)piperazin-1-yl)phenyl)amino)-9H-purin-9-yl)cyclobutyl)benzamide |
| 316 | 2,6-dichloro-N-((1s,3s)-3-(6-((4-(4-(7-(2-(2,6-dioxopiperidin-3-yl)-6-fluoro-1,3-dioxoisoindolin-5-yl)-7-azaspiro[3.5]nonan-2-yl)piperazin-1-yl)phenyl)amino)-9H-purin-9-yl)cyclobutyl)benzamide |
| 317 | 2,6-dichloro-N-((1s,3s)-3-(6-((4-(4-(7-(3-(2,6-dioxopiperidin-3-yl)-4-oxo-3,4-dihydrobenzo[d][1,2,3]triazin-6-yl)-7-azaspiro[3.5]nonan-2-yl)piperazin-1-yl)phenyl)amino)-9H-purin-9-yl)cyclobutyl)benzamide |
| 318 | 2,6-dichloro-N-((1s,3s)-3-(6-((4-(4-(7-((2-(2,6-dioxopiperidin-3-yl)-1,3-dioxoisoindolin-5-yl)glycyl)-7-azaspiro[3.5]nonan-2-yl)piperazin-1-yl)phenyl)amino)-9H-purin-9-yl)cyclobutyl)benzamide |
| 319 | N-((1s,3s)-3-(6-((4-(4-((4-(2-(2,6-dioxopiperidin-3-yl)-1,3-dioxoisoindolin-5-yl)piperazin-1-yl)methyl)piperidin-1-yl)phenyl)amino)-9H-purin-9-yl)cyclobutyl)-2-phenylacetamide |
| 320 | N-((1s,3s)-3-(6-((4-(4-((4-(3-(2,6-dioxopiperidin-3-yl)-4-oxo-3,4-dihydrobenzo[d][1,2,3]triazin-6-yl)piperazin-1-yl)methyl)piperidin-1-yl)phenyl)amino)-9H-purin-9-yl)cyclobutyl)-2-phenylacetamide |
| 321 | N-((1s,3s)-3-(6-((4-(4-((4-(3-(2,6-dioxopiperidin-3-yl)-4-oxo-3,4-dihydrobenzo[d][1,2,3]triazin-5-yl)piperazin-1-yl)methyl)piperidin-1-yl)phenyl)amino)-9H-purin-9-yl)cyclobutyl)-2-phenylacetamide |
| 322 | N-((1s,3s)-3-(6-((4-(4-((4-((2-(2,6-dioxopiperidin-3-yl)-1,3-dioxoisoindolin-4-yl)glycyl)piperazin-1-yl)methyl)piperidin-1-yl)phenyl)amino)-9H-purin-9-yl)cyclobutyl)-2-phenylacetamide |
| 323 | N-((1s,3s)-3-(6-((4-(4-(2-(1-(2-(2,6-dioxopiperidin-3-yl)-6-fluoro-1,3-dioxoisoindolin-5-yl)piperidin-4-yl)ethyl)piperazin-1-yl)phenyl)amino)-9H-purin-9-yl)cyclobutyl)-2-phenylacetamide |
| 324 | N-((1s,3s)-3-(6-((4-(4-(2-(1-(3-(2,6-dioxopiperidin-3-yl)-4-oxo-3,4-dihydrobenzo[d][1,2,3]triazin-6-yl)piperidin-4-yl)ethyl)piperazin-1-yl)phenyl)amino)-9H-purin-9-yl)cyclobutyl)-2-phenylacetamide |
| 325 | 2,6-dichloro-N-((1s,3s)-3-(6-((4-(4-((4-(2-((2-(2,6-dioxopiperidin-3-yl)-1,3-dioxoisoindolin-5-yl)oxy)acetyl)piperazin-1-yl)methyl)piperidin-1-yl)phenyl)amino)-9H-purin-9-yl)cyclobutyl)benzamide |
| 326 | N-((1s,3s)-3-(6-((4-(4-((4-(2-((2-(2,6-dioxopiperidin-3-yl)-1,3-dioxoisoindolin-4-yl)oxy)acetyl)piperazin-1-yl)methyl)piperidin-1-yl)phenyl)amino)-9H-purin-9-yl)cyclobutyl)-2-phenylacetamide |
| 327 | N-((1s,3s)-3-(6-((4-(4-(2-(1-(2-(2,6-dioxopiperidin-3-yl)-1,3-dioxoisoindolin-5-yl)piperidin-4-yl)ethyl)piperazin-1-yl)phenyl)amino)-9H-purin-9-yl)cyclobutyl)-2-phenylacetamide |
| 328 | N-((1s,3s)-3-(6-((4-(4-(2-(1-(1-(2-(2,6-dioxopiperidin-3-yl)-1,3-dioxoisoindolin-5-yl)azetidin-3-carbonyl)piperidin-4-yl)ethyl)piperazin-1-yl)phenyl)amino)-9H-purin-9-yl)cyclobutyl)-2-phenylacetamide |
| 329 | N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)phenyl)amino)-9H-purin-9-yl)cyclobutyl)-2-phenylacetamide |
| 330 | N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)phenyl)amino)-9H-purin-9-yl)cyclobutyl)-2-phenylacetamide |
| 331 | N-((1s,3s)-3-(6-((4-(4-((4-(1-(2-(2,6-dioxopiperidin-3-yl)-1,3-dioxoisoindolin-5-yl)azetidin-3-carbonyl)piperazin-1-yl)methyl)piperidin-1-yl)phenyl)amino)-9H-purin-9-yl)cyclobutyl)-2-phenylacetamide |
| 332 | N-((1s,3s)-3-(6-((4-(4-(1-(2-(2,6-dioxopiperidin-3-yl)-1,3-dioxoisoindolin-5-yl)piperidin-4-yl)piperazin-1-yl)phenyl)amino)-9H-purin-9-yl)cyclobutyl)-2-phenylacetamide |
| 333 | N-((1s,3s)-3-(6-((4-(4-(1-(2-(2,6-dioxopiperidin-3-yl)-6-fluoro-1,3-dioxoisoindolin-5-yl)piperidin-4-yl)piperazin-1-yl)phenyl)amino)-9H-purin-9-yl)cyclobutyl)-2-phenylacetamide |
| 334 | N-((1s,3s)-3-(6-((4-(4-(1-(3-(2,6-dioxopiperidin-3-yl)-4-oxo-3,4-dihydrobenzo[d][1,2,3]triazin-6-yl)piperidin-4-yl)piperazin-1-yl)phenyl)amino)-9H-purin-9-yl)cyclobutyl)-2-phenylacetamide |
| 335 | N-((1s,3s)-3-(6-((4-(2-(4-(2-(2,6-dioxopiperidin-3-yl)-1,3-dioxoisoindolin-5-yl)piperazin-1-yl)-7-azaspiro[3.5]nonan-7-yl)phenyl)amino)-9H-purin-9-yl)cyclobutyl)-2-phenylacetamide |
| 336 | N-((1s,3s)-3-(6-((4-(2-(4-(2-(2,6-dioxopiperidin-3-yl)-6-fluoro-1,3-dioxoisoindolin-5-yl)piperazin-1-yl)-7-azaspiro[3.5]nonan-7-yl)phenyl)amino)-9H-purin-9-yl)cyclobutyl)-2-phenylacetamide |
| 337 | N-((1s,3s)-3-(6-((4-(2-(4-(3-(2,6-dioxopiperidin-3-yl)-4-oxo-3,4-dihydrobenzo[d][1,2,3]triazin-6-yl)piperazin-1-yl)-7-azaspiro[3.5]nonan-7-yl)phenyl)amino)-9H-purin-9-yl)cyclobutyl)-2-phenylacetamide |
| 338 | N-((1s,3s)-3-(6-((4-(4-(2-(4-(2-(2,6-dioxopiperidin-3-yl)-1,3-dioxoisoindolin-5-yl)piperazin-1-yl)ethyl)piperidin-1-yl)phenyl)amino)-9H-purin-9-yl)cyclobutyl)-2-phenylacetamide |
| 339 | N-((1s,3s)-3-(6-((4-(4-(2-(4-(2-(2,6-dioxopiperidin-3-yl)-6-fluoro-1,3-dioxoisoindolin-5-yl)piperazin-1-yl)ethyl)piperidin-1-yl)phenyl)amino)-9H-purin-9-yl)cyclobutyl)-2-phenylacetamide |
| 340 | N-((1s,3s)-3-(6-((4-(4-(2-(4-(3-(2,6-dioxopiperidin-3-yl)-4-oxo-3,4-dihydrobenzo[d][1,2,3]triazin-6-yl)piperazin-1-yl)ethyl)piperidin-1-yl)phenyl)amino)-9H-purin-9-yl)cyclobutyl)-2-phenylacetamide |
| 341 | N-((1s,3s)-3-(6-((4-(4-((4-(2-(2,6-dioxopiperidin-3-yl)-6-fluoro-1,3-dioxoisoindolin-5-yl)piperazin-1-yl)methyl)piperidin-1-yl)phenyl)amino)-9H-purin-9-yl)cyclobutyl)-2-phenylacetamide |
| 342 | N-((1s,3s)-3-(6-((4-(4-(1-(1-(2-(2,6-dioxopiperidin-3-yl)-1,3-dioxoisoindolin-5-yl)azetidin-3-carbonyl)piperidin-4-yl)piperazin-1-yl)phenyl)amino)-9H-purin-9-yl)cyclobutyl)-2-phenylacetamide |
| 343 | N-((1s,3s)-3-(6-((4-(4-(1-(1-(2-(2,6-dioxopiperidin-3-yl)-1,3-dioxoisoindolin-5-yl)piperidin-4-carbonyl)piperidin-4-yl)piperazin-1-yl)phenyl)amino)-9H-purin-9-yl)cyclobutyl)-2-phenylacetamide |
| 344 | N-((1s,3s)-3-(6-((4-(4-(1-(2-(2,6-dioxopiperidin-3-yl)-1,3-dioxoisoindolin-5-yl)azetidin-3-yl)piperazin-1-yl)phenyl)amino)-9H-purin-9-yl)cyclobutyl)-2-phenylacetamide |
| 345 | N-((1s,3s)-3-(6-((4-(4-(1-(2-(2,6-dioxopiperidin-3-yl)-6-fluoro-1,3-dioxoisoindolin-5-yl)azetidin-3-yl)piperazin-1-yl)phenyl)amino)-9H-purin-9-yl)cyclobutyl)-2-phenylacetamide |
| 346 | N-((1s,3s)-3-(6-((4-(4-(1-(3-(2,6-dioxopiperidin-3-yl)-4-oxo-3,4-dihydrobenzo[d][1,2,3]triazin-6-yl)azetidin-3-yl)piperazin-1-yl)phenyl)amino)-9H-purin-9-yl)cyclobutyl)-2-phenylacetamide |
| 347 | N-((1s,3s)-3-(6-((4-(4-(3-(4-(2-(2,6-dioxopiperidin-3-yl)-1,3-dioxoisoindolin-5-yl)piperazin-1-yl)propyl)piperidin-1-yl)phenyl)amino)-9H-purin-9-yl)cyclobutyl)-2-phenylacetamide |
| 348 | N-((1s,3s)-3-(6-((4-(4-(3-(4-(2-(2,6-dioxopiperidin-3-yl)-6-fluoro-1,3-dioxoisoindolin-5-yl)piperazin-1-yl)propyl)piperidin-1-yl)phenyl)amino)-9H-purin-9-yl)cyclobutyl)-2-phenylacetamide |
| 349 | N-((1s,3s)-3-(6-((4-(4-(3-(4-(3-(2,6-dioxopiperidin-3-yl)-4-oxo-3,4-dihydrobenzo[d][1,2,3]triazin-6-yl)piperazin-1-yl)propyl)piperidin-1-yl)phenyl)amino)-9H-purin-9-yl)cyclobutyl)-2-phenylacetamide |
| 350 | N-((1s,3s)-3-(6-((4-(4-(1-(1-(2-(2,6-dioxopiperidin-3-yl)-1,3-dioxoisoindolin-5-yl)piperidin-4-carbonyl)azetidin-3-yl)piperazin-1-yl)phenyl)amino)-9H-purin-9-yl)cyclobutyl)-2-phenylacetamide |
| 351 | N-((1s,3s)-3-(6-((4-(4-(3-(1-(3-(2,6-dioxopiperidin-3-yl)-4-oxo-3,4-dihydrobenzo[d][1,2,3]triazin-6-yl)piperidin-4-yl)propyl)piperazin-1-yl)phenyl)amino)-9H-purin-9-yl)cyclobutyl)-2-phenylacetamide |
| 352 | N-((1s,3s)-3-(6-((4-(4-(7-(1-(2-(2,6-dioxopiperidin-3-yl)-1,3-dioxoisoindolin-5-yl)azetidin-3-carbonyl)-7-azaspiro[3.5]nonan-2-yl)piperazin-1-yl)phenyl)amino)-9H-purin-9-yl)cyclobutyl)-2-phenylacetamide |
| 353 | N-((1s,3s)-3-(6-((4-(4-(7-(1-(2-(2,6-dioxopiperidin-3-yl)-1,3-dioxoisoindolin-5-yl)piperidin-4-carbonyl)-7-azaspiro[3.5]nonan-2-yl)piperazin-1-yl)phenyl)amino)-9H-purin-9-yl)cyclobutyl)-2-phenylacetamide |
| 354 | N-((1s,3s)-3-(6-((4-(4-(3-(1-(1-(2-(2,6-dioxopiperidin-3-yl)-1,3-dioxoisoindolin-5-yl)piperidin-4-carbonyl)piperidin-4-yl)propyl)piperazin-1-yl)phenyl)amino)-9H-purin-9-yl)cyclobutyl)-2-phenylacetamide |
| 355 | N-((1s,3s)-3-(6-((2-(1-((1-(1-(2-(2,6-dioxopiperidin-3-yl)-1,3-dioxoisoindolin-5-yl)piperidin-4-carbonyl)piperidin-4-yl)methyl)piperidin-4-yl)ethyl)amino)-9H-purin-9-yl)cyclobutyl)-6-methylpicolinamide |
| 356 | N-((1s,3s)-3-(6-((4-(4-(4-(2-(2,6-dioxopiperidin-3-yl)-1,3-dioxoisoindolin-5-yl)piperazin-1-yl)piperidin-1-yl)phenyl)amino)-9H-purin-9-yl)cyclobutyl)-2-phenylacetamide |
| 357 | N-((1s,3s)-3-(6-((4-(4-(4-(3-(2,6-dioxopiperidin-3-yl)-4-oxo-3,4-dihydrobenzo[d][1,2,3]triazin-6-yl)piperazin-1-yl)piperidin-1-yl)phenyl)amino)-9H-purin-9-yl)cyclobutyl)-2-phenylacetamide |
| 358 | N-((1s,3s)-3-(6-((4-(3-(4-(2-(2,6-dioxopiperidin-3-yl)-1,3-dioxoisoindolin-5-yl)piperazin-1-yl)azetidin-1-yl)phenyl)amino)-9H-purin-9-yl)cyclobutyl)-2-phenylacetamide |
| 359 | N-((1s,3s)-3-(6-((4-(3-(4-(2-(2,6-dioxopiperidin-3-yl)-6-fluoro-1,3-dioxoisoindolin-5-yl)piperazin-1-yl)azetidin-1-yl)phenyl)amino)-9H-purin-9-yl)cyclobutyl)-2-phenylacetamide |
| 360 | N-((1s,3s)-3-(6-((4-(2-(4-(1-(2-(2,6-dioxopiperidin-3-yl)-1,3-dioxoisoindolin-5-yl)azetidin-3-carbonyl)piperazin-1-yl)-7-azaspiro[3.5]nonan-7-yl)phenyl)amino)-9H-purin-9-yl)cyclobutyl)-2-phenylacetamide |
| 361 | N-((1s,3s)-3-(6-((4-(2-(4-(1-(2-(2,6-dioxopiperidin-3-yl)-1,3-dioxoisoindolin-5-yl)piperidin-4-carbonyl)piperazin-1-yl)-7-azaspiro[3.5]nonan-7-yl)phenyl)amino)-9H-purin-9-yl)cyclobutyl)-2-phenylacetamide |
| 362 | N-((1s,3s)-3-(6-((4-(4-(2-(4-(1-(2-(2,6-dioxopiperidin-3-yl)-1,3-dioxoisoindolin-5-yl)azetidin-3-carbonyl)piperazin-1-yl)ethyl)piperidin-1-yl)phenyl)amino)-9H-purin-9-yl)cyclobutyl)-2-phenylacetamide |
| 363 | N-((1s,3s)-3-(6-((4-(4-(2-(4-(1-(2-(2,6-dioxopiperidin-3-yl)-1,3-dioxoisoindolin-5-yl)piperidin-4-carbonyl)piperazin-1-yl)ethyl)piperidin-1-yl)phenyl)amino)-9H-purin-9-yl)cyclobutyl)-2-phenylacetamide |
| 364 | N-((1s,3s)-3-(6-((4-(4-(4-(1-(2-(2,6-dioxopiperidin-3-yl)-1,3-dioxoisoindolin-5-yl)azetidin-3-carbonyl)piperazin-1-yl)piperidin-1-yl)phenyl)amino)-9H-purin-9-yl)cyclobutyl)-2-phenylacetamide |
| 365 | N-((1s,3s)-3-(6-((4-(4-(4-(1-(2-(2,6-dioxopiperidin-3-yl)-1,3-dioxoisoindolin-5-yl)piperidin-4-carbonyl)piperazin-1-yl)piperidin-1-yl)phenyl)amino)-9H-purin-9-yl)cyclobutyl)-2-phenylacetamide |
| 366 | N-((1s,3s)-3-(6-((4-(4-(3-(4-(1-(2-(2,6-dioxopiperidin-3-yl)-1,3-dioxoisoindolin-5-yl)azetidin-3-carbonyl)piperazin-1-yl)propyl)piperidin-1-yl)phenyl)amino)-9H-purin-9-yl)cyclobutyl)-2-phenylacetamide |
| 367 | N-((1s,3s)-3-(6-((4-(4-(3-(4-(1-(2-(2,6-dioxopiperidin-3-yl)-1,3-dioxoisoindolin-5-yl)piperidin-4-carbonyl)piperazin-1-yl)propyl)piperidin-1-yl)phenyl)amino)-9H-purin-9-yl)cyclobutyl)-2-phenylacetamide |
| 368 | N-((1s,3s)-3-(6-((4-(3-(4-(3-(2,6-dioxopiperidin-3-yl)-4-oxo-3,4-dihydrobenzo[d][1,2,3]triazin-6-yl)piperazin-1-yl)azetidin-1-yl)phenyl)amino)-9H-purin-9-yl)cyclobutyl)-2-phenylacetamide |
| 369 | N-((1s,3s)-3-(6-((4-(4-(4-(2-(2,6-dioxopiperidin-3-yl)-6-fluoro-1,3-dioxoisoindolin-5-yl)piperazin-1-yl)piperidin-1-yl)phenyl)amino)-9H-purin-9-yl)cyclobutyl)-2-phenylacetamide |
| 370 | 2,6-dichloro-N-((1s,3s)-3-(6-((4-(4-((4-(3-(2,6-dioxopiperidin-3-yl)-4-oxo-3,4-dihydrobenzo[d][1,2,3]triazin-6-yl)piperazin-1-yl)methyl)piperidin-1-yl)phenyl)amino)-9H-purin-9-yl)cyclobutyl)benzamide |
| 371 | 2,6-dichloro-N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)phenyl)amino)-9H-purin-9-yl)cyclobutyl)benzamide |
| 372 | N-((1s,3s)-3-(6-((4-(4-(3-(1-(1-(2-(2,6-dioxopiperidin-3-yl)-1,3-dioxoisoindolin-5-yl)azetidin-3-carbonyl)piperidin-4-yl)propyl)piperazin-1-yl)phenyl)amino)-9H-purin-9-yl)cyclobutyl)-2-phenylacetamide |
| 373 | N-((1s,3s)-3-(6-((4-(3-(4-(1-(2-(2,6-dioxopiperidin-3-yl)-1,3-dioxoisoindolin-5-yl)azetidin-3-carbonyl)piperazin-1-yl)azetidin-1-yl)phenyl)amino)-9H-purin-9-yl)cyclobutyl)-2-phenylacetamide |
| 374 | N-((1s,3s)-3-(6-((4-(3-(4-(1-(2-(2,6-dioxopiperidin-3-yl)-1,3-dioxoisoindolin-5-yl)azetidin-3-carbonyl)piperazin-1-yl)azetidin-1-yl)phenyl)amino)-9H-purin-9-yl)cyclobutyl)-2-phenylacetamide |
| 375 | N-((1s,3s)-3-(6-((4-(4-(2-(1-((1-(2-(2,6-dioxopiperidin-3-yl)-1,3-dioxoisoindolin-5-yl)azetidin-3-yl)methyl)piperidin-4-yl)ethyl)piperazin-1-yl)phenyl)amino)-9H-purin-9-yl)cyclobutyl)-2-phenylacetamide |
| 376 | 2,6-dichloro-N-((1s,3s)-3-(6-((4-(4-(7-(1-(2-(2,6-dioxopiperidin-3-yl)-1,3-dioxoisoindolin-5-yl)piperidin-4-carbonyl)-7-azaspiro[3.5]nonan-2-yl)piperazin-1-yl)phenyl)amino)-9H-purin-9-yl)cyclobutyl)benzamide |
| 377 | N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)-9H-purin-9-yl)cyclobutyl)-6-methylpicolinamide |
| 378 | N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)phenyl)amino)-9H-purin-9-yl)cyclobutyl)-6-methylpicolinamide |
| 379 | N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)phenyl)amino)-9H-purin-9-yl)cyclobutyl)-6-methylpicolinamide |
| 380 | N-((1s,3s)-3-(6-((4-(4-(2-(1-(2-(2,6-dioxopiperidin-3-yl)-1,3-dioxoisoindolin-5-yl)piperidin-4-yl)ethyl)piperazin-1-yl)phenyl)amino)-9H-purin-9-yl)cyclobutyl)-6-methylpicolinamide |
| 381 | N-((1s,3s)-3-(6-((4-(4-(2-(1-(2-(2,6-dioxopiperidin-3-yl)-6-fluoro-1,3-dioxoisoindolin-5-yl)piperidin-4-yl)ethyl)piperazin-1-yl)phenyl)amino)-9H-purin-9-yl)cyclobutyl)-6-methylpicolinamide |
| 382 | N-((1s,3s)-3-(6-((4-(4-(2-(1-(3-(2,6-dioxopiperidin-3-yl)-4-oxo-3,4-dihydrobenzo[d][1,2,3]triazin-6-yl)piperidin-4-yl)ethyl)piperazin-1-yl)phenyl)amino)-9H-purin-9-yl)cyclobutyl)-6-methylpicolinamide |
| 383 | 2,6-dichloro-N-((1s,3s)-3-(6-((4-(4-(7-(1-(2-(2,6-dioxopiperidin-3-yl)-1,3-dioxoisoindolin-5-yl)azetidin-3-carbonyl)-7-azaspiro[3.5]nonan-2-yl)piperazin-1-yl)phenyl)amino)-9H-purin-9-yl)cyclobutyl)benzamide |
| 384 | N-((1s,3s)-3-(6-((4-(4-(7-(2-(2,6-dioxopiperidin-3-yl)-1,3-dioxoisoindolin-5-yl)-7-azaspiro[3.5]nonan-2-yl)piperazin-1-yl)phenyl)amino)-9H-purin-9-yl)cyclobutyl)-6-methylpicolinamide |
| 385 | N-((1s,3s)-3-(6-((4-(4-(7-(2-(2,6-dioxopiperidin-3-yl)-6-fluoro-1,3-dioxoisoindolin-5-yl)-7-azaspiro[3.5]nonan-2-yl)piperazin-1-yl)phenyl)amino)-9H-purin-9-yl)cyclobutyl)-6-methylpicolinamide |
| 386 | N-((1s,3s)-3-(6-((4-(4-(7-(3-(2,6-dioxopiperidin-3-yl)-4-oxo-3,4-dihydrobenzo[d][1,2,3]triazin-6-yl)-7-azaspiro[3.5]nonan-2-yl)piperazin-1-yl)phenyl)amino)-9H-purin-9-yl)cyclobutyl)-6-methylpicolinamide |
| 387 | N-((1s,3s)-3-(6-((4-(4-(7-(1-(2-(2,6-dioxopiperidin-3-yl)-1,3-dioxoisoindolin-5-yl)azetidin-3-carbonyl)-7-azaspiro[3.5]nonan-2-yl)piperazin-1-yl)phenyl)amino)-9H-purin-9-yl)cyclobutyl)-6-methylpicolinamide |
| 388 | N-((1s,3s)-3-(6-((4-(4-(2-(1-(1-(2-(2,6-dioxopiperidin-3-yl)-1,3-dioxoisoindolin-5-yl)azetidin-3-carbonyl)piperidin-4-yl)ethyl)piperazin-1-yl)phenyl)amino)-9H-purin-9-yl)cyclobutyl)-6-methylpicolinamide |
| 389 | N-((1s,3s)-3-(6-((4-(4-(2-(1-(1-(2-(2,6-dioxopiperidin-3-yl)-1,3-dioxoisoindolin-5-yl)piperidin-4-carbonyl)piperidin-4-yl)ethyl)piperazin-1-yl)phenyl)amino)-9H-purin-9-yl)cyclobutyl)-6-methylpicolinamide |
| 390 | N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)phenyl)amino)-9H-purin-9-yl)cyclobutyl)-6-fluoropicolinamide |
| 391 | 6-chloro-N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)phenyl)amino)-9H-purin-9-yl)cyclobutyl)picolinamide |
| 392 | 6-cyano-N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)phenyl)amino)-9H-purin-9-yl)cyclobutyl)picolinamide |
| 393 | N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)phenyl)amino)-9H-purin-9-yl)cyclobutyl)-6-(trifluoromethyl)picolinamide |
| 394 | 2,6-dichloro-N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)phenyl)amino)-9H-purin-9-yl)cyclobutyl)benzamide |
| 395 | 2,6-dichloro-N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)phenyl)amino)-9H-purin-9-yl)cyclobutyl)benzamide |
| 396 | 2,6-dichloro-N-((1s,3s)-3-(6-((2-(1-((1-(2-(2,6-dioxopiperidin-3-yl)-1,3-dioxoisoindolin-5-yl)piperidin-4-yl)methyl)piperidin-4-yl)ethyl)amino)-9H-purin-9-yl)cyclobutyl)benzamide |
| 397 | 2,6-dichloro-N-((1s,3s)-3-(6-((2-(1-((1-(2-(2,6-dioxopiperidin-3-yl)-6-fluoro-1,3-dioxoisoindolin-5-yl)piperidin-4-yl)methyl)piperidin-4-yl)ethyl)amino)-9H-purin-9-yl)cyclobutyl)benzamide |
| 398 | 2,6-dichloro-N-((1s,3s)-3-(6-((2-(1-((1-(3-(2,6-dioxopiperidin-3-yl)-4-oxo-3,4-dihydrobenzo[d][1,2,3]triazin-6-yl)piperidin-4-yl)methyl)piperidin-4-yl)ethyl)amino)-9H-purin-9-yl)cyclobutyl)benzamide |
| 399 | 2,6-dichloro-N-((1s,3s)-3-(6-((2-(1-((1-(1-(2-(2,6-dioxopiperidin-3-yl)-1,3-dioxoisoindolin-5-yl)azetidin-3-carbonyl)piperidin-4-yl)methyl)piperidin-4-yl)ethyl)amino)-9H-purin-9-yl)cyclobutyl)benzamide |
| 400 | 2,6-dichloro-N-((1s,3s)-3-(6-((2-(1-((1-(1-(2-(2,6-dioxopiperidin-3-yl)-1,3-dioxoisoindolin-5-yl)piperidin-4-carbonyl)piperidin-4-yl)methyl)piperidin-4-yl)ethyl)amino)-9H-purin-9-yl)cyclobutyl)benzamide |
| 401 | N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)phenyl)amino)-9H-purin-9-yl)cyclobutyl)-6-methylpicolinamide |
| 402 | N-((1s,3s)-3-(6-((2-(1-((1-((1-(2-(2,6-dioxopiperidin-3-yl)-1,3-dioxoisoindolin-5-yl)azetidin-3-yl)methyl)piperidin-4-yl)methyl)piperidin-4-yl)ethyl)amino)-9H-purin-9-yl)cyclobutyl)-6-methylpicolinamide |
| 403 | N-((1s,3s)-3-(6-((4-(4-(7-(1-(2-(2,6-dioxopiperidin-3-yl)-1,3-dioxoisoindolin-5-yl)piperidin-4-carbonyl)-7-azaspiro[3.5]nonan-2-yl)piperazin-1-yl)phenyl)amino)-9H-purin-9-yl)cyclobutyl)-6-methylpicolinamide |
| 404 | N-((1s,3s)-3-(6-((4-(4-(2-(1-(1-(2-(2,6-dioxopiperidin-3-yl)-1,3-dioxoisoindolin-5-yl)piperidin-4-carbonyl)piperidin-4-yl)ethyl)piperazin-1-yl)phenyl)amino)-9H-purin-9-yl)cyclobutyl)-2-phenylacetamide |
| 405 | 2,6-dichloro-N-((1s,3s)-3-(6-((4-(4-(2-(1-(2-(2,6-dioxopiperidin-3-yl)-1,3-dioxoisoindolin-5-yl)piperidin-4-yl)ethyl)piperazin-1-yl)phenyl)amino)-9H-purin-9-yl)cyclobutyl)benzamide |
| 406 | 2,6-dichloro-N-((1s,3s)-3-(6-((4-(4-(2-(1-(2-(2,6-dioxopiperidin-3-yl)-6-fluoro-1,3-dioxoisoindolin-5-yl)piperidin-4-yl)ethyl)piperazin-1-yl)phenyl)amino)-9H-purin-9-yl)cyclobutyl)benzamide |
| 407 | 2,6-dichloro-N-((1s,3s)-3-(6-((4-(4-(2-(1-(3-(2,6-dioxopiperidin-3-yl)-4-oxo-3,4-dihydrobenzo[d][1,2,3]triazin-6-yl)piperidin-4-yl)ethyl)piperazin-1-yl)phenyl)amino)-9H-purin-9-yl)cyclobutyl)benzamide |
| 408 | N-((1s,3s)-3-(6-((4-(4-(1-(4-(2-(2,6-dioxopiperidin-3-yl)-1,3-dioxoisoindolin-5-yl)butyl)piperidin-4-yl)piperazin-1-yl)phenyl)amino)-9H-purin-9-yl)cyclobutyl)-2-phenylacetamide |
| 409 | N-((1s,3s)-3-(6-((2-(1-((1-(2-(2,6-dioxopiperidin-3-yl)-6-fluoro-1,3-dioxoisoindolin-5-yl)piperidin-4-yl)methyl)piperidin-4-yl)ethyl)amino)-9H-purin-9-yl)cyclobutyl)-2-phenylacetamide |
| 410 | N-((1s,3s)-3-(6-((2-(1-((1-(3-(2,6-dioxopiperidin-3-yl)-4-oxo-3,4-dihydrobenzo[d][1,2,3]triazin-6-yl)piperidin-4-yl)methyl)piperidin-4-yl)ethyl)amino)-9H-purin-9-yl)cyclobutyl)-2-phenylacetamide |
| 411 | N-((1s,3s)-3-(6-((2-(1-((1-(1-(2-(2,6-dioxopiperidin-3-yl)-1,3-dioxoisoindolin-5-yl)azetidin-3-carbonyl)piperidin-4-yl)methyl)piperidin-4-yl)ethyl)amino)-9H-purin-9-yl)cyclobutyl)-2-phenylacetamide |
| 412 | 2,6-dichloro-N-((1s,3s)-3-(6-((4-(4-(2-(1-(1-(2-(2,6-dioxopiperidin-3-yl)-1,3-dioxoisoindolin-5-yl)azetidin-3-carbonyl)piperidin-4-yl)ethyl)piperazin-1-yl)phenyl)amino)-9H-purin-9-yl)cyclobutyl)benzamide |
| 413 | 2,6-dichloro-N-((1s,3s)-3-(6-((4-(4-(2-(1-(1-(2-(2,6-dioxopiperidin-3-yl)-1,3-dioxoisoindolin-5-yl)piperidin-4-carbonyl)piperidin-4-yl)ethyl)piperazin-1-yl)phenyl)amino)-9H-purin-9-yl)cyclobutyl)benzamide |
| 414 | 6-cyano-N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)-9H-purin-9-yl)cyclobutyl)picolinamide |
| 415 | N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)-9H-purin-9-yl)cyclobutyl)-6-(trifluoromethyl)picolinamide |
| 416 | 6-chloro-N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)-9H-purin-9-yl)cyclobutyl)picolinamide |
| 417 | 6-chloro-N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)phenyl)amino)-9H-purin-9-yl)cyclobutyl)picolinamide |
| 418 | 6-cyano-N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)phenyl)amino)-9H-purin-9-yl)cyclobutyl)picolinamide |
| 419 | 6-chloro-N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)phenyl)amino)-9H-purin-9-yl)cyclobutyl)picolinamide |
| 420 | 6-cyano-N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)phenyl)amino)-9H-purin-9-yl)cyclobutyl)picolinamide |
| 421 | N-((1s,3s)-3-(6-((2-(1-((1-(1-(2-(2,6-dioxopiperidin-3-yl)-1,3-dioxoisoindolin-5-yl)piperidin-4-carbonyl)piperidin-4-yl)methyl)piperidin-4-yl)ethyl)amino)-9H-purin-9-yl)cyclobutyl)-2-phenylacetamide |
| 422 | N-((1s,3s)-3-(6-((2-(1-((1-(3-(2-(2,6-dioxopiperidin-3-yl)-1,3-dioxoisoindolin-4-yl)propyl)piperidin-4-yl)methyl)piperidin-4-yl)ethyl)amino)-9H-purin-9-yl)cyclobutyl)-2-phenylacetamide |
| 423 | N-((1s,3s)-3-(6-((2-(1-((1-(2-(2,6-dioxopiperidin-3-yl)-1,3-dioxoisoindolin-5-yl)piperidin-4-yl)methyl)piperidin-4-yl)ethyl)amino)-9H-purin-9-yl)cyclobutyl)-2-phenylacetamide |
| 424 | N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)phenyl)amino)-9H-purin-9-yl)cyclobutyl)-6-(trifluoromethyl)picolinamide |
| 425 | N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)phenyl)amino)-9H-purin-9-yl)cyclobutyl)-6-(trifluoromethyl)picolinamide |
| 426 | N-((1s,3s)-3-(6-((4-(4-((1-(3-(2-(2,6-dioxopiperidin-3-yl)-1,3-dioxoisoindolin-4-yl)propyl)piperidin-4-yl)methyl)piperazin-1-yl)phenyl)amino)-9H-purin-9-yl)cyclobutyl)-6-(trifluoromethyl)picolinamide |
| 427 | N-((1s,3s)-3-(6-((4-(4-((1-(4-(2-(2,6-dioxopiperidin-3-yl)-1,3-dioxoisoindolin-4-yl)butyl)piperidin-4-yl)methyl)piperazin-1-yl)phenyl)amino)-9H-purin-9-yl)cyclobutyl)-6-(trifluoromethyl)picolinamide |
| 428 | N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)-9H-purin-9-yl)cyclobutyl)-6-fluoropicolinamide |
| 429 | N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)phenyl)amino)-9H-purin-9-yl)cyclobutyl)-6-fluoropicolinamide |
| 430 | N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)phenyl)amino)-9H-purin-9-yl)cyclobutyl)-6-fluoropicolinamide |
| 431 | N-((1s,3s)-3-(6-((4-(4-((1-(3-(2-(2,6-dioxopiperidin-3-yl)-1,3-dioxoisoindolin-4-yl)propyl)piperidin-4-yl)methyl)piperazin-1-yl)phenyl)amino)-9H-purin-9-yl)cyclobutyl)-6-fluoropicolinamide |
| 432 | N-((1s,3s)-3-(6-((4-(4-((1-(4-(2-(2,6-dioxopiperidin-3-yl)-1,3-dioxoisoindolin-4-yl)butyl)piperidin-4-yl)methyl)piperazin-1-yl)phenyl)amino)-9H-purin-9-yl)cyclobutyl)-6-fluoropicolinamide |
| 433 | N-((1s,3s)-3-(6-((4-(4-(2-(1-(2-(4-(2,6-dioxopiperidin-3-yl)phenoxy)acetyl)piperidin-4-yl)ethyl)piperazin-1-yl)phenyl)amino)-9H-purin-9-yl)cyclobutyl)-2-phenylacetamide |
| 434 | 2,6-dichloro-N-((1s,3s)-3-(6-((4-(4-(2-(1-(2-(4-(2,6-dioxopiperidin-3-yl)phenoxy)acetyl)piperidin-4-yl)ethyl)piperazin-1-yl)phenyl)amino)-9H-purin-9-yl)cyclobutyl)benzamide |
| 435 | N-((1s,3s)-3-(6-((4-(4-(7-(2-(4-(2,6-dioxopiperidin-3-yl)phenoxy)acetyl)-7-azaspiro[3.5]nonan-2-yl)piperazin-1-yl)phenyl)amino)-9H-purin-9-yl)cyclobutyl)-6-methylpicolinamide |
| 436 | N-((1s,3s)-3-(6-((4-(4-((1-(2-(4-(2,6-dioxopiperidin-3-yl)phenoxy)acetyl)piperidin-4-yl)methyl)piperazin-1-yl)phenyl)amino)-9H-purin-9-yl)cyclobutyl)-6-(trifluoromethyl)picolinamide |
| 437 | 2,6-dichloro-N-((1s,3s)-3-(6-((4-(4-((1-(2-(4-(2,6-dioxopiperidin-3-yl)phenoxy)acetyl)piperidin-4-yl)methyl)piperazin-1-yl)phenyl)amino)-9H-purin-9-yl)cyclobutyl)benzamide |
| 438 | N-((1s,3s)-3-(6-((4-(4-(2-(1-(2-(4-(2,6-dioxopiperidin-3-yl)phenoxy)acetyl)piperidin-4-yl)ethyl)piperazin-1-yl)phenyl)amino)-9H-purin-9-yl)cyclobutyl)-6-(trifluoromethyl)picolinamide |
| 439 | N-((1s,3s)-3-(6-((4-(4-(7-(2-(4-(2,6-dioxopiperidin-3-yl)phenoxy)acetyl)-7-azaspiro[3.5]nonan-2-yl)piperazin-1-yl)phenyl)amino)-9H-purin-9-yl)cyclobutyl)-6-(trifluoromethyl)picolinamide |
| 440 | 6-chloro-N-((1s,3s)-3-(6-((4-(4-((1-(2-(4-(2,6-dioxopiperidin-3-yl)phenoxy)acetyl)piperidin-4-yl)methyl)piperazin-1-yl)phenyl)amino)-9H-purin-9-yl)cyclobutyl)picolinamide |
| 441 | 6-cyano-N-((1s,3s)-3-(6-((4-(4-((1-(2-(2,6-dioxopiperidin-3-yl)-1-oxoisoindolin-5-yl)piperidin-4-yl)methyl)piperazin-1-yl)phenyl)amino)-9H-purin-9-yl)cyclobutyl)picolinamide |
| 442 | 2,6-dichloro-N-((1s,3s)-3-(6-((4-(4-(7-(2-(4-(2,6-dioxopiperidin-3-yl)phenoxy)acetyl)-7-azaspiro[3.5]nonan-2-yl)piperazin-1-yl)phenyl)amino)-9H-purin-9-yl)cyclobutyl)benzamide |
| 443 | N-((1s,3s)-3-(6-((4-(4-((1-(2-(4-(2,6-dioxopiperidin-3-yl)phenoxy)acetyl)piperidin-4-yl)methyl)piperazin-1-yl)phenyl)amino)-9H-purin-9-yl)cyclobutyl)-6-methylpicolinamide |
| 444 | N-((1s,3s)-3-(6-((4-(4-(2-(1-(2-(4-(2,6-dioxopiperidin-3-yl)phenoxy)acetyl)piperidin-4-yl)ethyl)piperazin-1-yl)phenyl)amino)-9H-purin-9-yl)cyclobutyl)-6-methylpicolinamide |
| 445 | N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)-2-methylphenyl)amino)-9H-purin-9-yl)cyclobutyl)-6-methylpicolinamide |
| 446 | N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)-2-methylphenyl)amino)-9H-purin-9-yl)cyclobutyl)-6-methylpicolinamide |
| 447 | N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)-2-methylphenyl)amino)-9H-purin-9-yl)cyclobutyl)-6-methylpicolinamide |
| 448 | N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)-2-methylphenyl)amino)-9H-purin-9-yl)cyclobutyl)-6-methylpicolinamide |
| 449 | N-((1s,3s)-3-(6-((4-(4-(2-(1-(1-(2-(2,6-dioxopiperidin-3-yl)-6-fluoro-1,3-dioxoisoindolin-5-yl)piperidin-4-carbonyl)piperidin-4-yl)ethyl)piperazin-1-yl)phenyl)amino)-9H-purin-9-yl)cyclobutyl)-6-methylpicolinamide |
| 450 | N-((1s,3s)-3-(6-((2-(1-((1-(1-(2-(2,6-dioxopiperidin-3-yl)-6-fluoro-1,3-dioxoisoindolin-5-yl)piperidin-4-carbonyl)piperidin-4-yl)methyl)piperidin-4-yl)ethyl)amino)-9H-purin-9-yl)cyclobutyl)-6-methylpicolinamide |
| 451 | 6-chloro-N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)phenyl)amino)-9H-purin-9-yl)cyclobutyl)picolinamide |
| 452 | 6-chloro-N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)phenyl)amino)-9H-purin-9-yl)cyclobutyl)picolinamide |
| 453 | 6-chloro-N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)phenyl)amino)-9H-purin-9-yl)cyclobutyl)picolinamide |
| 454 | 6-cyano-N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)phenyl)amino)-9H-purin-9-yl)cyclobutyl)picolinamide |
| 455 | 6-cyano-N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)phenyl)amino)-9H-purin-9-yl)cyclobutyl)picolinamide |
| 456 | N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)phenyl)amino)-9H-purin-9-yl)cyclobutyl)-2-phenylacetamide |
| 457 | N-((1s,3s)-3-(6-((4-(4-(2-(1-(1-(2-(2,6-dioxopiperidin-3-yl)-6-fluoro-1,3-dioxoisoindolin-5-yl)piperidin-4-carbonyl)piperidin-4-yl)ethyl)piperazin-1-yl)phenyl)amino)-9H-purin-9-yl)cyclobutyl)-2-phenylacetamide |
| 458 | N-((1s,3s)-3-(6-((4-(4-(3-(1-(2-(2,6-dioxopiperidin-3-yl)-6-fluoro-1,3-dioxoisoindolin-5-yl)piperidin-4-yl)propyl)piperazin-1-yl)phenyl)amino)-9H-purin-9-yl)cyclobutyl)-2-phenylacetamide |
| 459 | N-((1s,3s)-3-(6-((4-(4-(3-(1-(1-(2-(2,6-dioxopiperidin-3-yl)-6-fluoro-1,3-dioxoisoindolin-5-yl)piperidin-4-carbonyl)piperidin-4-yl)propyl)piperazin-1-yl)phenyl)amino)-9H-purin-9-yl)cyclobutyl)-2-phenylacetamide |
| 460 | N-((1s,3s)-3-(6-((4-(4-(7-(1-(2-(2,6-dioxopiperidin-3-yl)-6-fluoro-1,3-dioxoisoindolin-5-yl)piperidin-4-carbonyl)-7-azaspiro[3.5]nonan-2-yl)piperazin-1-yl)phenyl)amino)-9H-purin-9-yl)cyclobutyl)-2-phenylacetamide |
| 461 | N-((1s,3s)-3-(6-((2-(1-((1-(1-(2-(2,6-dioxopiperidin-3-yl)-6-fluoro-1,3-dioxoisoindolin-5-yl)piperidin-4-carbonyl)piperidin-4-yl)methyl)piperidin-4-yl)ethyl)amino)-9H-purin-9-yl)cyclobutyl)-2-phenylacetamide |
| 462 | 2,6-dichloro-N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)phenyl)amino)-9H-purin-9-yl)cyclobutyl)benzamide |
| 463 | 2,6-dichloro-N-((1s,3s)-3-(6-((4-(4-(2-(1-(1-(2-(2,6-dioxopiperidin-3-yl)-6-fluoro-1,3-dioxoisoindolin-5-yl)piperidin-4-carbonyl)piperidin-4-yl)ethyl)piperazin-1-yl)phenyl)amino)-9H-purin-9-yl)cyclobutyl)benzamide |
| 464 | 2,6-dichloro-N-((1s,3s)-3-(6-((4-(4-(7-(1-(2-(2,6-dioxopiperidin-3-yl)-6-fluoro-1,3-dioxoisoindolin-5-yl)piperidin-4-carbonyl)-7-azaspiro[3.5]nonan-2-yl)piperazin-1-yl)phenyl)amino)-9H-purin-9-yl)cyclobutyl)benzamide |
| 465 | N-((1s,3s)-3-(6-((4-(4-(7-(1-(2-(2,6-dioxopiperidin-3-yl)-6-fluoro-1,3-dioxoisoindolin-5-yl)piperidin-4-carbonyl)-7-azaspiro[3.5]nonan-2-yl)piperazin-1-yl)phenyl)amino)-9H-purin-9-yl)cyclobutyl)-6-methylpicolinamide |
| 466 | N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)phenyl)amino)-9H-purin-9-yl)cyclobutyl)-6-fluoropicolinamide |
| 467 | N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)phenyl)amino)-9H-purin-9-yl)cyclobutyl)-6-fluoropicolinamide |
| 468 | N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)phenyl)amino)-9H-purin-9-yl)cyclobutyl)-6-fluoropicolinamide |
| 469 | N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)phenyl)amino)-9H-purin-9-yl)cyclobutyl)-6-(trifluoromethyl)picolinamide |
| 470 | N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)phenyl)amino)-9H-purin-9-yl)cyclobutyl)-6-(trifluoromethyl)picolinamide |
| 471 | N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)phenyl)amino)-9H-purin-9-yl)cyclobutyl)-6-(trifluoromethyl)picolinamide |
| 472 | 6-cyano-N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)phenyl)amino)-9H-purin-9-yl)cyclobutyl)picolinamide |
| 473 | 2,6-dichloro-N-((1s,3s)-3-(6-((4-(4-((4-(1-(2-(2,6-dioxopiperidin-3-yl)-1,3-dioxoisoindolin-5-yl)azetidin-3-carbonyl)piperazin-1-yl)methyl)piperidin-1-yl)phenyl)amino)-9H-purin-9-yl)cyclobutyl)benzamide |
| 474 | 2,6-dichloro-N-((1s,3s)-3-(6-((4-(4-((4-(1-(2-(2,6-dioxopiperidin-3-yl)-1,3-dioxoisoindolin-5-yl)piperidin-4-carbonyl)piperazin-1-yl)methyl)piperidin-1-yl)phenyl)amino)-9H-purin-9-yl)cyclobutyl)benzamide |
| 475 | 2,6-dichloro-N-((1s,3s)-3-(6-((4-(4-((4-(1-(2-(2,6-dioxopiperidin-3-yl)-6-fluoro-1,3-dioxoisoindolin-5-yl)piperidin-4-carbonyl)piperazin-1-yl)methyl)piperidin-1-yl)phenyl)amino)-9H-purin-9-yl)cyclobutyl)benzamide |
| 476 | N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)phenyl)amino)-9H-purin-9-yl)cyclobutyl)-6-methylpicolinamide |
| 477 | N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)-2-methylphenyl)amino)-9H-purin-9-yl)cyclobutyl)-6-methylpicolinamide |
| 478 | N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)-2-methylphenyl)amino)-9H-purin-9-yl)cyclobutyl)-6-methylpicolinamide |
| 479 | N-((1s,3s)-3-(6-((4-(4-(7-(4-(4-(2-(2,6-dioxopiperidin-3-yl)-1,3-dioxoisoindolin-5-yl)piperazin-1-yl)but-2-en-1-yl)-7-azaspiro[3.5]nonan-2-yl)piperazin-1-yl)phenyl)amino)-9H-purin-9-yl)cyclobutyl)-2-phenylacetamide |

**[Table 2]**

| Compound # | Structural Formula |
|---|---|
| 1 | 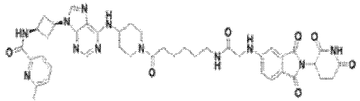 |
| 2 | 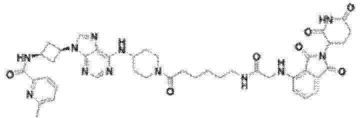 |
| 3 | 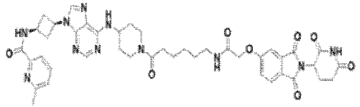 |
| 4 | 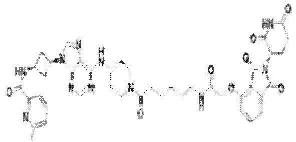 |
| 5 | 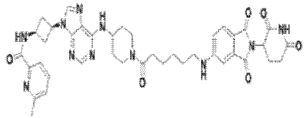 |
| 6 | 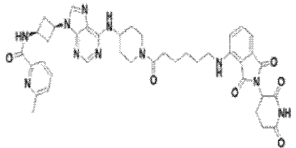 |
| 7 | 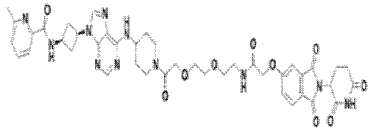 |
| 8 | 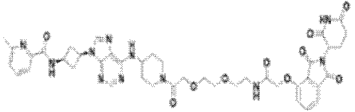 |
| 9 | 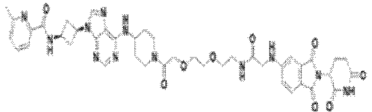 |
| 10 | 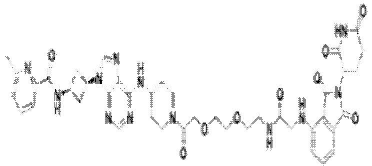 |
| 11 | 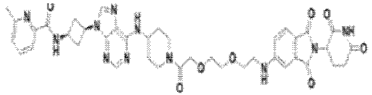 |
| 12 | 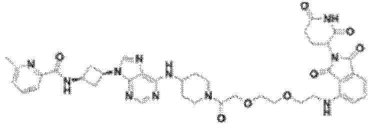 |
| 13 | 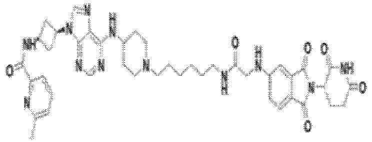 |
| 14 | 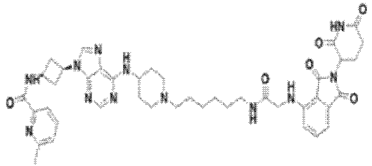 |
| 15 | 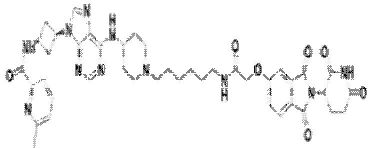 |
| 16 | 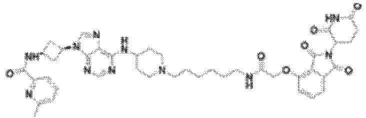 |
| 17 | 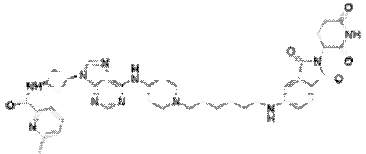 |
| 18 | 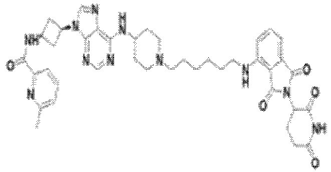 |
| 19 | 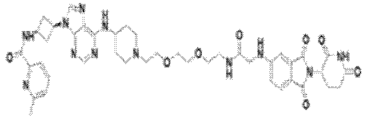 |
| 20 | 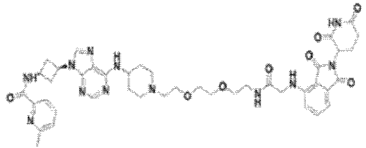 |
| 21 | 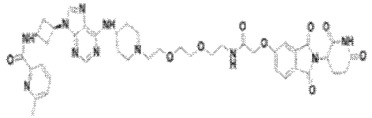 |
| 22 | 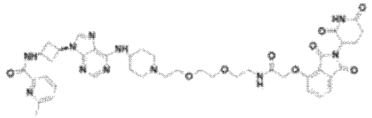 |
| 23 | 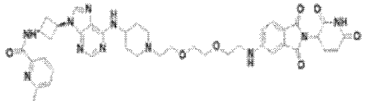 |
| 24 | 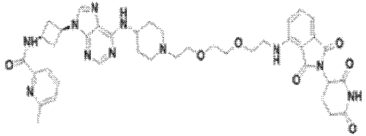 |
| 25 | 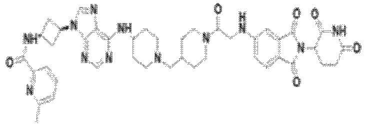 |
| 26 | 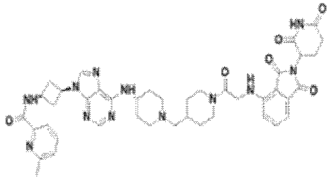 |
| 27 | 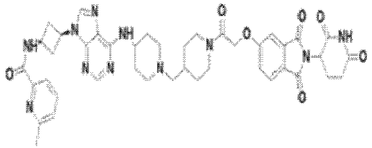 |
| 28 | 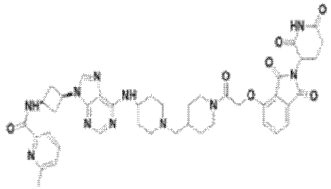 |
| 29 | 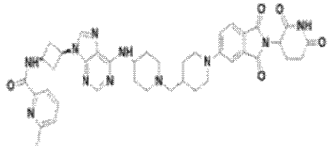 |
| 30 | 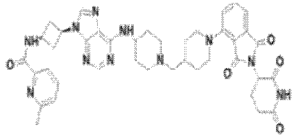 |

**[Table 3]**

| | |
|---|---|
| 31 | 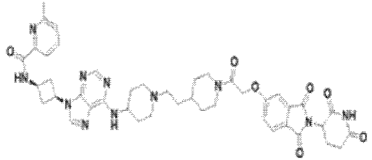 |
| 32 | 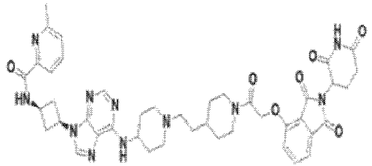 |
| 33 | 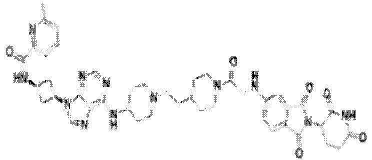 |
| 34 | 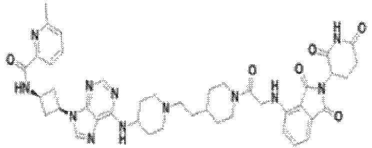 |
| 35 | 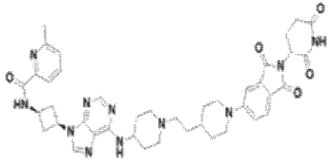 |
| 36 | 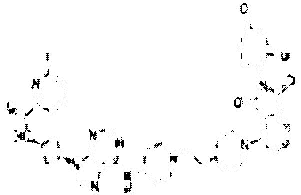 |
| 37 | 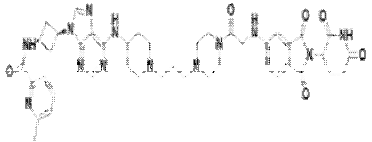 |
| 38 | 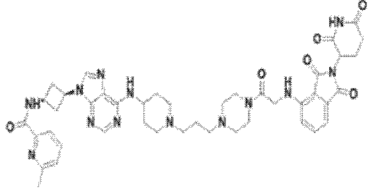 |
| 39 | 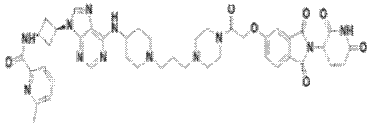 |
| 40 | 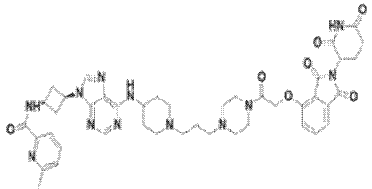 |
| 41 | 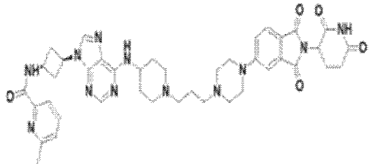 |
| 42 | 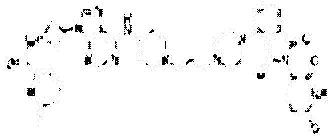 |
| 43 | 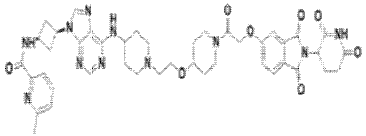 |
| 44 | 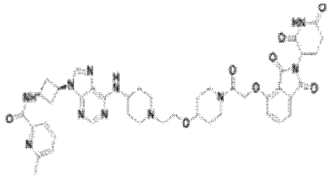 |
| 45 | 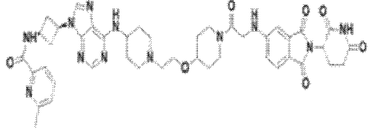 |
| 46 | 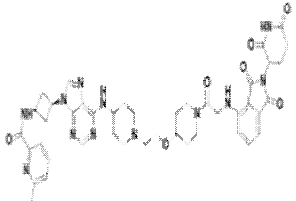 |
| 47 | 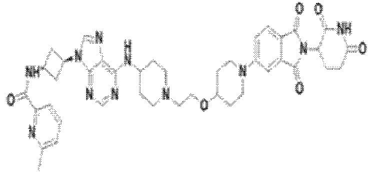 |
| 48 | 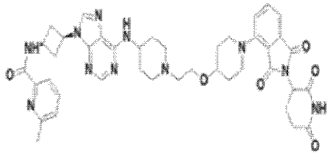 |
| 49 | 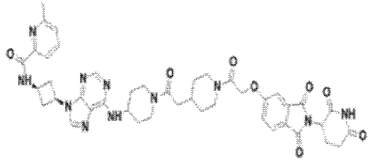 |
| 50 | 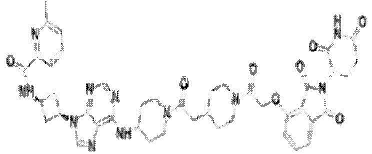 |
| 51 | 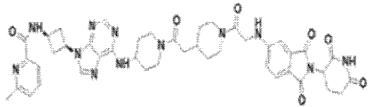 |
| 52 | 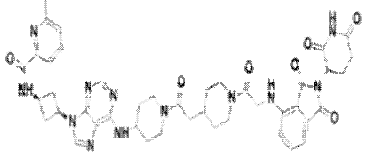 |
| 53 | 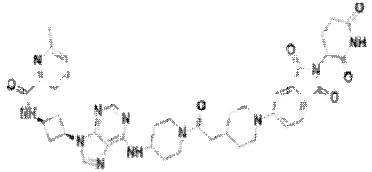 |
| 54 | 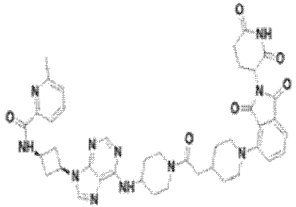 |
| 55 | 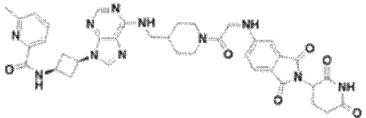 |
| 56 | 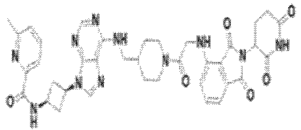 |
| 57 | 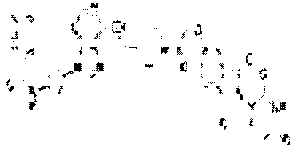 |
| 58 | 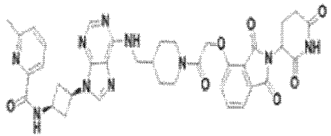 |

**[Table 4]**

| | |
|---|---|
| 59 | 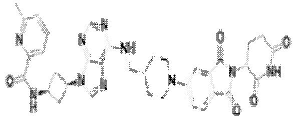 |
| 60 | 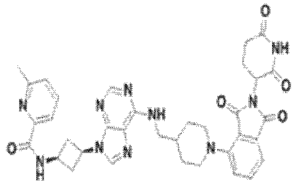 |
| 61 | 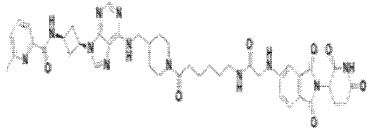 |
| 62 | 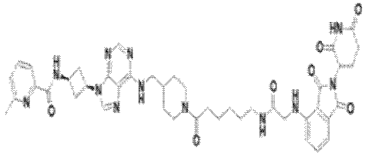 |
| 63 | 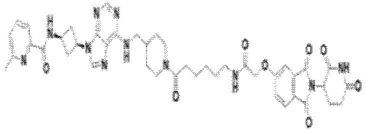 |
| 64 | 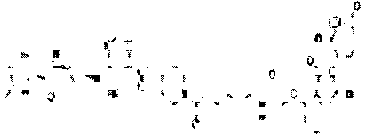 |
| 65 | 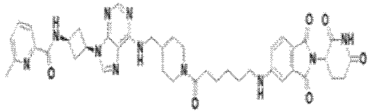 |
| 66 | 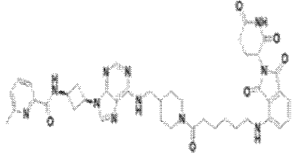 |
| 67 | 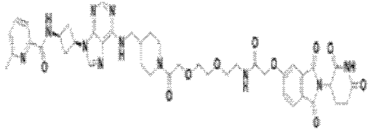 |
| 68 | 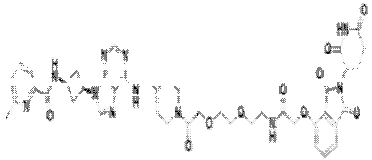 |
| 69 | 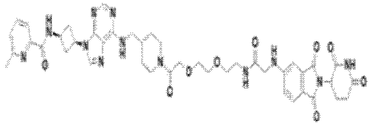 |
| 70 | 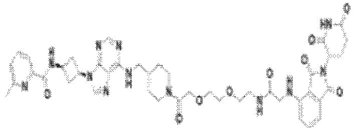 |
| 71 | 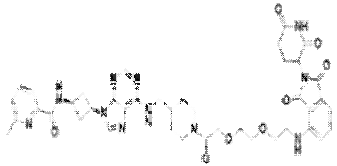 |
| 72 | 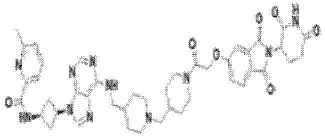 |
| 73 | 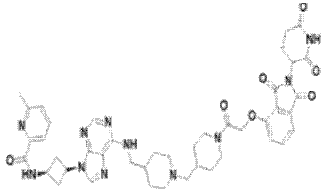 |
| 74 | 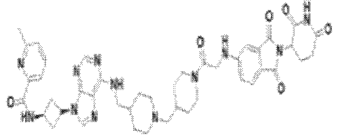 |
| 75 | 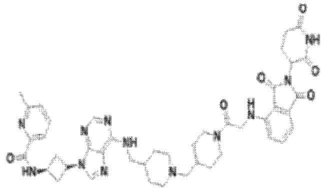 |
| 76 | 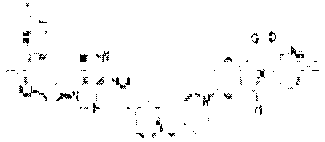 |
| 77 | 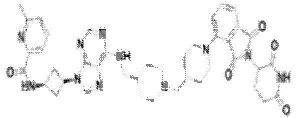 |
| 78 | 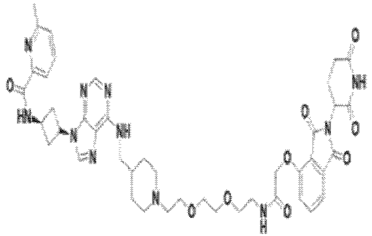 |
| 79 | 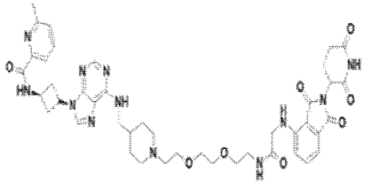 |
| 80 | 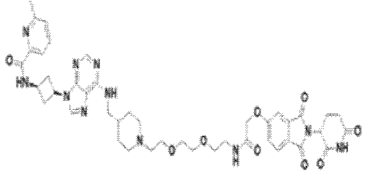 |
| 81 | 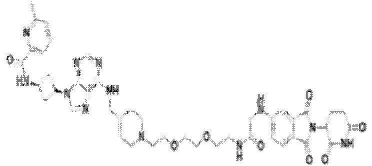 |
| 82 | 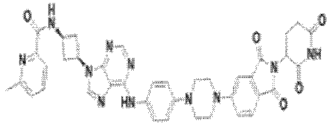 |
| 83 | 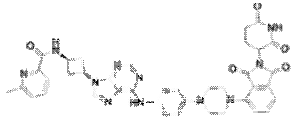 |
| 84 | 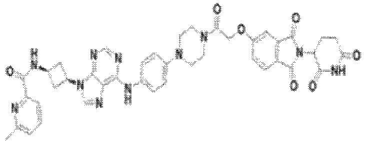 |
| 85 | 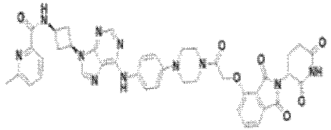 |
| 86 | 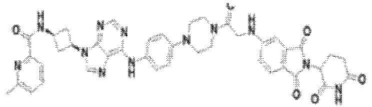 |
| 87 | 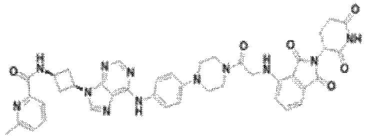 |

**[Table 5]**

| | |
|---|---|
| 88 | 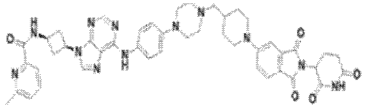 |
| 89 | 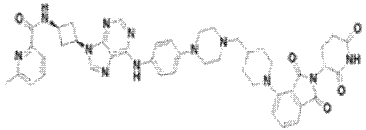 |
| 90 | 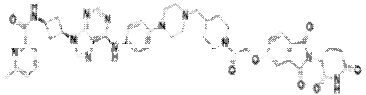 |
| 91 | 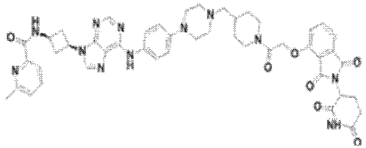 |
| 92 | 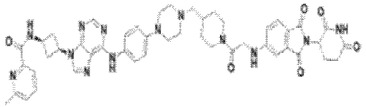 |
| 93 | 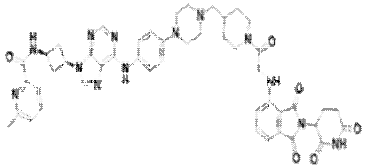 |
| 94 | 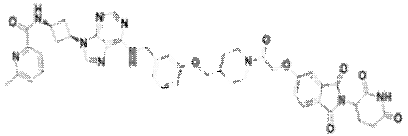 |
| 95 | 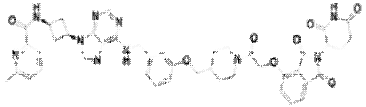 |
| 96 | 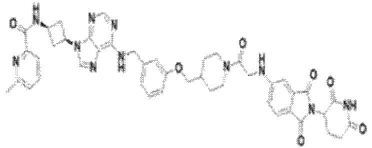 |
| 97 | 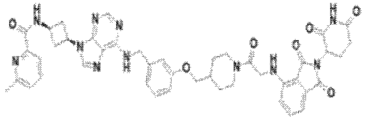 |
| 98 | 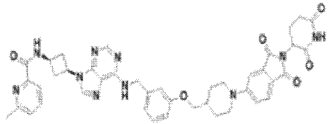 |
| 99 | 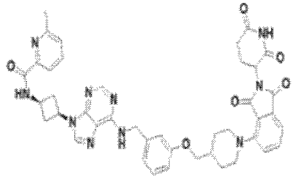 |
| 100 | 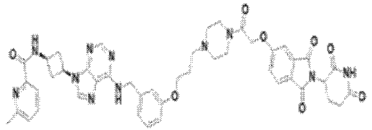 |
| 101 | 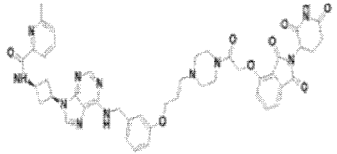 |
| 102 | 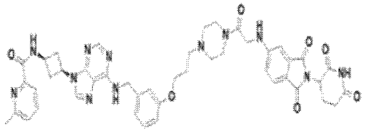 |
| 103 | 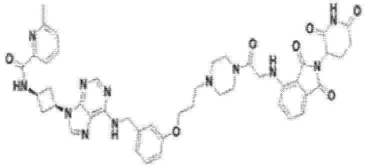 |
| 104 | 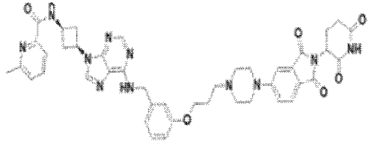 |
| 105 | 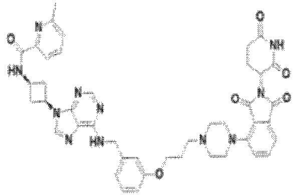 |
| 106 | 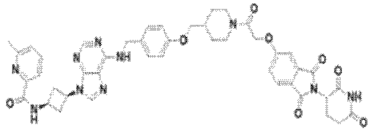 |
| 107 | 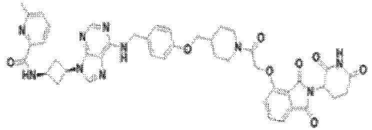 |
| 108 | 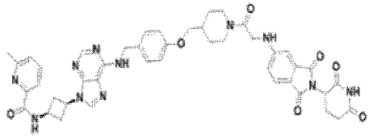 |
| 109 | 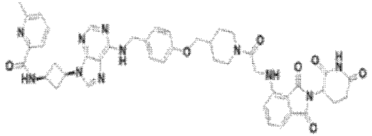 |
| 110 | 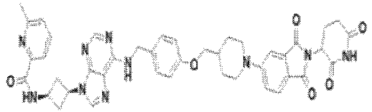 |
| 111 | 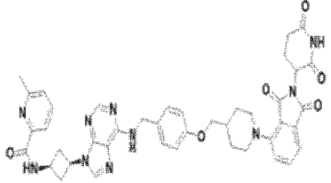 |
| 112 | 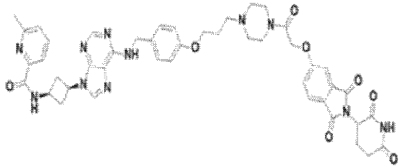 |
| 113 | 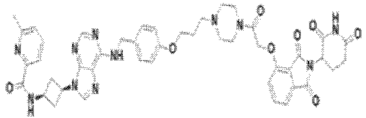 |
| 114 | 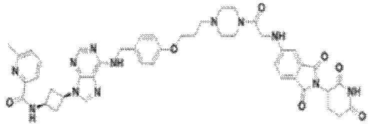 |
| 115 | 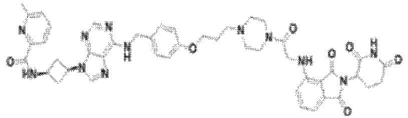 |
| 116 | 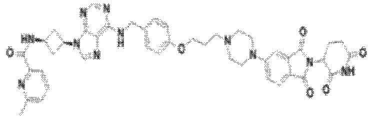 |
| 117 | 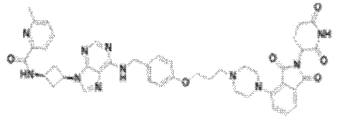 |
| 118 | 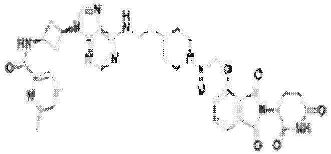 |

**[Table 6]**

| | |
|---|---|
| 119 | 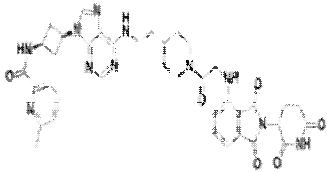 |
| 120 | 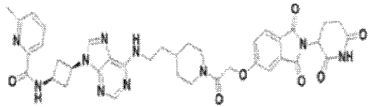 |
| 121 | 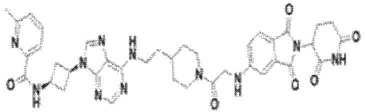 |
| 122 | 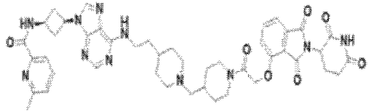 |
| 123 | 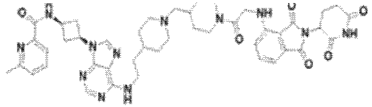 |
| 124 | 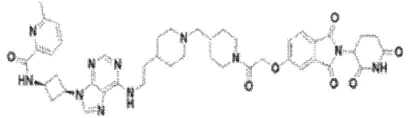 |
| 125 | 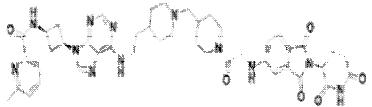 |
| 126 | 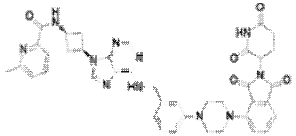 |
| 127 | 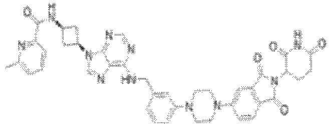 |
| 128 | 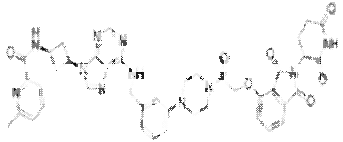 |
| 129 | 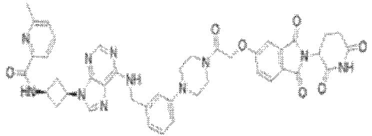 |
| 130 | 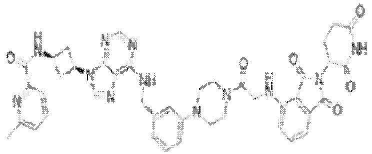 |
| 131 | 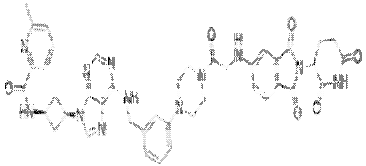 |
| 132 | 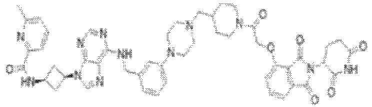 |
| 133 | 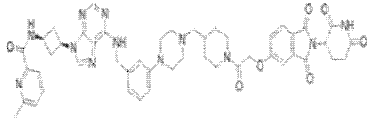 |
| 134 | 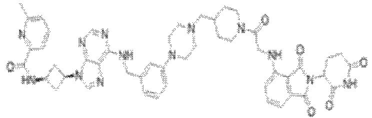 |
| 135 | 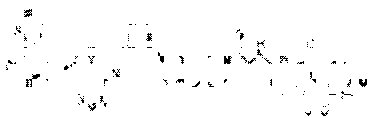 |
| 136 | 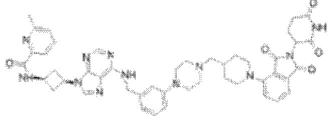 |
| 137 | 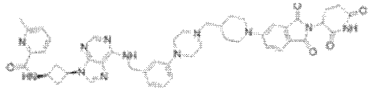 |
| 138 | 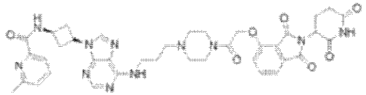 |
| 139 | 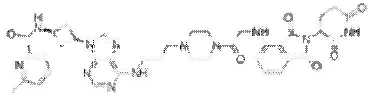 |
| 140 | 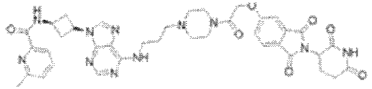 |
| 141 | 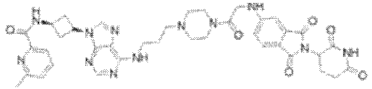 |
| 142 | 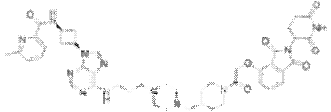 |
| 143 | 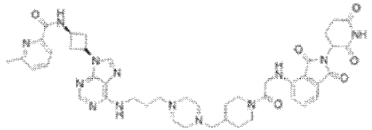 |
| 144 | 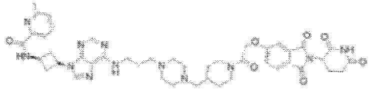 |
| 145 | 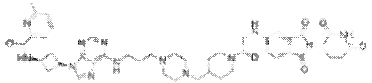 |
| 146 | 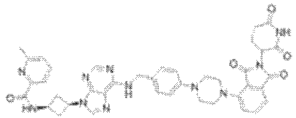 |
| 147 | 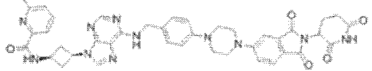 |
| 148 | 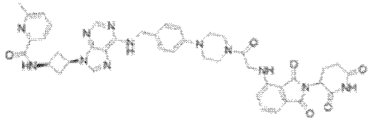 |
| 149 | 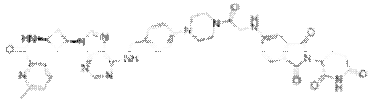 |
| 150 | 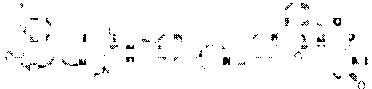 |
| 151 | 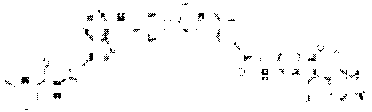 |
| 152 | 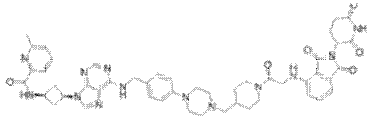 |
| 153 | 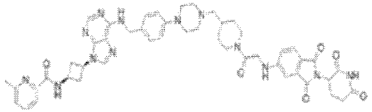 |
| 154 | 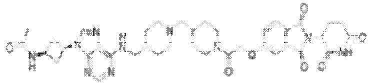 |
| 155 | 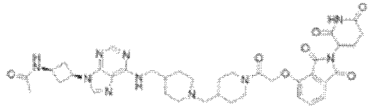 |
| 156 | 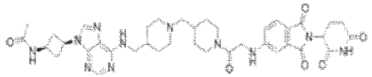 |
| 157 | 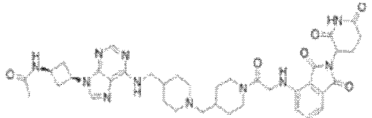 |

**[Table 7]**

| | |
|---|---|
| 158 | 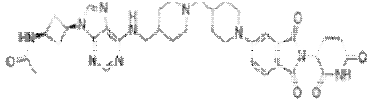 |
| 159 | 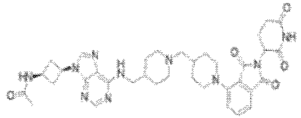 |
| 160 | 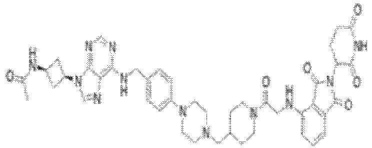 |
| 161 | 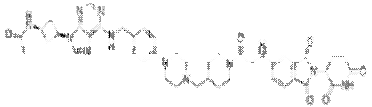 |
| 162 | 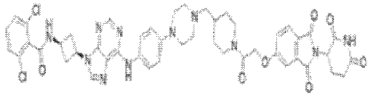 |
| 163 | 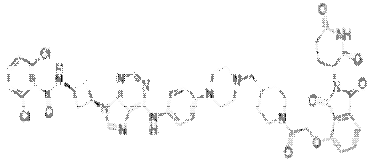 |
| 164 | 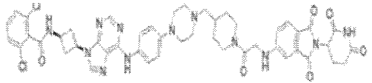 |
| 165 | 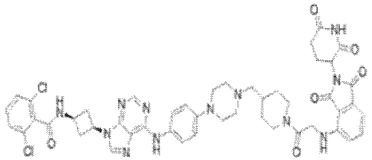 |
| 166 | 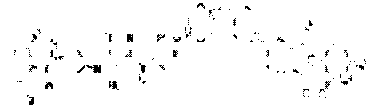 |
| 167 | 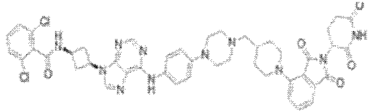 |
| 168 | 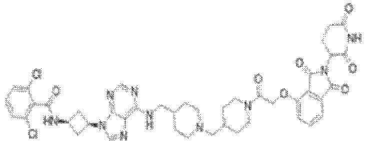 |
| 169 | 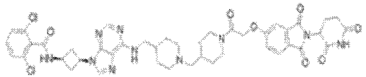 |
| 170 | 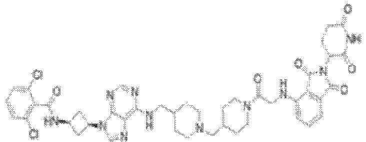 |
| 171 | 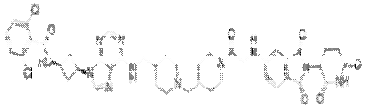 |
| 172 | 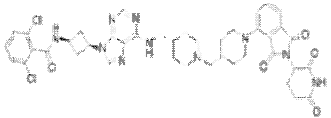 |
| 173 | 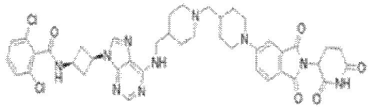 |
| 174 | 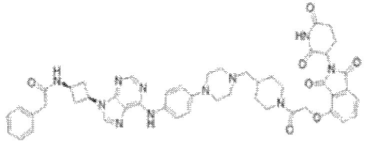 |
| 175 | 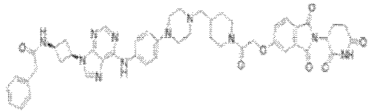 |
| 176 | 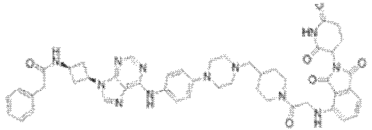 |
| 177 | 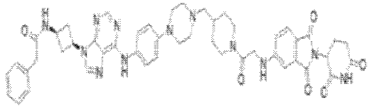 |
| 178 | 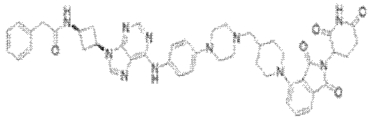 |
| 179 | 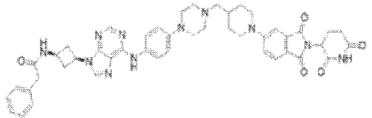 |
| 180 | 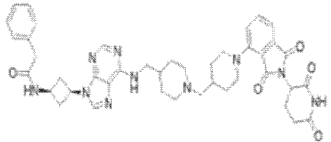 |
| 181 | 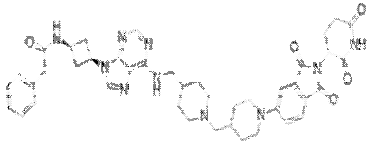 |
| 182 | 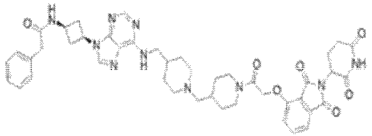 |
| 183 | 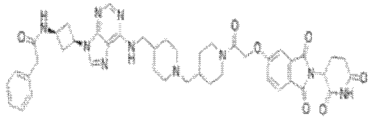 |
| 184 | 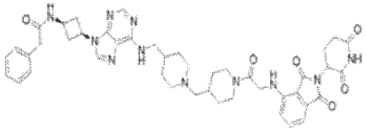 |
| 185 | 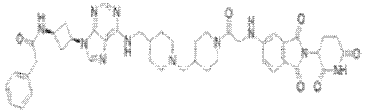 |
| 186 | 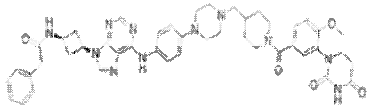 |
| 187 | 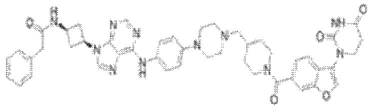 |
| 188 | 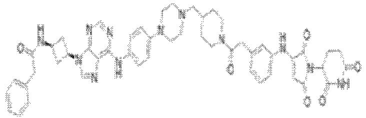 |
| 189 | 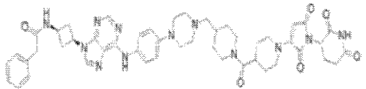 |
| 190 | 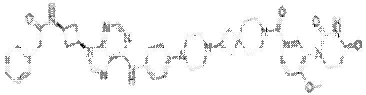 |
| 191 | 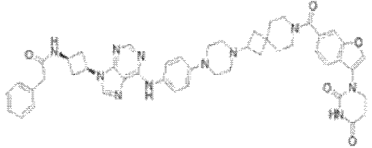 |
| 192 | 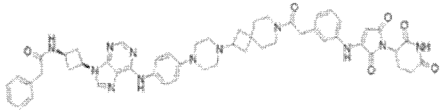 |
| 193 | 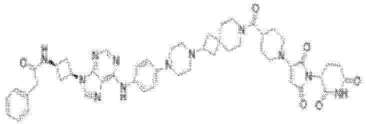 |

**[Table 8]**

| | |
|---|---|
| 194 | 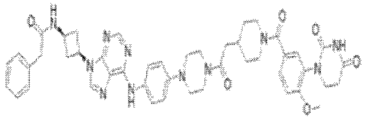 |
| 195 | 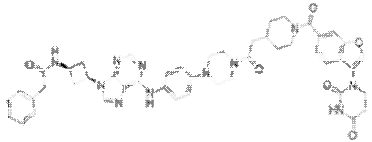 |
| 196 | 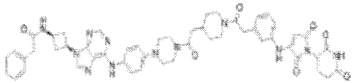 |
| 197 | 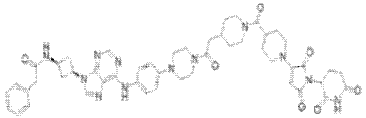 |
| 198 | 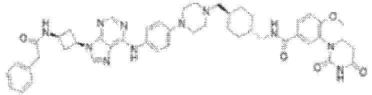 |
| 199 | 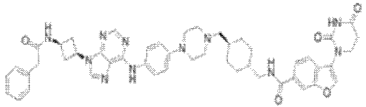 |
| 200 | 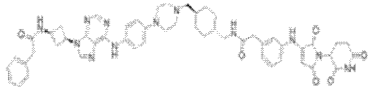 |
| 201 | 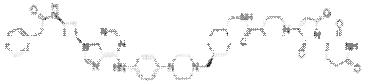 |
| 202 | 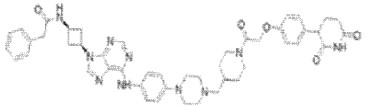 |
| 203 | 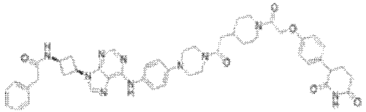 |
| 204 | 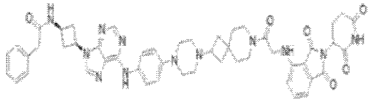 |
| 205 | 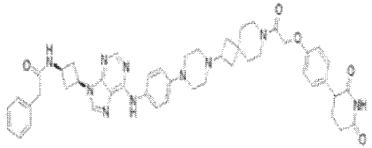 |
| 206 | 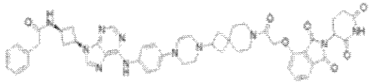 |
| 207 | 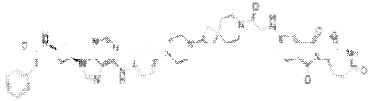 |
| 208 | 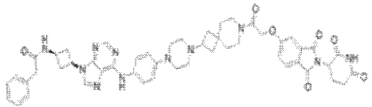 |
| 209 | 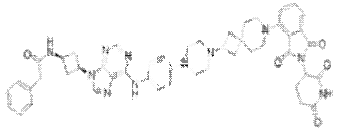 |
| 210 | 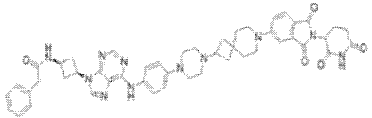 |
| 211 | 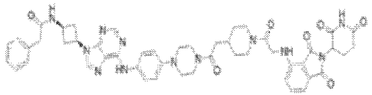 |
| 212 | 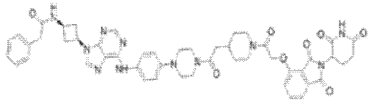 |
| 213 | 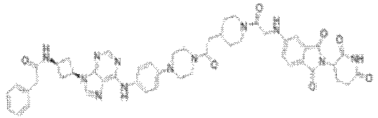 |
| 214 | 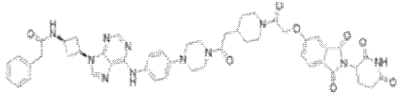 |
| 215 | 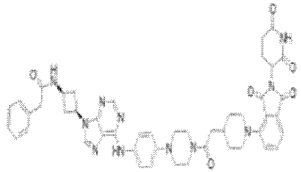 |
| 216 | 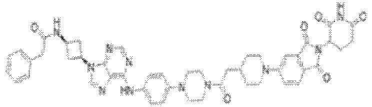 |
| 217 | 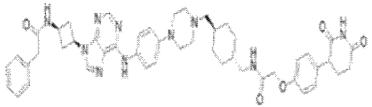 |
| 218 | 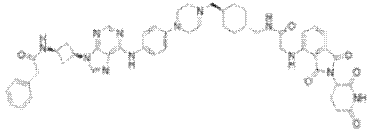 |
| 219 | 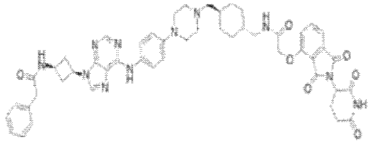 |
| 220 | 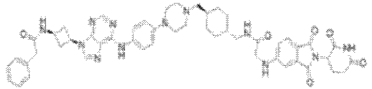 |
| 221 | 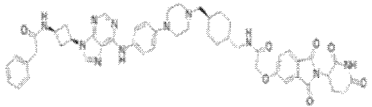 |
| 222 | 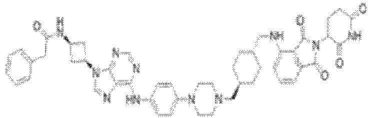 |
| 223 | 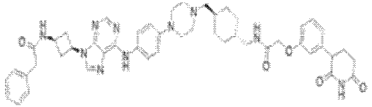 |
| 224 | 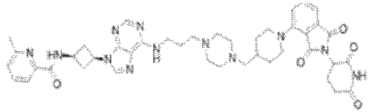 |
| 225 | 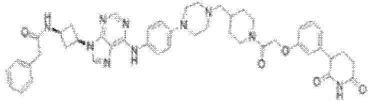 |
| 226 | 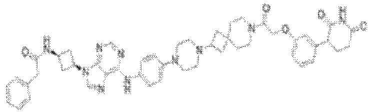 |
| 227 | 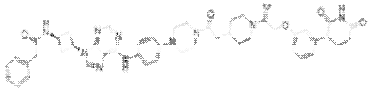 |
| 228 | 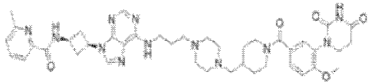 |
| 229 | 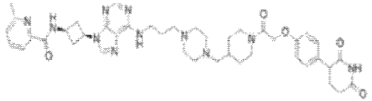 |
| 230 | 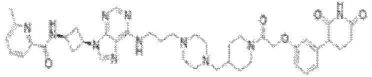 |
| 231 | 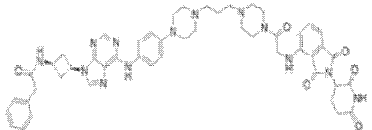 |
| 232 | 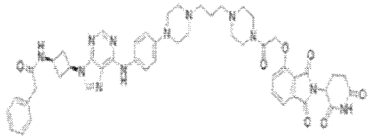 |

**[Table 9]**

| | |
|---|---|
| 233 | 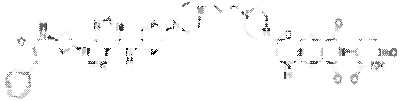 |
| 234 | 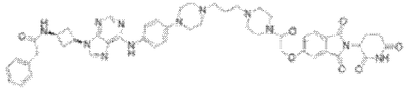 |
| 235 | 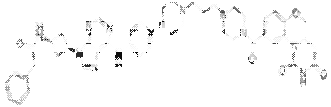 |
| 236 | 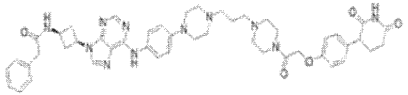 |
| 237 | 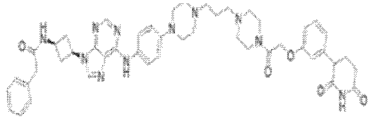 |
| 238 | 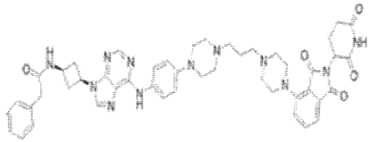 |
| 239 | 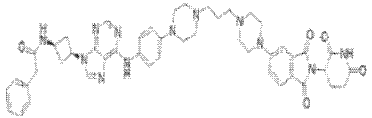 |
| 240 | 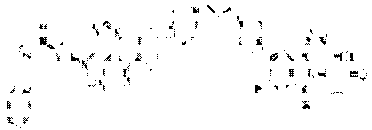 |
| 241 | 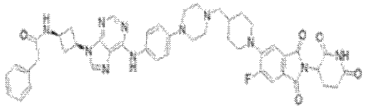 |
| 242 | 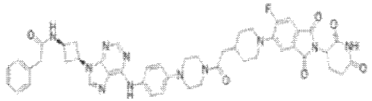 |
| 243 | 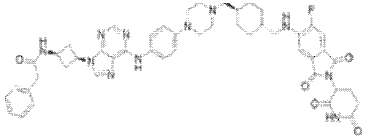 |
| 244 | 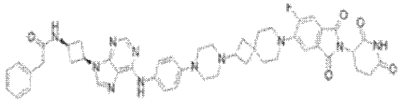 |
| 245 | 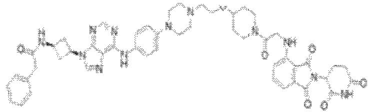 |
| 246 | 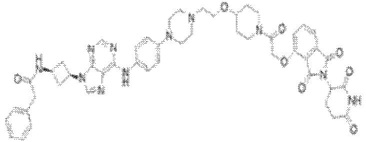 |
| 247 | 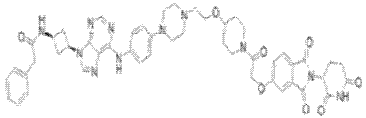 |
| 248 | 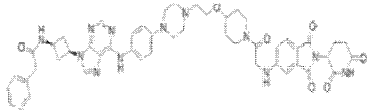 |
| 249 | 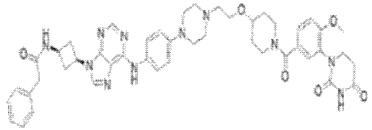 |
| 250 | 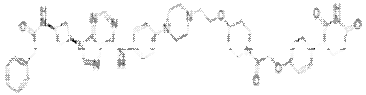 |
| 251 | 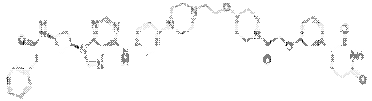 |
| 252 | 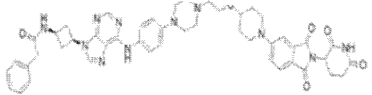 |
| 253 | 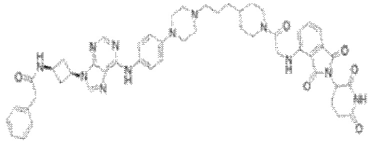 |
| 254 | 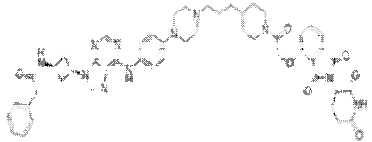 |
| 255 | 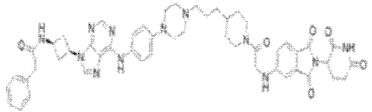 |
| 256 | 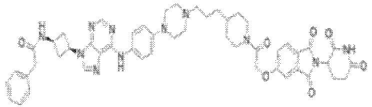 |
| 257 | 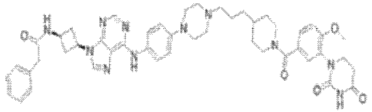 |
| 258 | 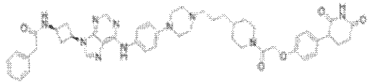 |
| 259 | 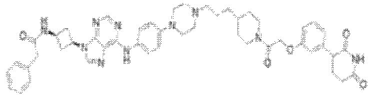 |
| 260 | 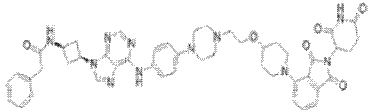 |
| 261 | 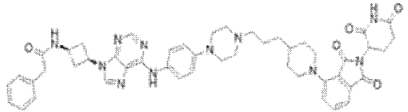 |
| 262 | 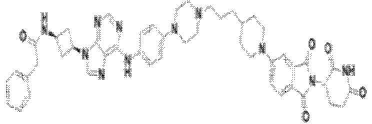 |
| 263 | 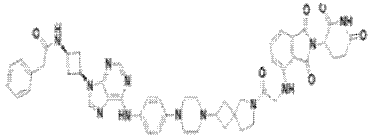 |
| 264 | 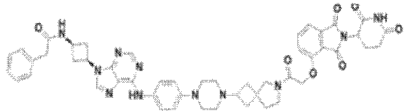 |
| 265 | 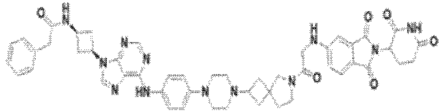 |
| 266 | 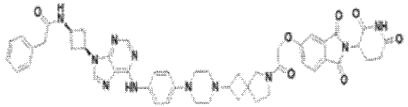 |
| 267 | 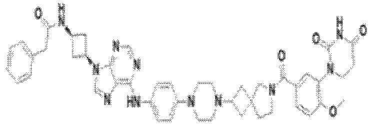 |
| 268 | 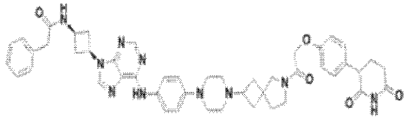 |
| 269 | 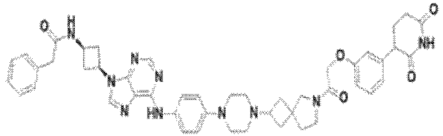 |
| 270 | 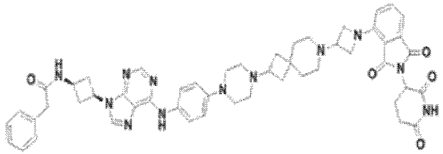 |

**[Table 10]**

| | |
|---|---|
| 271 | 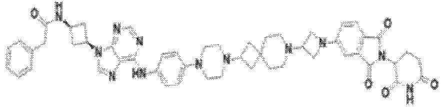 |
| 272 | 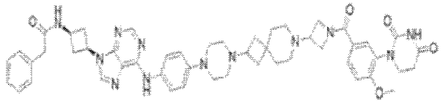 |
| 273 | 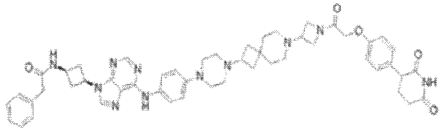 |
| 274 | 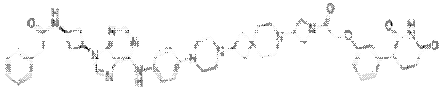 |
| 275 | 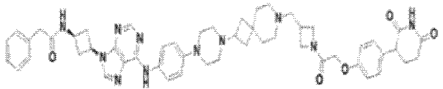 |
| 276 | 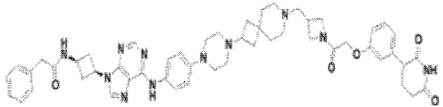 |
| 277 | 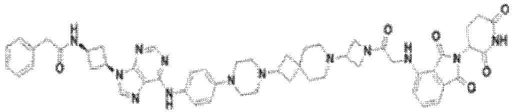 |
| 278 | 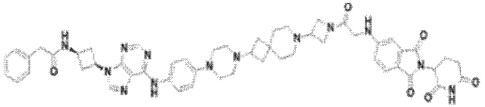 |
| 279 | 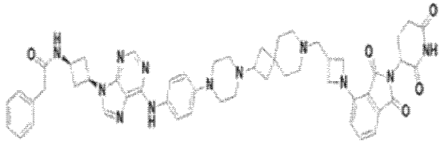 |
| 280 | 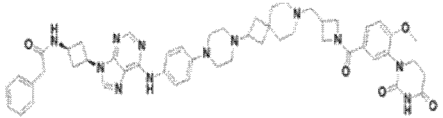 |
| 281 | 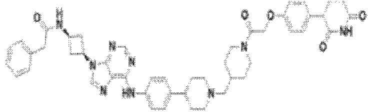 |
| 282 | 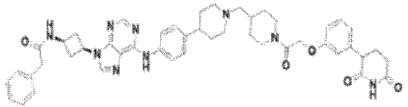 |
| 283 | 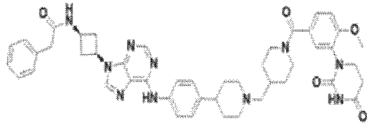 |
| 284 | 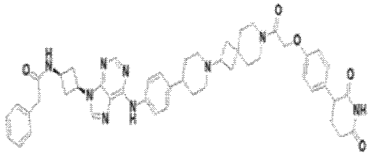 |
| 285 | 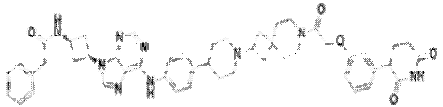 |
| 286 | 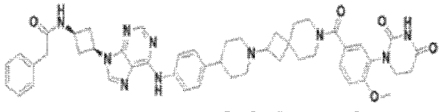 |
| 287 | 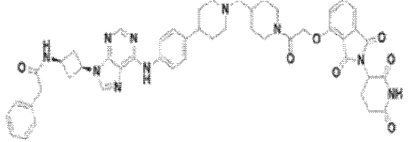 |
| 288 | 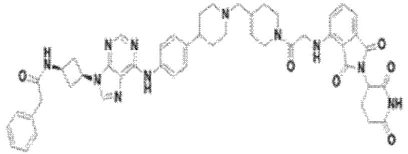 |
| 289 | 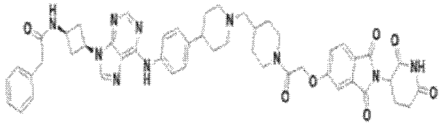 |
| 290 | 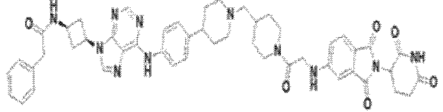 |
| 291 | 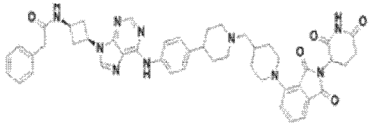 |
| 292 | 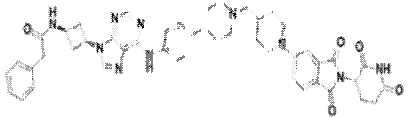 |
| 293 | 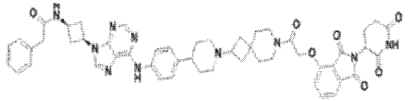 |
| 294 | 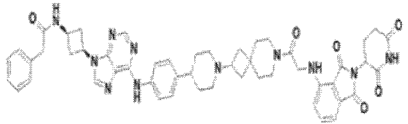 |
| 295 | 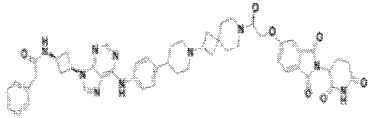 |
| 296 | 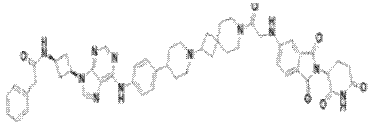 |
| 297 | 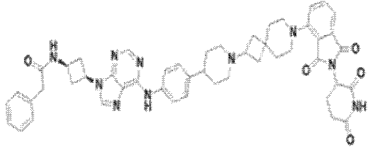 |
| 298 | 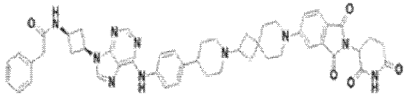 |
| 299 | 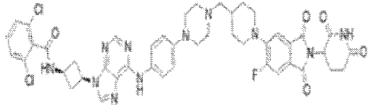 |
| 300 | 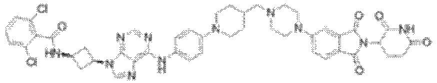 |
| 301 | 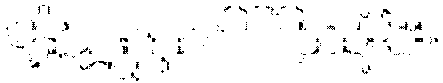 |
| 302 | 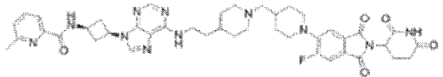 |
| 303 | 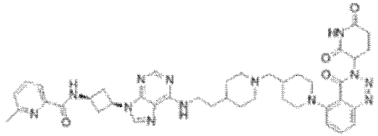 |
| 304 | 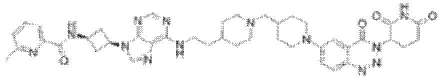 |
| 305 | 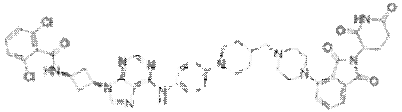 |
| 306 | 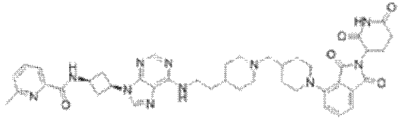 |
| 307 | 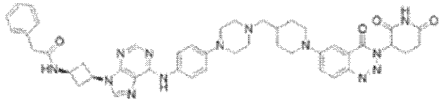 |
| 308 | 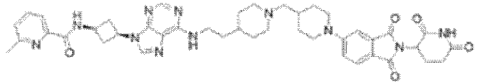 |
| 309 | 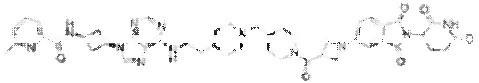 |

**[Table 11]**

| | |
|---|---|
| 310 | 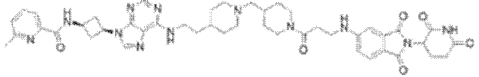 |
| 311 | 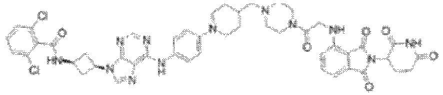 |
| 312 | 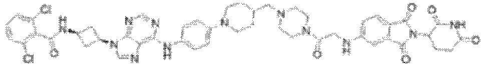 |
| 313 | 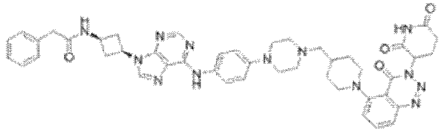 |
| 314 | 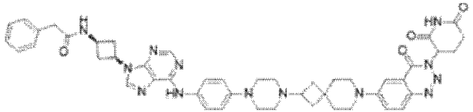 |
| 315 | 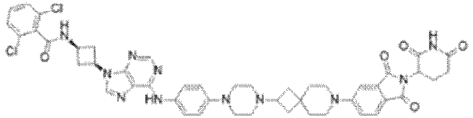 |
| 316 | 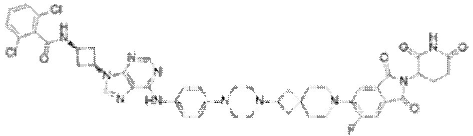 |
| 317 | 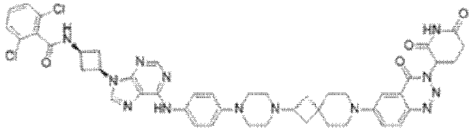 |
| 318 | 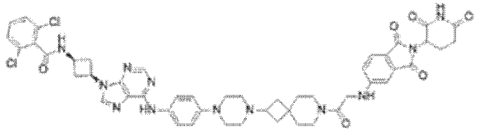 |
| 319 | 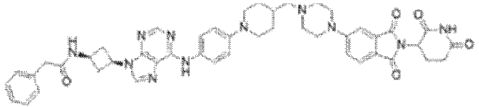 |
| 320 | 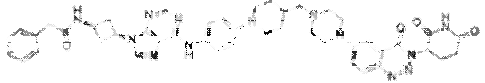 |
| 321 | 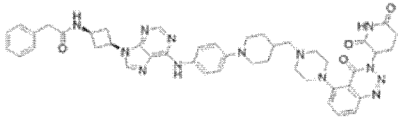 |
| 322 | 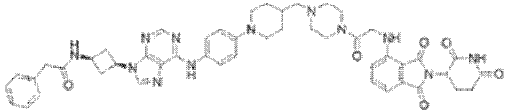 |
| 323 | 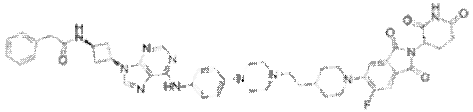 |
| 324 | 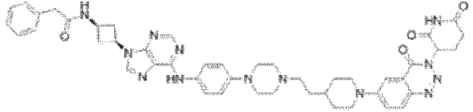 |
| 325 | 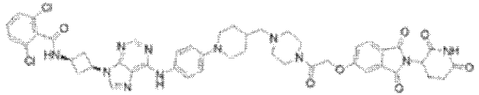 |
| 326 | 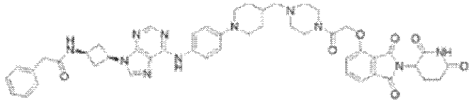 |
| 327 | 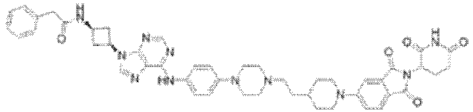 |
| 328 | 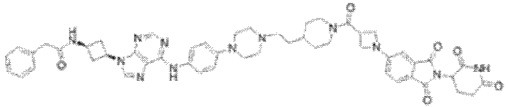 |
| 329 | 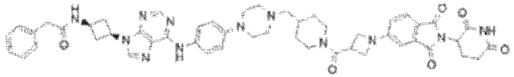 |
| 330 | 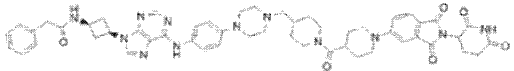 |
| 331 | 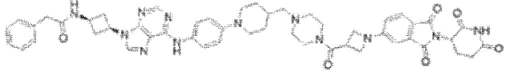 |
| 332 | 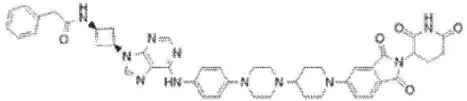 |
| 333 | 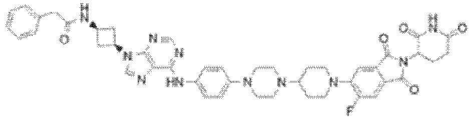 |
| 334 | 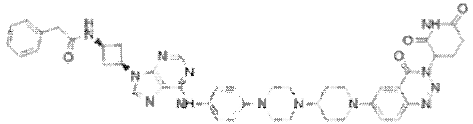 |
| 335 | 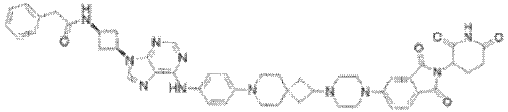 |
| 336 | 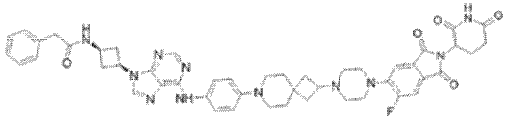 |
| 337 | 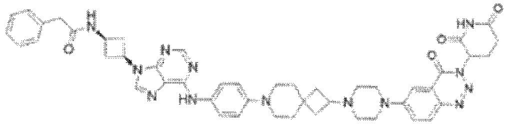 |
| 338 | 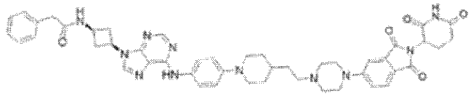 |
| 339 | 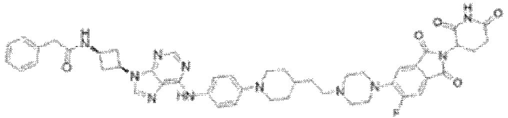 |
| 340 | 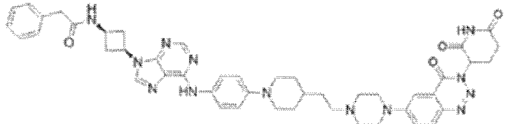 |
| 341 | 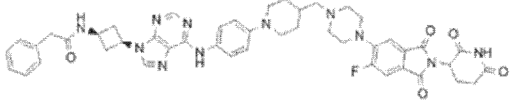 |
| 342 | 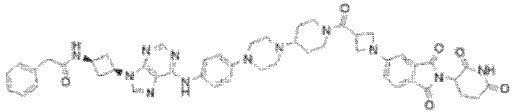 |
| 343 | 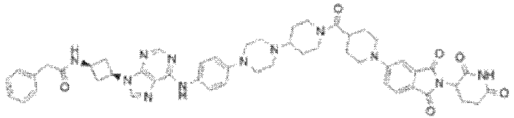 |
| 344 | 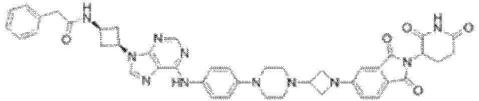 |
| 345 | 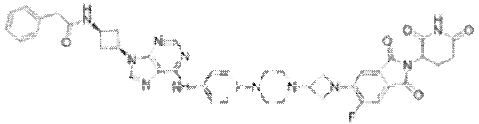 |
| 346 | 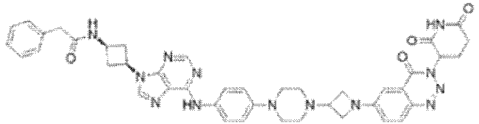 |
| 347 | 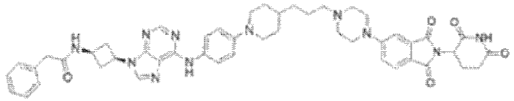 |
| 348 | 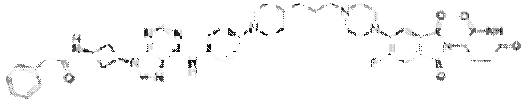 |
| 349 | 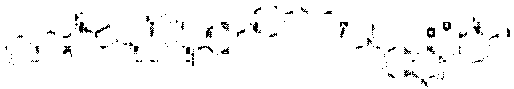 |
| 350 | 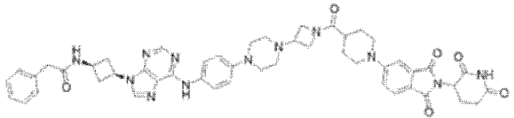 |

**[Table 12]**

| | |
|---|---|
| 351 | 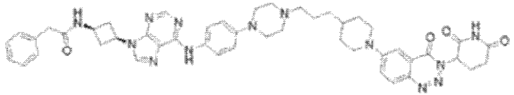 |
| 352 | 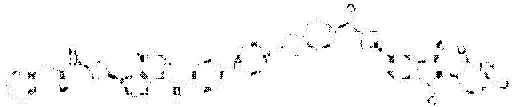 |
| 353 | 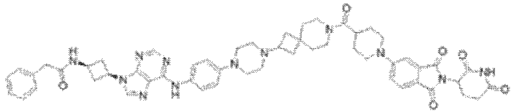 |
| 354 | 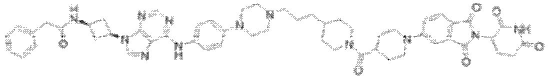 |
| 355 | 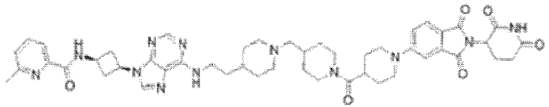 |
| 356 | 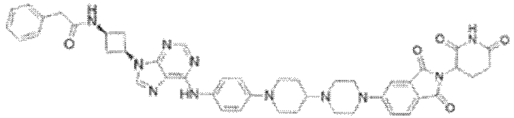 |
| 357 | 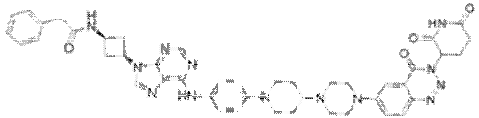 |
| 358 | 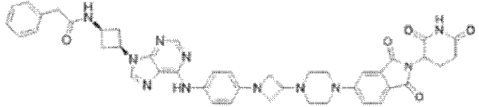 |
| 359 | 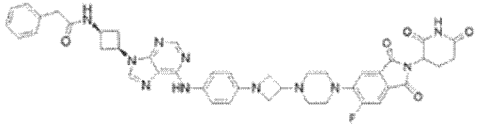 |
| 360 | 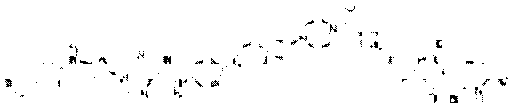 |
| 361 | 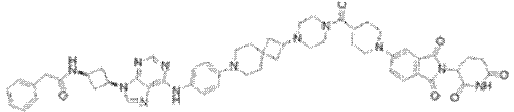 |
| 362 | 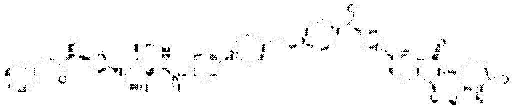 |
| 363 | 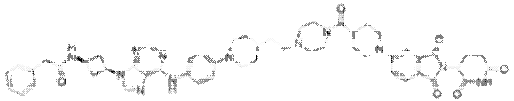 |
| 364 | 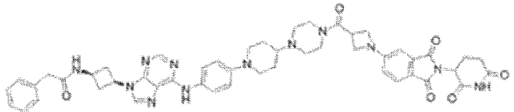 |
| 365 | 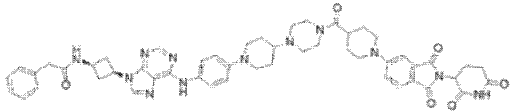 |
| 366 | 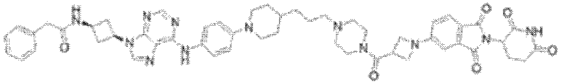 |
| 367 | 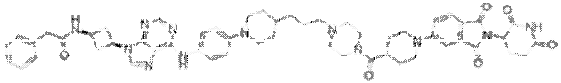 |
| 368 | 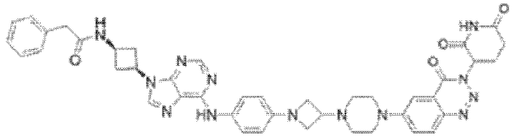 |
| 369 | 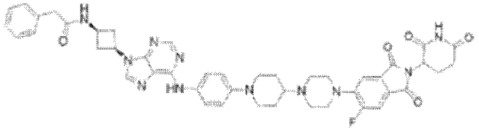 |
| 370 | 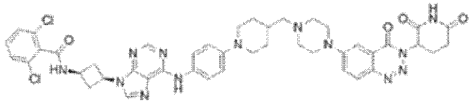 |
| 371 | 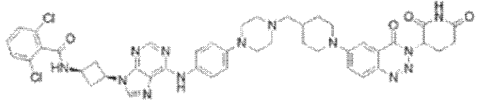 |
| 372 | 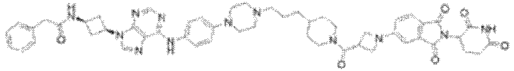 |
| 373 | 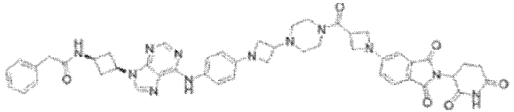 |
| 374 | 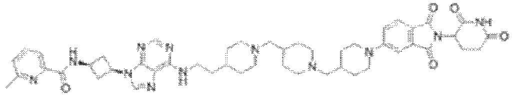 |
| 375 | 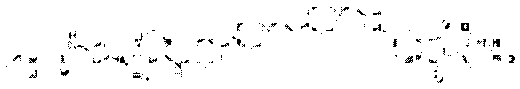 |
| 376 | 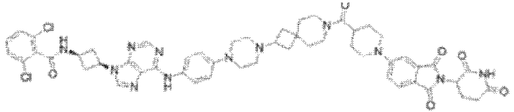 |
| 377 | 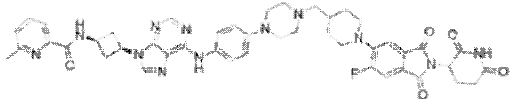 |
| 378 | 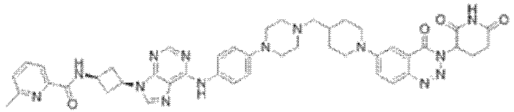 |
| 379 | 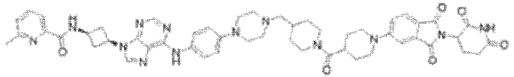 |
| 380 | 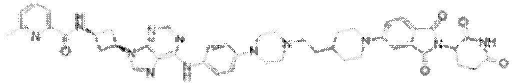 |
| 381 | 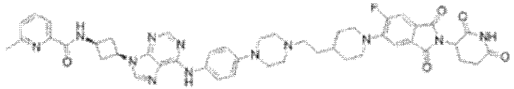 |
| 382 | 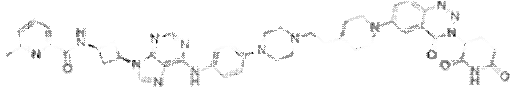 |
| 383 | 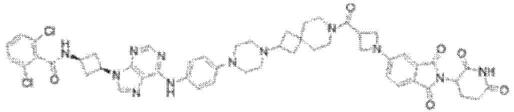 |
| 384 | 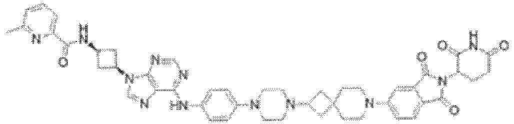 |
| 385 | 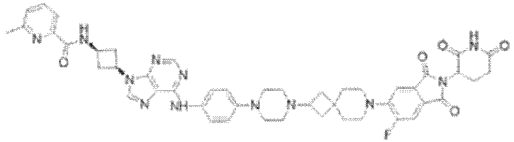 |
| 386 | 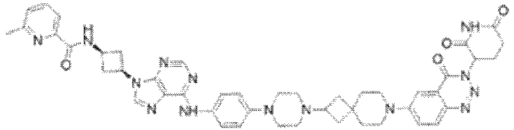 |
| 387 | 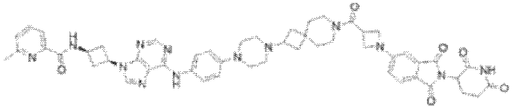 |
| 388 | 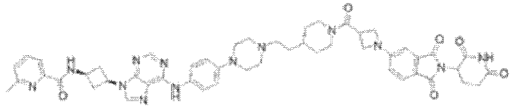 |
| 389 | 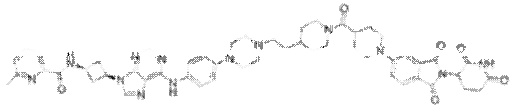 |
| 390 | 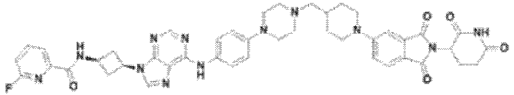 |
| 391 | 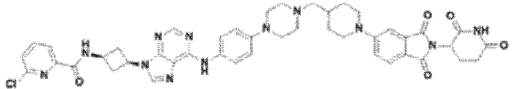 |

**[Table 13]**

| | |
|---|---|
| 392 | 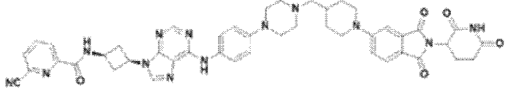 |
| 393 | 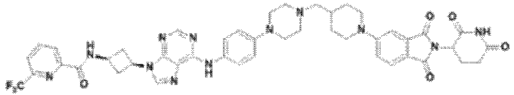 |
| 394 | 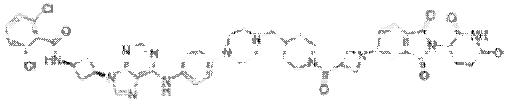 |
| 395 | 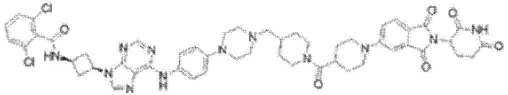 |
| 396 | 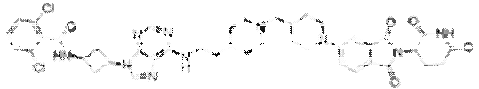 |
| 397 | 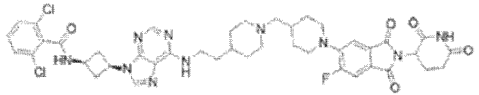 |
| 398 | 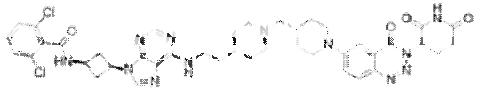 |
| 399 | 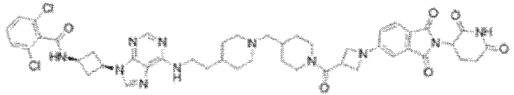 |
| 400 | 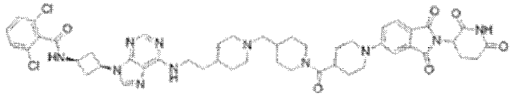 |
| 401 | 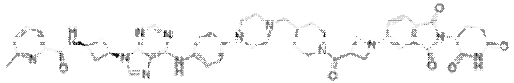 |
| 402 | 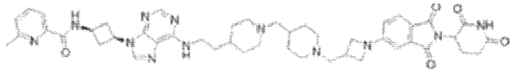 |
| 403 | 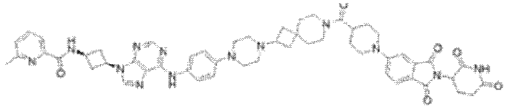 |
| 404 | 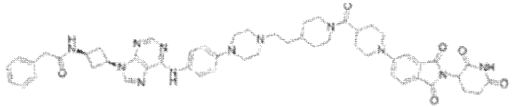 |
| 405 | 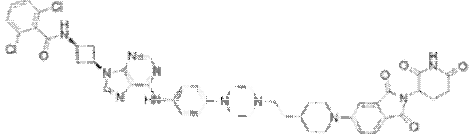 |
| 406 | 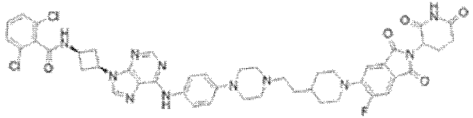 |
| 407 | 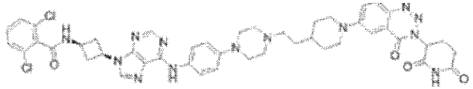 |
| 408 | 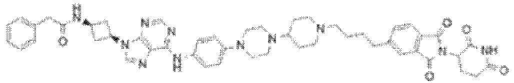 |
| 409 | 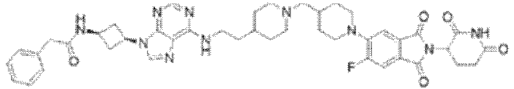 |
| 410 | 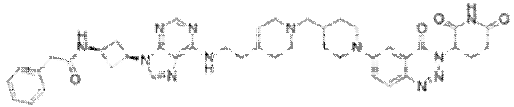 |
| 411 | 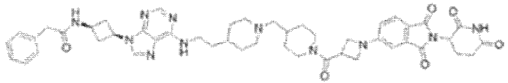 |
| 412 | 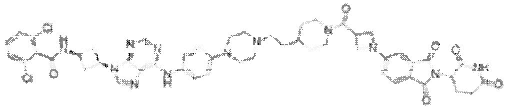 |
| 413 | 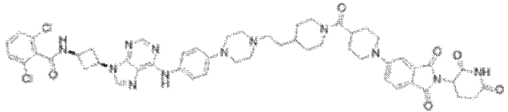 |
| 414 | 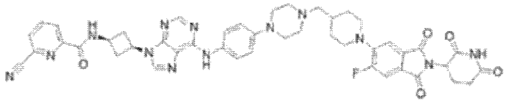 |
| 415 | 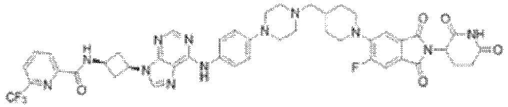 |
| 416 | 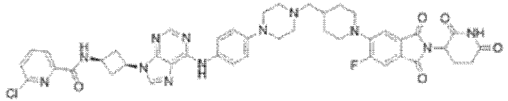 |
| 417 | 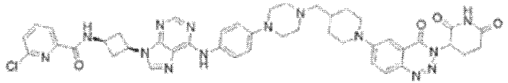 |
| 418 | 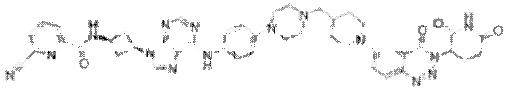 |
| 419 | 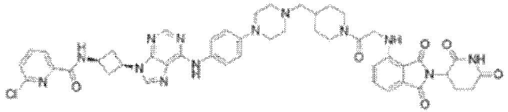 |
| 420 | 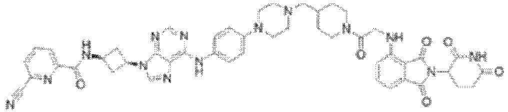 |
| 421 | 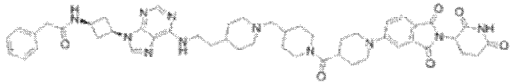 |
| 422 | 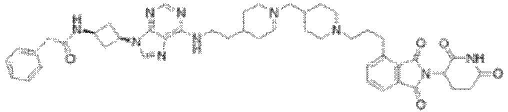 |
| 423 | 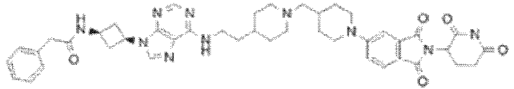 |
| 424 | 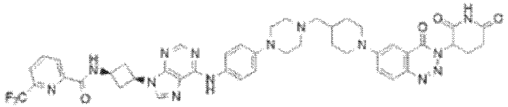 |
| 425 | 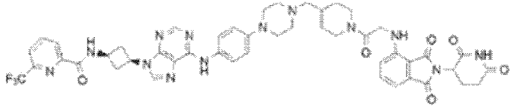 |
| 426 | 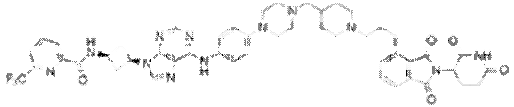 |
| 427 | 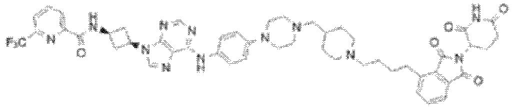 |
| 428 | 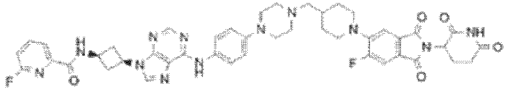 |
| 429 | 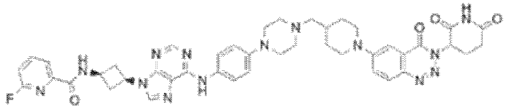 |
| 430 | 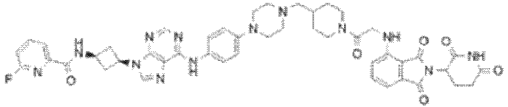 |
| 431 | 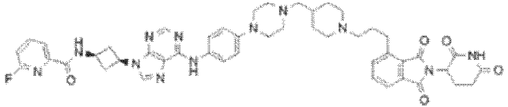 |
| 432 | 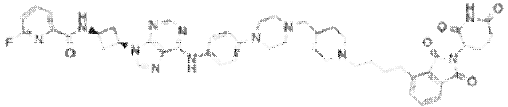 |
| 433 | 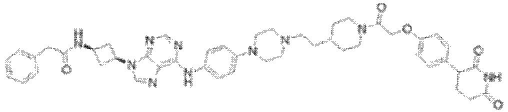 |
| 434 | 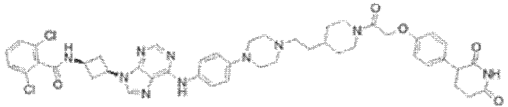 |

**[Table 14]**

| | |
|---|---|
| 435 | 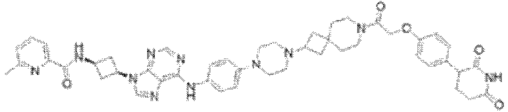 |
| 436 | 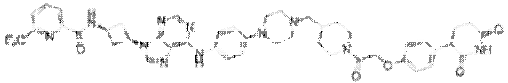 |
| 437 | 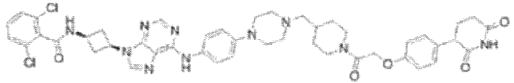 |
| 438 | 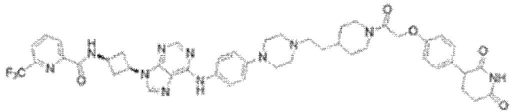 |
| 439 | 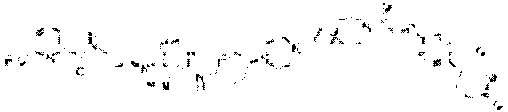 |
| 440 | 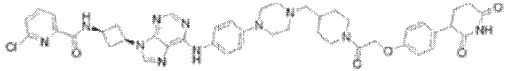 |
| 441 | 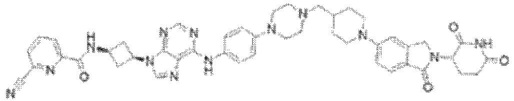 |
| 442 | 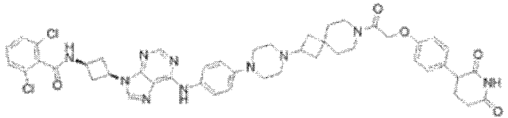 |
| 443 | 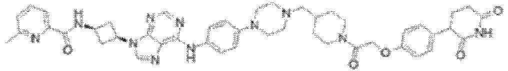 |
| 444 | 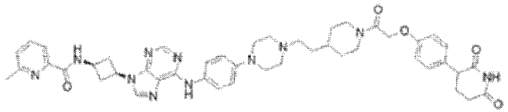 |
| 445 | 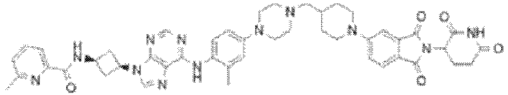 |
| 446 | 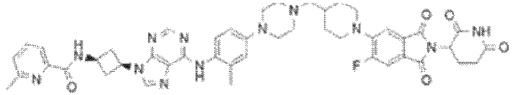 |
| 447 | 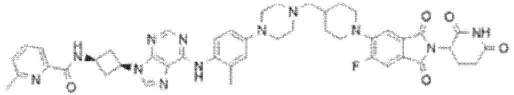 |
| 448 | 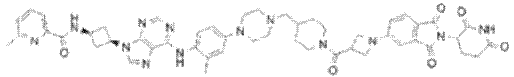 |
| 449 | 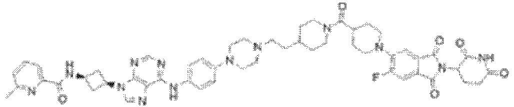 |
| 450 | 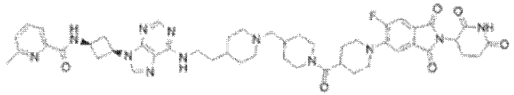 |
| 451 | 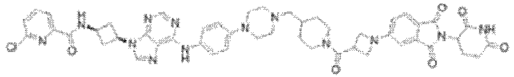 |
| 452 | 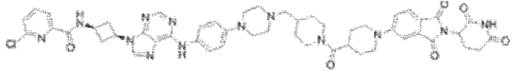 |
| 453 | 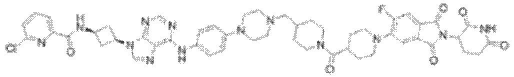 |
| 454 | 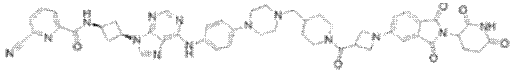 |
| 455 | 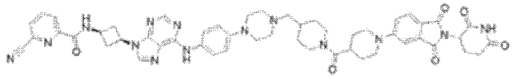 |
| 456 | 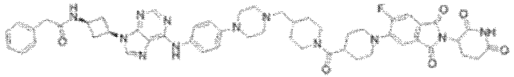 |
| 457 | 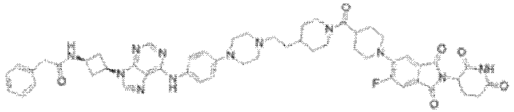 |
| 458 | 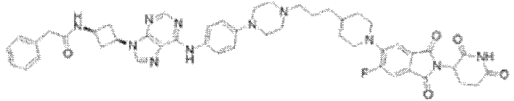 |
| 459 | 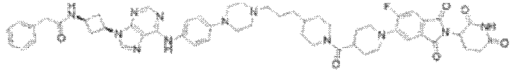 |
| 460 | 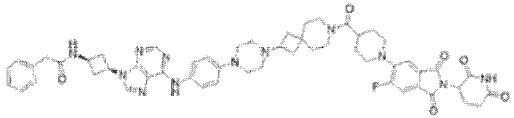 |
| 461 | 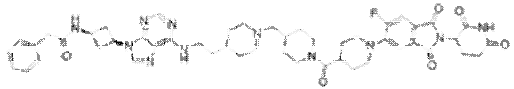 |
| 462 | 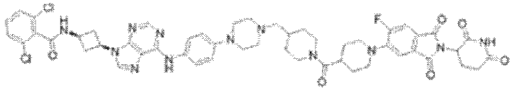 |
| 463 | 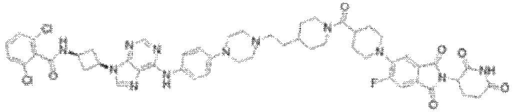 |
| 464 | 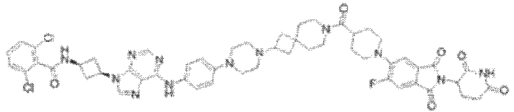 |
| 465 | 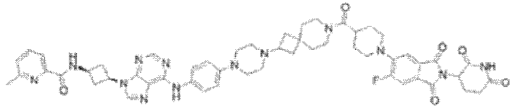 |
| 466 | 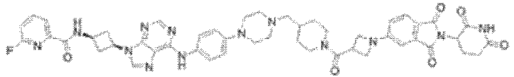 |
| 467 | 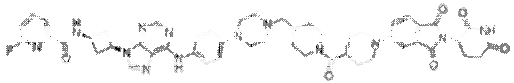 |
| 468 | 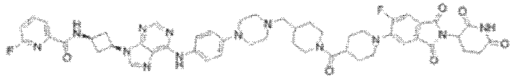 |
| 469 | 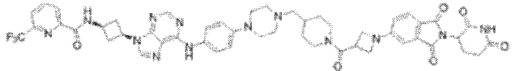 |
| 470 | 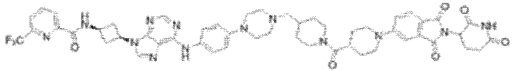 |
| 471 | 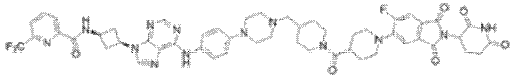 |
| 472 | 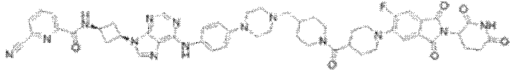 |
| 473 | 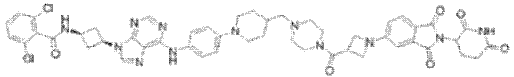 |
| 474 | 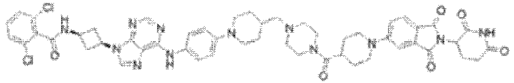 |
| 475 | 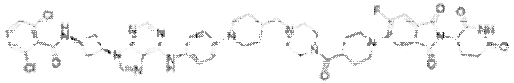 |
| 476 | 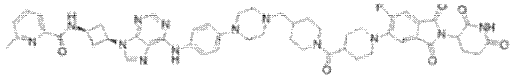 |
| 477 | 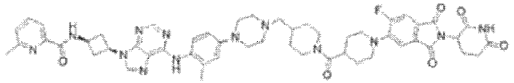 |
| 478 | 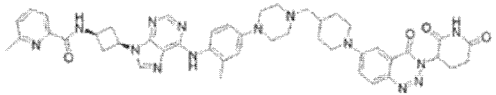 |
| 479 | 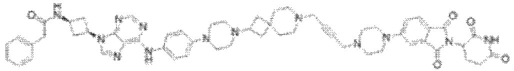 |

Among the compounds according to the present disclosure above, Compounds 25, 31, 33, 34, 35, 38, 41, 45, 74, 75, 88, 89, 90, 91, 92, 93, 96, 108, 109, 114, 115, 116, 117, 122, 123, 124, 125, 134, 135, 137, 145, 148, 149, 150, 151, 152, 153, 162, 163, 164, 165, 166, 167, 169, 170, 171, 174, 176, 178, 179, 182, 185, 189, 191, 193, 202, 204, 205, 217, 226, 236, 258, 259, 301, 307, 330, 353, 354, 378, 379, 393, 428, 429, *etc.* may be more preferable for the purpose of the present invention, but the compounds are not limited thereto.

The compound of the present invention may exist in a form of a pharmaceutically acceptable salt. Useful salts include acid salts formed by pharmaceutically acceptable free acids. As used herein, the term "pharmaceutically acceptable salt" refers to a form of salt that is relatively non-toxic and harmless at an effective concentration to a patient and refers to any organic or inorganic addition salt in which the side effects of the salt do not reduce the beneficial effects of the compound represented by Formula 1.

Acid addition salts are prepared by conventional methods, for example, by dissolving the compound in an excess of aqueous acid solution and precipitating the salt using a water-miscible organic solvent, such as methanol, ethanol, acetone, or acetonitrile. The compound, and an acid in water or alcohol (for example, glycol monomethyl ether) in equal molar amounts are heated, and subsequently, the mixture may be evaporated to dryness, or the precipitated salt may be suction filtered.

In particular, as the free acids, organic acids and inorganic acids may be used; inorganic acids such as hydrochloric acid, phosphoric acid, sulfuric acid, nitric acid, and tartaric acid may be used, and organic acids such as methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, and hydroiodic acid may be used, but the free acids are not limited thereto.

In addition, a pharmaceutically acceptable metal salt may be prepared using a base. An alkali metal salt or alkaline earth metal salt is obtained, for example, by dissolving the compound in an excess of an alkali metal hydroxide or alkaline earth metal hydroxide solution, filtering out the undissolved compound salt, and evaporating and drying the filtrate. In particular, as the metal salts, it is pharmaceutically suitable to manufacture sodium, potassium, or calcium salts, but the metal salts are not limited thereto. Also, the corresponding silver salt may be obtained by reacting an alkali metal or alkaline earth metal salt with a suitable silver salt (for example, silver nitrate).

The pharmaceutically acceptable salt of the compound of the present invention includes salts of acidic or basic groups that may be present in the compounds of Formula 1 above, unless indicated otherwise. For example, pharmaceutically acceptable salts may include sodium, calcium, and potassium salts of the hydroxy group, and other pharmaceutically acceptable salts of the amino group may include hydrobromide, sulfate, hydrogen sulfate, phosphate, hydrogen phosphate, dihydrogen phosphate, acetate, succinate, citrate, tartrate, lactate, mandelate, methanesulfonate (mesylate), and p-toluenesulfonate (tosylate) salts; these salts may be prepared by salt preparation methods known in the art.

Any salt of the compound of Formula 1 may be used as the salt of the compound of the present invention without limitation, as long as the salt is pharmaceutically acceptable and exhibits pharmacological activity equivalent to that of the compound of Formula 1.

In addition, the compound represented by Formula 1 above according to the present invention includes not only pharmaceutically acceptable salts thereof, but also solvates such as hydrates that can be prepared therefrom, and all possible stereoisomers without limitation. The solvates and stereoisomers of the compound represented by Formula 1 above may be prepared from the compound represented by Formula 1 using a method known in the art.

Furthermore, the compound represented by Formula 1 above according to the present invention may be manufactured in crystalline or amorphous form, and if manufactured in crystalline form, the compound may be optionally hydrated or solvated. In the present invention, compounds containing various amounts of water may be included in addition to the stoichiometric hydrate of the compound represented by Formula 1. The solvates of the compound represented by Formula 1 above according to the present invention include both the stoichiometric solvates and non-stoichiometric solvates.

The second aspect of the present invention provides a method for preparing a compound of the first embodiment or a pharmaceutically acceptable salt thereof, comprising the following steps: a first step of reacting a compound of the following Formula 4 with a derivative of moiety A, comprising an amine group at one end and a protecting group at the other end, and deprotecting the compound to prepare a compound of Formula 5; a second step of reacting the compound of Formula 5 with a derivative of an L1 moiety, comprising a reactive functional group X₂ at one end and a protecting group at the other end, and deprotecting the compound to prepare a compound of Formula 6; and a third step of reacting the compound of Formula 5 with a CRBN binder, which includes a reactive functional group X₃ at one end. 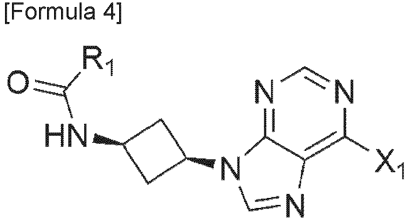 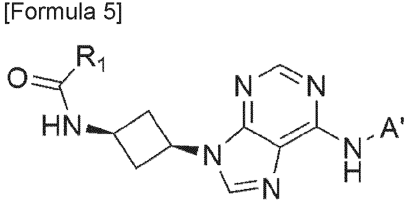 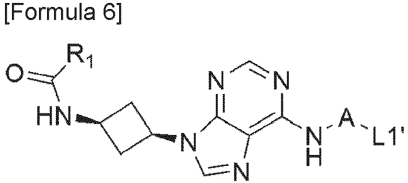 wherein in Formulas 4 to 6 above:
X₁, X₂, and X₃ are independently halogen or hydroxy;
the protecting group is tert-butoxycarbonyl;
and A' and L1' may be identical to A and L1, respectively, or may be in the form of a salt thereof.
R₁, A, and L1 not defined above are as defined in the first aspect.

Specifically, the CRBN binder may be any one selected from the group consisting of 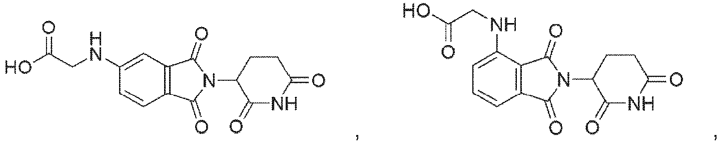 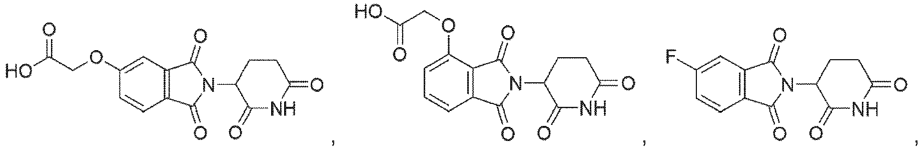 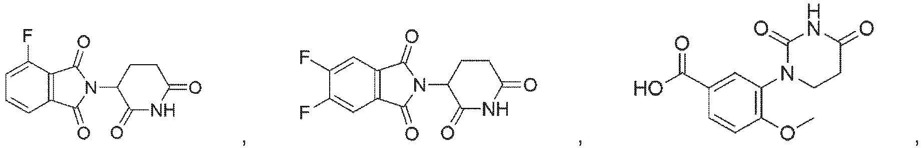 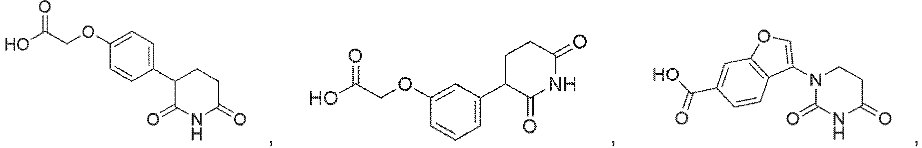 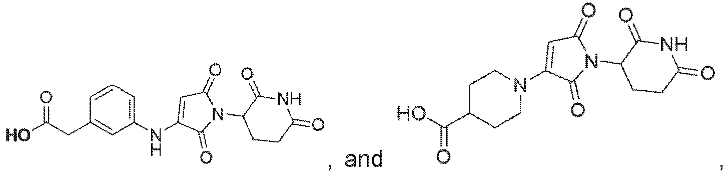 but is not limited thereto.

For example, the first step may be performed by: heating to react a compound, in which X₁ is a halogen, at a temperature of 70°C to 100°C in a lower alcohol solvent; and deprotecting by treating with a strong acid solution in an organic solvent, but is not limited thereto.

For example, the second step may be performed by:
i) reacting a compound, in which X₂ is hydroxy, with 1-ethyl-3-(3-dimethylaminopropyl)carbodiimide (EDCI), hydroxybenzotriazole (HOBt), and N,N-diisopropylethylamine (DIPEA) in an organic solvent at a temperature of 15°C to 40°C;
ii) reacting a compound, in which X₂ is halogen, in an organic solvent under basic conditions at a temperature of 40°C to 80°C;
iii) reacting a compound, in which X₂ is oxo, with DIPEA and sodium triacetoxyborohydride (NaBH(OAc)₃) at a temperature of 15°C to 40°C; or
iv) reacting a compound, in which X₂ is oxo, with sodium cyanoborohydride (NaBH₃CN) in a lower alcohol solvent in the presence of acetic acid at a temperature of 15°C to 40°C, and
deprotecting the compound using treatment with a strong acid solution in an organic solvent, but is not limited thereto.

Further, depending on the linker type to be introduced, the second step may be performed with two or more identical or different reactions selected from the above-described four reactions in a consecutive manner, but is not limited thereto.

For example, the third step may be performed by:
i) reacting a compound, in which X3 is hydroxy, with EDCL, HOBt, and DIPEA in an organic solvent at a temperature of 15°C to 40°C; or
ii) reacting a compound, in which X3 is halogen, with DIPEA in an organic solvent at a temperature of 70°C to 100°C, but is not limited thereto.

As described above, the reactions in each step may be performed in lower alcohols or organic solvents. For example, the lower alcohol solvent refers to an C₁₋₄ alkyl alcohol and may be methanol or ethanol, but is not limited thereto. In addition, the organic solvent may be dimethylformamide (DMF), dichloromethane (DCM), dimethyl sulfoxide (DMSO), *etc.,* which may be used alone or in combination, but is not limited thereto.

As the strong acid solution, a solution containing hydrochloric acid may be used, and the basic conditions may be achieved by using potassium carbonate (K₂CO₃), but is not limited thereto.

The specific reactions to perform the first to third steps are merely illustrative and are not intended to limit the scope of the present invention. Any similar reactions known in the art may be used without limitation. In addition, the preparation method of the present invention may further include processes such as separation, purification, and/or washing after each step, but is not limited thereto.

The third aspect of the present invention provides a composition comprising the compound of the first embodiment or a pharmaceutically acceptable salt thereof, and a pharmaceutically acceptable carrier.

The fourth aspect of the present invention provides a pharmaceutical composition for inhibiting or degrading cyclin-dependent kinase 2 (CDK2), cyclin-dependent kinase 9 (CDK9), or both, comprising the compound of the first embodiment or a pharmaceutically acceptable salt thereof as an active ingredient.

The fifth aspect of the present invention provides a pharmaceutical composition for preventing or treating cancer or HIV infection, comprising the compound of the first aspect or a pharmaceutically acceptable salt thereof as an active ingredient.

As used herein, the terms "compound of the first embodiment", and "pharmaceutically acceptable salt" are as described above.

In a specific embodiment of the present invention, the effect of degrading CDK2, CDK9, or both, or inhibiting the activity thereof has been confirmed. This finding suggests that a composition comprising the compound of the present invention can efficiently inhibit or degrade CDK2, CDK9, or both, and further suggests that it may be effectively used in the prevention or treatment of diseases caused by these enzymes.

As used herein, the term "prevention" refers to any activity of inhibiting or delaying the occurrence, spread, and recurrence of cancer or viral infection through administering the composition of the present invention, and the term "treatment" refers to any activity improving or beneficially changing the symptoms of diseases by administering the composition of the present invention.

Diseases that may be prevented or treated by administering the pharmaceutical composition of the present invention include cancer or human immunodeficiency virus (HIV) infection. The cancer may be, for example, one or more selected from the group consisting of pseudomyxoma, Intrahepatic cholangiocarcinoma, hepatoblastoma, liver cancer, thyroid cancer, colorectal cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell cancer, male breast cancer, brain cancer, pituitary adenoma, multiple myeloma, gallbladder cancer, biliary tract cancer, colon cancer, chronic myeloid leukemia, chronic lymphocytic leukemia, retinoblastoma, choroidal melanoma, diffuse large B-cell lymphoma, ampulla of Vater, bladder cancer, peritoneal cancer, parathyroid cancer, adrenal cancer, paranasal sinus cancer, non-small cell lung cancer, non-Hodgkin's lymphoma, tongue cancer, astrocytoma, small cell lung cancer, pediatric brain cancer, pediatric lymphoma, pediatric leukemia, small intestine cancer, meningioma, esophageal cancer, glioma, neuroblastoma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, stomach cancer, gastric carcinoid, gastrointestinal stromal cancer, Wilms' cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cancer, acoustic neuroma, pancreatic cancer, salivary gland cancer, Kaposi's sarcoma, Paget's disease, tonsil cancer, squamous cell carcinoma, pulmonary adenocarcinoma, lung cancer, lung squamous cell carcinoma, skin cancer, anal cancer, rhabdomyosarcoma, laryngeal cancer, pleural cancer, and thymic cancer. Specifically, the cancer may be prostate cancer, lymphoma (such as B-cell lymphoma, Burkitt lymphoma, or follicular lymphoma), glioblastoma, neuroblastoma, ovarian cancer, hepatocellular carcinoma, liver cancer, breast cancer, leukemia (such as chronic lymphocytic leukemia (CLL) or acute myeloid leukemia), pancreatic cancer, colon cancer, lung cancer, colorectal cancer, or multiple myeloma, but is not limited thereto. Meanwhile, HIV infection may be acquired immune deficiency syndrome (AIDS), but is not limited thereto. Furthermore, HIV may refer to Human Immunodeficiency Virus-1 (HIV-1), the most common and pathogenic variant, but is not limited thereto.

Preferably, the pharmaceutical composition according to the present invention may contain, as an active ingredient, a compound represented by Formula 1 or a pharmaceutically acceptable salt thereof in an amount of 0.1 wt% to 75 wt% based on the total weight of the composition, more preferably 1 wt% to 50 wt%.

The composition of the present invention may further comprise pharmaceutically acceptable carriers, diluents, or excipients, and may be formulated into various forms such as powers, granules, tablets, capsules, suspensions, emulsions, syrups, aerosols, *etc.,* according to conventional methods for each intended use. It may be administered orally or through various routes including intravenous, intraperitoneal, subcutaneous, rectal, and local administration. Examples of suitable carriers, excipients, or diluents that may be included in such a composition may include lactose, dextrose, sucrose, sorbitol, mannitol, xylitol, erythritol, maltitol, starch, acacia gum, alginate, gelatin, calcium phosphate, calcium silicate, cellulose, methylcellulose, amorphous cellulose, polyvinylpyrrolidone, water, methyl hydroxybenzoate, propyl hydroxybenzoate, talc, magnesium stearate, and mineral oil. In addition, the composition of the present invention may further include fillers, anti-coagulants, lubricants, moisturizing agents, fragrances, emulsifiers, preservatives, *etc.*

Solid preparations for oral administration include tablets, pills, powders, granules, capsules, *etc.,* and these solid preparations are formulated by mixing at least one or more excipients, such as starch, calcium carbonate, sucrose, lactose, and gelatin, in the composition. Further, in addition to simple excipients, lubricants such as magnesium stearate and talc may be used.

Examples of oral liquid preparations may include suspensions, solutions, emulsions, syrups, *etc.,* and in addition to commonly used simple diluents such as water and liquid paraffin, various excipients such as wetting agents, sweeteners, flavoring agents, and preservatives may be included.

Preparations for non-oral administration include sterilized aqueous solvents, non-aqueous solvents, suspensions, emulsions, freeze-dried preparations, and suppositories. As non-aqueous solvents and suspending agents, propylene glycol, polyethylene glycol, vegetable oils such as olive oil, injectable esters such as ethyl oleate, *etc.* may be used. As bases for suppositories, Witepsol, Macrogol, Tween 61, cocoa butter, lauric acid, glycerogelatin, *etc.* may be used. Meanwhile, the injections may comprise conventional additives such as solubilizing agents, isotonic agents, suspending agents, emulsifiers, stabilizing agents, and preservatives.

The sixth aspect of the present invention provides a method for preventing or treating cancer or HIV infection, comprising administering a pharmaceutical composition of the fifth aspect to a subject in need thereof.

As used herein, the term "compound of the first embodiment", and "pharmaceutically acceptable salt" are as described above.

As used herein, the term "subject" refers to any animal, including humans, monkeys, cows, horses, sheep, pigs, chickens, turkeys, cats, dogs, mice, rats, rabbits, or guinea pigs, that can develop or be infected with the viral infection. The pharmaceutical composition of the present invention can effectively prevent or treat the disease by administering it to the subject. The pharmaceutical composition of the present invention may be administered concurrently with existing treatments.

The term "administering" as used herein refers to provision of a specified substance to a patient by any appropriate means, and the route of administration of the composition of the present invention may be through any general route that may reach the target tissue. It may be administered intraperitoneally, intravenously, intramuscularly, subcutaneously, intradermally, orally, locally, intranasally, intrapulmonary, and intrarectally, but the route of administration is not limited thereto. In addition, the pharmaceutical composition of the present invention may also be administered by any device capable of transporting the active substance to a target cell. Desirable methods of administration and formulations include intravenous injection, subcutaneous injection, intradermal injection, intramuscular injection, drip injection, *etc.* The injections may be manufactured using aqueous solvents such as saline solution and Ringer's solution, non-aqueous solvents such as vegetable oils, high-grade fatty acid esters (for example, ethyl oleate), alcohols (for example, ethanol, benzyl alcohol, propylene glycol, or glycerin), *etc.* The injection may also contain pharmaceutical carriers such as stabilizing agents (for example, ascorbic acid, sodium hydrosulfite, sodium pyrosulfite, BHA, tocopherol, or EDTA), emulsifiers, buffering agents for pH adjustment, and preservatives to inhibit microbial growth (for example, phenylmercuric nitrate, thimerosal, benzalkonium chloride, phenol, cresol, benzyl alcohol, *etc*.)*.*

In the present invention, the term "therapeutically effective amount", which is used in conjunction with active ingredient, refers to the amount of a triazolomethylurea derivative compound or a pharmaceutically acceptable salt thereof that is effective in preventing or treating a target disease.

The pharmaceutical composition of the present invention may further comprise, as an active ingredient, a known drug that is used in preventing or treating each disease in addition to the compound of the present invention or a pharmaceutically acceptable salt thereof, depending on the type of disease to be prevented or treated. For example, when used in preventing or treating a viral infection, a known drug may be further included in addition to the compound of the present invention or a pharmaceutically acceptable salt thereof, and the pharmaceutical composition of the present invention may be used in combination with other known treatments for the infection.

In particular, the composition of the present invention is administered in a pharmaceutically effective amount. As used herein, the term "pharmaceutically effective amount" refers to an amount that is sufficient to treat a disease with a reasonable benefit-risk ratio applicable to medical therapy and without causing significant side effects. The effective dose level may be determined based on factors including the patient's health conditions, type and severity of disease, drug activity, sensitivity to the drug, administration method, administration time, route of administration and elimination rate, treatment duration, and combination or concurrent use of drugs, as well as other well-known factors in the medical field. The composition of the present invention may be administered as a stand-alone treatment or in combination with other treatments, and may be administered sequentially or simultaneously with conventional treatments. The composition of the present invention may be administered in single or multiple doses. It is important to administer the minimum amount that can achieve the maximum effect without side effects, taking all the above-mentioned factors into consideration. This may be readily determined by a person of ordinary skill in the art.

For example, the administered dose may vary depending on factors such as the route of administration, severity of disease, sex, weight, and age, and thus the administered dose does not limit the scope of the present invention by any means.

Specifically, the effective amount of the compound in the composition of the present invention may vary depending on the patient's age, gender, and weight, and may be administered in a range of 1 mg to 100 mg, preferably 5 mg to 60 mg, per kg of body weight, daily or every other day, or divided into 1 to 3 doses per day. However, the administered dose may vary depending on factors such as the route of administration, severity of disease, gender, weight, and age, and thus the administered dose does not limit the scope of the present invention by any means.

### [Mode for Carrying Out the Invention]

Hereinafter, the present invention will be described in detail with reference to Examples and the like to facilitate understanding of the present invention. However, Examples according to the present invention may be modified into other various forms, and the scope of the present invention should not be construed as being limited to the following embodiments. Examples of the present invention are provided to more fully describe the invention to a person of ordinary skill in the art to which the present invention pertains.

### Preparation of compounds of present invention

The synthetic procedures of certain compounds of the present invention are described below, and compounds not mentioned below may be prepared in a similar manner by substituting the starting materials, intermediates, and/or reactants.

Certain intermediates, especially CDK inhibitor moieties, may be prepared by the method disclosed in Korean Patent No. 1893879, and the entire disclosure thereof is incorporated herein by reference.

### <Substances>

The structural formulas and names of the CRBN binder compounds exemplarily used in Examples of the present invention are listed below. 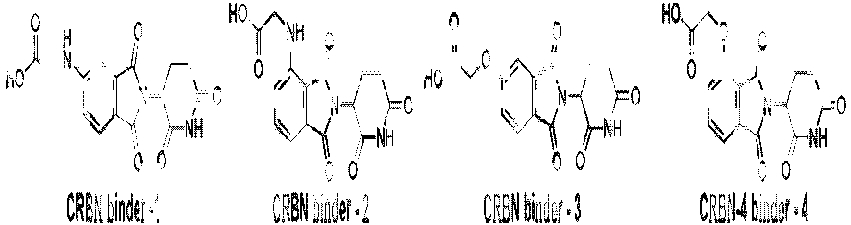 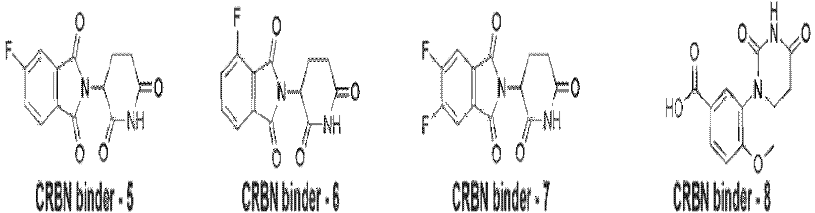 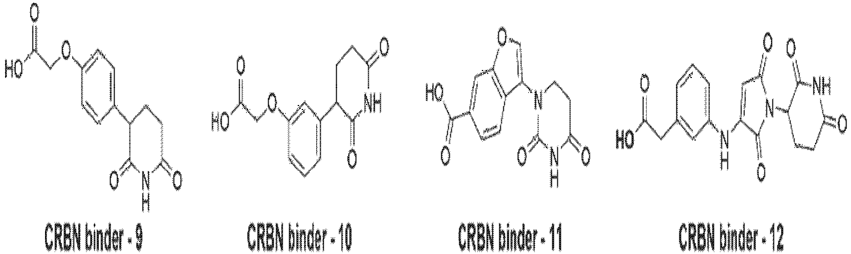 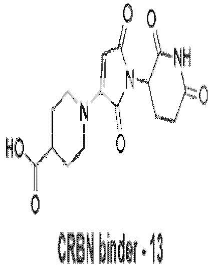
**CRBN Binder-1:** (2-(2,6-dioxopiperidin-3-yl)-1,3-dioxoisoindolin-5-yl)glycine
**CRBN Binder-2:** (2-(2,6-dioxopiperidin-3-yl)-1,3-dioxoisoindolin-4-yl)glycine
**CRBN Binder-3:** 2-((2-(2,6-dioxopiperidin-3-yl)-1,3-dioxoisoindolin-5-yl)oxy)acetic acid
**CRBN Binder-4:** 2-((2-(2,6-dioxopiperidin-3-yl)-1,3-dioxoisoindolin-4-yl)oxy)acetic acid
**CRBN Binder-5:** 2-(2,6-dioxopiperidin-3-yl)-5-fluoroisoindoline-1,3-dione
**CRBN Binder-6:** 2-(2,6-dioxopiperidin-3-yl)-4-fluoroisoindoline-1,3-dione
**CRBN Binder-7:** 2-(2,6-dioxopiperidin-3-yl)-5,6-difluoroisoindoline-1,3-dione
**CRBN Binder-8:** 3-(2,4-dioxotetrahydropyrimidin-1(2H)-yl)-4-methoxybenzoic acid
**CRBN Binder-9:** 2-(4-(2,6-dioxopiperidin-3-yl)phenoxy)acetic acid
**CRBN Binder-10:** 2-(3-(2,6-dioxopiperidin-3-yl)phenoxy)acetic acid
**CRBN Binder-11:** 3-(2,4-dioxotetrahydropyrimidin-1(2H)-yl)benzofuran-6-carboxylic acid
**CRBN Binder-12:** 2-(3-((1-(2,6-dioxopiperidin-3-yl)-2,5-dioxo-2,5-dihydro-1H-pyrrol-3-yl)amino)phenyl)acetic acid
**CRBN Binder-13:** 1-(1-(2,6-dioxopiperidin-3-yl)-2,5-dioxo-2,5-dihydro-1H-pyrrol-3-yl)piperidin-4-carboxylic acid

### Example 1: Synthesis of N-((1r,3r)-3-(6-((1-(6-(2-((2-(2,6-dioxopiperidin-3-yl)-1,3-dioxoisoindolin-4-yl)amino)acetamido)hexanoyl)piperidin-4-yl)amino)-9H-purin-9-yl)cyclobutyl)-6-methylpicolinamide (Compound 1)

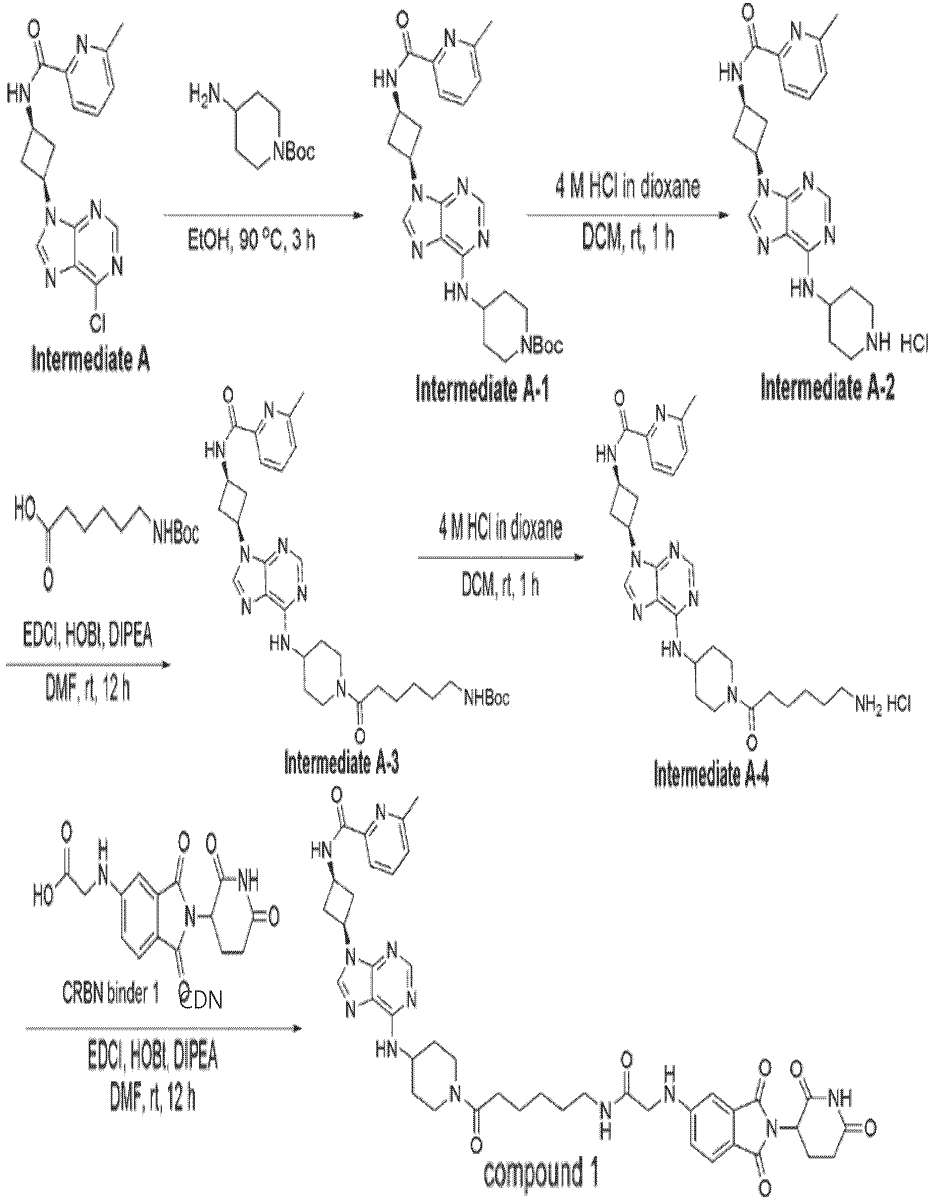

### Step 1: Synthesis of tert-butyl 4-((9-((1s,3s)-3-(6-methylpicolinamido)cyclobutyl)-9H-purin-6-yl)amino)piperidine-1-carboxylate (Intermediate A-1)

N-((1s,3s)-3-(6-chloro-9H-purin-9-yl)cyclobutyl)-6-methylpicolinamide (Intermediate A, 583 mg, 1.70 mmol) was dissolved in ethanol (7 mL). Then, *tert-butyl* 4-aminopiperidine-1-carboxylate (680 mg, 3.40 mmol) was added, followed by stirring at 90°C for 3 hours. Upon completion of the reaction, the reaction product was concentrated under reduced pressure and separated and purified by silica gel column chromatography to obtain the desired Intermediate A-1 (698 mg, 1.19 mmol, 81%) as a white solid.

¹H NMR (300 MHz, Chloroform-d) δ 8.84 (d, *J* = 8.5 Hz, 1H), 8.46 (s, 1H), 8.02 (d, *J* = 7.6 Hz, 1H), 7.89 (s, 1H), 7.74 (t, *J* = 7.7 Hz, 1H), 7.31 (d, *J* = 7.7 Hz, 1H), 5.68 (d, *J* = 8.1 Hz, 1H), 4.79 (t, *J* = 8.2 Hz, 1H), 4.60 (q, *J* = 8.1 Hz, 1H), 4.39 (s, 1H), 4.23-3.98 (m, 2H), 3.26-3.09 (m, 2H), 3.07-2.89 (m, 4H), 2.64 (s, 3H), 2.18-2.06 (m, 2H), 1.54 (s, 2H), 1.48 (s, 9H).

### Step 1-1: Synthesis of 6-methyl-N-((1s,3s)-3-(6-(piperidin-4-yl amino)-9H-purin-9-yl)cyclobutyl)picolinamide hydrochloride (Intermediate A-2)

Intermediate A-1 (100 mg, 0.43 mmol) was dissolved in DCM (2 mL). 4N hydrochloric acid/1,4-dioxene (1.08 mL) was then added, followed by stirring at room temperature for 1 hour. The reaction mixture was concentrated under reduced pressure to obtain the desired Intermediate A-2 (143 mg, 0.35 mmol, fullweight) as a white solid.

¹H NMR (300 MHz, DMSO-*d*₆) δ 9.70 (s, 1H), 9.14 (d, *J* = 9.0 Hz, 1H), 8.46 (s, 2H), 7.97-7.89 (m, 2H), 7.52 (dd, *J* = 5.8, 3.0 Hz, 1H), 4.93 (p, *J* = 8.4 Hz, 1H), 4.62-4.49 (m, 1H), 3.57 (s, 3H), 3.35 (s, 2H), 2.97-2.86 (m, 4H), 2.12-2.04 (m, 2H), 2.00-1.85 (m, 2H), 1.85-1.73 (m, 2H).

### Step 2: Synthesis of tert-butyl (6-(4-((9-((1s,3s)-3-(6-methylpicolinamido)cyclobutyl)-9H-purin-6-yl)amino)piperidin-1-yl)-6-oxohexyl)carbamate (Intermediate A-3)

Intermediate A-2 (156 mg, 0.25 mmol), 6-((*tert*-butoxycarbonyl)amino)hexanoic acid (82 mg, 0.25 mmol), EDCI. HCl (53 mg, 0.28 mmol), and HOBt. H2O (40 mg, 0.28 mmol) were dissolved in DMF (1 mL). DIPEA (0.2 mL, 1.25 mmol) was then added, followed by stirring at room temperature for 12 hours. The reaction mixture was diluted with water and then extracted with ethyl acetate. The organic layer was washed with brine, and the residue was dried over anhydrous magnesium sulfate and filtered. The filtrate was concentrated under reduced pressure, and then separated and purified by silica gel column chromatography to obtain the desired Intermediate A-3 (78 mg, 0.13 mmol, 51%) as a white solid.

¹H NMR (300 MHz, Chloroform-d) δ 8.85 (d, *J* = 8.5 Hz, 1H), 8.48 (s, 1H), 8.04 (d, *J* = 7.7 Hz, 1H), 7.92 (s, 1H), 7.77 (t, *J* = 7.7 Hz, 1H), 7.33 (d, *J* = 7.8 Hz, 1H), 5.87 (d, *J* = 8.0 Hz, 1H), 4.86-4.74 (m, 1H), 4.67-4.57 (m, 3H), 4.50 (s, 1H), 3.97-3.85 (m, 1H), 3.19-3.10 (m, 4H), 3.06-2.92 (m, 3H), 2.66 (s, 3H), 2.38 (t, *J* = 7.5 Hz, 2H), 2.23-2.12 (m, 2H), 1.68-1.65 (m, 2H), 1.56-1.48 (m, 4H), 1.46 (s, 9H), 1.44-1.38 (m, 2H), 1.32-1.21 (m, 2H).

### Step 2-2: Synthesis of N-((1s,3s)-3-(6-((1-(6-aminohexanoyl)piperidin-4-yl)amino)-9H-purin-9-yl)cyclobutyl)-6-methylpicolinamide hydrochloride (Intermediate A-4)

Intermediate A-3 (77 mg, 0.11 mmol) was dissolved in DCM (2 mL). 4N hydrochloric acid/1,4-dioxene (0.27 mL) was then added, followed by stirring at room temperature for 1 hour. The reaction mixture was concentrated under reduced pressure to obtain the desired Intermediate A-4 as a white solid.

¹H NMR (500 MHz, Methanol-*d*₄) δ 8.75 (s, 1H), 8.57-8.41 (m, 3H), 8.00 (d, *J* = 8.3 Hz, 1H), 5.14-5.01 (m, 1H), 4.68-4.57 (m, 1H), 3.63-3.57 (m, 1H), 3.28 (s, 2H), 3.22 (t, *J* = 8.5 Hz, 2H), 3.20-3.14 (m, 2H), 3.09-3.01 (m, 3H), 2.98 (t, *J* = 7.7 Hz, 2H), 2.88 (s, 3H), 2.47-2.36 (m, 2H), 2.24-2.16 (m, 2H), 1.88 (s, 2H), 1.77-1.69 (m, 2H), 1.62 (s, 2H).

### Step 3: Synthesis of N-((1r,3r)-3-(6-((1-(6-(2-((2-(2,6-dioxopiperidin-3-yl)-1,3-dioxoisoindolin-4-yl)amino)acetamido)hexanoyl)piperidin-4-yl)amino)-9H-purin-9-yl)cyclobutyl)-6-methylpicolinamide (Compound 1)

Intermediate A-4 (10 mg, 0.019 mmol), (2-(2,6-dioxopiperidin-3-yl)-1,3-dioxoisoindolin-5-yl)glycine (6 mg, 0.019 mmol), EDCI. HCl (4 mg, 0.021 mmol), and HOBt. H2O (3 mg, 0.021 mmol) were dissolved in DMF (1 mL). DIPEA (0.02 mL, 0.095 mmol) was then added, followed by stirring at room temperature for 12 hours. The reaction mixture was diluted with water and then extracted with ethyl acetate. The organic layer was washed with brine, and the residue was dried over anhydrous magnesium sulfate and filtered. The filtrate was concentrated under reduced pressure, and then separated and purified by silica gel column chromatography to obtain the desired Compound 1 (6.6 mg, 41%) as a yellow solid.

### Example 2: Synthesis of N-((1r,3r)-3-(6-(((1-(6-(2-((2-(2,6-dioxopiperidin-3-yl)-1,3-dioxoisoindolin-4-yl)amino)acetamido)hexanoyl)piperidin-4-yl)methyl)amino)-9H-purin-9-yl)cyclobutyl)-6-methylpicolinamide (Compound 2)

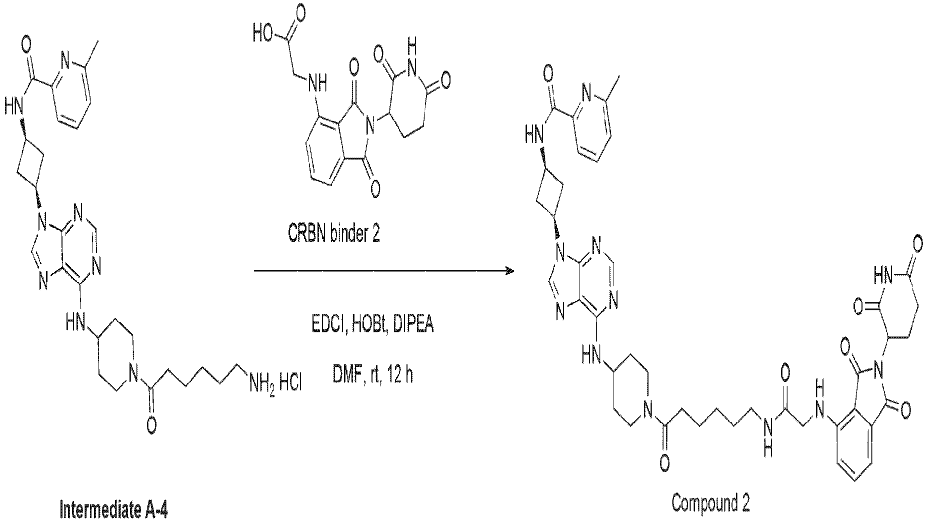

Compound 2 was synthesized in a similar manner to Example 1, except that (2-(2,6-dioxopiperidin-3-yl)-1,3-dioxoisoindolin-4-yl)glycine (CRBN Binder 2) was used instead of CRBN Binder 1.

### Example 3: Synthesis of N-((1r,3r)-3-(6-((1-(6-(2-((2-(2,6-dioxopiperidin-3-yl)-1,3-dioxoisoindolin-5-yl)oxy)acetamido)hexanoyl)piperidin-4-yl)amino)-9H-purin-9-yl)cyclobutyl)-6-methylpicolinamide (Compound 3)

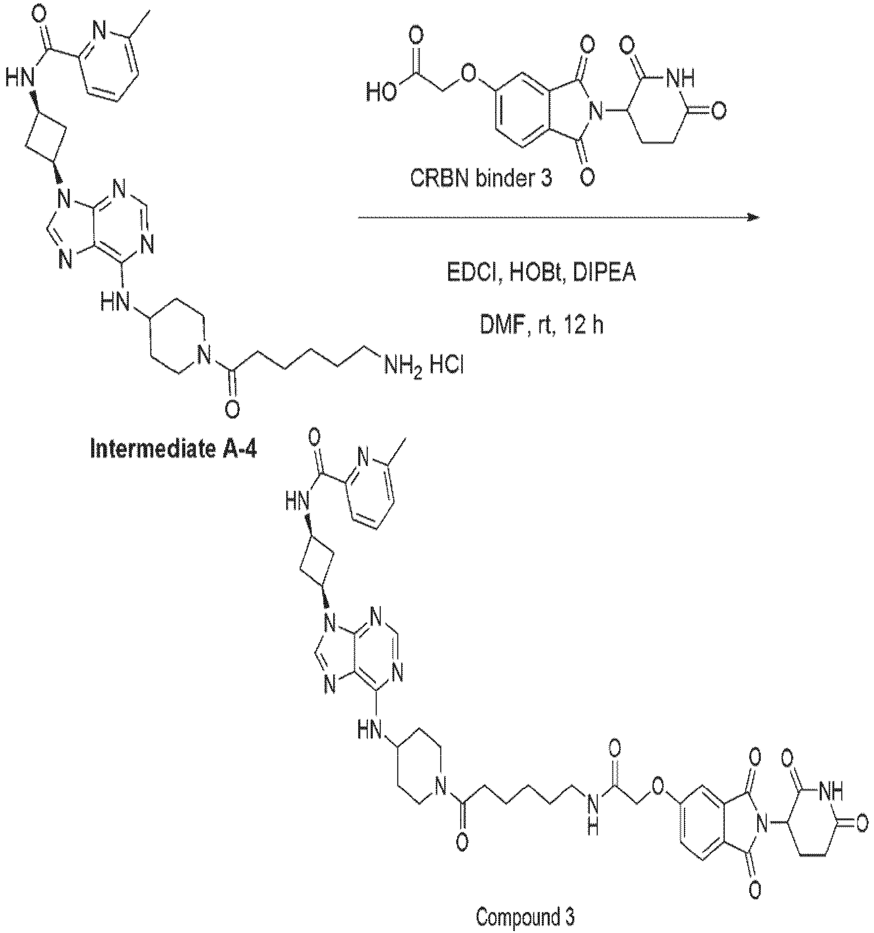

Compound 3 was synthesized in a similar manner to Example 1, except that 2-((2-(2,6-dioxopiperidin-3-yl)-1,3-dioxoisoindolin-5-yl)oxy)acetic acid (CRBN Binder 3) was used instead of CRBN Binder 1.

### Example 4: Synthesis of N-((1r,3r)-3-(6-((1-(6-(2-((2-(2,6-dioxopiperidin-3-yl)-1,3-dioxoisoindolin-4-yl)oxy)acetamido)hexanoyl)piperidin-4-yl)amino)-9H-purin-9-yl)cyclobutyl)-6-methylpicolinamide (Compound 4)

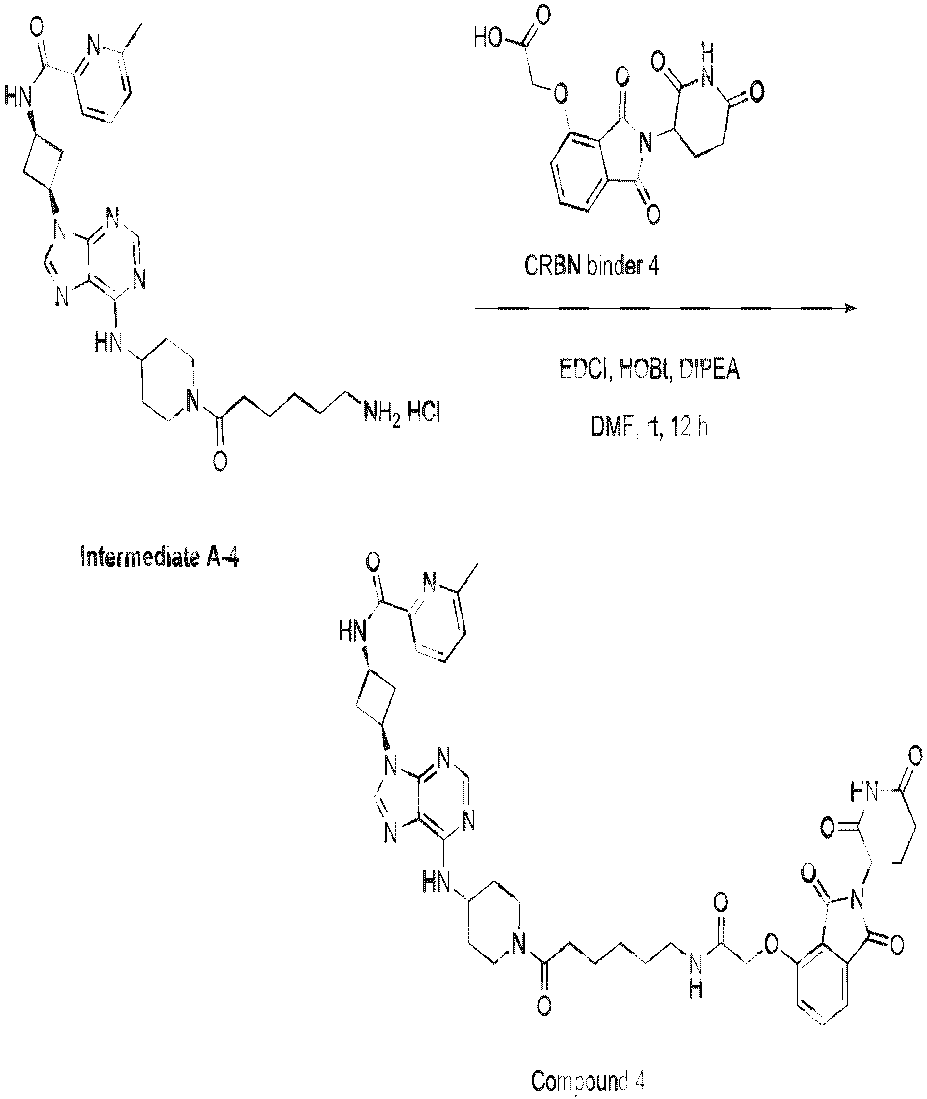

### Compound 4 was synthesized in a similar manner to Example 1, except that 2-((2-(2,6-dioxopiperidin-3-yl)-1,3-dioxoisoindolin-4-yl)oxy)acetic acid (CRBN Binder 4) was used instead of CRBN Binder 1.

### Example 5: Synthesis of N-((1r,3r)-3-(6-((1-(6-((2-(2,6-dioxopiperidin-3-yl)-1,3-dioxoisoindolin-5-yl)amino)hexanoyl)piperidin-4-yl)amino)-9H-purin-9-yl)cyclobutyl)-6-methylpicolinamide (Compound 5)

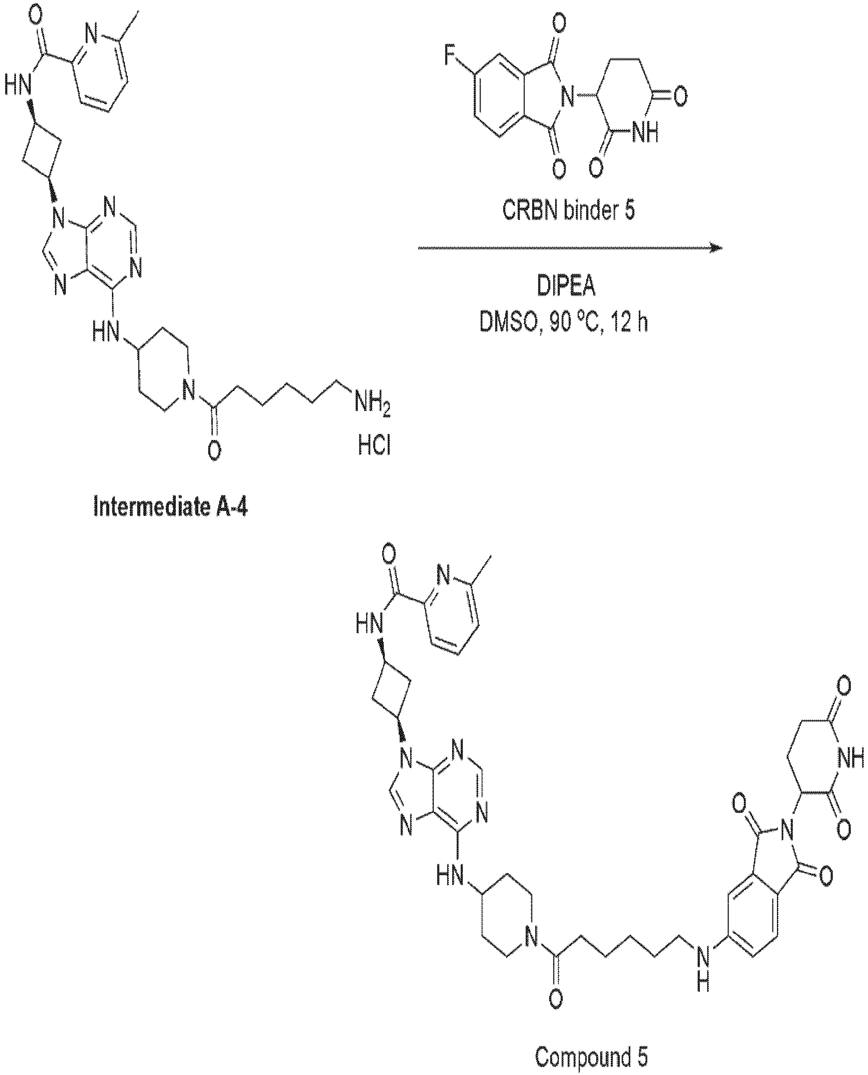

Intermediate A-4 (40 mg, 0.08 mmol) was dissolved in DMSO (1 mL). 2-(2,6-dioxopiperidin-3-yl)-5-fluoroisoindoline-1,3-dione (22 mg, 0.08 mmol) and DIPEA (0.04 mL, 0.23 mmol) were then added, followed by stirring at 90°C for 12 hours. The reaction mixture was diluted with water and then extracted with ethyl acetate. The organic layer was washed with brine, and the residue was dried over anhydrous magnesium sulfate and filtered. The filtrate was concentrated, and then separated and purified by silica gel column chromatography to obtain the desired Compound 5 (6.7 mg, 11%) as a yellow solid.

### Example 6: Synthesis of N-((1r,3r)-3-(6-((1-(6-((2-(2,6-dioxopiperidin-3-yl)-1,3-dioxoisoindolin-4-yl)amino)hexanoyl)piperidin-4-yl)amino)-9H-purin-9-yl)cyclobutyl)-6-methylpicolinamide (Compound 6)

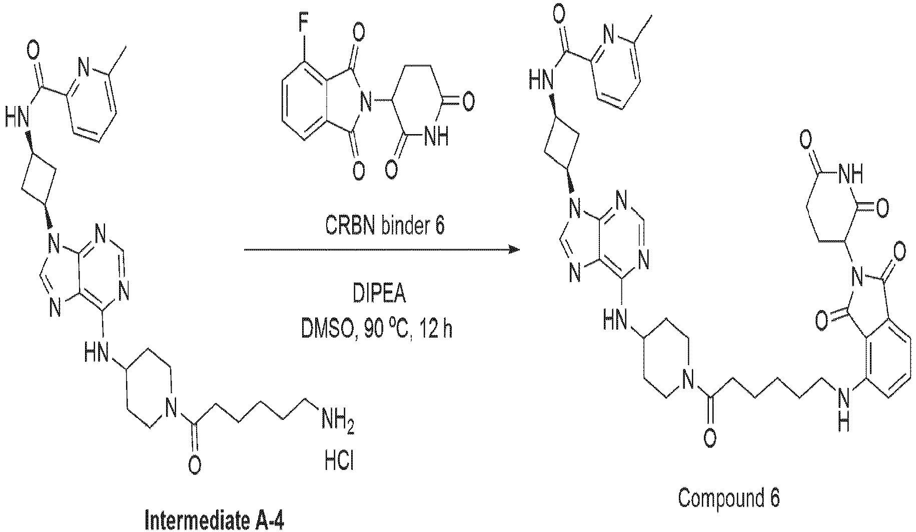

Compound 6 was synthesized in a similar manner to Compound 5, except that 2-(2,6-dioxopiperidin-3-yl)-4-fluoroisoindoline-1,3-dione (CRBN Binder 6) was used instead of CRBN Binder 5.

Compounds 7 to 153 were synthesized in the same or similar manner to Examples 1 to 6 above using corresponding linkers. Hereinafter, the synthesis methods of additionally used intermediates and compounds are exemplarily described, and other compounds for which no specific method is provided were synthesized using the corresponding intermediates and appropriate reactions.

### Example 7: Synthesis of N-((1s,3s)-3-(6-(((1-((1-(2-((2-(2,6-dioxopiperidin-3-yl)-1,3-dioxoisoindolin-5-yl)oxy)acetyl)piperidin-4-yl)methyl)piperidin-4-yl)methyl)amino)-9H-purin-9-yl)cyclobutyl)acetamide (Compound 154)

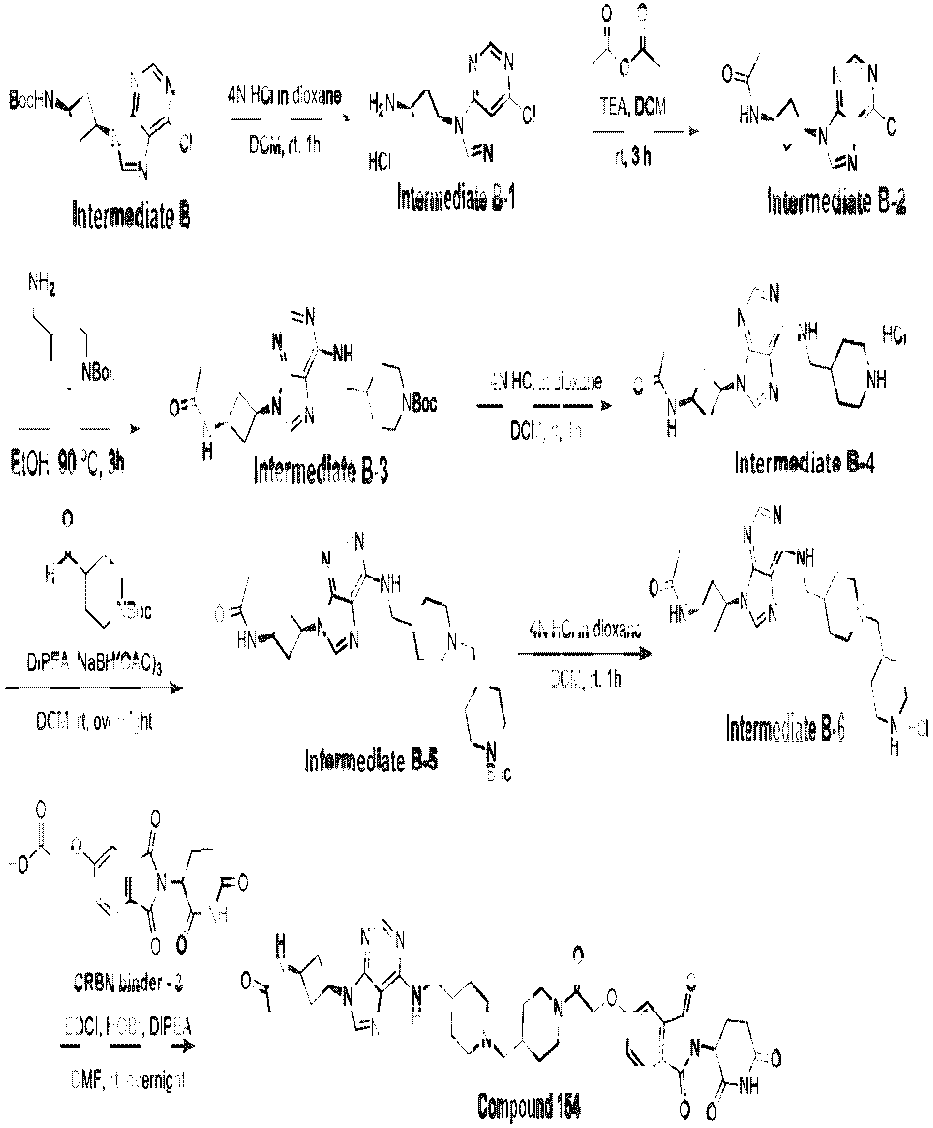

### Step 1: Synthesis of (1s,3s)-3-(6-chloro-9H-purin-9-yl)cyclobutan-1-amine hydrochloride (Intermediate B-1)

Intermediate B (*tert*-butyl (1s,3s)-3-(6-chloro-9H-purin-9-yl)cyclobutylcarbamate; 5.7g, 17.6 mmol) was dissolved in DCM (20 mL). 4N hydrochloric acid/1,4-dioxene (53 mmol) was then added, followed by stirring at room temperature for 1 hour. The reaction mixture was concentrated under reduced pressure to obtain the desired Intermediate B-1 (3.9 g) as a white solid.

¹H H NMR (300 MHz, DMSO) δ 9.21 (s, 1H), 8.79 (s, 1H), 8.57 (s, 3H), 5.04 (p, *J =* 8.5 Hz, 1H), 3.80-3.62 (m, 1H), 3.42-2.85 (m, 4H).

### Step 2: Synthesis of N-((1s,3s)-3-(6-chloro-9H-purin-9-yl)cyclobutyl)acetamide (Intermediate B-2)

Intermediate B-1 (650 mg, 2.4988 mmol) was dissolved in DCM (10 mL). TEA(1.044 mL) was then added, followed by addition of acetic anhydride (0.354 mL), followed by stirring at room temperature for 1 hour. Upon completion of the reaction, the reaction mixture was diluted with water and then extracted with ethyl acetate. The organic layer was washed with brine, and the residue was dried over anhydrous magnesium sulfate and filtered. The filtrate was concentrated, and then separated and purified by silica gel column chromatography to obtain the desired Intermediate B-2 (170 mg, 22%).

¹H NMR (500 MHz, Chloroform-d) δ 8.78 (s, 1H), 8.25 (s, 1H), 6.34 (d, *J* = 8.2 Hz, 1H), 4.85-4.76 (m, 1H), 4.50-4.40 (m, 1H), 3.15-3.08 (m, 2H), 2.96-2.89 (m, 2H), 2.07 (s, 3H).

### Step 3: Synthesis of tert-butyl 4-(((9-((1s,3s)-3-acetamidocyclobutyl)-9H-purin-6-yl)amino)methyl)piperidine-1-carboxylate (Intermediate B-3)

Reaction was carried out in a similar manner to Step 1 of Example 1 above, except that Intermediate B-2 (170 mg, 0.6398 mmol) and *tert*-butyl 4-(aminomethyl)piperidine-1-carboxylate (136 mg, 0.9597 mmol) were used as reactants. The reaction mixture was separated and purified to obtain the desired Intermediate B-3 (256 mg, 90%) as a white solid.

¹H NMR (500 MHz, Chloroform-d) δ 8.40 (s, 1H), 7.76 (s, 1H), 6.85 (d, *J* = 8.7 Hz, 1H), 5.85 (s, 1H), 4.73-4.63 (m, 1H), 4.53-4.42 (m, 1H), 4.15 (s, 2H), 3.59 (s, 2H), 3.54-3.50 (m, 1H), 3.13-3.05 (m, 2H), 2.91-2.83 (m, 2H), 2.72 (s, 2H), 2.07 (s, 3H), 1.83-1.78 (m, 2H), 1.47 (s, 9H), 1.30-1.20 (m, 2H).

### Step 4: Synthesis of N-((1s,3s)-3-(6-((piperidin-4-ylmethyl)amino)-9H-purin-9-yl)cyclobutyl)acetamide hydrochloride (Intermediate B-4)

Reaction was carried out in a similar manner to Step 1-1 of Example 1 above, except that Intermediate B-3 (250mg, 0.5636 mmol) was used as a reactant to obtain the desired Intermediate B-4 (214 mg) as a white solid.

¹H NMR (500 MHz, Methanol-*d*₄) δ 8.54 (d, *J* = 4.3 Hz, 1H), 8.44 (d, *J* = 13.8 Hz, 1H), 4.33-4.25 (m, 1H), 4.19-4.13 (m, 1H), 3.64-3.58 (m, 1H), 3.51-3.44 (m, 2H), 3.10-3.01 (m, 4H), 2.84-2.75 (m, 2H), 2.16-2.09 (m, 2H), 2.03 (s, 3H), 1.63-1.62 (m, 3H), 1.62-1.57 (m, 1H).

### Step 5: Synthesis of tert-butyl 4-((4-(((9-((1s,3s)-3-acetamidocyclobutyl)-9H-purin-6-yl)amino)methyl)piperidin-1-yl)methyl)piperidine-1-carboxylate (Intermediate B-5)

Intermediate B-4 (210 mg, 0.5527 mmol) was dissolved in DCM (20 mL). DIPEA (144 µL) and *tert-*butyl 4-formylpiperidine-1-carboxylate (188 mg, 0.8291 mmol) was then added, followed by stirring. NaBH(OAC)3 (152 mg, 0.7185 mmol) was then added, followed by stirring at room temperature overnight. Upon completion of the reaction, the solvent was removed by concentration under reduced pressure, and the reaction mixture was separated and purified by silica gel column chromatography to obtain the desired Intermediate B-5 (0.264 g).

¹H NMR (500 MHz, Methanol-d4) δ 8.26 (d, J = 8.5 Hz, 2H), 4.83-4.74 (m, 1H), 4.33-4.24 (m, 1H), 4.08 (d, J = 13.2 Hz, 2H), 3.79-3.71 (m, 1H), 3.60-3.51 (m, 2H), 3.46 (d, J = 12.0 Hz, 2H), 3.28-3.21 (m, 1H), 3.02-2.94 (m, 2H), 2.82 (d, J = 6.9 Hz, 2H), 2.80-2.68 (m, 5H), 2.00 (s, 3H), 1.98 (s, 1H), 1.94 (s, 2H), 1.85-1.78 (m, 2H), 1.72-1.61 (m, 2H), 1.44 (s, 9H), 1.21-1.11 (m, 2H).

### Step 6: Synthesis of N-((1s,3s)-3-(6-(((1-(piperidin-4-ylmethyl)piperidin-4-yl)methyl)amino)-9H-purin-9-yl)cyclobutyl)acetamide hydrochloride (Intermediate B-6)

The reaction was carried out in a similar manner to Step 2-1 of Example 1 above, except that Intermediate B-5 (260mg, 0.48 mmol) was used as a reactant to obtain the desired Intermediate B-6 (211 mg) as a white solid.

¹H NMR (500 MHz, Methanol-d4) δ 8.52 (s, 1H), 8.44 (d, J = 13.9 Hz, 1H), 4.31-4.22 (m, 1H), 3.78-3.71 (m, 2H), 3.61 (d, J = 6.7 Hz, 1H), 3.46 (d, J = 12.8 Hz, 2H), 3.16-3.00 (m, 8H), 2.81-2.73 (m, 2H), 2.32 (s, 1H), 2.15 (d, J = 15.0 Hz, 4H), 2.01 (s, 3H), 1.90-1.80 (m, 2H), 1.62 (s, 2H), 1.61-1.52 (m, 2H), 1.42-1.38 (m, 1H).

### Step 7: Synthesis of N-((1s,3s)-3-(6-(((1-((1-(2-((2-(2,6-dioxopiperidin-3-yl)-1,3-dioxoisoindolin-5-yl)oxy)acetyl)piperidin-4-yl)methyl)piperidin-4-yl)methyl)amino)-9H-purin-9-yl)cyclobutyl)acetamide (Compound 154)

The reaction was carried out in a similar manner to Step 3 of Example 1 above, except that Intermediate B-6 (20 mg, 0.0419 mmol) and CRBN Binder 3 (16 mg, 0.0503 mmol) were used as reactants. The reaction mixture was separated and purified to obtain the desired Compound 154 (6 mg, 20%) as a white solid.

### Example 8: Synthesis of N-((1s,3s)-3-(6-(((1-((1-(2-((2-(2,6-dioxopiperidin-3-yl)-1,3-dioxoisoindolin-4-yl)oxy)acetyl)piperidin-4-yl)methyl)piperidin-4-yl)methyl)amino)-9H-purin-9-yl)cyclobutyl)acetamide (Compound 155)

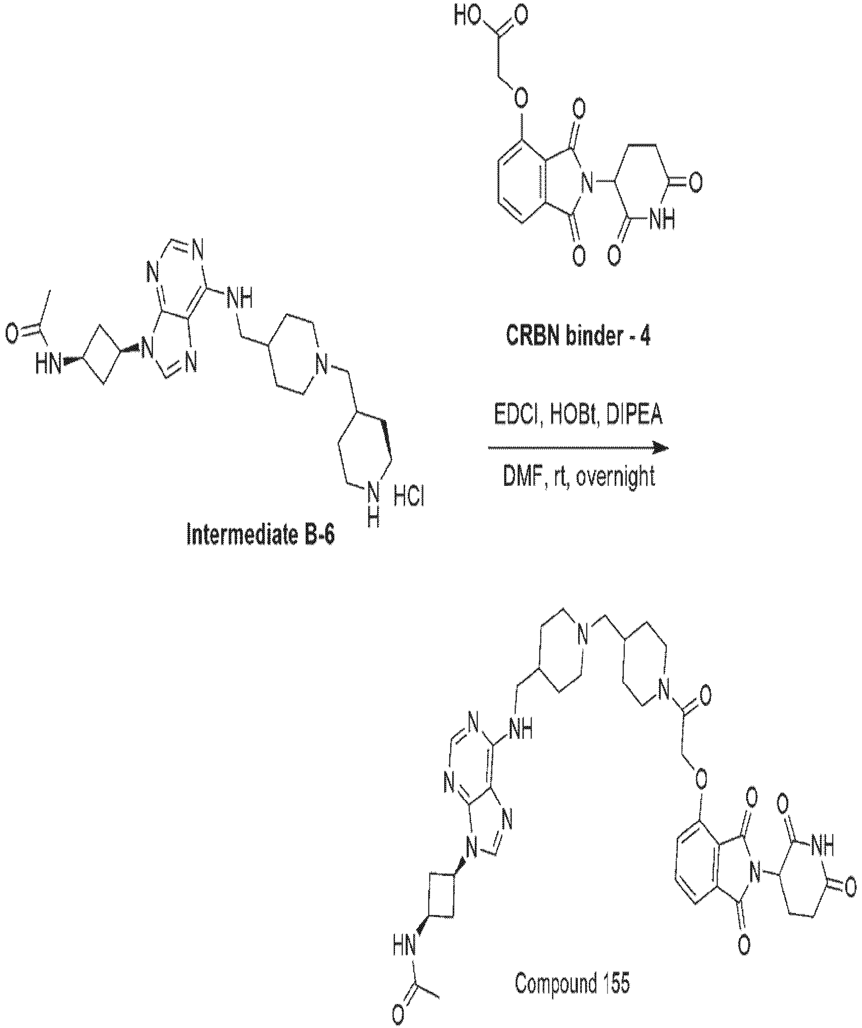

Compound 155 was synthesized in a similar manner to Compound 154, except that CRBN Binder 4 was used instead of CRBN Binder 3.

### Example 9: Synthesis of N-((1s,3s)-3-(6-(((1-((1-((2-(2,6-dioxopiperidin-3-yl)-1,3-dioxoisoindolin-5-yl)glycyl)piperidin-4-yl)methyl)piperidin-4-yl)methyl)amino)-9H-purin-9-yl)cyclobutyl)acetamide (Compound 156)

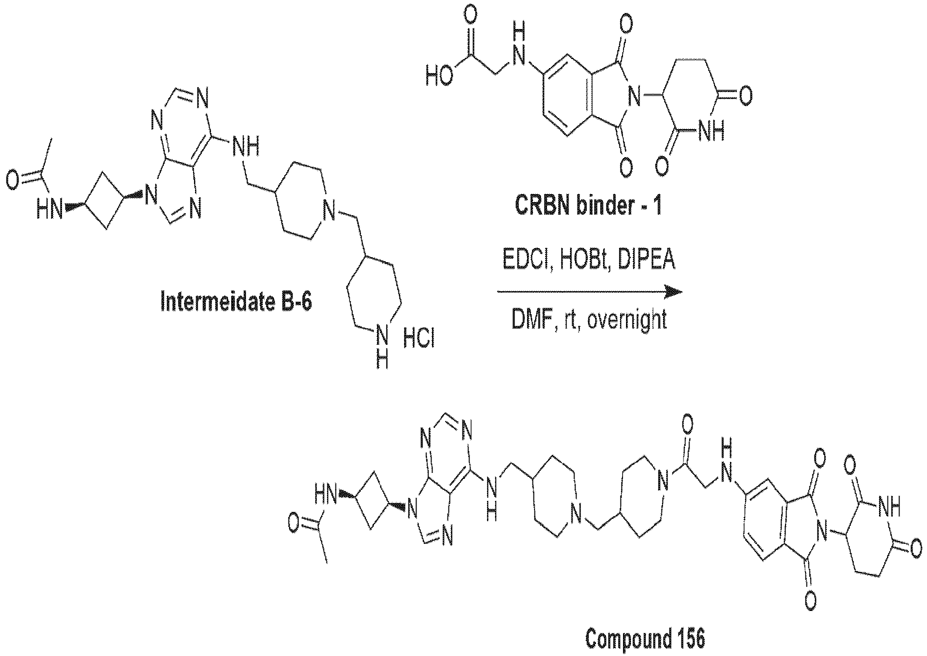

Compound 156 was synthesized in a similar manner to Compound 154, except that CRBN Binder 1 was used instead of CRBN Binder 3.

### Example 10: Synthesis of N-((1s,3s)-3-(6-(((1-((1-((2-(2,6-dioxopiperidin-3-yl)-1,3-dioxoisoindolin-4-yl)glycyl)piperidin-4-yl)methyl)piperidin-4-yl)methyl)amino)-9H-purin-9-yl)cyclobutyl)acetamide (Compound 157)

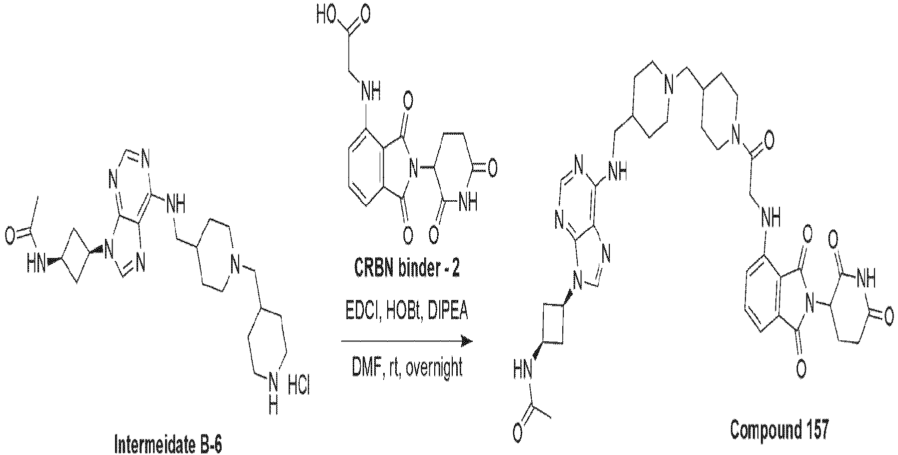

Compound 157 was synthesized in a similar manner to Compound 154, except that CRBN Binder 2 was used instead of CRBN Binder 3.

### Example 11: Synthesis of N-((1s,3s)-3-(6-(((1-((1-(2-(2,6-dioxopiperidin-3-yl)-1,3-dioxoisoindolin-5-yl)piperidin-4-yl)methyl)piperidin-4-yl)methyl)amino)-9H-purin-9-yl)cyclobutyl)acetamide (Compound 158)

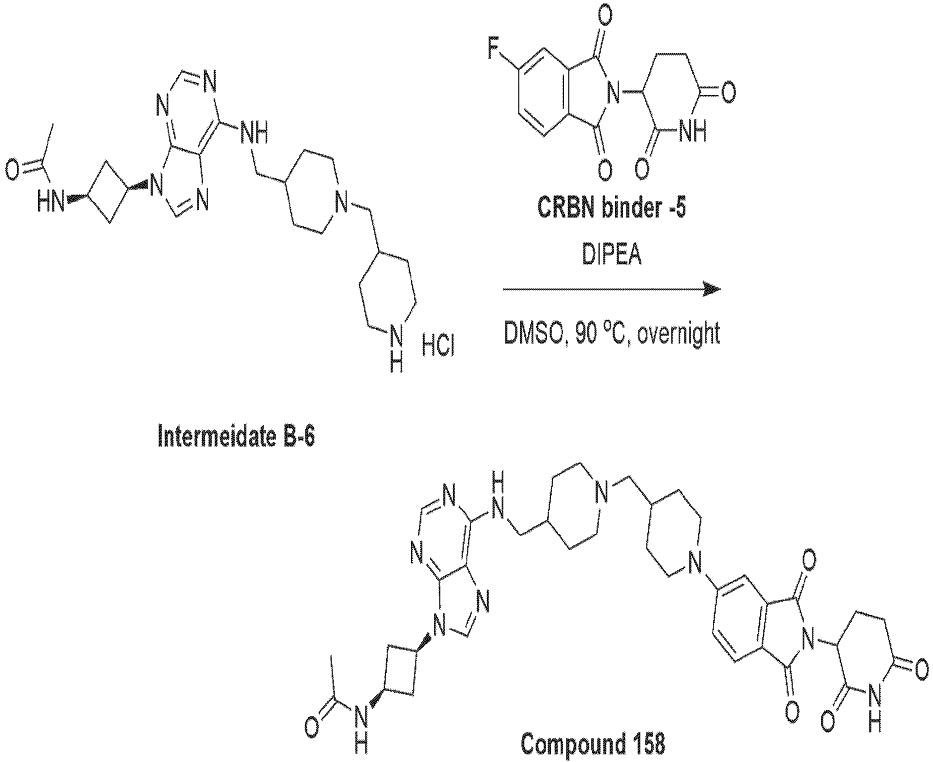

Compound 158 was synthesized in a similar manner to Example 5, except that Intermediate B-6 was used instead of Intermediate A-4.

### Example 12: Synthesis of N-((1s,3s)-3-(6-(((1-((1-(2-(2,6-dioxopiperidin-3-yl)-1,3-dioxoisoindolin-4-yl)piperidin-4-yl)methyl)piperidin-4-yl)methyl)amino)-9H-purin-9-yl)cyclobutyl)acetamide (Compound 159)

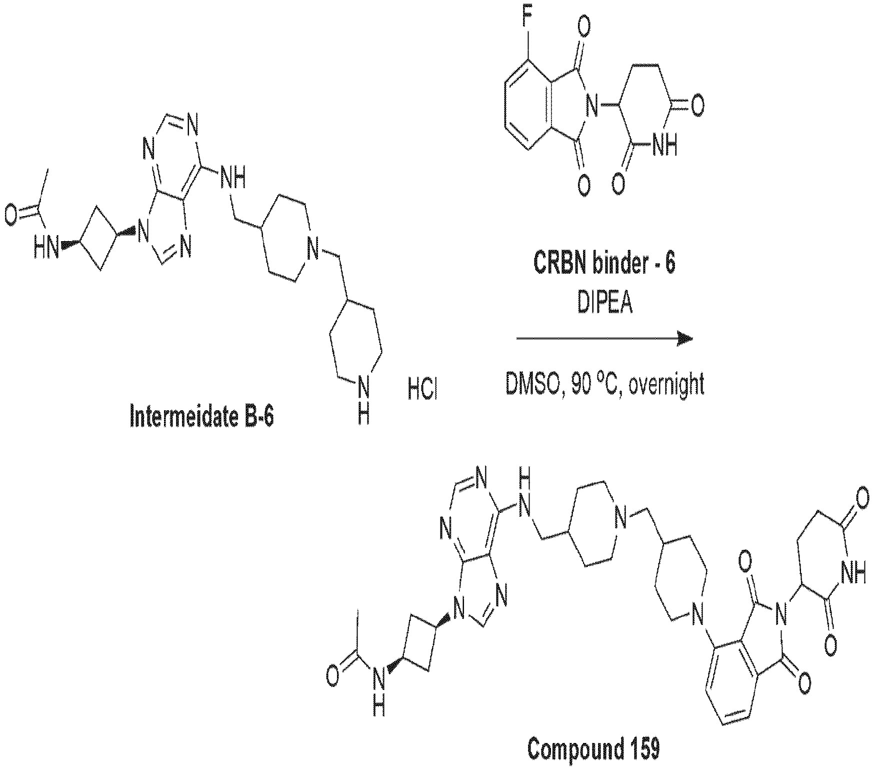

Compound 159 was synthesized in a similar manner to Compound 154, except that CRBN Binder 6 was used instead of CRBN Binder 3.

Compounds 160 to 185 were synthesized in the same or similar manner to Compounds 155 to 160 above using corresponding linkers.

### Preparation Example 1: Synthesis of 2-phenyl-N-((1s,3s)-3-(6-((4-(4-(piperidin-4-ylmethyl)piperazin-1-yl)phenyl)amino)-9H-purin-9-yl)cyclobutyl)acetamide hydrochloride (Intermediate C-6)

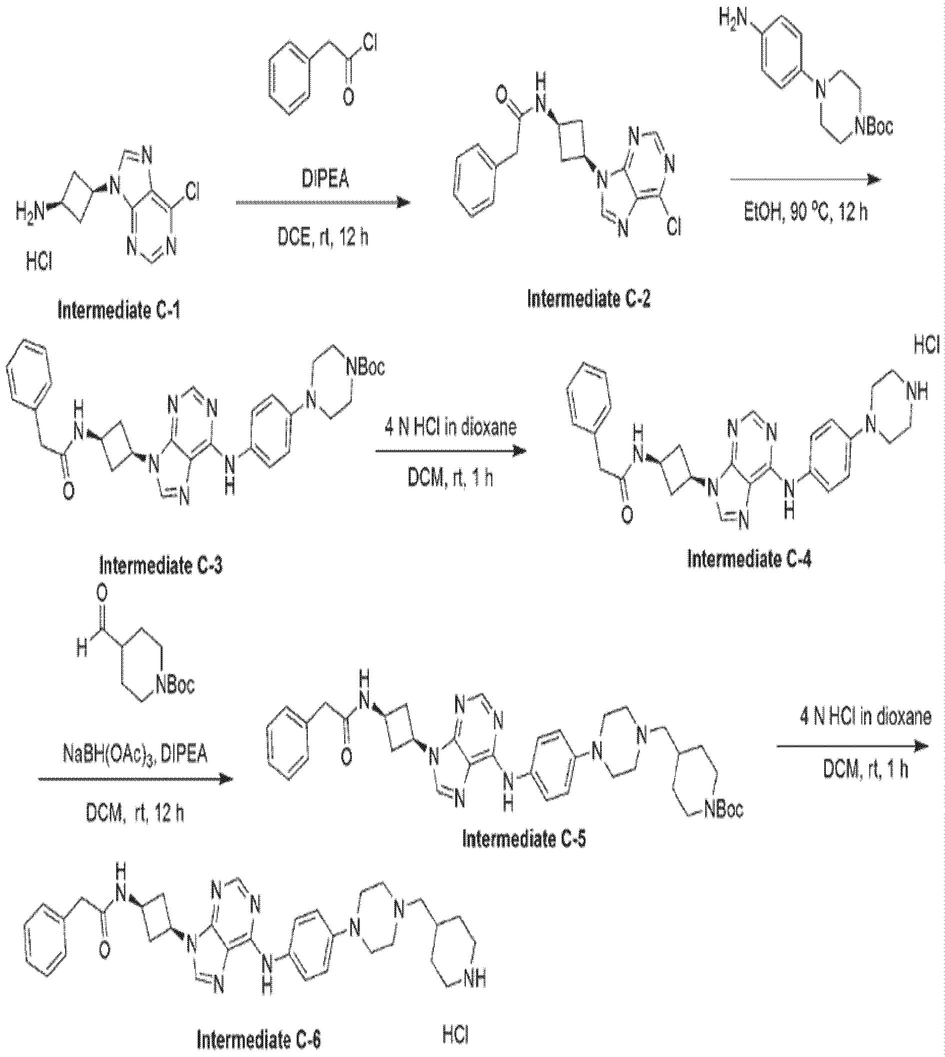

### Step 1: Synthesis of N-((1s,3s)-3-(6-chloro-9H-purin-9-yl)cyclobutyl)-2-phenylacetamide (Intermediate C-2)

Intermediate B-1 (500 mg, 1.92 mmol) was dissolved in dichloroethane (15 mL). Then, 2-phenylacetic acid chloride (508 µL, 3.84 mmol) and DIPEA (1.4 mL, 7.68 mmol) were added, followed by stirring at room temperature for 12 hours. Upon completion of the reaction, the reaction mixture was diluted with water and then extracted with ethyl acetate. The organic layer was washed with brine, and the residue was dried over anhydrous magnesium sulfate and filtered. The filtrate was concentrated, and then separated and purified by silica gel column chromatography to obtain the desired Intermediate C-2 (265 mg, 40%).

¹H NMR (500 MHz, CDCl₃) δ 8.50 (s, 1H), 8.18 (s, 1H), 7.50 - 7.32 (m, 5H), 6.34 - 6.19 (m, 1H), 4.78 (p, *J* = 8.3 Hz, 1H), 4.48 (q, *J* = 8.2 Hz, 1H), 3.67 (s, 2H), 3.11 (m, 2H), 2.81 (m, 2H).

### Step 2: Synthesis of tert-butyl 4-(4-((9-((1s,3s)-3-(2-phenylacetamido) cyclobutyl)-9H-purin-6-yl)amino)phenyl)piperazine-1-carboxylate (Intermediate C-3)

The reaction was carried out in a similar manner to Step 1 of Example 1 above, except that Intermediate C-2 (750mg, 2.194 mmol) and *tert-*butyl 4-(4-aminophenyl)piperizine-1-carboxylate (1.1 g, 3.949 mmol) were used as reactants. The reaction mixture was separated and purified to obtain the desired Intermediate C-3 (1.0 g) as an ivory solid.

¹H NMR (500 MHz, CDCl₃) δ 8.25 (s, 1H), 7.78 (s, 1H), 7.71 - 7.54 (m, 3H), 7.42 - 7.37 (m, 5H), 7.01 - 6.92 (m, 2H), 6.69 (d, *J* = 8.6 Hz, 1H), 4.66 (p, *J* = 8.1 Hz, 1H), 4.46 (h, *J* = 8.1 Hz, 1H), 3.63 (s, 2H), 3.59 (t, *J* = 5.2 Hz, 4H), 3.11 (t, *J* = 5.2 Hz, 4H), 3.09 - 3.00 (m, 2H), 2.84 - 2.72 (m, 2H), 1.49 (s, 9H).

### Step 3: Synthesis of 2-phenyl-N-((1s,3s)-3-(6-((4-(piperazin-1-yl)phenyl)amino)-9H-purin-9-yl)cyclobutyl)acetamide hydrochloride (Intermediate C-4)

The reaction was carried out in a similar manner to Step 1-1 of Example 1 above, except that Intermediate C-3 (1.0 g, 1.71 mmol) was used as a reactant to obtain the desired Intermediate C-4 (850 mg) as a light green solid.

¹H NMR (500 MHz, CD3OD) δ 8.58 (s, 1H), 8.29 (s, 1H), 7.49 - 7.43 (m, 2H), 7.34 (m, 4H), 7.30 - 7.24 (m, 3H), 4.99 - 4.93 (m, 1H), 4.36 - 4.21 (m, 1H), 3.56 (m, 6H), 3.44 (m, 4H), 3.05 (m, 2H), 2.80 (m, 2H).

### Step 4: Synthesis of tert-butyl 4-((4-(4-((9-((1s,3s)-3-(2-phenylacetamido)cyclobutyl)-9H-purin-6-yl)amino)phenyl)piperazin-1-yl)methyl)piperidine-1-carboxylate (Intermediate C-5)

Intermediate C-4 (100 mg, 0.192 mmol) was dissolved in DCM (10 mL). DIPEA (100 µL, 0.576 mmol) and *tert-*butyl 4-formylpiperidine-1-carboxylate (82 mg, 0.385 mmol) was then added, followed by stirring. NaBH(OAC)₃ (142 mg, 0.672 mmol) was then added, followed by stirring at room temperature for 12 hours. Upon completion of the reaction, the solvent was removed by concentration under reduced pressure, and the reaction mixture was separated and purified by silica gel column chromatography to obtain the desired Intermediate C-5 (90 mg).

¹H NMR (300 MHz, CD3OD) δ 8.32 (s, 1H), 8.25 (s, 1H), 7.70 - 7.56 (m, 2H), 7.41 - 7.18 (m, 5H), 7.10 - 6.96 (m, 2H), 4.81 (q, *J* = 8.5 Hz, 1H), 4.31 (p, *J* = 8.2 Hz, 1H), 4.09 (d, *J* = 13.1 Hz, 2H), 3.55 (s, 2H), 3.21 (t, *J* = 5.0 Hz, 4H), 3.00 (m, 2H), 2.76 (m, 2H), 2.65 (t, *J* = 5.1 Hz, 4H), 2.31 (d, *J* = 6.6 Hz, 2H), 1.81 (d, *J* = 12.1 Hz, 3H), 1.48 (s, 9H), 1.21 - 1.01 (m, 1H).

### Step 5: Synthesis of 2-phenyl-N-((1s,3s)-3-(6-((4-(4-(piperidin-4-ylmethyl)piperazin-1-yl)phenyl)amino)-9H-purin-9-yl)cyclobutyl)acetamide hydrochloride (Intermediate C-6)

The reaction was carried out in a similar manner to Step 2-1 of Example 1 above, except that Intermediate C-5 (90 mg, 0.132 mmol) was used as a reactant to obtain the desired Intermediate C-6 (75 mg) as a white solid.

¹H NMR (500 MHz, CD3OD) δ 8.59 (s, 1H), 8.30 (s, 1H), 7.54 - 7.42 (m, 2H), 7.36 - 7.33 (m, 4H), 7.31 - 7.23 (m, 3H), 4.99 - 4.94 (m, 1H), 4.34 - 4.24 (m, 1H), 3.99 (d, *J* = 13.0 Hz, 2H), 3.83 (d, *J* = 11.7 Hz, 2H), 3.56 (s, 2H), 3.52 - 3.36 (m, 6H), 3.28 (d, *J* = 6.7 Hz, 2H), 3.13 (m, 2H), 3.09 - 3.01 (m, 2H), 2.81 (m, 2H), 2.45 - 2.35 (m, 1H), 2.21 (d, *J* = 13*.*8 Hz, 2H), 1.70 - 1.58 (m, 2H).

### Preparation Example 2: Synthesis of N-((1s,3s)-3-(6-((4-(4-(7-azaspiro[3.5]nonane-2-yl)piperazin-1-yl)phenyl)amino)-9H-purin-9-yl)cyclobutyl)-2-phenylacetamide hydrochloride (Intermediate C-8)

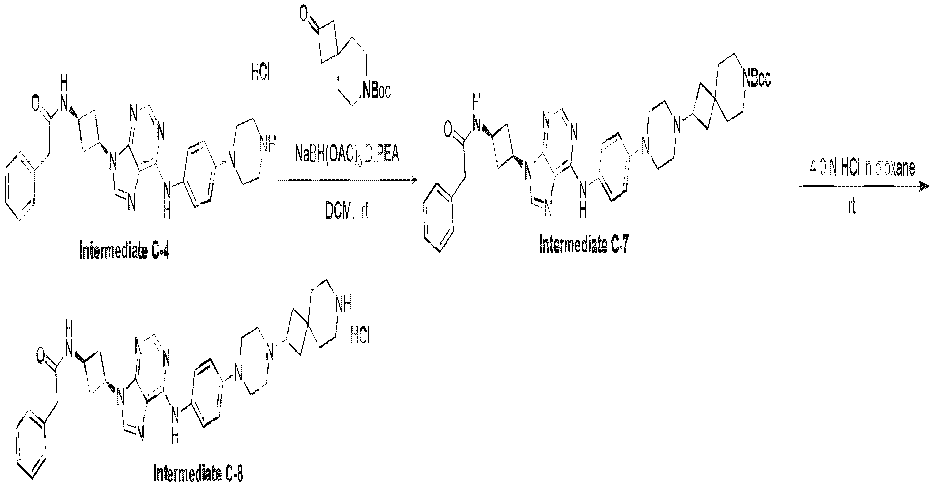

### Step 1: Synthesis of tert-butyl 2-(4-(4-((9-((1s,3s)-3-(2-phenylacetamido)cyclobutyl)-9H-purin-6-yl)amino)phenyl)piperazin-1-yl)-7-azaspiro[3.5]nonane-7-carboxylate (Intermediate C-7)

Intermediate C-7 was synthesized in a similar manner to Step 4 of Preparation Example 1 above.

¹H NMR (500 MHz, DMSO-*d*₆) δ 9.65 (s, 1H), 8.47 (d, *J* = 7.7 Hz, 1H), 8.40 (s, 1H), 8.32 (s, 1H), 7.72 (d, *J* = 8.7 Hz, 2H), 7.34 - 7.20 (m, 5H), 6.91 (d, *J* = 9.0 Hz, 2H), 4.76 (p, *J* = 8.6 Hz, 1H), 4.16 (h, *J* = 8.2 Hz, 1H), 3.43 (s, 2H), 3.29 (t, *J* = 5.7 Hz, 2H), 3.20 (m,2H), 3.09 (m, 4H), 2.85 (m, 2H), 2.71 (t, *J* = 7.7 Hz, 1H), 2.67 - 2.57 (m, 2H), 2.38 (m, 4H), 1.98 (t, *J =* 9.4 Hz, 2H), 1.57 (t, *J =* 9.9 Hz, 2H), 1.49 (t, *J* = 5.6 Hz, 2H).

### Step 2: Synthesis of N-((1s,3s)-3-(6-((4-(4-(7-azaspiro[3.5]nonane-2-yl)piperazin-1-yl)phenyl)amino)-9H-purin-9-yl)cyclobutyl)-2-phenylacetamide hydrochloride (Intermediate C-8)

Intermediate C-7 was synthesized in a similar manner to Step 5 of Preparation Example 1 above.

¹H NMR (500 MHz, DMSO-*d*₆) δ 11.71 (s, 1H), 10.33 (s, 1H), 8.79 (s, 2H), 8.68 (s, 1H), 8.60 (d, *J* = 7.6 Hz, 1H), 8.38 (s, 1H), 7.71 (d, *J* = 8.3 Hz, 2H), 7.35 - 7.28 (m, 4H), 7.24 (t, *J* = 6.8 Hz, 1H), 7.05 (d, *J* = 8.8 Hz, 2H), 4.80 (p, *J* = 8.5 Hz, 1H), 4.17 (h, *J* = 8.1 Hz, 1H), 3.81 (d, *J =* 12.9 Hz, 4H), 3.44 (s, 3H), 3.41 (m, 1H), 3.18 (t, *J =* 12.4 Hz, 2H), 3.08 - 2.93 (m, 6H), 2.92 - 2.83 (m, 2H), 2.65 (m, 2H), 2.34 (m, 2H), 2.22 (t, *J =* 10.1 Hz, 2H), 1.82 (t, *J* = 5.7 Hz, 2H), 1.75 (t, *J =* 5.7 Hz, 2H).

### Preparation Example 3: Synthesis of 2-phenyl-N-((1s,3s)-3-(6-((4-(4-(2-(piperidin-4-yl)acetyl)piperazin-1-yl)phenyl)amino)-9H- purin-9-yl)cyclobutyl)acetamide hydrochloride (Intermediate C-10)

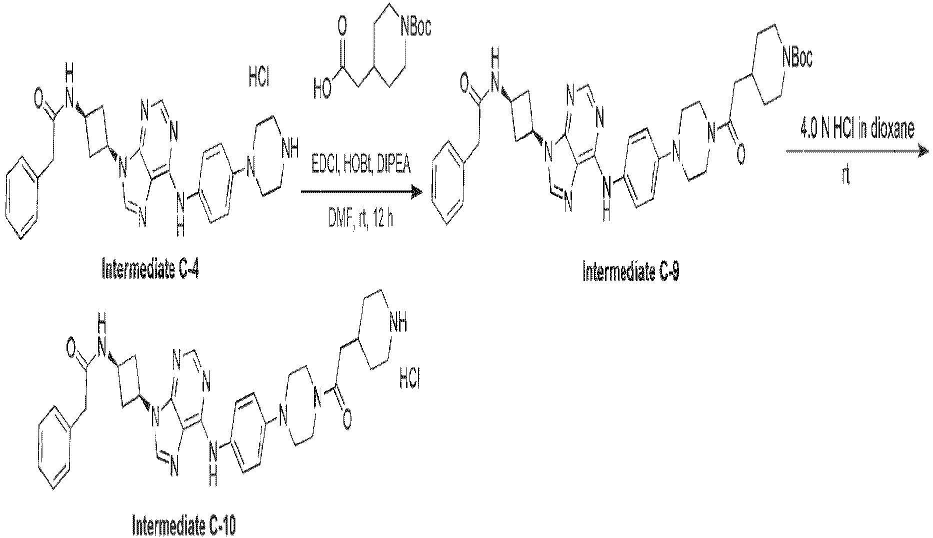

### Step 1: Synthesis of tert-butyl 4-(2-oxo-2-(4-(4-((9-((1s,3s)-3-(2-phenylacetamido) cyclobutyl)-9H-purin-6-yl)amino)phenyl)piperazin-1-yl)ethyl)piperidine-1-carboxylate (Intermediate C-9)

The reaction was carried out in a similar manner to Step 2-1 of Example 1 above, except that Intermediate C-4 (100 mg, 0.192 mmol) and 2-(1-(*tert-*butoxycarbonyl)piperidin-4-yl)acetic acid (56 mg, 0.231 mmol) were used as reactants. The reaction mixture was separated and purified to obtain the desired Intermediate C-9 (105 mg, 79%) as a white solid.

¹H NMR (400 MHz, CD3OD) δ 8.33 (s, 1H), 8.26 (s, 1H), 7.67 (d, *J* = 9.0 Hz, 2H), 7.34 (m, 4H), 7.31 - 7.22 (m, 1H), 7.11 - 7.03 (m, 2H), 4.83 (m, 1H), 4.31 (m, 1H), 4.09 (d, *J* = 13.2 Hz, 2H), 3.77 (m, 4H), 3.55 (s, 2H), 3.18 (dt, *J* = 16.0, 5.2 Hz, 4H), 3.01 (m, 2H), 2.77 (m, 2H), 2.42 (d, *J* = 7.0 Hz, 2H), 2.00 (m, 1H), 1.82 - 1.70 (m, 2H), 1.47 (s, 9H), 1.19 (m, 2H).

### Step 2: Synthesis of 2-phenyl-N-((1s,3s)-3-(6-((4-(4-(2-(piperidin-4-yl)acetyl)piperazin-1-yl)phenyl)amino)-9H-purin-9-yl)cyclobutyl)acetamide hydrochloride (Intermediate C-10)

Intermediate C-7 was synthesized in a similar manner to Step 5 of Preparation Example 1 above.

¹H NMR (400 MHz, CD3OD) δ 8.63 (s, 1H), 8.36 (s, 1H), 7.61 (d, *J* = 8.9 Hz, 1H), 7.46 (d, *J* = 9.0 Hz, 2H), 7.34 (m, 4H), 7.30 - 7.24 (m, 1H), 4.98 - 4.93 (m, 1H), 4.28 (m, 1H), 3.91 (m, 4H), 3.54 (m, 4H), 3.48 (t, *J* = 5.1 Hz, 1H), 3.42 (m, 2H), 3.12 - 2.99 (m, 4H), 2.80 (m, 2H), 2.53 (d, *J* = 6.8 Hz, 2H), 2.24 - 2.13 (m, 1H), 2.06 (d, *J* = 14.0 Hz, 2H), 1.61 - 1.45 (m, 2H).

### Preparation Example 4: Synthesis of N-((1s,3s)-3-(6-((4-(4-(((1r,4r)-4-(aminomethyl)cyclohexyl)methyl)piperazin-1-yl)phenyl)amino)-9H-purin-9-yl)cyclobutyl)-2-phenylacetamide hydrochloride (Intermediate C-12)

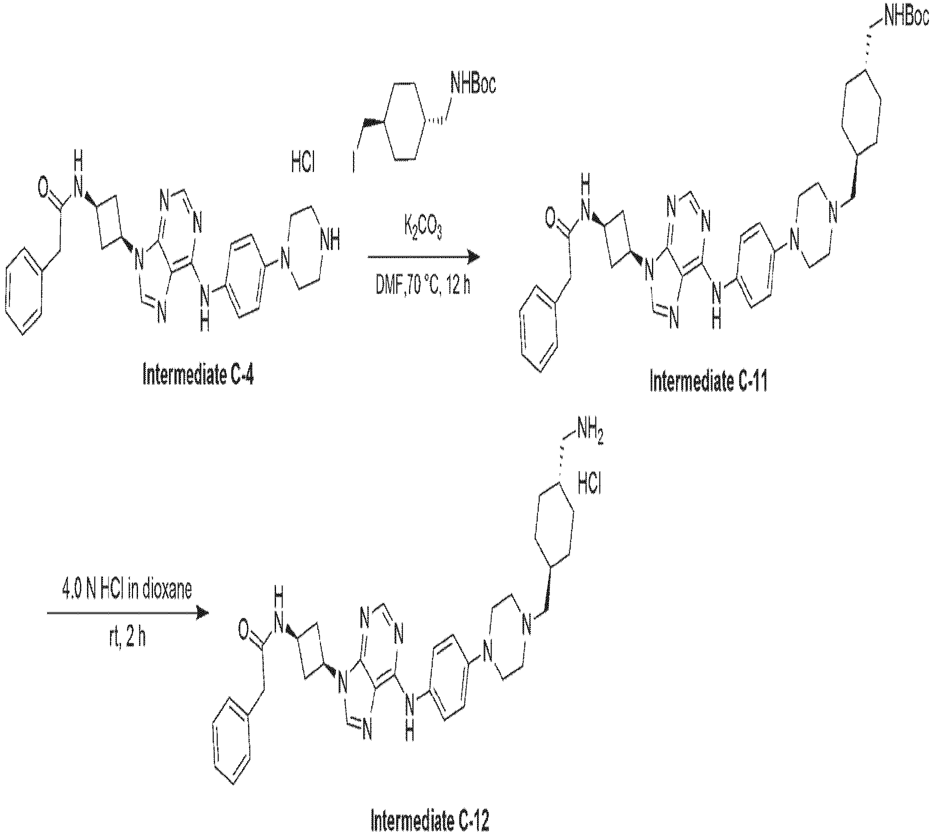

### Step 1: Synthesis of tert-butyl (((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)carbamate (Intermediate C-11)

Intermediate C-4 (30 mg, 0.0577 mmol) was dissolved in DMF (5 mL). *Tert-*butyl (((1r,4r)-4-(iodomethyl)cyclohexyl)methyl)carbamate (24 mg, 0.0693 mmol) and potassium carbonate (20 mg, 0.144 mmol) were then added, followed by stirring at 70°C for 12 hours. The reaction mixture was diluted with water and then extracted with ethyl acetate. The organic layer was washed with brine, and the residue was dried over anhydrous magnesium sulfate and filtered. The filtrate was concentrated under reduced pressure, and then separated and purified by silica gel column chromatography to obtain the desired Intermediate C-11 (17 mg, 34%) as a white solid.

¹H NMR (400 MHz, CDCl₃) δ 8.27 (s, 1H), 7.83 (s, 1H), 7.70 - 7.60 (m, 3H), 7.46 - 7.31 (m, 5H), 6.97 (d, *J* = 8.6 Hz, 2H), 6.78 (d, *J* = 8.6 Hz, 1H), 4.67 (m, 2H), 4.47 (p, *J* = 8.1 Hz, 1H), 3.64 (s, 2H), 3.42 (m, 3H), 3.17 - 2.73 (m, 10H), 2.52 (s, 2H), 2.02 (m, 3H), 1.87 - 1.56 (m, 4H), 1.46 (s, 9H), 1.16 - 0.85 (m, 4H).

### Step 2: Synthesis of N-((1s,3s)-3-(6-((4-(4-(((1r,4r)-4-(aminomethyl)cyclohexyl)methyl)piperazin-1-yl)phenyl)amino)-9H-purin-9-yl)cyclobutyl)-2-phenylacetamide hydrochloride (Intermediate C-12)

Intermediate C-7 was synthesized in a similar manner to Step 5 of Preparation Example 1 above.

¹H NMR (500 MHz, CD3OD) δ 8.57 (s, 1H), 8.29 (m, 1H), 7.55 - 7.23 (m, 9H), 4.28 (m, 1H), 3.98 (d, *J* = 10.3 Hz, 2H), 3.84 - 3.59 (m, 5H), 3.55 (s, 2H), 3.41 (m, 3H), 3.17 (d, *J* = 6.8 Hz, 2H), 3.05 (m, 2H), 2.82 (m, 4H), 2.00 (m, 4H), 1.69 (m, 1H), 1.26 (m, 5H).

### Preparation Example 5: Synthesis of 6-methyl-N-((1s,3s)-3-(6-((3-(4-(piperidin-4-ylmethyl)piperazin-1-yl)propyl)amino)-9H-purin-9-yl)cyclobutyl)picolinamide hydrochloride (Intermediate D-4)

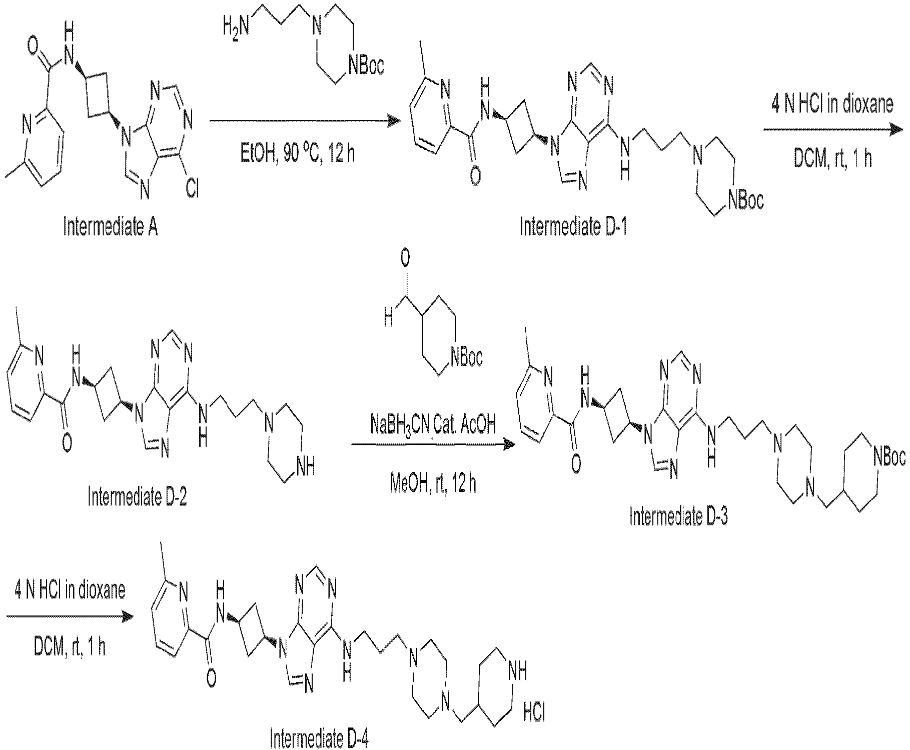

### Step 1: Synthesis of tert-butyl 4-(3-((9-((1s,3s)-3-(6-methylpicolinamido)cyclobutyl)-9H-purin-6-yl)amino)propyl)piperazine-1-carboxylate (Intermediate D-1)

The reaction was carried out in a similar manner to Step 1 of Example 1 above, except that *tert-*butyl 4-(3-aminophenyl)piperizine-1-carboxylate was used as a reactant, to obtain the desired Intermediate D-1.

¹H NMR (400 MHz, CDCl₃) δ 8.79 (d, *J* = 8.6 Hz, 1H), 8.37 (s, 1H), 7.94 (d, *J* = 7.7 Hz, 1H), 7.80 (s, 1H), 7.67 (t, *J =* 7.7 Hz, 1H), 7.23 (d, *J* = 7.7 Hz, 1H), 7.00 (s, 1H), 4.71 (p, *J* = 8.3 Hz, 1H), 4.53 (h, *J* = 8.2 Hz, 1H), 3.67 (m, 2H), 3.52 (t, *J* = 5.1 Hz, 4H), 3.18 - 3.03 (m, 2H), 2.88 (m, 2H), 2.57 (m, 5H), 2.51 - 2.38 (m, 4H), 1.87 (p, *J* = 6.5 Hz, 2H), 1.40 (s, 9H).

### Step 2: Synthesis of 6-methyl-N-((1s,3s)-3-(6-((3-(piperazin-1-yl)propyl)amino)-9H-purin-9-yl)cyclobutyl)picolinamide (Intermediate D-2)

Intermediate D-2 was synthesized in a similar manner to Step 1-1 of Example 1 above.

¹H NMR (300 MHz, CD3OD) δ 8.33 (s, 1H), 8.29 (s, 1H), 7.93 (d, J = 7.8 Hz, 1H), 7.85 (t, J = 7.7 Hz, 1H), 7.46 (dd, J = 7.6, 1.2 Hz, 1H), 4.88 (m, 1H), 4.61 (p, J = 8.2 Hz, 1H), 3.68 (m, 2H), 3.15 - 3.03 (m, 2H), 2.98 (m, 2H), 2.89 (t, J = 5.0 Hz, 4H), 2.66 (s, 3H), 2.61 - 2.41 (m, 6H), 1.93 (p, J = 6.9 Hz, 2H).

### Step 3: Synthesis of tert-butyl 4-((4-(3-((9-((1s,3s)-3-(6-methylpicolinamido)cyclobutyl)-9H-purin-6-yl)amino)propyl)piperazin-1-yl)methyl)piperidine-1-carboxylate (Intermediate D-3)

Intermediate D-2 (150 mg, 0.333 mmol) was dissolved in methanol (25 mL). *Tert-*butyl 4-formylpiperidine-1-carboxylate (106 mg, 0.50 mmol) and acetic acid (0.5 mL) were then added, followed by stirring for 30 minutes. NaBH₃CN (125 mg, 1.98 mmol) was then slowly added to the reaction solution, followed by stirring at room temperature for 12 hours. The reaction solution was concentrated under reduced pressure, diluted with saturated sodium bicarbonate solution, and extracted with ethyl acetate. The filtrate was concentrated under reduced pressure, and then separated and purified by silica gel column chromatography to obtain the desired Intermediate D-3 (138 mg, 90%) as a white solid (138 mg, 90% yield).

¹H NMR (500 MHz, CD3OD) δ 8.34 (s, 1H), 8.30 (s, 1H), 7.94 (d, *J* = 7.8 Hz, 1H), 7.86 (t, *J* = 7.8 Hz, 1H), 7.47 (d, *J* = 7.8 Hz, 1H), 4.89 (m, 1H), 4.61 (m, 1H), 4.07 (d, *J* = 13.2 Hz, 2H), 3.67 (m, 2H), 3.14 - 3.05 (m, 3H), 2.97 (m, 3H), 2.62 (m, 14H), 2.25 (d, *J* = 6.5 Hz, 2H), 1.94 (m, 2H), 1.77 (d, *J* = 12.2 Hz, 3H), 1.47 (s, 9H), 1.15 - 0.99 (m, 2H).

### Step 4: Synthesis of 6-methyl-N-((1s,3s)-3-(6-((3-(4-(piperidin-4-ylmethyl)piperazin-1-yl)propyl)amino)-9H-purin-9-yl)cyclobutyl)picolinamide hydrochloride (Intermediate D-4)

Intermediate D-4 was synthesized in a similar manner to Step 5 of Preparation Example 1 above.

¹H NMR (500 MHz, CD3OD) δ 8.88 (s, 0.5H), 8.73 (s, 0.5H), 8.64 - 8.43 (m, 3H), 8.07 (d, *J* = 7.8 Hz, 1H), 5.17 - 5.01 (m, 1H), 4.64 (m, 1H), 4.29 (m, 1H), 3.84 (m, 9H), 3.58 - 3.42 (m, 4H), 3.21 - 3.02 (m, 6H), 2.91 (s, 3H), 2.37 (m, 3H), 2.24 (d, *J =* 14.2 Hz, 2H), 1.59 (m, 2H).

### Preparation Example 6: Synthesis of 2-phenyl-N-((1s,3s)-3-(6-((4-(4-(3-(piperazin-1-yl)propyl)piperazin-1-yl)phenyl)amino)-9H-purin-9-yl)cyclobutyl)acetamide hydrochloride (Intermediate C-14)

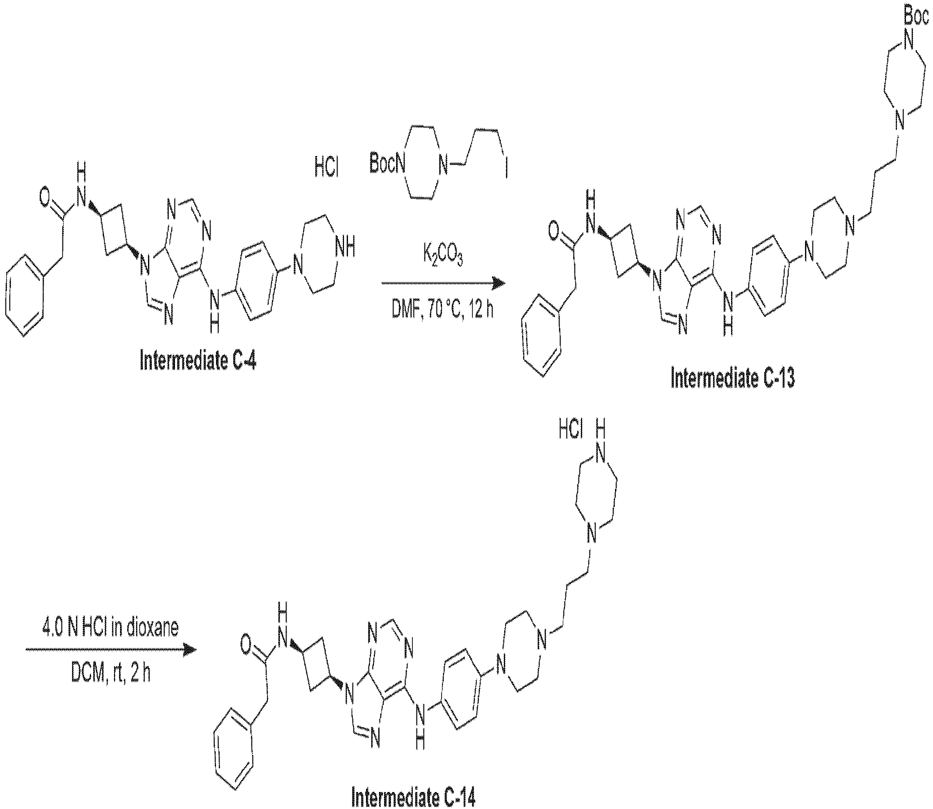

### Step 1: Synthesis of tert-butyl 4-(3-(4-(4-((9-((1s,3s)-3-(2-phenylacetamido)cyclobutyl)-9H-purin-6-yl)amino)phenyl)piperazin-1-yl)propyl)piperazine-1-carboxylate (Intermediate C-13)

Intermediate C-13 was synthesized in a similar manner to Step 1 of Preparation Example 4 above.

¹H NMR (300 MHz, CD3OD) δ 8.32 (s, 1H), 8.25 (s, 1H), 7.65 (d, *J* = 9.0 Hz, 2H), 7.38 - 7.19 (m, 5H), 7.13 - 6.97 (m, 2H), 4.81 (q, *J* = 8.6 Hz, 1H), 4.31 (p, *J* = 8.2 Hz, 1H), 3.55 (s, 2H), 3.47 (t, *J* = 4.8 Hz, 4H), 3.23 (t, *J* = 5.0 Hz, 4H), 3.06 - 2.94 (m, 2H), 2.84 - 2.64 (m, 6H), 2.47 (m, 8H), 1.80 (m, 2H), 1.48 (s, 9H).

### Step 2: Synthesis of 2-phenyl-N-((1s,3s)-3-(6-((4-(4-(3-(piperazin-1-yl)propyl)piperazin-1-yl)phenyl)amino)-9H-purin-9-yl)cyclobutyl)acetamide hydrochloride (Intermediate C-14)

Intermediate C-14 was synthesized in a similar manner to Step 5 of Preparation Example 1 above.

¹H NMR (400 MHz, CD3OD) δ 8.57 (s, 1H), 8.29 (s, 1H), 7.46 (d, *J* = 8.7 Hz, 2H), 7.33 (m, 4H), 7.27 (m, 3H), 5.00 - 4.93 (m, 1H), 4.28 (m, 1H), 4.02 (s, 3H), 3.87 - 3.65 (m, 14H), 3.62 - 3.58 (m, 2H), 3.55 (s, 2H), 3.47 (m, 5H), 3.05 (m, 2H), 2.80 (m, 2H), 2.55 - 2.37 (m, 2H).

### Preparation Example 7: Synthesis of 2-phenyl-N-((1s,3s)-3-(6-((4-(4-(2-(piperidin-4-yloxy)ethyl)piperazin-1-yl)phenyl)amino)-9H-purin-9-yl)cyclobutyl)acetamide hydrochloride (Intermediate C-16)

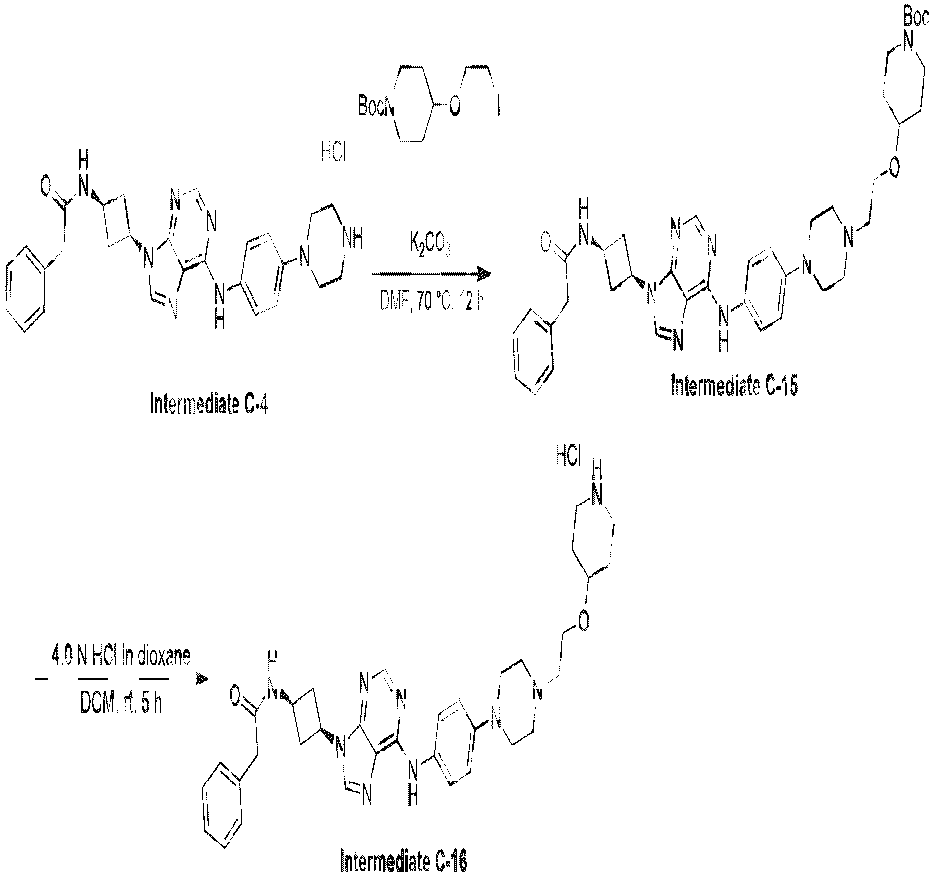

### Step 1: Synthesis of tert-butyl 4-(2-(4-(4-((9-((1s,3s)-3-(2-phenylacetamido)cyclobutyl)-9H-purin-6-yl)amino)phenyl)piperazin-1-yl)ethoxy)piperidine-1-carboxylate (Intermediate C-15)

Intermediate C-13 was synthesized in a similar manner to Step 1 of Preparation Example 4 above.

¹H NMR (300 MHz, CD3OD) δ 8.32 (s, 1H), 8.26 (s, 1H), 7.65 (d, *J* = 8.9 Hz, 2H), 7.41 - 7.23 (m, 5H), 7.04 (d, *J* = 9.1 Hz, 2H), 4.82 (m, 1H), 4.31 (m, 1H), 3.83 - 3.67 (m, 6H), 3.55 (s, 3H), 3.23 (t, *J* = 5.1 Hz, 4H), 3.17 (m, 2H), 3.07 - 2.92 (m, 2H), 2.82 - 2.65 (m, 8H), 1.95 - 1.82 (m, 2H), 1.54 (m, 2H), 1.48 (s, H).

### Step 2: Synthesis of 2-phenyl-N-((1s,3s)-3-(6-((4-(4-(2-(piperidin-4-yloxy)ethyl)piperazin-1-yl)phenyl)amino)-9H-purin-9-yl)cyclobutyl)acetamide hydrochloride (Intermediate C-16)

Intermediate C-14 was synthesized in a similar manner to Step 5 of Preparation Example 1 above.

¹H NMR (400 MHz, CD3OD) δ 8.57 (s, 1H), 8.29 (s, 1H), 7.46 (d, *J* = 8.7 Hz, 2H), 7.33 (m, 4H), 7.28 - 7.22 (m, 3H), 4.95 (q, *J* = 8.2, 7.7 Hz, 1H), 4.28 (h, *J = 7.8,* 7.4 Hz, 1H), 3.99 (t, *J* = 5.0 Hz, 4H), 3.89 - 3.74 (m, 4H), 3.69 (m, 1H), 3.55 (m, 4H), 3.48 - 3.38 (m, 5H), 3.16 (m, 2H), 3.09 - 2.98 (m, 2H), 2.79 (m, 2H), 2.12 (m, 2H), 1.99 (m, 2H).

### Preparation Example 8: Synthesis of 2-phenyl-N-((1s,3s)-3-(6-((4-(4-(3-(piperidin-4-yl)propyl)piperazin-1-yl)phenyl)amino)-9H-purin-9-yl)cyclobutyl)acetamide hydrochloride (Intermediate C-18)

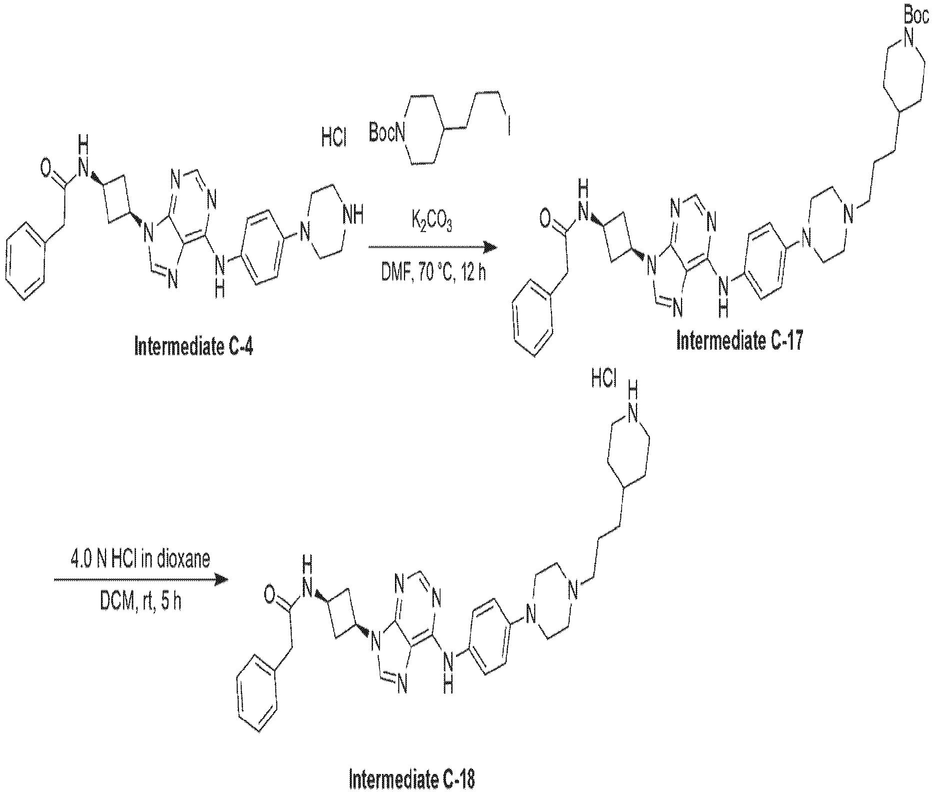

### Step 1: Synthesis of tert-butyl 4-(3-(4-(4-((9-((1s,3s)-3-(2-phenylacetamido)cyclobutyl)-9H-purin-6-yl)amino)phenyl)piperazin-1-yl)propyl)piperidine-1-carboxylate (Intermediate C-17)

Intermediate C-17 was synthesized in a similar manner to Step 1 of Preparation Example 4 above.

1H NMR (300 MHz, CD3OD) δ 8.33 (s, 1H), 8.26 (s, 1H), 7.66 (d, J = 8.9 Hz, 2H), 7.34 (m, 4H), 7.31 - 7.22 (m, 1H), 7.05 (d, J = 9.1 Hz, 2H), 4.81 (q, J = 8.1 Hz, 1H), 4.29 (q, J = 8.2 Hz, 1H), 4.08 (d, J = 12.9 Hz, 2H), 3.55 (s, 2H), 3.24 (t, J = 5.0 Hz, 4H), 3.01 (m, 2H), 2.86 - 2.67 (m, 8H), 2.53 - 2.43 (m, 2H), 1.74 (d, J = 13.0 Hz, 2H), 1.68 - 1.57 (m, 3H), 1.47 (s, 8H), 1.31 (m, 2H), 1.09 (m, 2H).

### Step 2: Synthesis of 2-phenyl-N-((1s,3s)-3-(6-((4-(4-(3-(piperidin-4-yl)propyl)piperazin-1-yl)phenyl)amino)-9H-purin-9-yl)cyclobutyl)acetamide hydrochloride (Intermediate C-18)

Intermediate C-18 was synthesized in a similar manner to Step 1 of Preparation Example 4 above.

¹H NMR (400 MHz, CD3OD) δ 8.59 (s, 1H), 8.30 (s, 1H), 7.46 (d, *J* = 8.8 Hz, 2H), 7.33 (m, 4H), 7.27 (m, 3H), 4.95 (m, 1H), 4.29 (p, *J* = 8.2 Hz, 1H), 4.00 (d, *J = 9.7* Hz, 2H), 3.76 (m, 3H), 3.69 (m, 1H), 3.64 - 3.58 (m, 1H), 3.56 (s, 2H), 3.43 (d, *J* = 12.9 Hz, 2H), 3.31 (m, 1H), 3.26 (m, 2H), 3.09 - 2.97 (m, 4H), 2.80 (m, 2H), 2.03 (d, *J* = 14.1 Hz, 2H), 1.93 (m, 2H), 1.73 (m, 1H), 1.47 (m, 4H).

### Preparation Example 9: Synthesis of N-((1s,3s)-3-(6-((4-(4-(6-azaspiro[3.4]octane-2-yl)piperazin-1-yl)phenyl)amino)-9H-purin-9-yl)cyclobutyl)-2-phenylacetamide hydrochloride (Intermediate C-20)

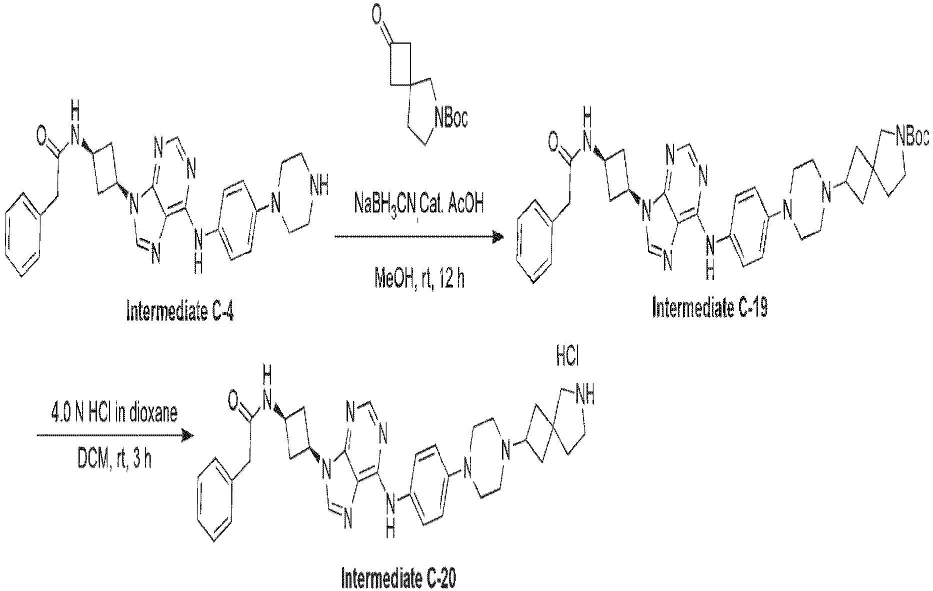

### Step 1: Synthesis of tert-butyl 2-(4-(4-((9-((1s,3s)-3-(2-phenylacetamido)cyclobutyl)-9H-purin-6-yl)amino)phenyl)piperazin-1-yl)-6-azaspiro[3.4]octane-6-carboxylate (Intermediate C-19)

Intermediate C-19 was synthesized in a similar manner to Step 2 of Preparation Example 5 above.

¹H NMR (400 MHz, CD3OD) δ 8.32 (s, 1H), 8.25 (s, 1H), 7.65 (d, *J* = 8.9 Hz, 2H), 7.34 (m, 4H), 7.27 (m, 1H), 7.04 (d, *J* = 8.7 Hz, 2H), 4.81 (m, 1H), 4.31 (p, *J = 8.2* Hz, 1H), 3.55 (s, 2H), 3.37 (m, 3H), 3.24 (m, 5H), 3.07 - 2.95 (m, 2H), 2.87 (m, 1H), 2.77 (m, 2H), 2.59 (t, *J* = 4.9 Hz, 4H), 2.16 (m, 2H), 1.98 (m, 3H), 1.89 (m, 1H), 1.48 (m, 9H).

### Step 2: Synthesis of N-((1s,3s)-3-(6-((4-(4-(6-azaspiro[3.4]octane-2-yl)piperazin-1-yl)phenyl)amino)-9H-purin-9-yl)cyclobutyl)-2-phenylacetamide hydrochloride (Intermediate C-20)

Intermediate C-20 was synthesized in a similar manner to Step 5 of Preparation Example 1 above.

¹H NMR (400 MHz, CD3OD) δ 8.57 (s, 1H), 8.29 (s, 1H), 7.48 - 7.42 (m, 2H), 7.33 (m, 4H), 7.27 (d, *J* = 9.0 Hz, 2H), 5.00 - 4.92 (m, 1H), 4.28 (p, *J* = 8.2 Hz, 1H), 4.07 - 3.86 (m, 4H), 3.62 (m, 2H), 3.55 (s, 2H), 3.42 - 3.35 (m, 4H), 3.16 (t, *J* = 11.8 Hz, 2H), 3.09 - 2.99 (m, 2H), 2.83 - 2.59 (m, 5H), 2.54 (t, *J* = 10.1 Hz, 1H), 2.21 (q, *J* = 7.2 Hz, 2H).

### Preparation Example 10: Synthesis of N-((1s,3s)-3-(6-((4-(4-(7-(azetidin-3-yl)-7-azaspiro[3.5]nonane-2-yl)piperazin-1-yl)phenyl)amino)-9H-purin-9-yl)cyclobutyl)-2-phenylacetamide hydrochloride (Intermediate C-24)

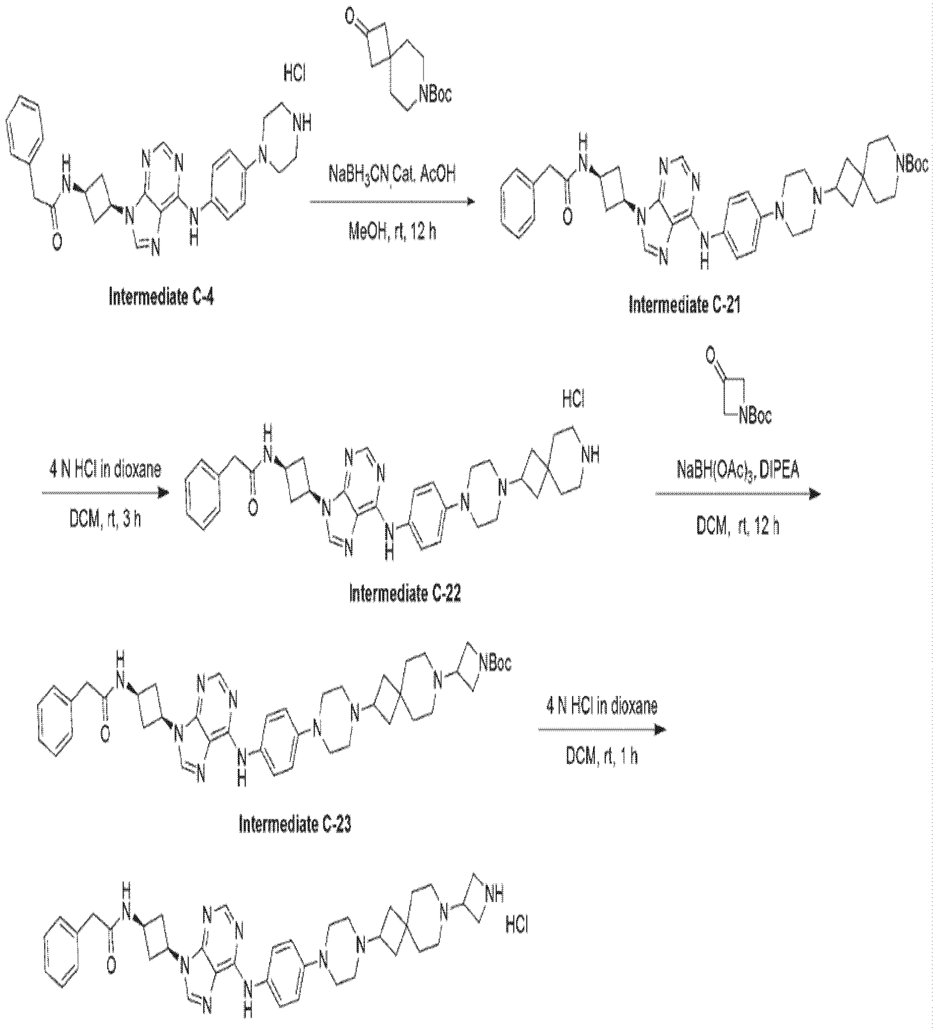

### Step 1: Synthesis of tert-butyl 2-(4-(4-((9-((1s,3s)-3-(2-phenylacetamido)cyclobutyl)-9H-purin-6-yl)amino)phenyl)piperazin-1-yl)-7-azaspiro[3.5]nonane-7-carboxylate (Intermediate C-21)

Intermediate C-21 was synthesized in a similar manner to Step 2 of Preparation Example 5 above.

¹H NMR (500 MHz, DMSO-d₆) δ 9.65 (s, 1H), 8.47 (d, *J* = 7.7 Hz, 1H), 8.40 (s, 1H), 8.32 (s, 1H), 7.72 (d, *J* = 8.7 Hz, 2H), 7.34 - 7.20 (m, 5H), 6.91 (d, *J* = 9.0 Hz, 2H), 4.76 (p, *J* = 8.6 Hz, 1H), 4.16 (h, *J* = 8.2 Hz, 1H), 3.43 (s, 2H), 3.29 (t, *J* = 5.7 Hz, 2H), 3.20 (m,2H), 3.09 (m, 4H), 2.85 (m, 2H), 2.71 (t, *J* = 7.7 Hz, 1H), 2.67 - 2.57 (m, 2H), 2.38 (m, 4H), 1.98 (t, *J =* 9.4 Hz, 2H), 1.57 (t, *J* = 9.9 Hz, 2H), 1.49 (t, *J* = 5.6 Hz, 2H).

### Step 2: Synthesis of N-((1s,3s)-3-(6-((4-(4-(7-azaspiro[3.5]nonane-2-yl)piperazin-1-yl)phenyl)amino)-9H-purin-9-yl)cyclobutyl)-2-phenylacetamide hydrochloride (Intermediate C-22)

Intermediate C-22 was synthesized in a similar manner to Step 5 of Preparation Example 1 above.

¹H NMR (500 MHz, DMSO-d₆) δ 11.71 (s, 1H), 10.33 (s, 1H), 8.79 (s, 2H), 8.68 (s, 1H), 8.60 (d, *J* = 7.6 Hz, 1H), 8.38 (s, 1H), 7.71 (d, *J* = 8.3 Hz, 2H), 7.35 - 7.28 (m, 4H), 7.24 (t, *J* = 6.8 Hz, 1H), 7.05 (d, *J* = 8.8 Hz, 2H), 4.80 (p, *J* = 8.5 Hz, 1H), 4.17 (h, *J* = 8.1 Hz, 1H), 3.81 (d, *J =* 12.9 Hz, 4H), 3.44 (s, 3H), 3.41 (m, 1H), 3.18 (t, *J* = 12.4 Hz, 2H), 3.08 - 2.93 (m, 6H), 2.92 - 2.83 (m, 2H), 2.65 (m, 2H), 2.34 (m, 2H), 2.22 (t, *J* = 10.1 Hz, 2H), 1.82 (t, *J* = 5.7 Hz, 2H), 1.75 (t, *J* = 5.7 Hz, 2H).

### Step 3: Synthesis of tert-butyl 3-(2-(4-(4-((9-((1s,3s)-3-(2-phenylacetamido)cyclobutyl)-9H-purin-6-yl)amino)phenyl)piperazin-1-yl)-7-azaspiro[3.5]nonane-7-yl)azetidine-1-carboxylate (Intermediate C-23)

Intermediate C-23 was synthesized in a similar manner to Step 4 of Preparation Example 1 above.

¹H NMR (400 MHz, CD3OD) δ 8.32 (s, 1H), 8.25 (s, 1H), 7.66 (d, *J* = 8.8 Hz, 2H), 7.40 - 7.18 (m, 5H), 7.04 (d, *J* = 8.8 Hz, 2H), 4.81 (q, *J* = 8.3 Hz, 1H), 4.31 (m, 1H), 3.97 (m, 2H), 3.78 (m, 2H), 3.55 (s, 2H), 3.24 (m, 4H), 3.13 - 2.95 (m, 3H), 2.90 (t, *J =* 8.0 Hz, 1H), 2.76 (m, 2H), 2.62 (m, 4H), 2.47 - 2.21 (m, 2H), 2.09 (m, 2H), 1.73 (m, 4H), 1.65 (t, *J* = 5.5 Hz, 2H), 1.46 (s, 9H), 1.39 (d, *J* = 6.4 Hz, 2H).

### Step 4: Synthesis of N-((1s,3s)-3-(6-((4-(4-(7-(azetidin-3-yl)-7-azaspiro[3.5]nonane-2-yl)piperazin-1-yl)phenyl)amino)-9H-purin-9-yl)cyclobutyl)-2-phenylacetamide hydrochloride (Intermediate C-24)

Intermediate C-24 was synthesized in a similar manner to Step 5 of Preparation Example 1 above.

¹H NMR (400 MHz, CD3OD) δ 8.55 (s, 1H), 8.28 (s, 1H), 7.45 (d, *J* = 8.8 Hz, 2H), 7.38 - 7.18 (m, 7H), 4.95 (m, 2H), 4.71 (m, 2H), 4.47 - 4.35 (m, 3H), 4.28 (p, *J =* 8.4 Hz, 1H), 4.00 (d, *J* = 13.4 Hz, 2H), 3.89 (m, 1H), 3.76 (t, *J* = 5.7 Hz, 1H), 3.72 - 3.58 (m, 6H), 3.55 (s, 2H), 3.19 - 2.99 (m, 5H), 2.79 (m, 2H), 2.58 (m, 1H), 2.39 (m, 2H), 2.23 - 2.05 (m, 4H).

### Preparation Example 11: Synthesis of N-((1s,3s)-3-(6-((4-(4-(7-(azetidin-3-ylmethyl)-7-azaspiro[3.5]nonane-2-yl)piperazin-1-yl)phenyl)amino)-9H-purin-9-yl)cyclobutyl)-2-phenylacetamide hydrochloride (Intermediate C-25)

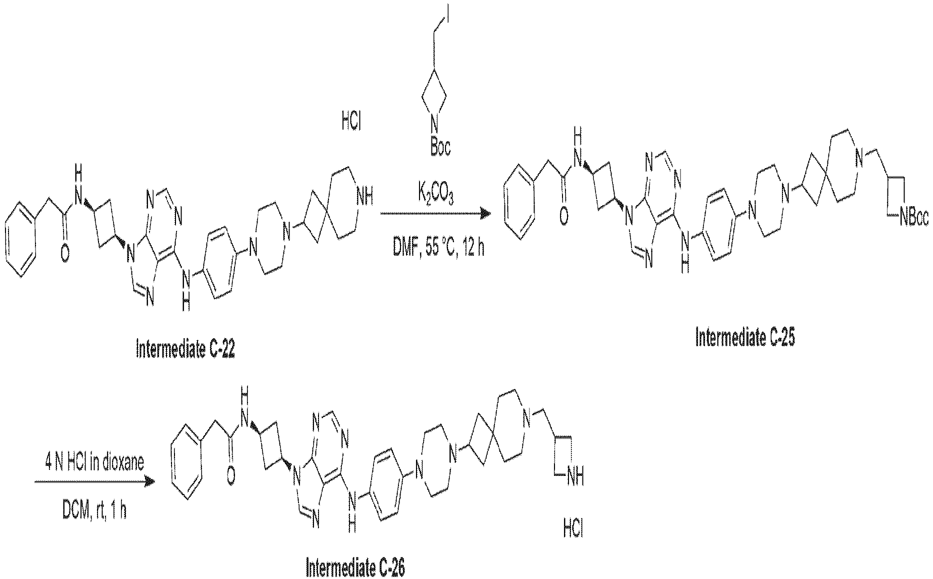

### Step 1: Synthesis of tert-butyl 3-((2-(4-(4-((9-((1s,3s)-3-(2-phenylacetamido)cyclobutyl)-9H-purin-6-yl)amino)phenyl)piperazin-1-yl)-7-azaspiro[3.5]nonane-7-yl)methyl)azetidine-1-carboxylate (Intermediate C-25)

Intermediate C-25 was synthesized in a similar manner to Step 1 of Preparation Example 4 above.

¹H NMR (300 MHz, CD3OD) δ 8.32 (s, 1H), 8.26 (s, 1H), 7.65 (d, *J* = 9.0 Hz, 2H), 7.34 (m, 4H), 7.27 (m, 1H), 7.04 (d, *J* = 9.0 Hz, 2H), 4.83 (m, 1H), 4.63 (s, 4H), 4.31 (p, *J* = 8.5 Hz, 1H), 4.06 (t, *J* = 8.4 Hz, 2H), 3.69 - 3.59 (m, 2H), 3.55 (s, 2H), 3.23 (m, 4H), 3.01 (m, 2H), 2.89 - 2.66 (m, 6H), 2.59 (m, 4H), 2.46 (m, 2H), 2.16 - 2.00 (m, 2H), 1.74 (m, 3H), 1.66 (t, *J* = 5.8 Hz, 2H), 1.45 (s, 9H).

### Step 2: Synthesis of N-((1s,3s)-3-(6-((4-(4-(7-(azetidin-3-ylmethyl)-7-azaspiro[3.5]nonane-2-yl)piperazin-1-yl)phenyl)amino)-9H-purin-9-yl)cyclobutyl)-2-phenylacetamide (Intermediate C-26)

Intermediate C-26 was synthesized in a similar manner to Step 5 of Preparation Example 1 above.

¹H NMR (400 MHz, CD3OD) δ 8.56 (s, 1H), 8.28 (s, 1H), 7.46 (d, *J* = 8.5 Hz, 2H), 7.33 (m, 4H), 7.26 (d, *J =* 8.6 Hz, 3H), 4.89 (m, 2H), 4.26 (m, 4H), 4.15 - 3.95 (m, 4H), 3.90 (m, 1H), 3.76 (m, 1H), 3.72 - 3.60 (m, 5H), 3.54 (m, 4H), 3.45 (m, 2H), 3.10 (m, 6H), 2.79 (m, 2H), 2.61 (m, 1H), 2.40 (m, 3H), 2.08 (m, 4H).

### Preparation Example 12: Synthesis of 2-phenyl-N-((1s,3s)-3-(6-((4-(1-(piperidin-4-ylmethyl)piperidin-4-yl)phenyl)amino)-9H-purin-9-yl)cyclobutyl)acetamide hydrochloride (Intermediate E-4)

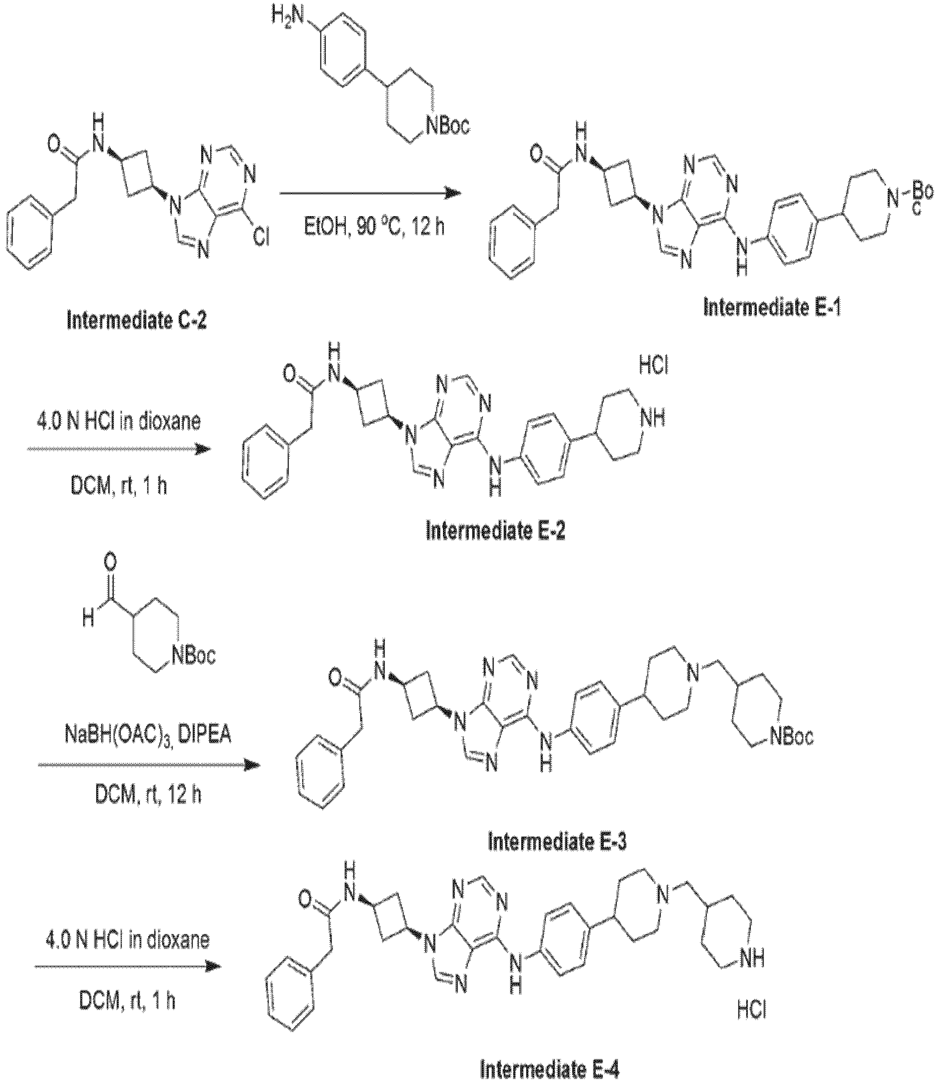

### Step 1: Synthesis of tert-butyl 4-(4-((9-((1s,3s)-3-(2-phenylacetamido)cyclobutyl)-9H-purin-6-yl)amino)phenyl)piperidine-1-carboxylate (Intermediate E-1)

Intermediate E-1 was synthesized in a similar manner to Step 2 of Preparation Example 1 above.

¹H NMR (500 MHz, DMSO-d₆) δ 9.83 (s, 1H), 8.48 (d, *J* = 7.7 Hz, 1H), 8.45 (s, 1H), 8.38 (s, 1H), 7.85 (d, *J* = 8.6 Hz, 2H), 7.39 - 7.15 (m, 7H), 4.78 (p, *J* = 8.6 Hz, 1H), 4.16 (q, *J* = 8.1 Hz, 1H), 4.08 (m, 2H), 3.44 (s, 2H), 2.92 - 2.74 (m, 4H), 2.72 - 2.59 (m, 3H), 1.76 (d, *J* = 12.8 Hz, 2H), 1.50 (m, 2H), 1.43 (s, 9H).

### Step 2: Synthesis of 2-phenyl-N-((1s,3s)-3-(6-((4-(piperidin-4-yl)phenyl)amino)-9H-purin-9-yl)cyclobutyl)acetamide hydrochloride (Intermediate E-2)

Intermediate E-2 was synthesized in a similar manner to Step 3 of Preparation Example 1 above.

¹H NMR (400 MHz, CD3OD) δ 8.36 (s, 1H), 8.26 (s, 1H), 7.77 - 7.68 (m, 2H), 7.38 - 7.22 (m, 7H), 4.85 - 4.74 (m, 1H), 4.30 (p, *J* = 8.3 Hz, 1H), 3.54 (s, 2H), 3.21 - 3.13 (m, 2H), 3.00 (m, 2H), 2.76 (m, 4H), 2.71 - 2.62 (m, 1H).

### Step 3: Synthesis of tert-butyl 4-((4-(4-((9-((1s,3s)-3-(2-phenylacetamido)cyclobutyl)-9H-purin-6-yl)amino)phenyl)piperidin-1-yl)methyl)piperidine-1-carboxylate (Intermediate E-3)

Intermediate E-3 was synthesized in a similar manner to Step 4 of Preparation Example 1 above.

¹H NMR (400 MHz, CD3OD) δ 8.37 (s, 1H), 8.28 (s, 1H), 7.74 (d, *J* = 8.5 Hz, 2H), 7.34 (m, 4H), 7.28 (m, 3H), 4.85 - 4.76 (m, 1H), 4.30 (h, *J = 7.9* Hz, 1H), 4.11 (d, *J* = 13.2 Hz, 2H), 3.55 (s, 2H), 3.19 (d, *J* = 11.5 Hz, 2H), 3.08 - 2.98 (m, 2H), 2.77 (m, 2H), 2.64 (m, 1H), 2.44 (d, *J* = 6.7 Hz, 2H), 2.31 (m, 2H), 1.93 - 1.74 (m, 7H), 1.48 (s, 9H), 1.14 (m, 2H).

### Step 4: Synthesis of 2-phenyl-N-((1s,3s)-3-(6-((4-(1-(piperidin-4-ylmethyl)piperidin-4-yl)phenyl)amino)-9H-purin-9-yl)cyclobutyl)acetamide hydrochloride (Intermediate E-4)

Intermediate E-4 was synthesized in a similar manner to Step 5 of Preparation Example 1 above.

¹H NMR (500 MHz, CD3OD) δ 8.61 (s, 1H), 8.32 (s, 1H), 7.58 (d, *J* = 8.6 Hz, 2H), 7.54 (d, *J* = 8.5 Hz, 2H), 7.34 (m, 4H), 7.30 - 7.23 (m, 1H), 4.99 - 4.93 (m, 1H), 4.33 - 4.23 (m, 1H), 3.82 (d, *J* = 12.0 Hz, 2H), 3.56 (s, 2H), 3.49 (d, *J* = 12.9 Hz, 2H), 3.28 - 3.18 (m, 4H), 3.16 - 2.99 (m, 5H), 2.81 (m, 2H), 2.41 - 2.29 (m, 3H), 2.19 (t, *J =* 16.3 Hz, 4H), 1.61 (m, 2H).

### Preparation Example 13: Synthesis of N-((1s,3s)-3-(6-((4-(1-(7-azaspiro[3.5]nonane-2-yl)piperidin-4-yl)phenyl)amino)-9H-purin-9-yl)cyclobutyl)-2-phenylacetamide hydrochloride (Intermediate E-6)

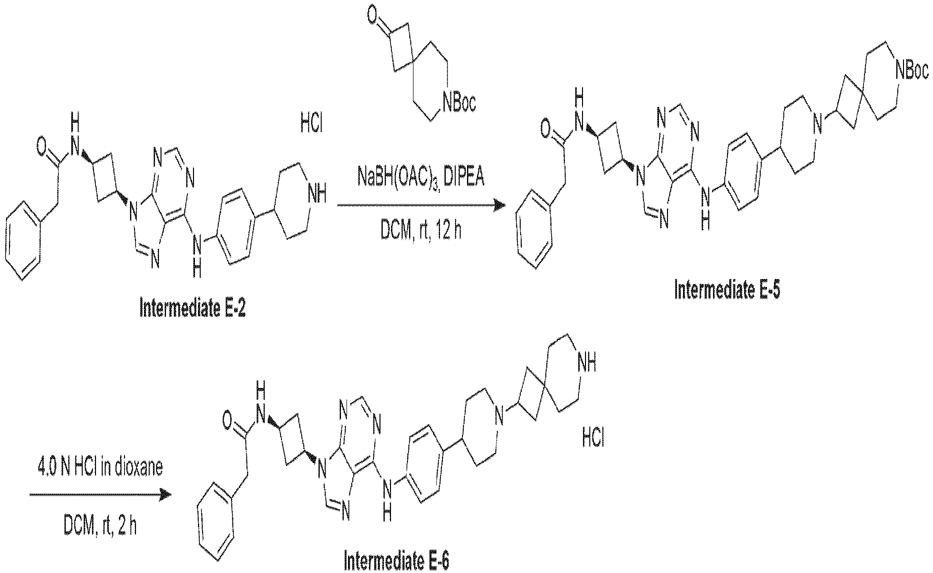

### Step 1: Tert-butyl 2-(4-(4-((9-((1s,3s)-3-(2-phenylacetamido)cyclobutyl)-9H-purin-6-yl)amino)phenyl)piperidin-1-yl)-7-azaspiro[3.5]nonane-7-carboxylate (Intermediate E-5)

Intermediate E-5 was synthesized in a similar manner to Step 4 of Preparation Example 1 above.

¹H NMR (400 MHz, CD3OD) δ 8.38 (s, 1H), 8.28 (s, 1H), 7.77 (d, *J* = 8.6 Hz, 2H), 7.34 (m, 4H), 7.32 - 7.24 (m, 3H), 4.85 - 4.78 (m, 1H), 4.36 - 4.25 (m, 1H), 3.55 (s, 2H), 3.42 (m, 1H), 3.37 (m, 2H), 3.26 (d, *J* = 11.3 Hz, 2H), 3.06 - 2.96 (m, 2H), 2.77 (m, H), 2.33 (m, 2H), 2.21 (t, *J* = 10.1 Hz, 2H), 2.06 - 1.77 (m, 7H), 1.63 (t, *J* = 5.6 Hz, 2H), 1.59 - 1.54 (m, 2H), 1.47 (s, 9H).

### Step 2: Synthesis of N-((1s,3s)-3-(6-((4-(1-(7-azaspiro[3.5]nonane-2-yl)piperidin-4-yl)phenyl)amino)-9H-purin-9-yl)cyclobutyl)-2-phenylacetamide hydrochloride (Intermediate E-6)

Intermediate E-6 was synthesized in a similar manner to Step 5 of Preparation Example 1 above.

¹H NMR (500 MHz, CD3OD) δ 8.55 (s, 1H), 8.27 (s, 1H), 7.53 (d, *J* = 8.5 Hz, 2H), 7.50 (d, *J* = 8.6 Hz, 2H), 7.30 (m, 4H), 7.23 (m, 1H), 4.91 (m, 1H), 4.24 (p, *J = 8.3* Hz, 1H), 3.80 - 3.71 (m, 1H), 3.61 (d, *J* = 11.9 Hz, 2H), 3.51 (s, 2H), 3.18 (t, *J* = 5.9 Hz, 2H), 3.12 (t, *J =* 5.7 Hz, 2H), 3.06 - 2.93 (m, 5H), 2.76 (m, 2H), 2.41 (t, *J* = 10.3 Hz, 2H), 2.34 (t, *J* = 10.6 Hz, 2H), 2.17 (m, 4H), 1.92 (m, 4H).

### Example 13: Synthesis of N-((1s,3s)-3-(6-(4-(4-((1-(3-(2,4-dioxotetrahydropyrimidin-1(2H)-yl)-4-methoxybenzoyl)piperidin-4-yl)methyl)piperazin-1-yl)phenylamino)-9H-purin-9-yl)cyclobutyl)-2-phenylacetamide (Compound 186)

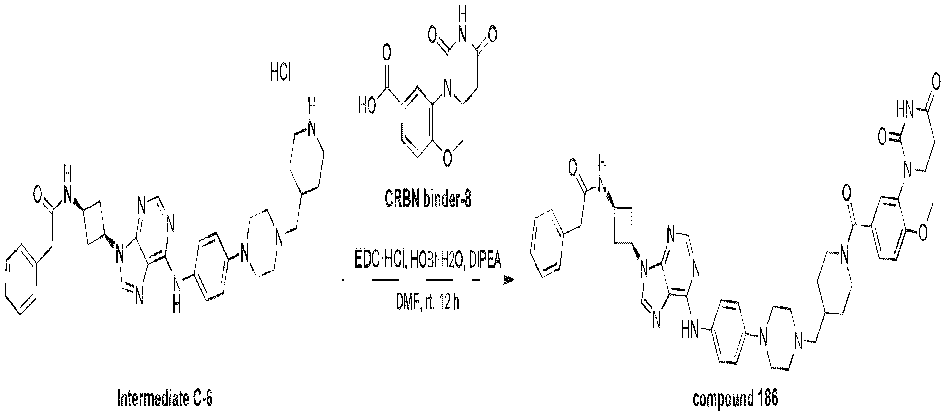

Reaction was carried out in a similar manner to Step 3 of Example 1 above, except that Intermediate C-6 (10 mg, 0.0162 mmol) and 3-(2,4-dioxotetrahydropyrimidin-1(2H)-yl)-4-methoxybenzoic acid (CRBN Binder 8; 5.2 mg, 0.0194 mmol) were used as reactants. The reaction mixture was separated and purified to obtain the desired Compound 186 (9 mg, 69%) as a white solid.

¹H NMR (500 MHz, DMSO-d₆) δ 10.37 (s, 1H), 9.86 (s, 2H), 8.56 - 8.40 (m, 2H), 8.35 (s, 0.5H), 8.23 (d, *J* = 2.5 Hz, 0.5H), 7.78 (d, *J* = 8.5 Hz, 1H), 7.43 - 7.15 (m, 8H), 7.02 (d, *J* = 8.6 Hz, 1H), 4.85 - 4.68 (m, 1H), 4.4 (m,1H), 4.16 (m, 1H), 3.86 (s, 3H), 3.76 (d, *J* = 10.9 Hz, 2H), 3.61 (t, *J* = 6.8 Hz, 4H), 3.15 (m, 7H), 2.93 - 2.81 (m, 2H), 2.73 - 2.60 (m, 5H), 2.18 (m, 1H), 1.88 (m, 3H), 1.23 (m, 4H).

Compounds 187 to 479 were synthesized in the same or similar manner to Compound 186, using the corresponding starting materials and CRBN binders.

### Experimental Example 1: Compound Identification

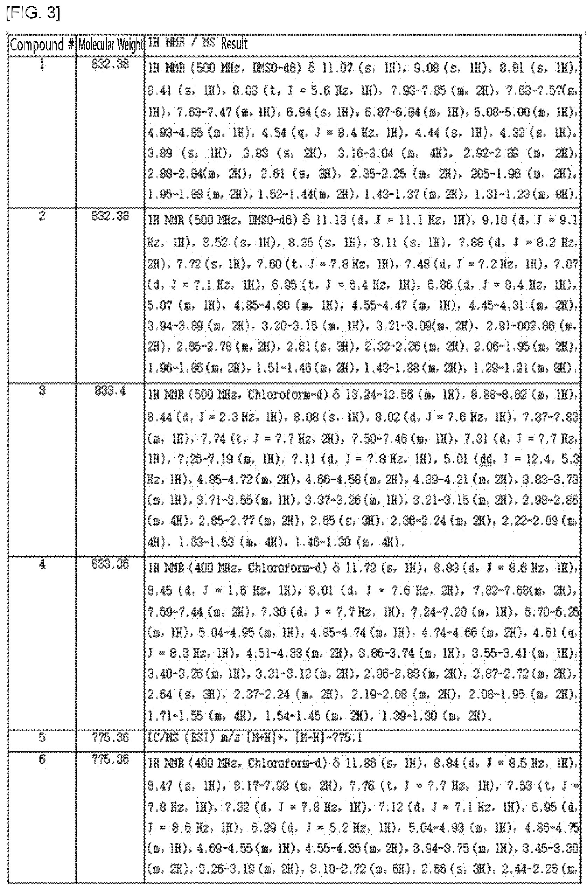

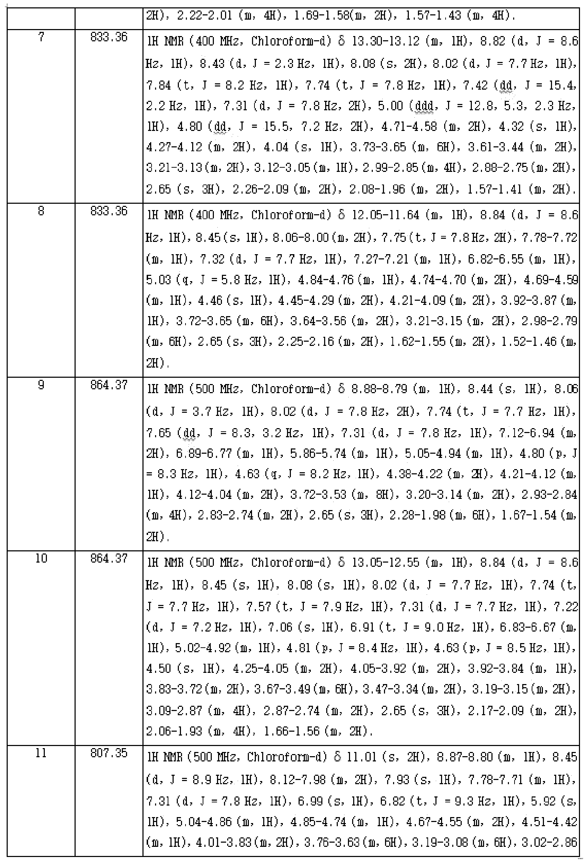

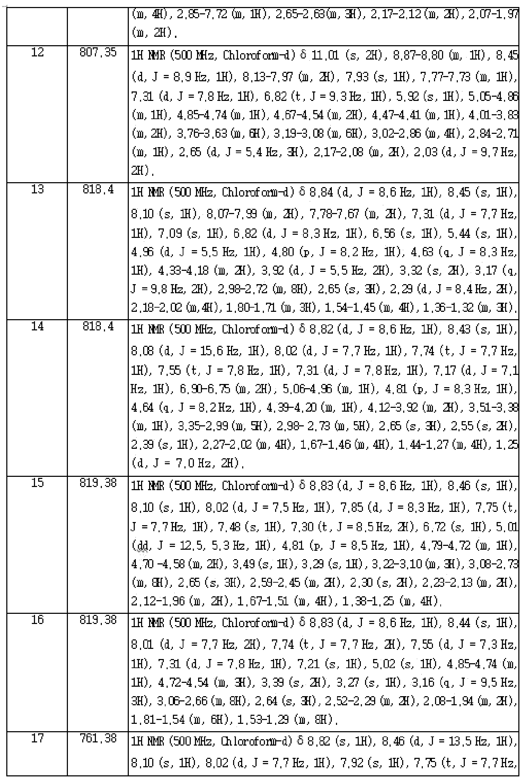

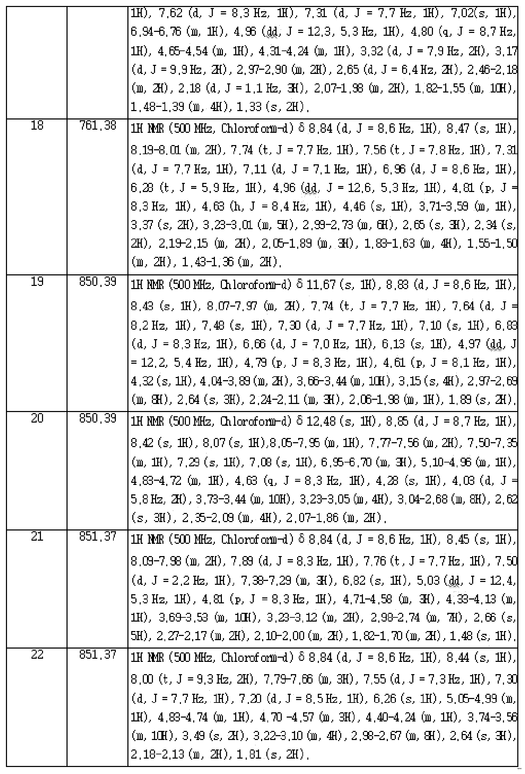

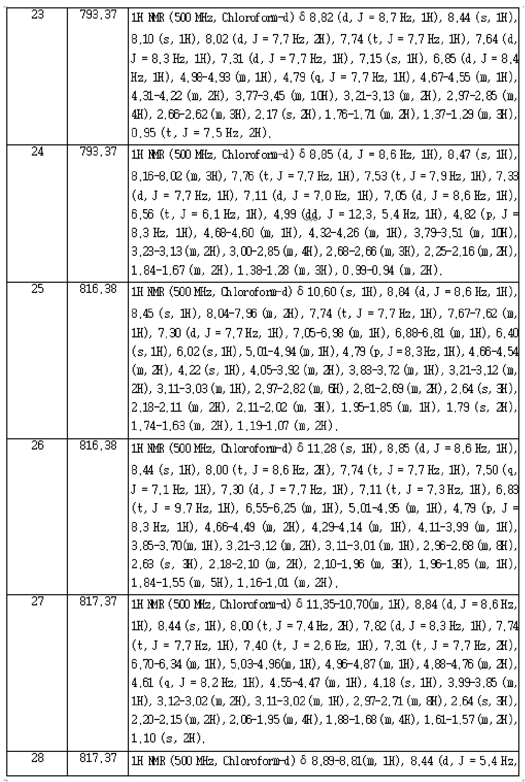

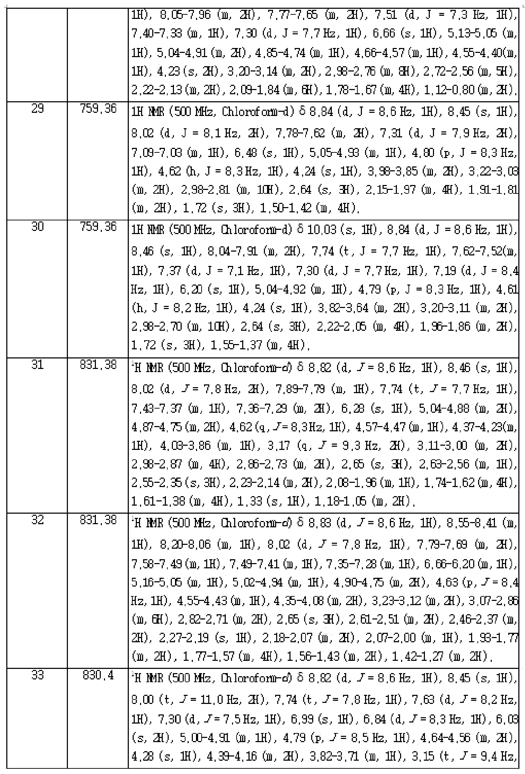

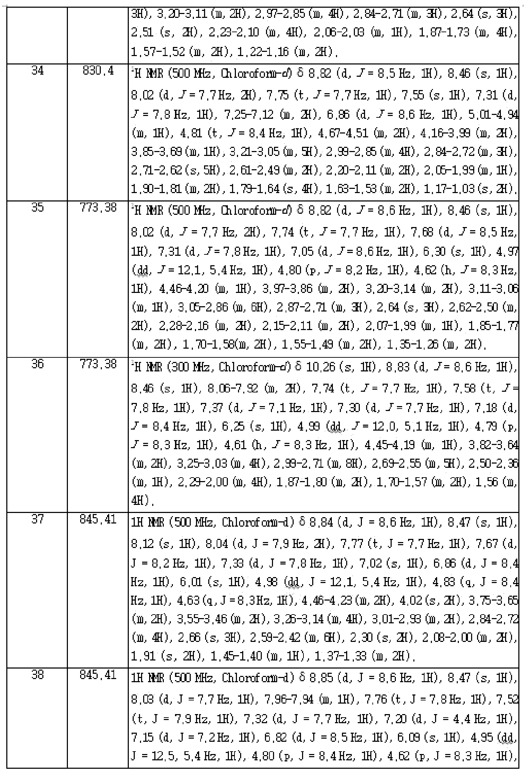

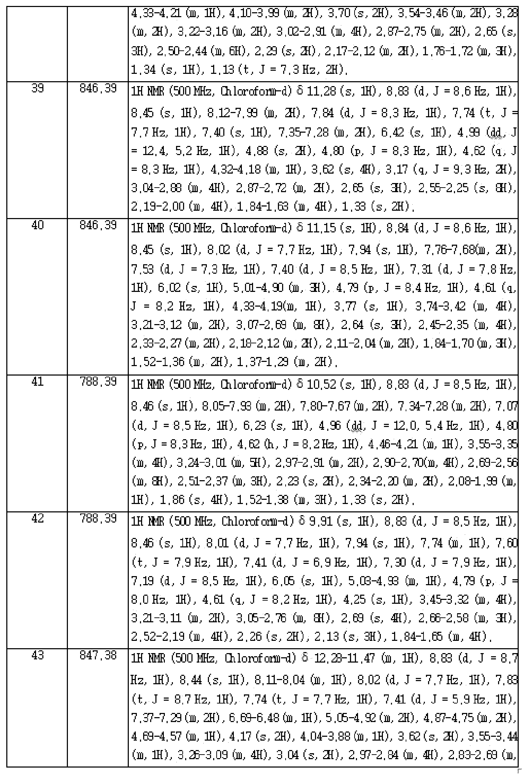

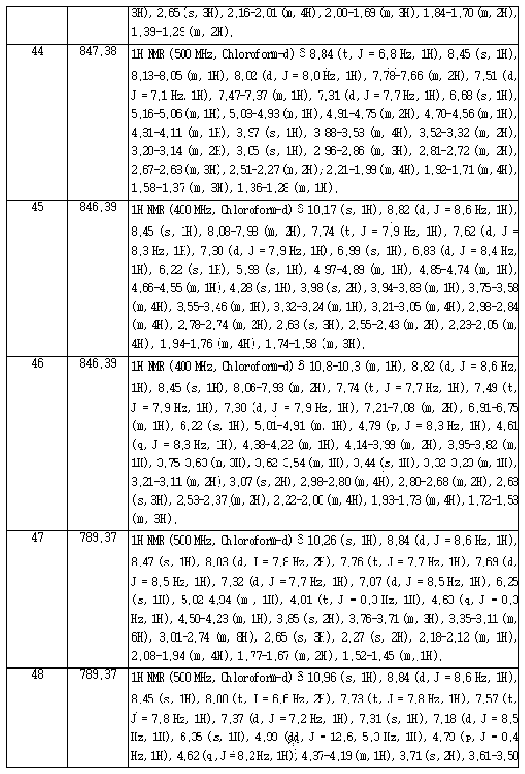

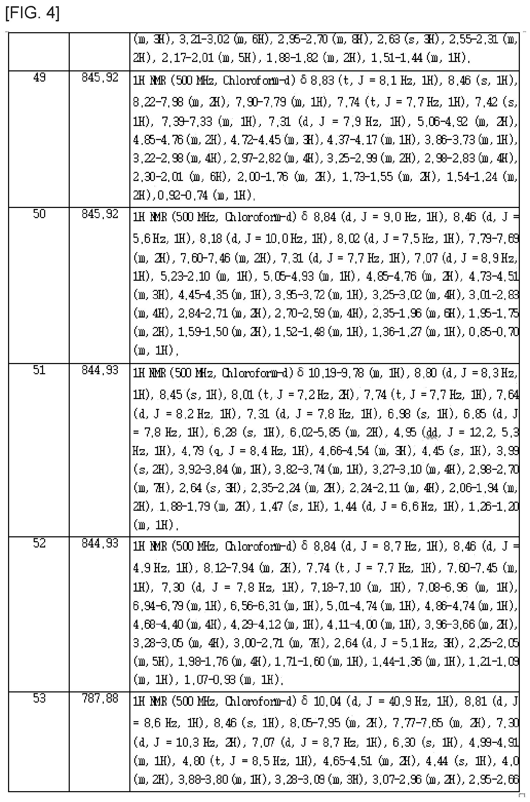

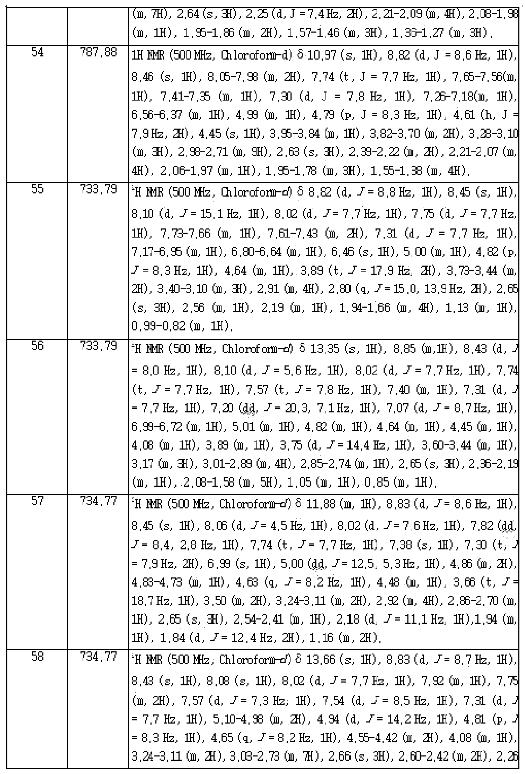

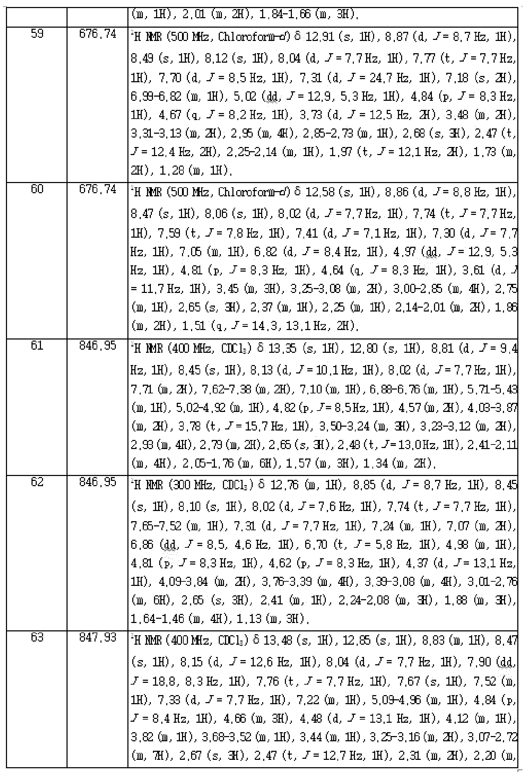

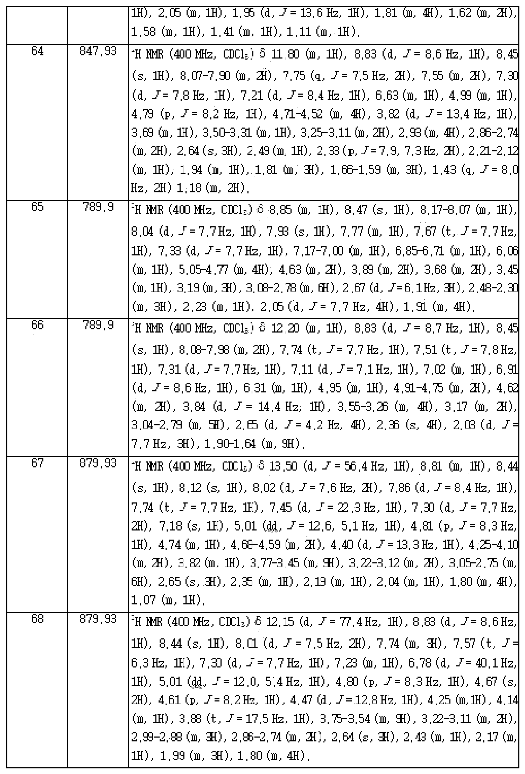

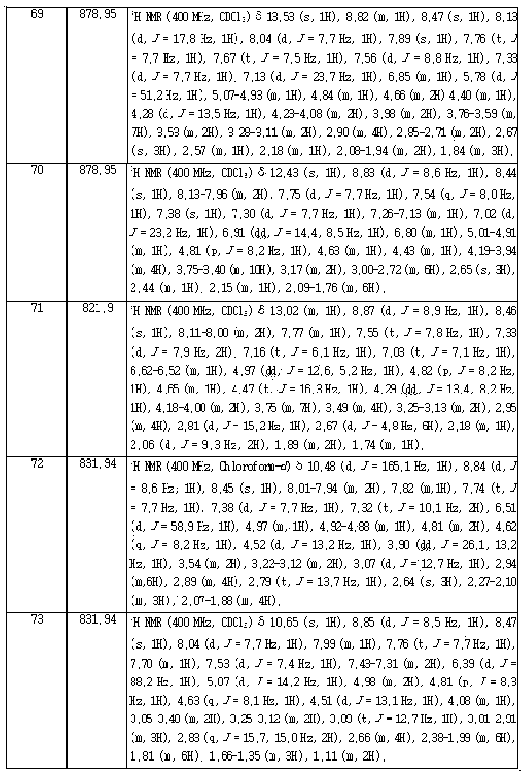

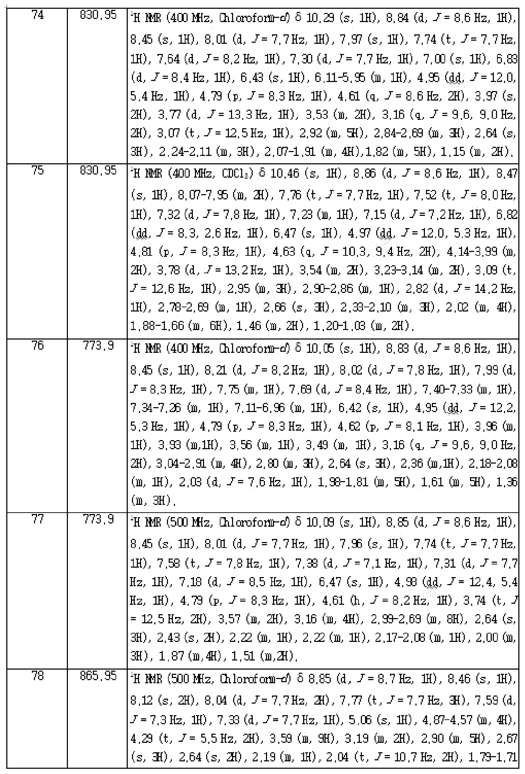

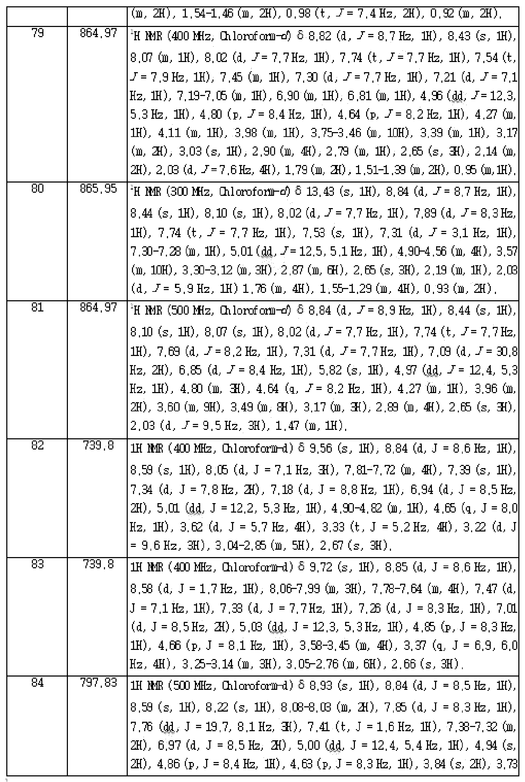

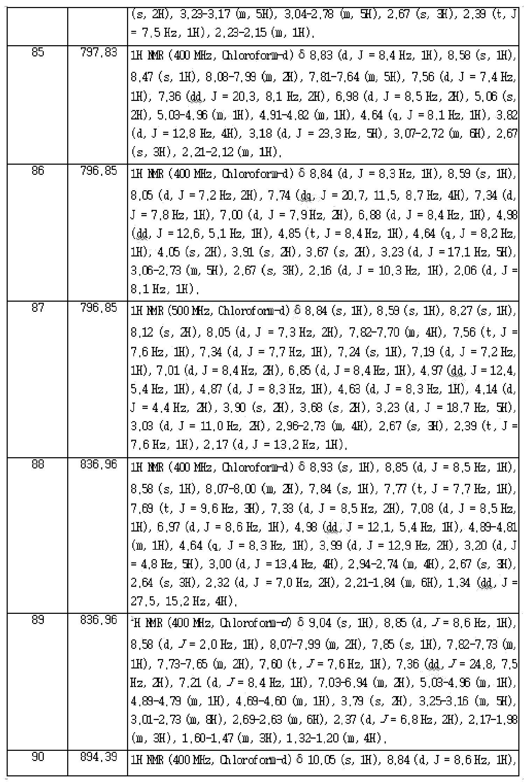

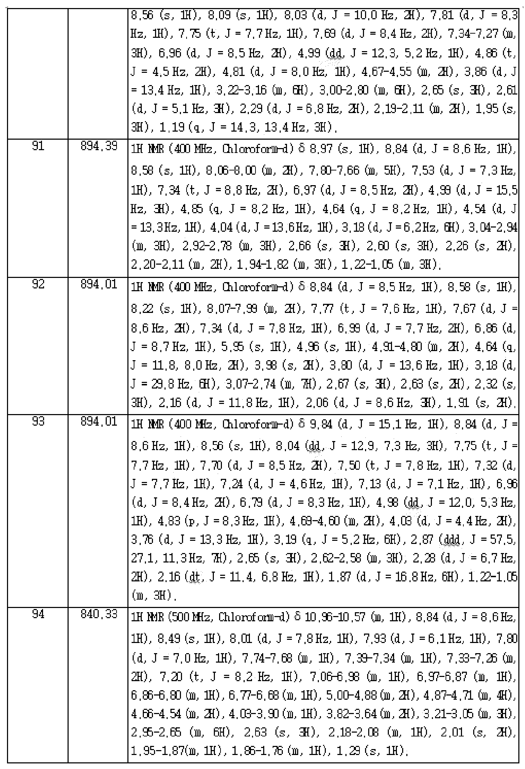

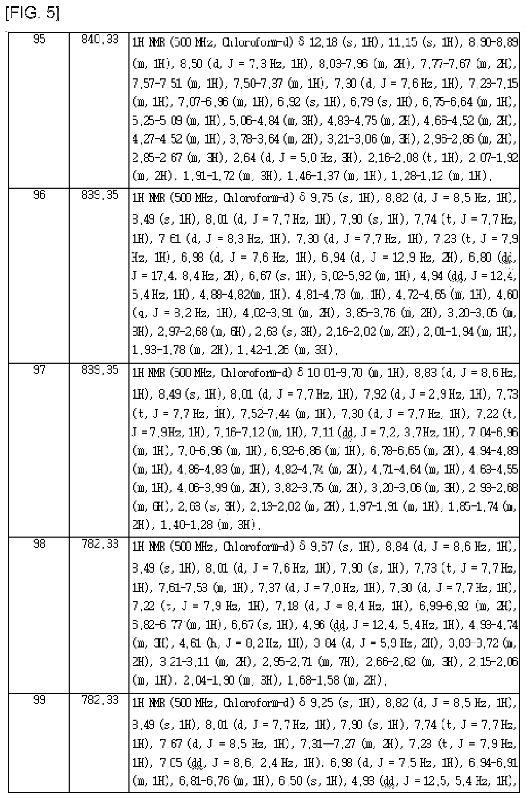

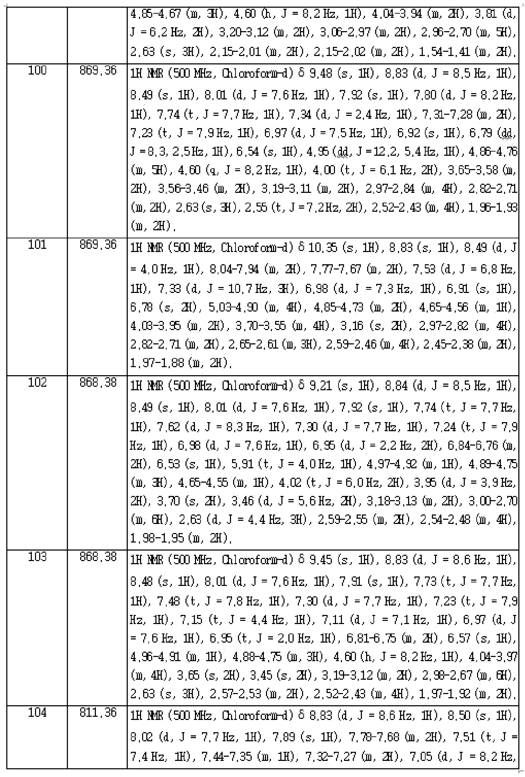

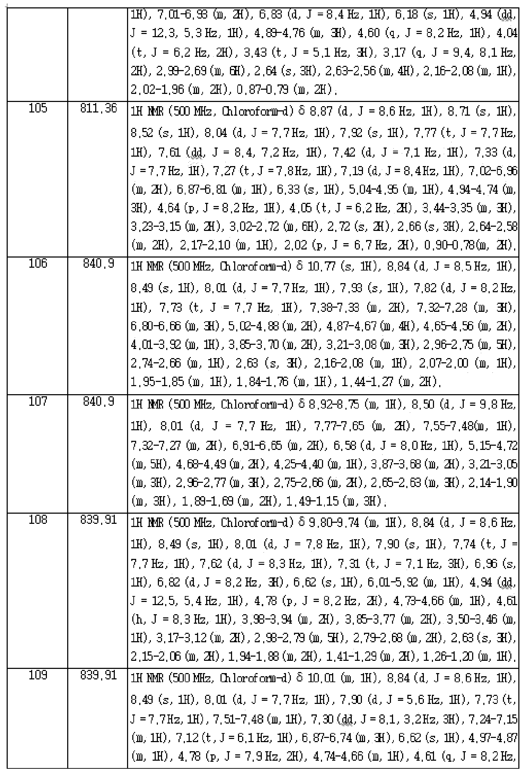

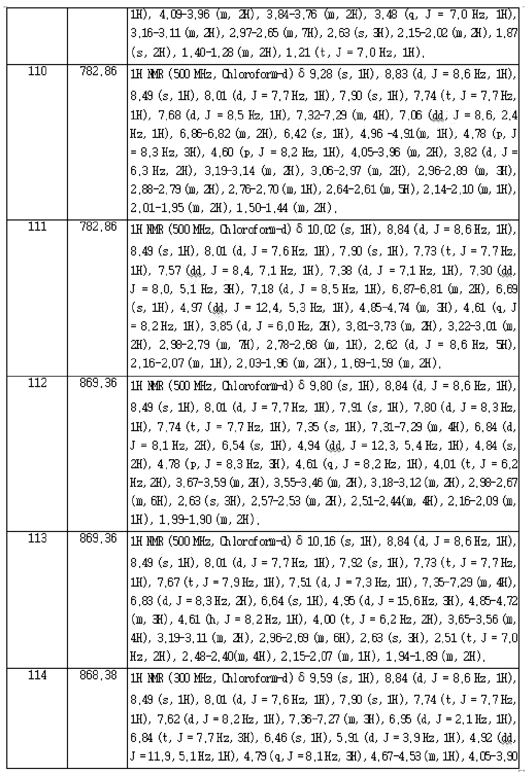

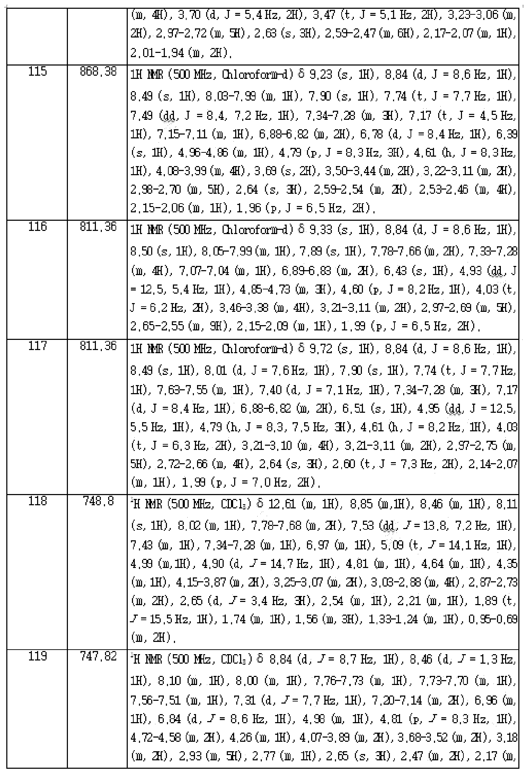

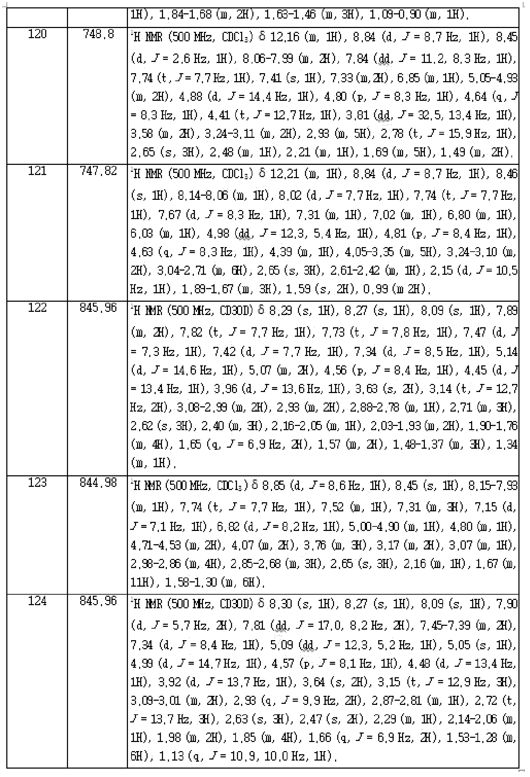

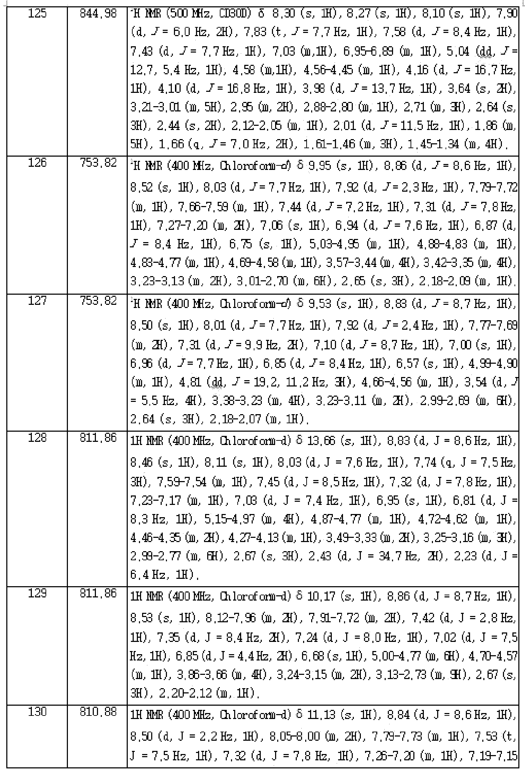

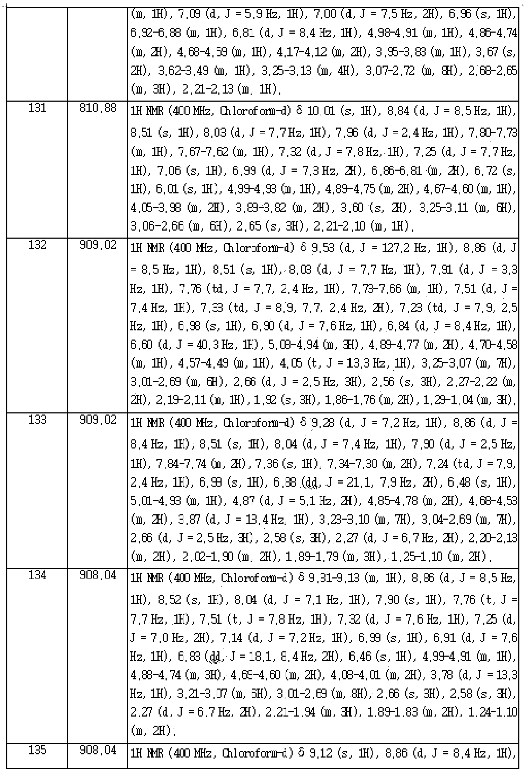

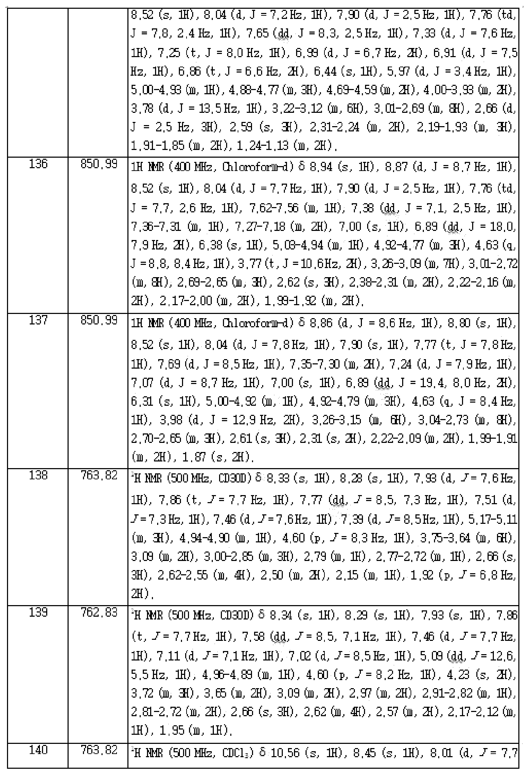

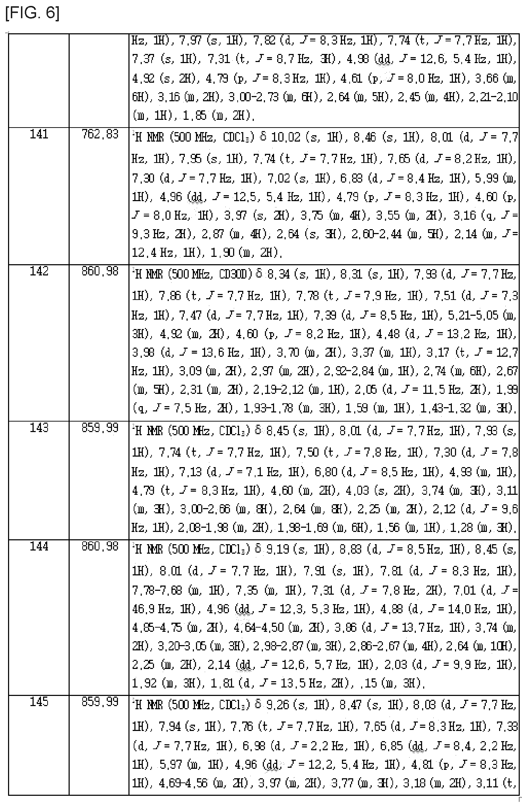

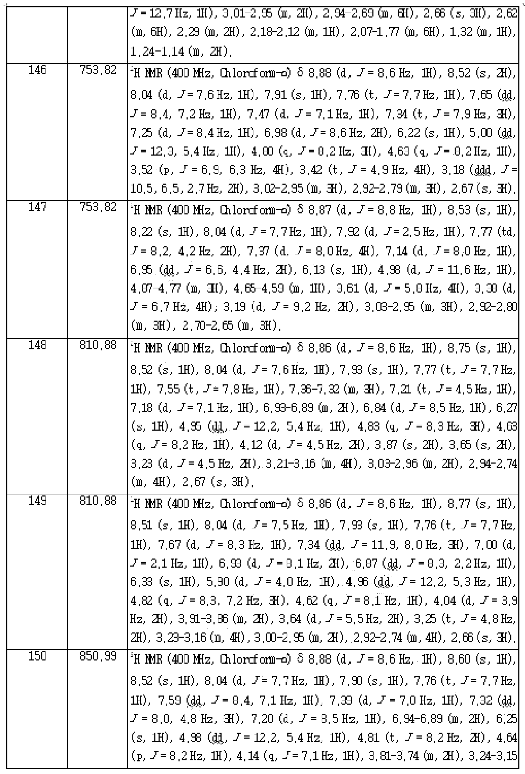

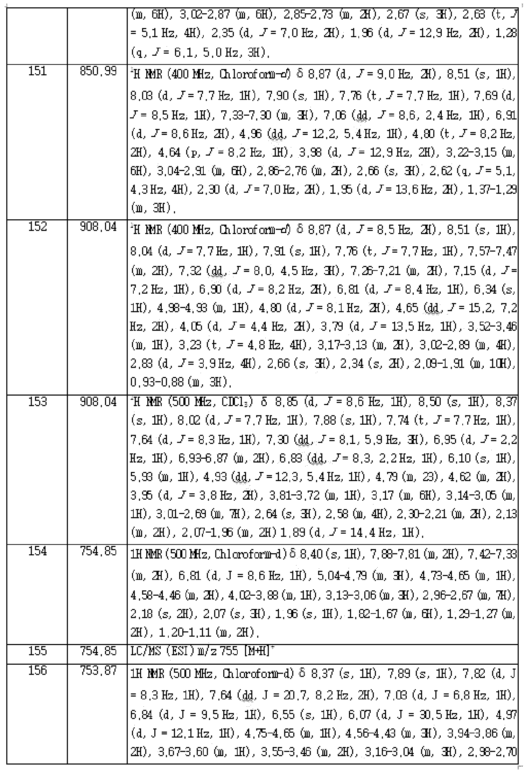

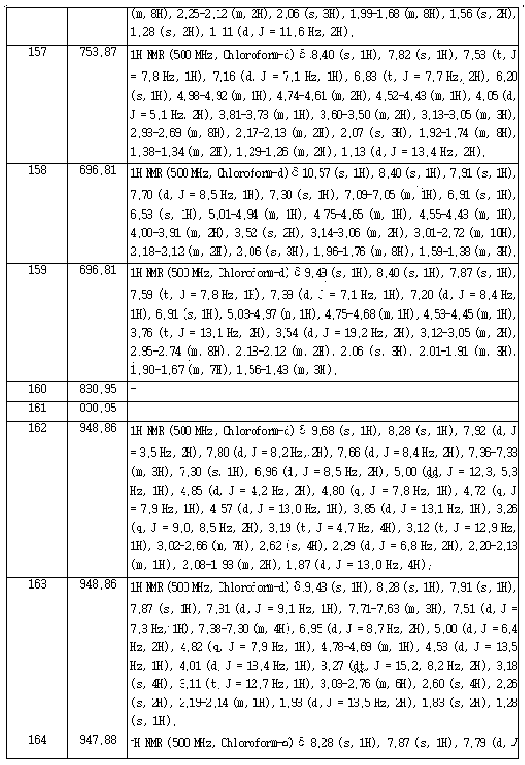

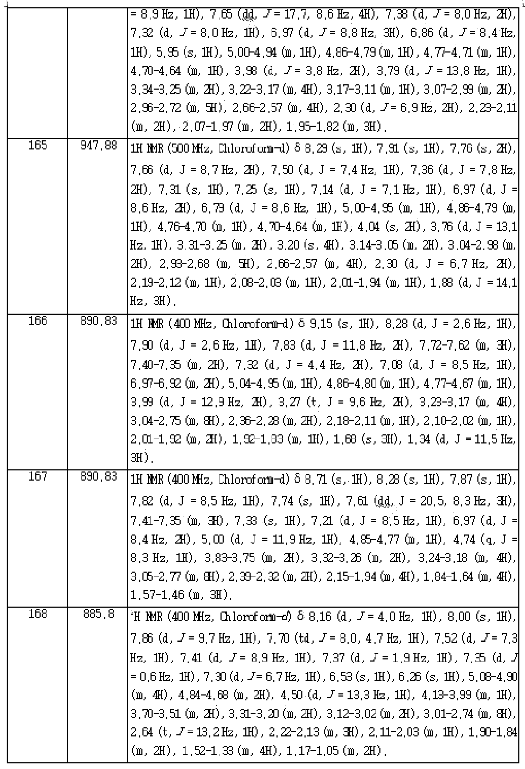

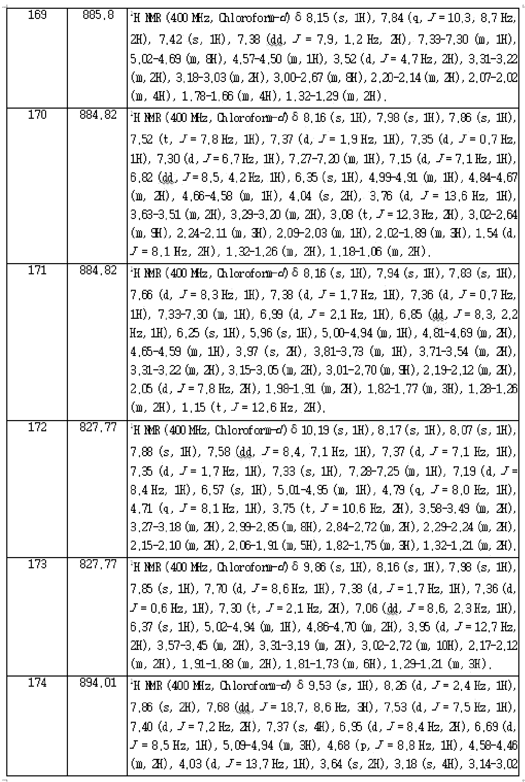

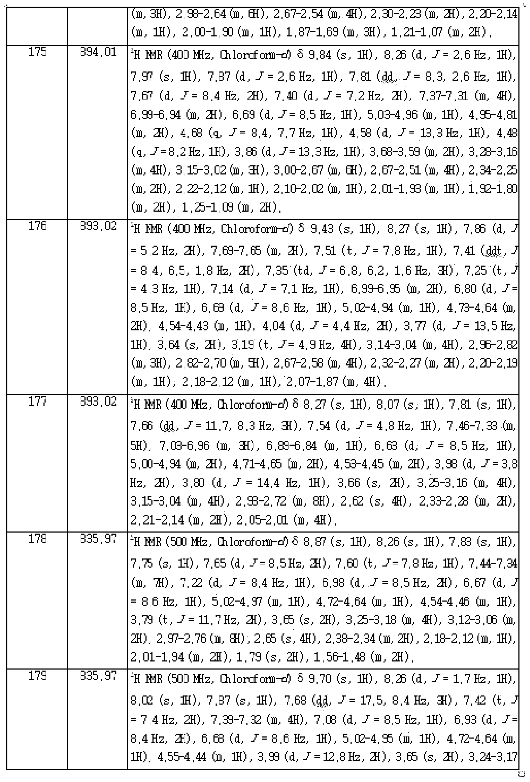

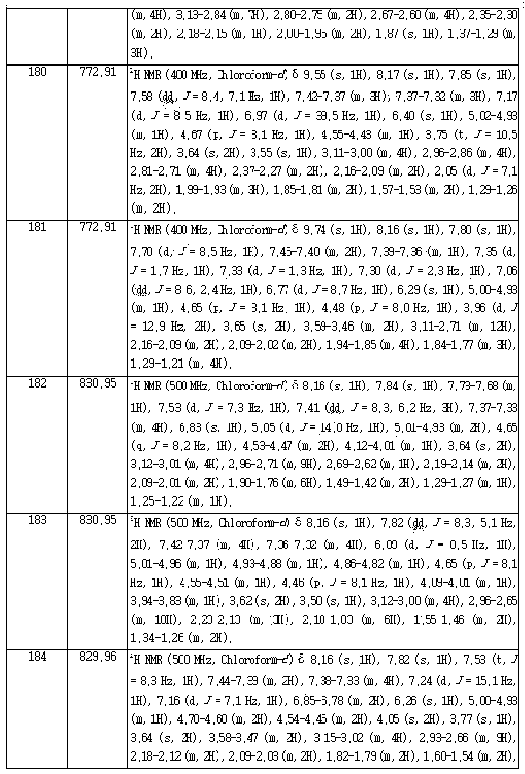

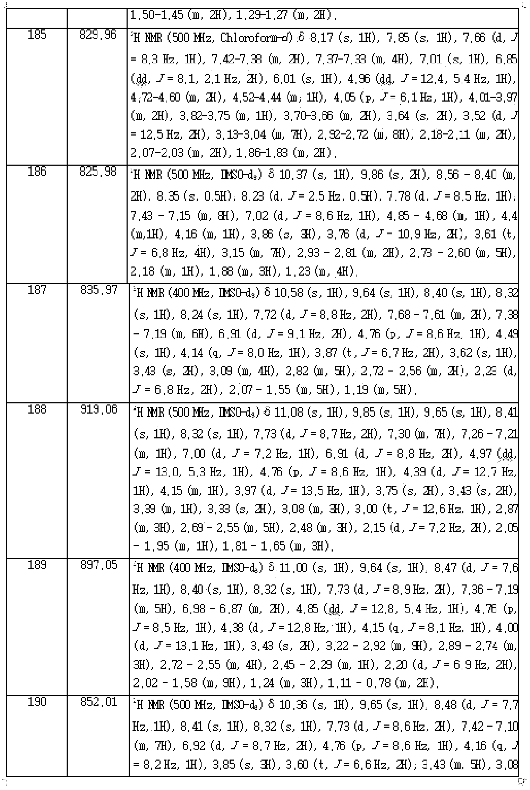

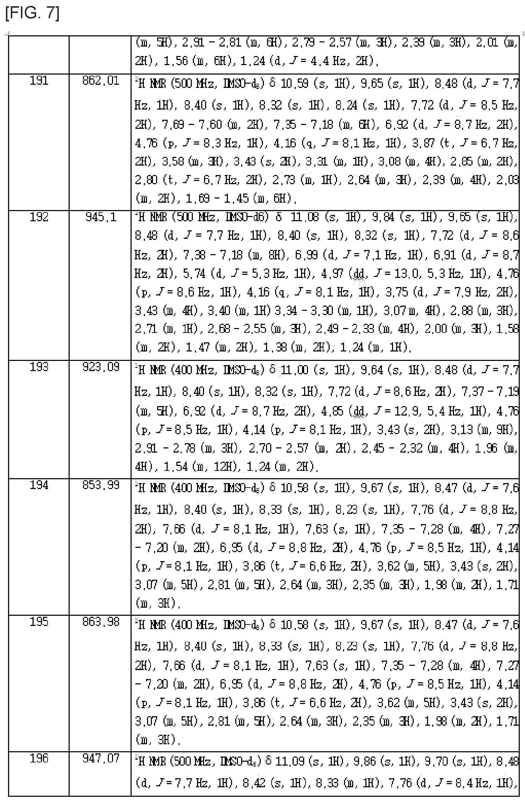

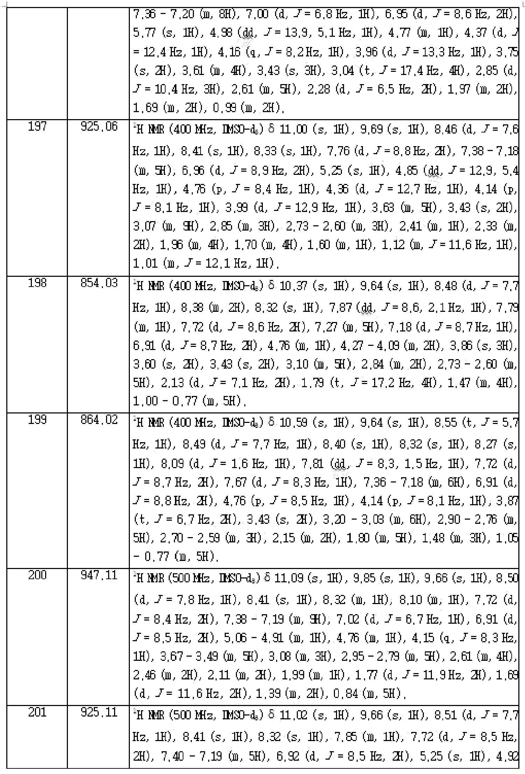

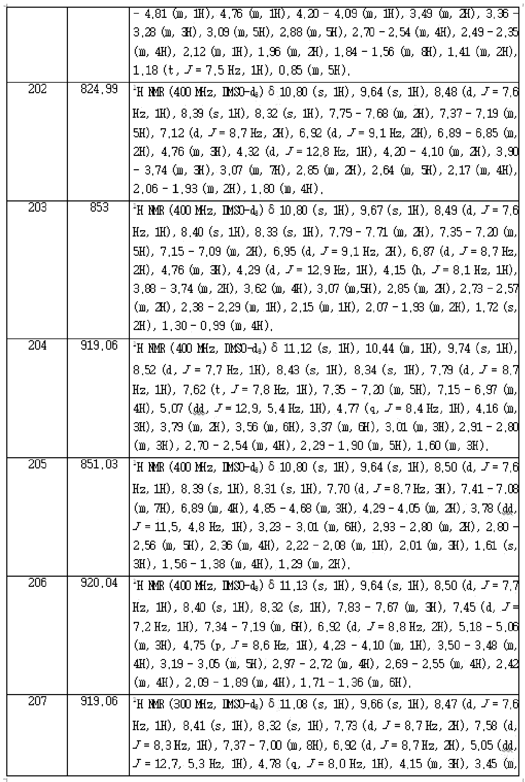

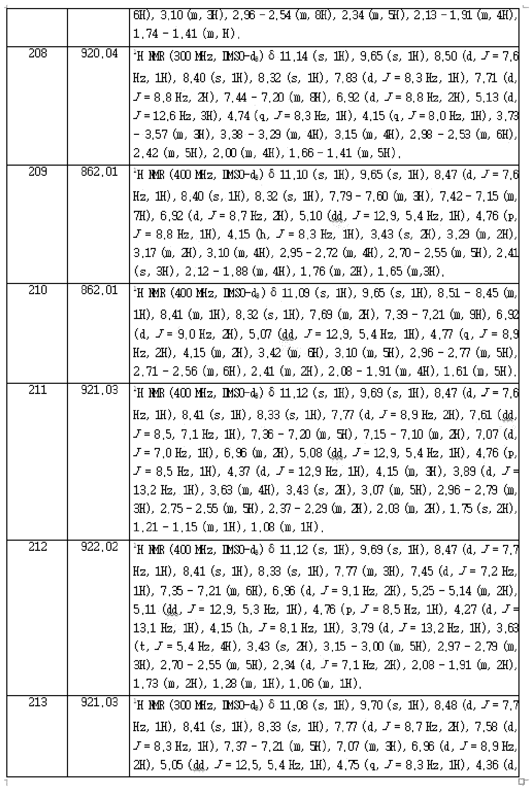

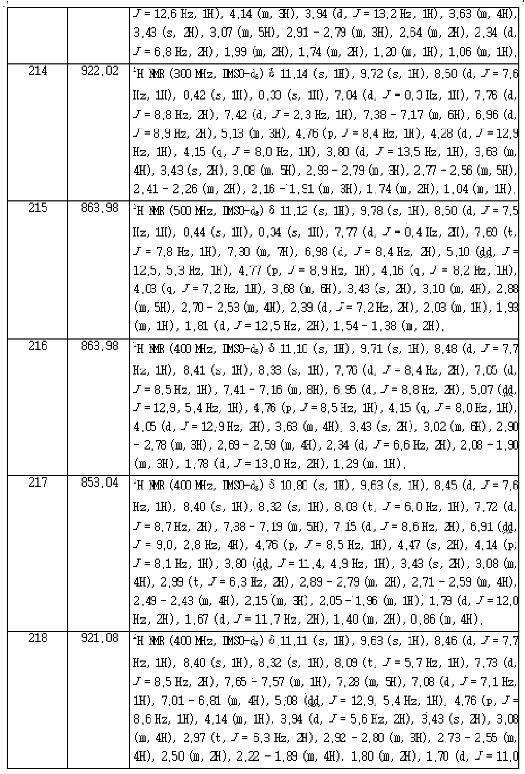

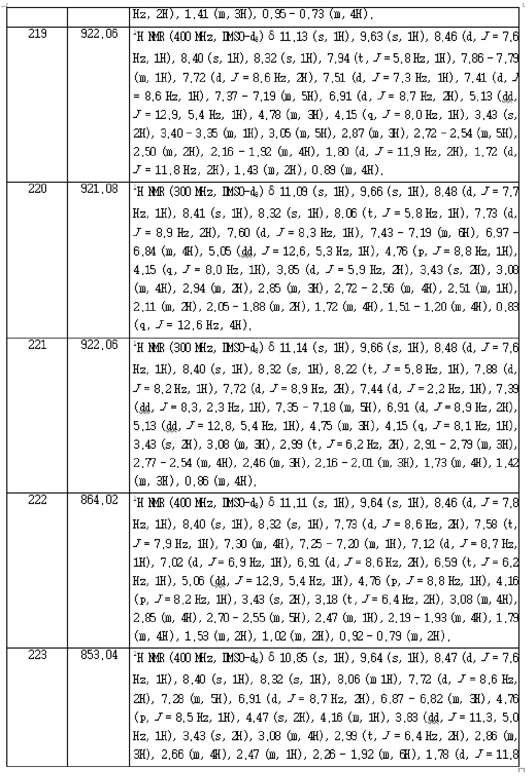

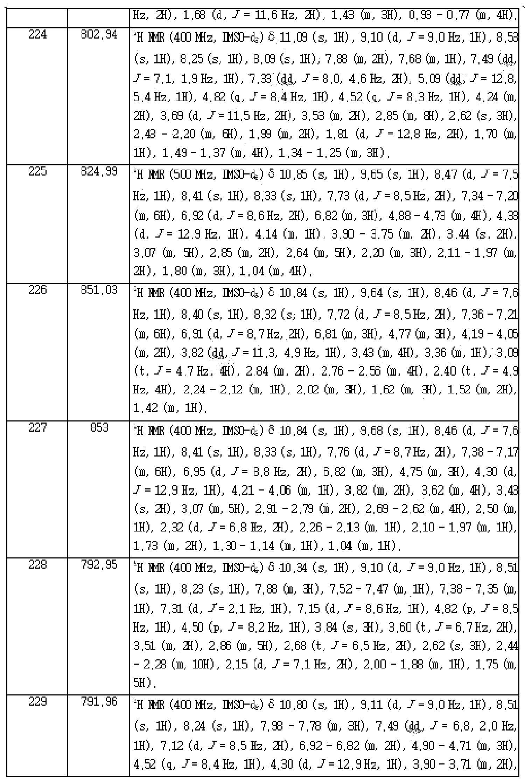

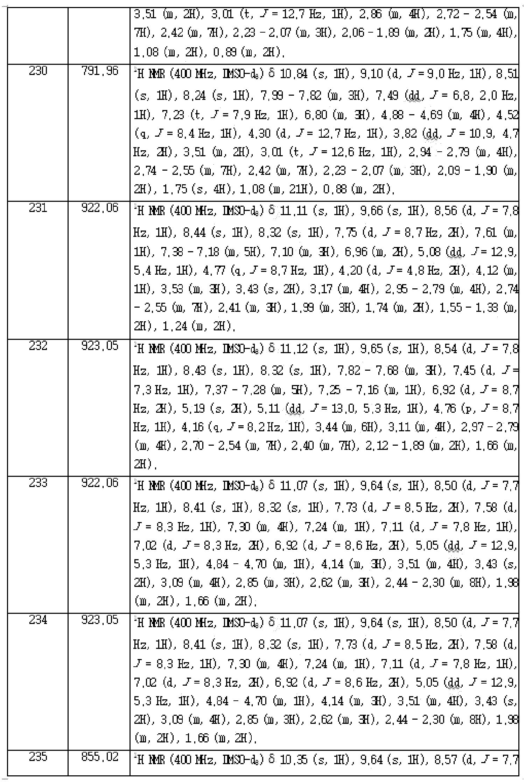

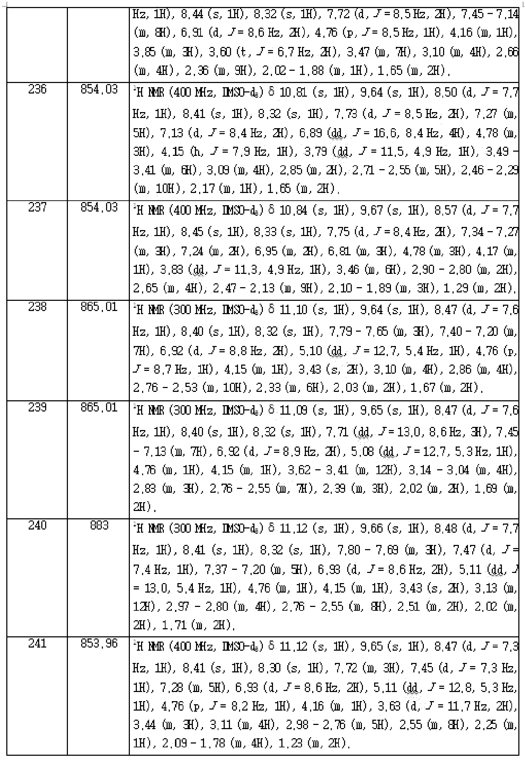

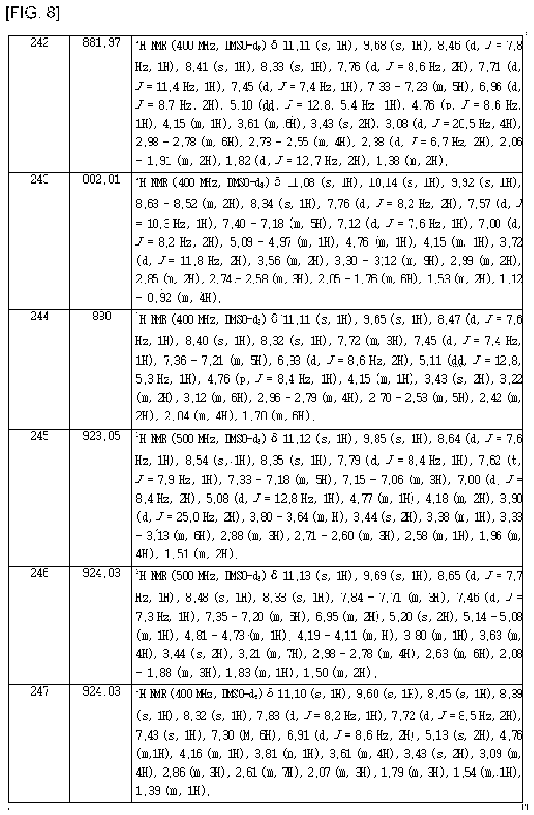

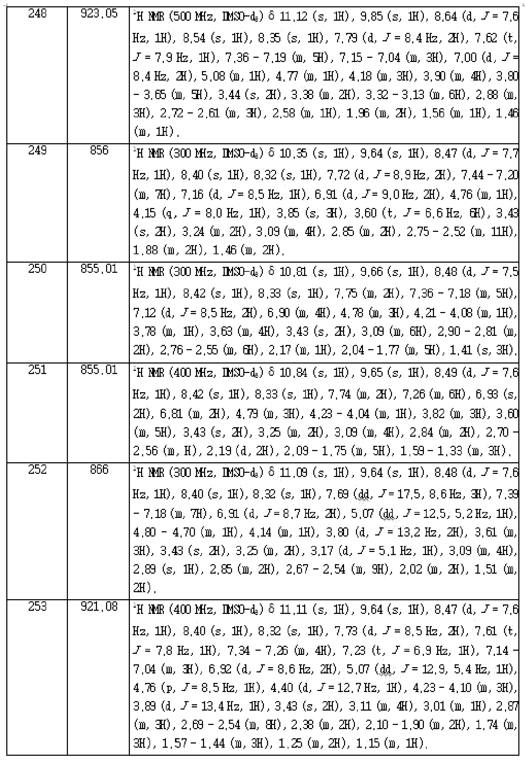

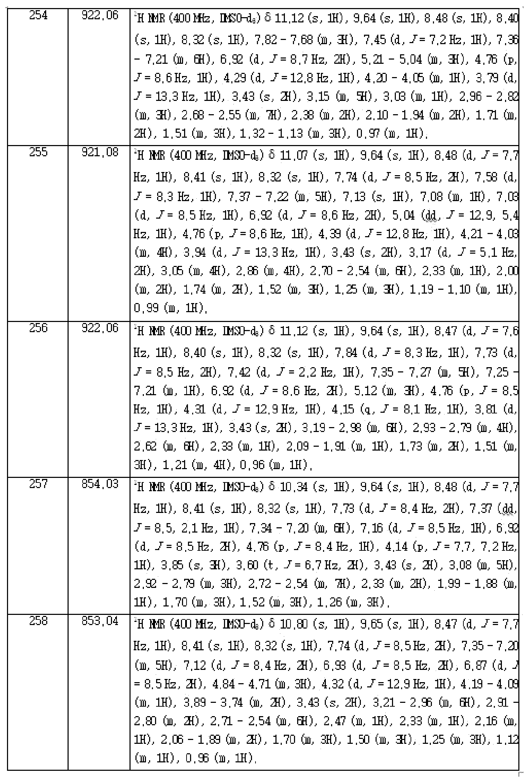

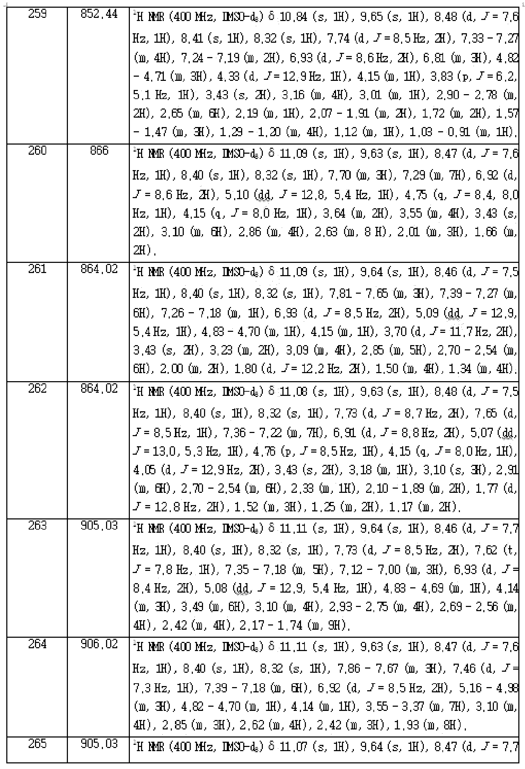

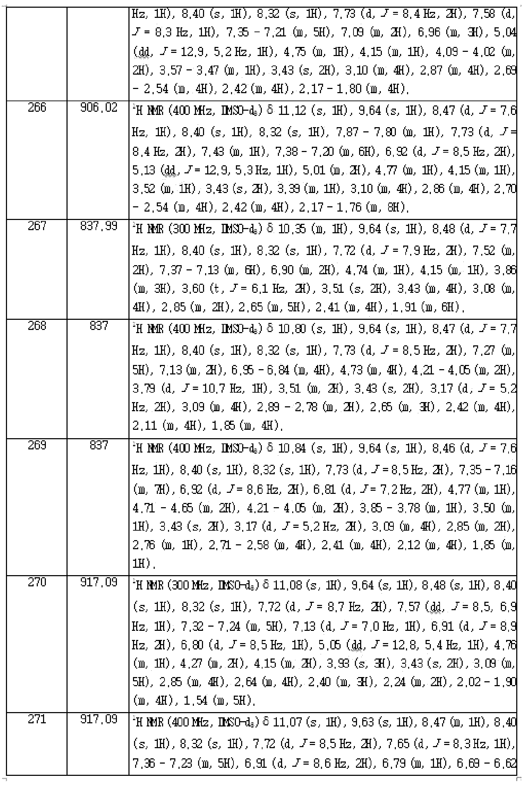

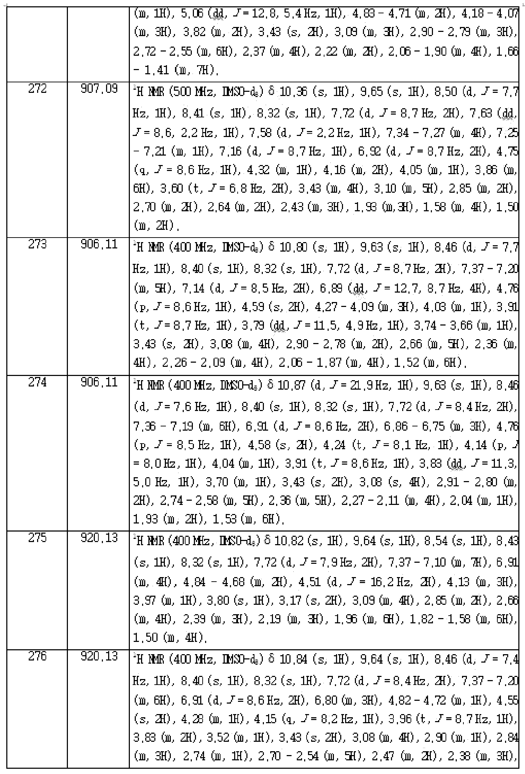

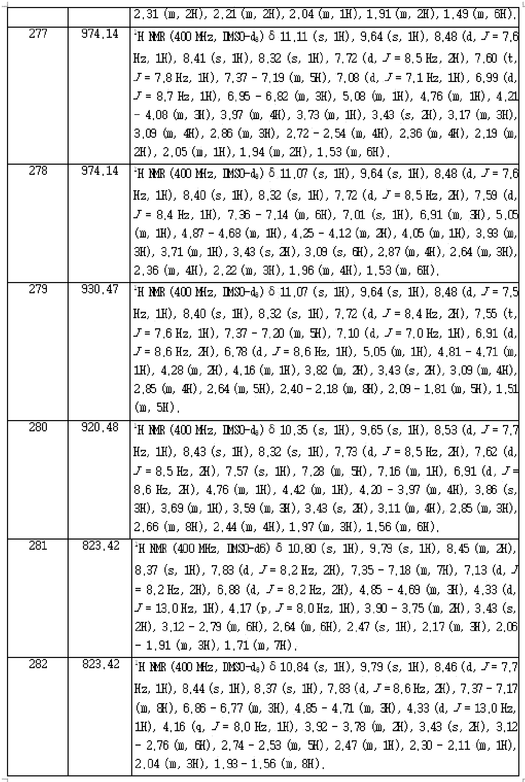

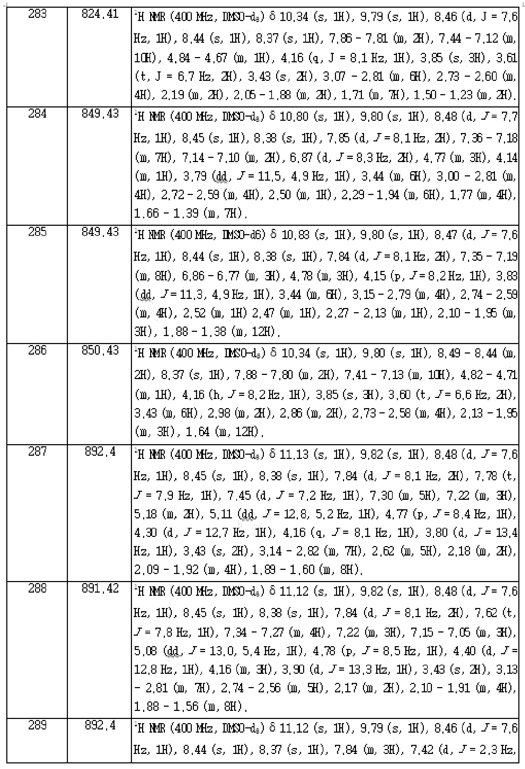

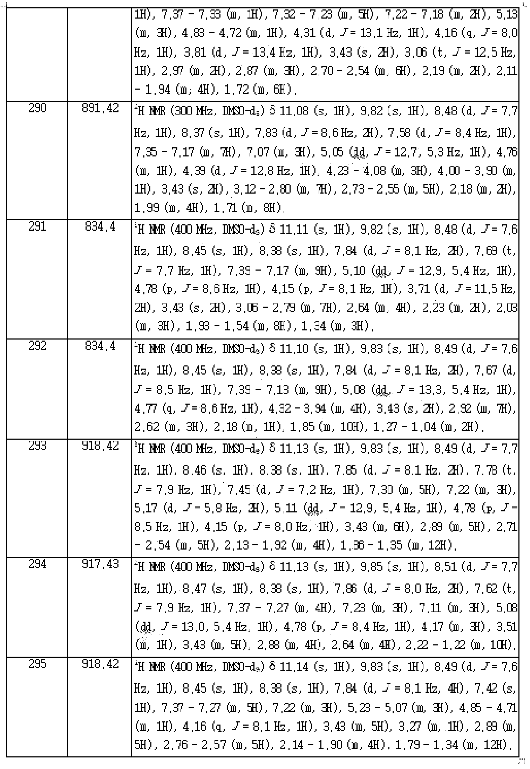

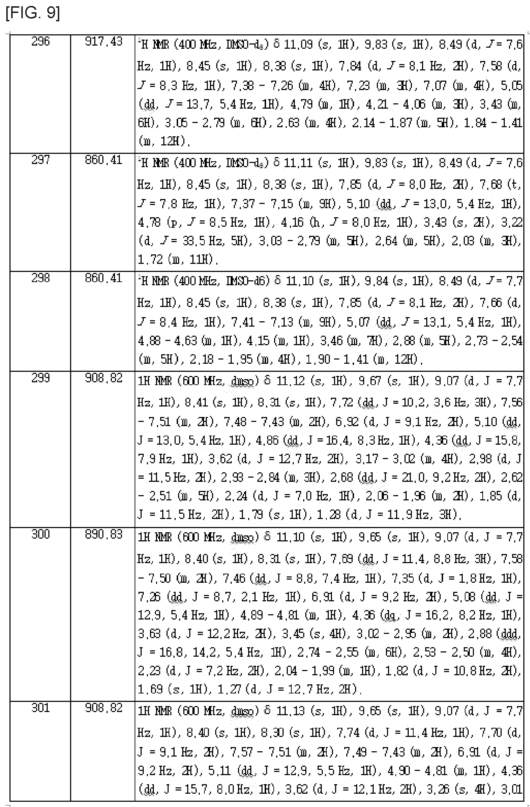

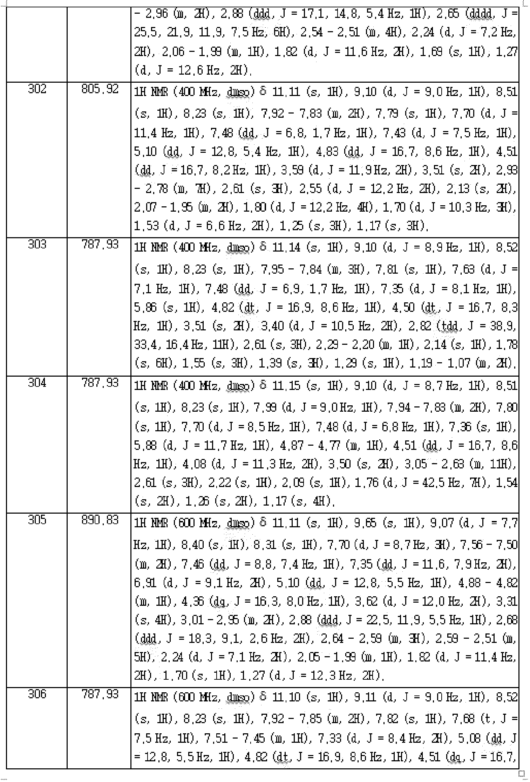

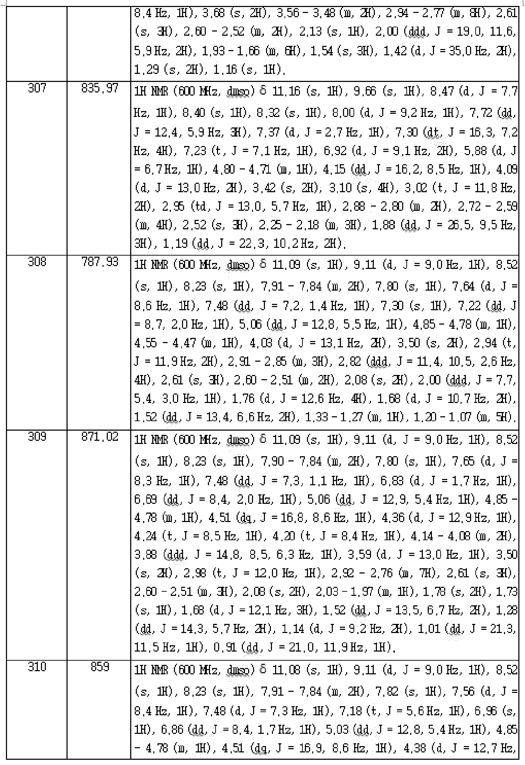

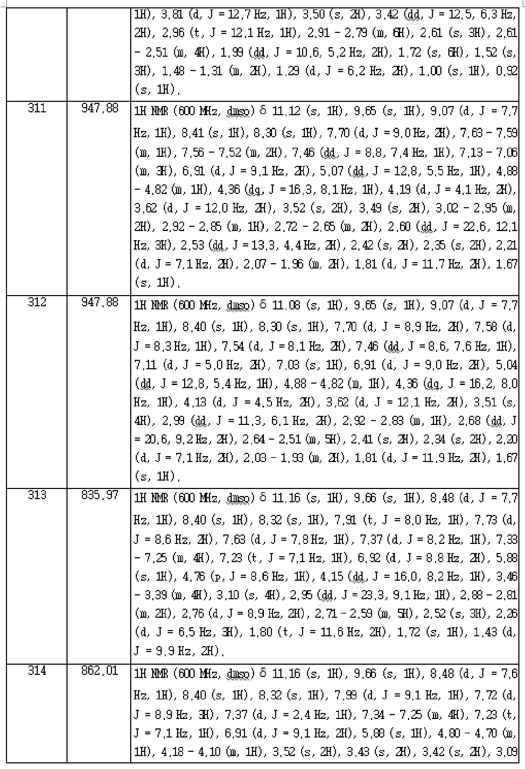

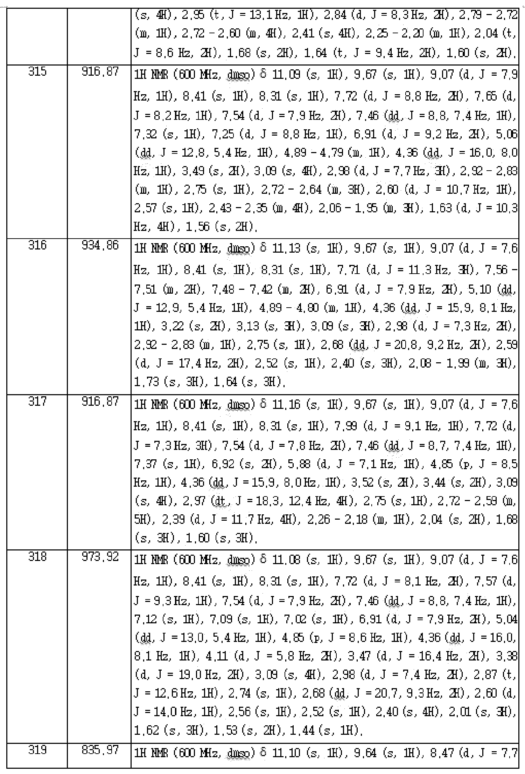

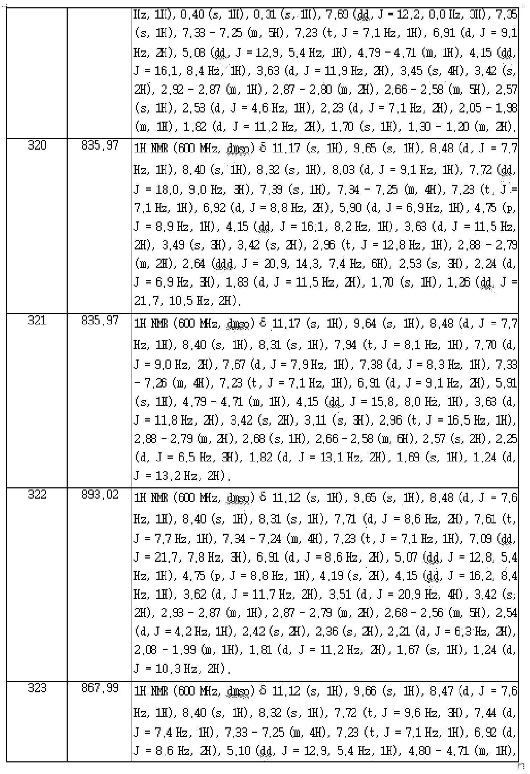

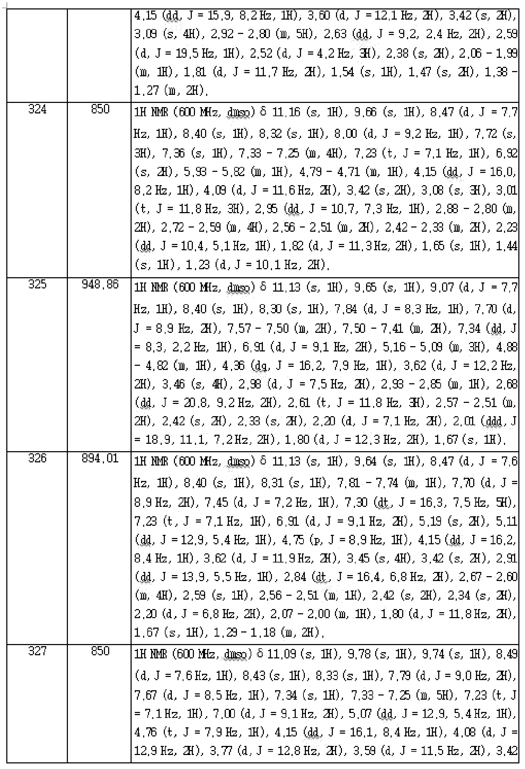

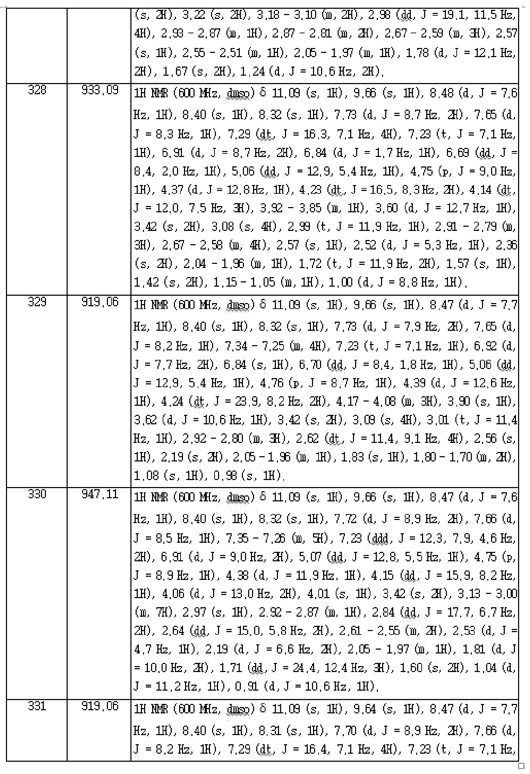

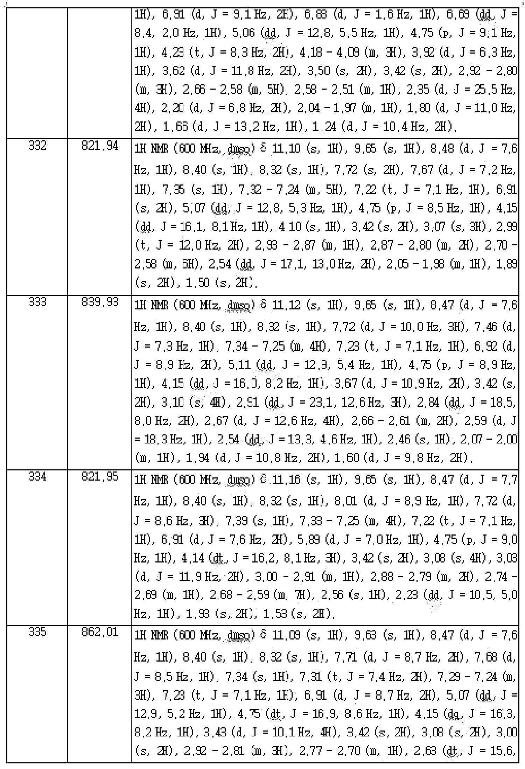

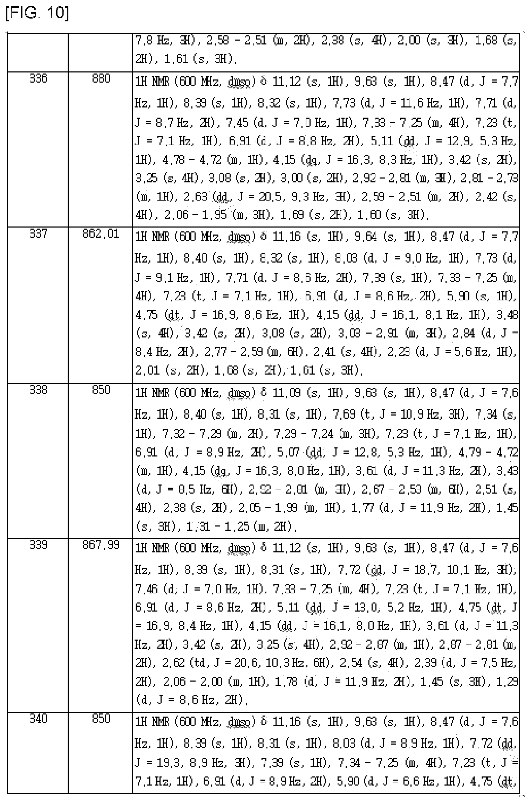

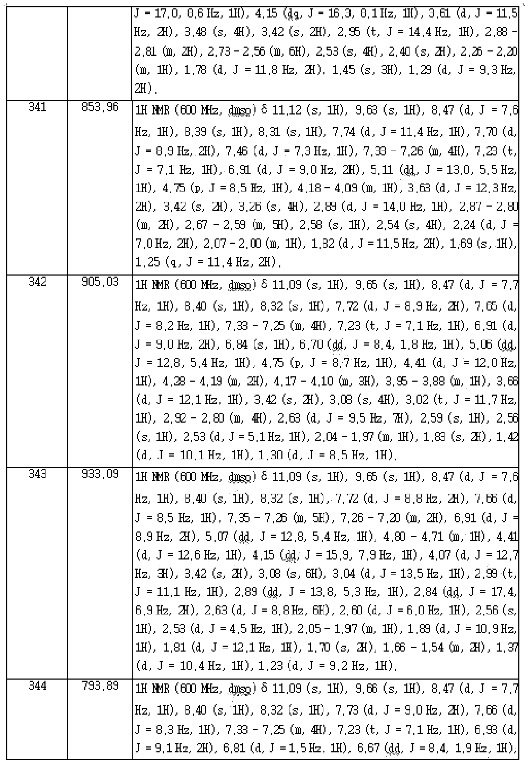

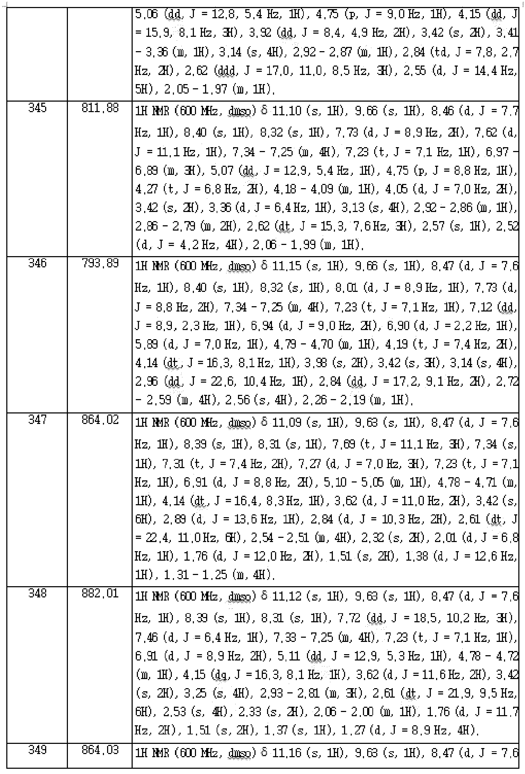

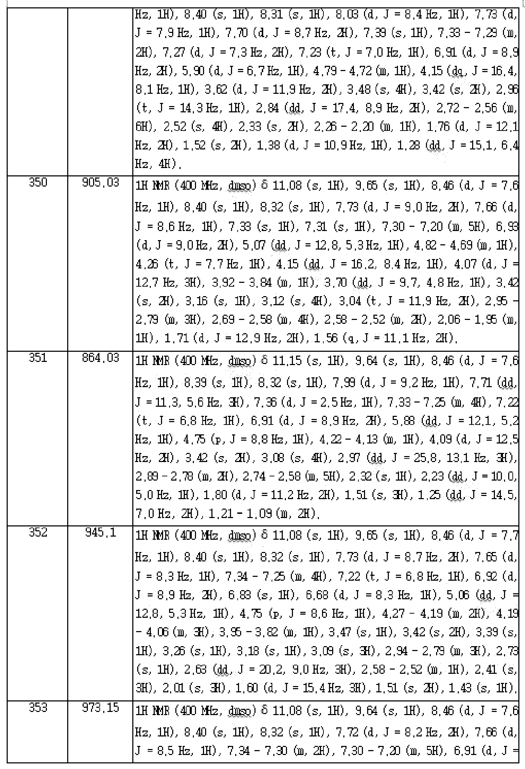

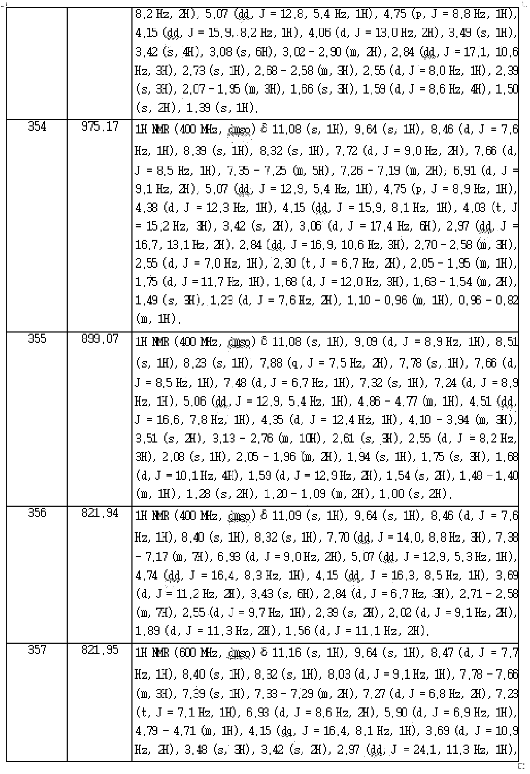

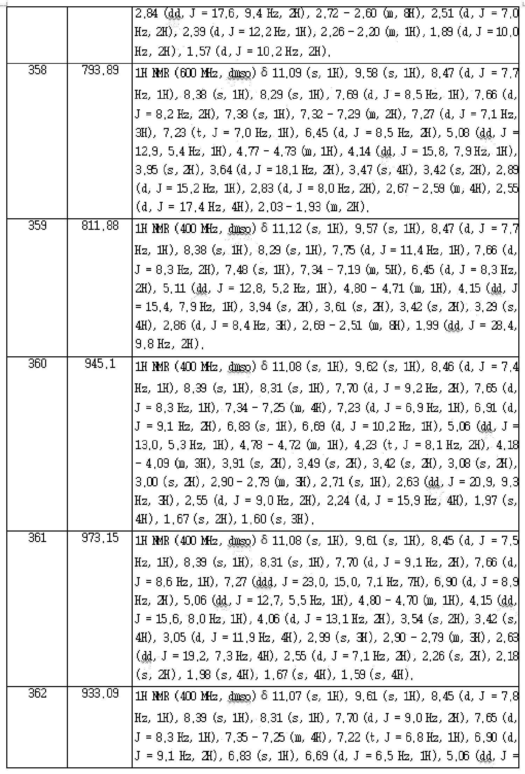

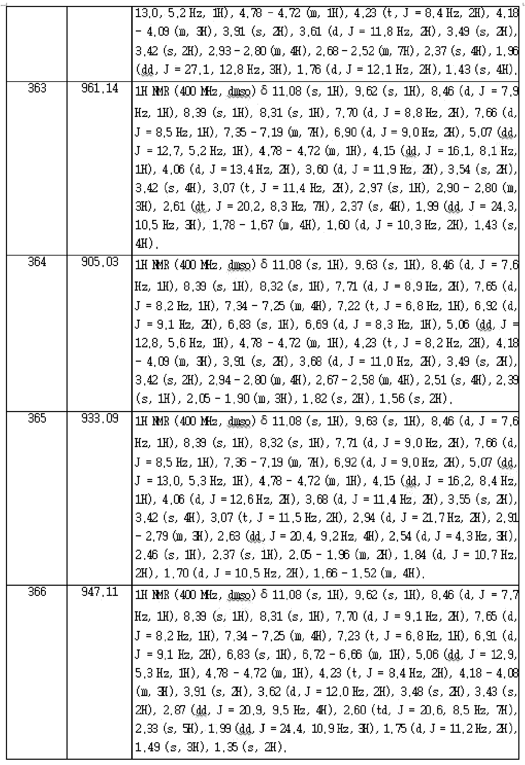

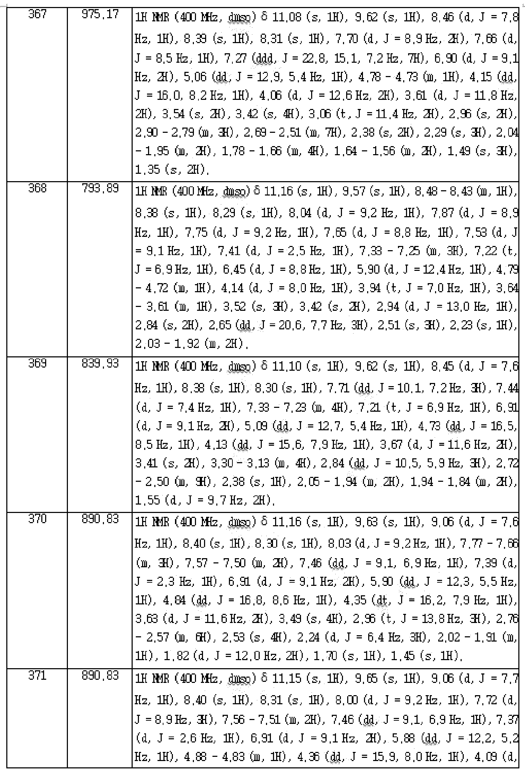

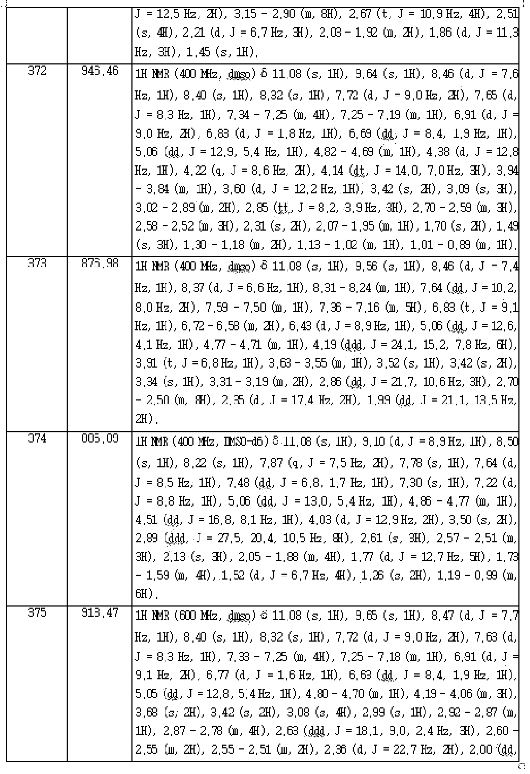

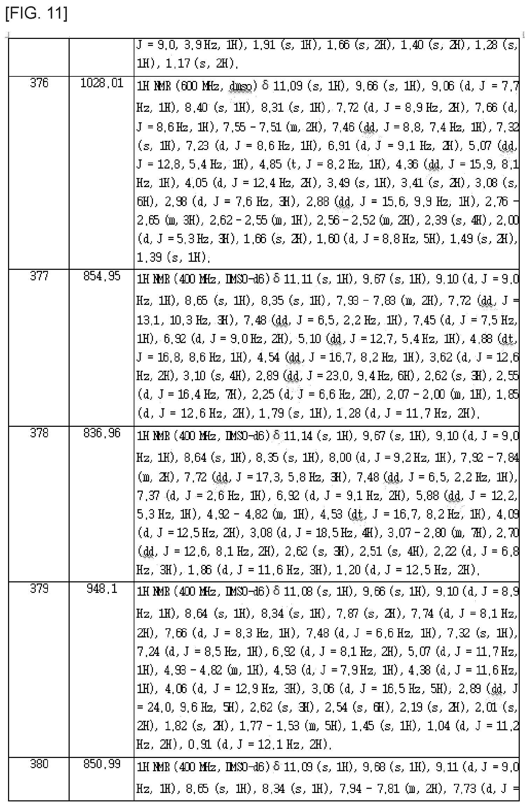

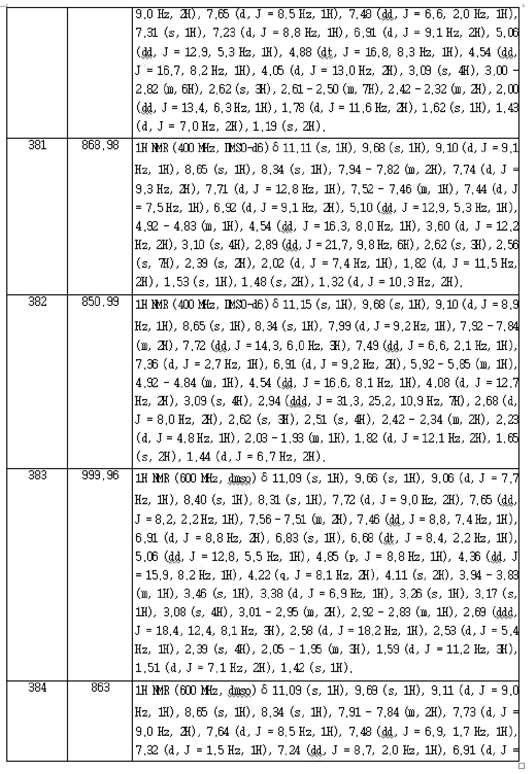

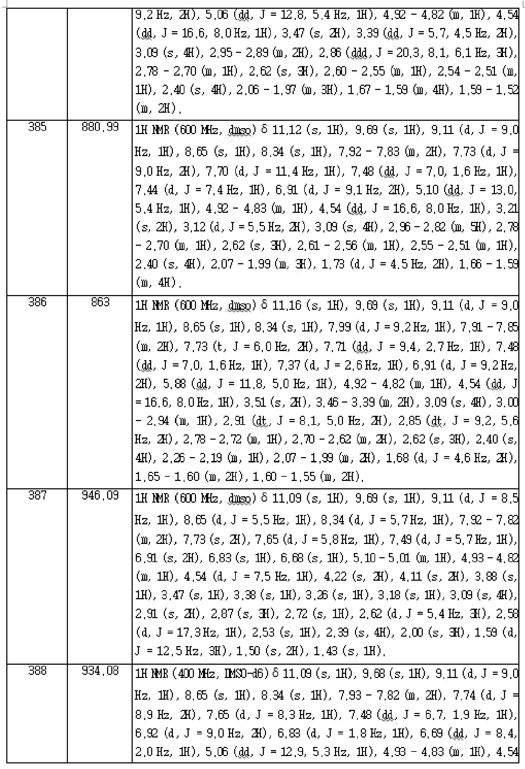

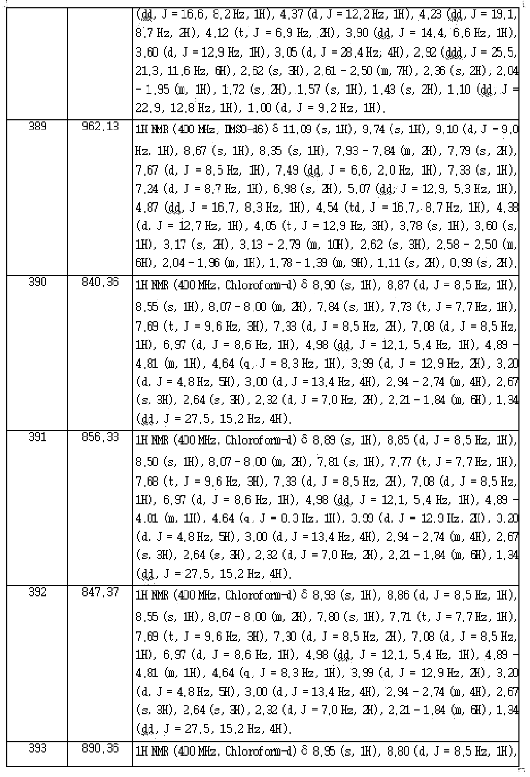

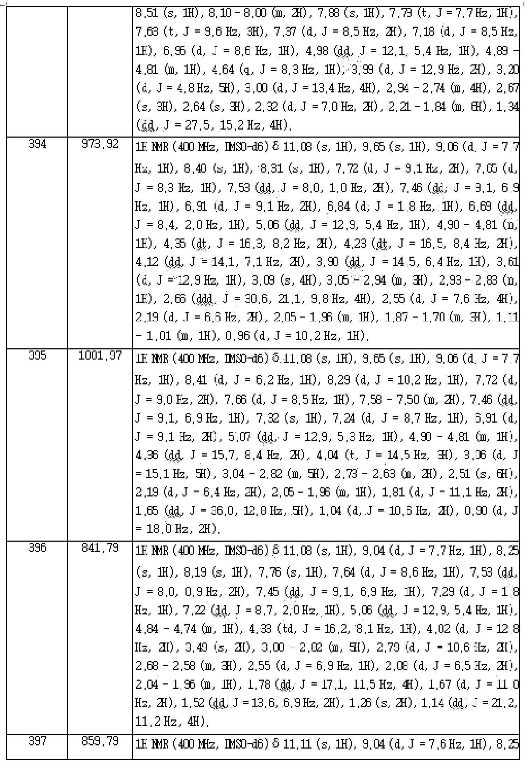

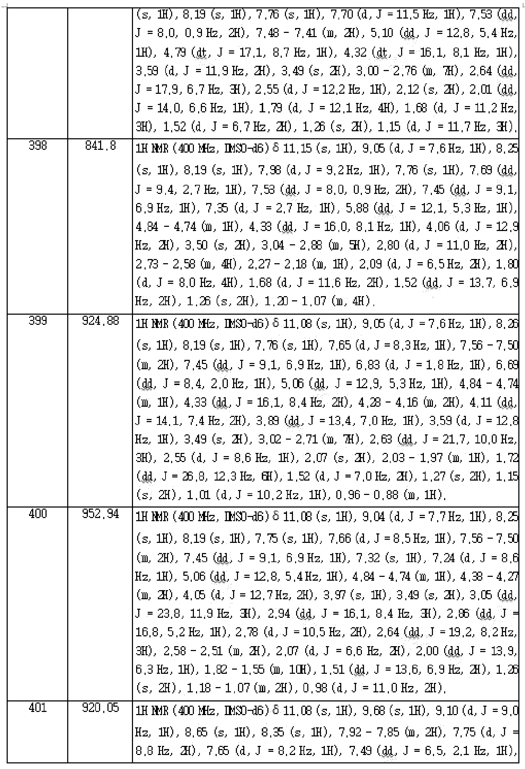

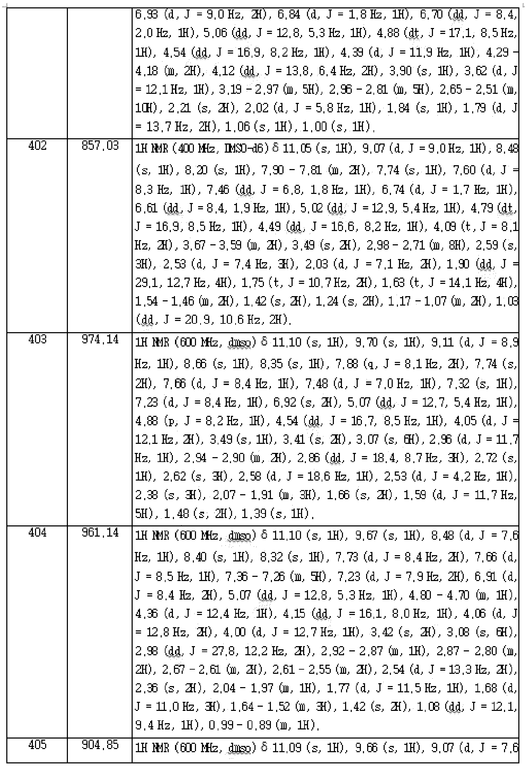

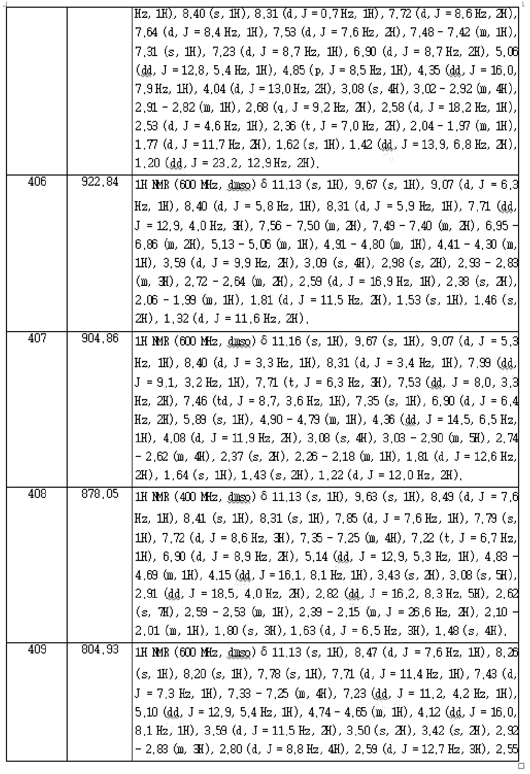

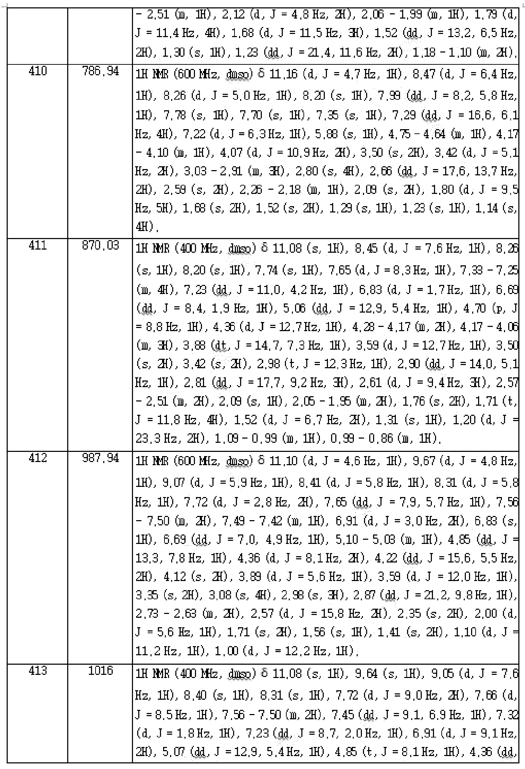

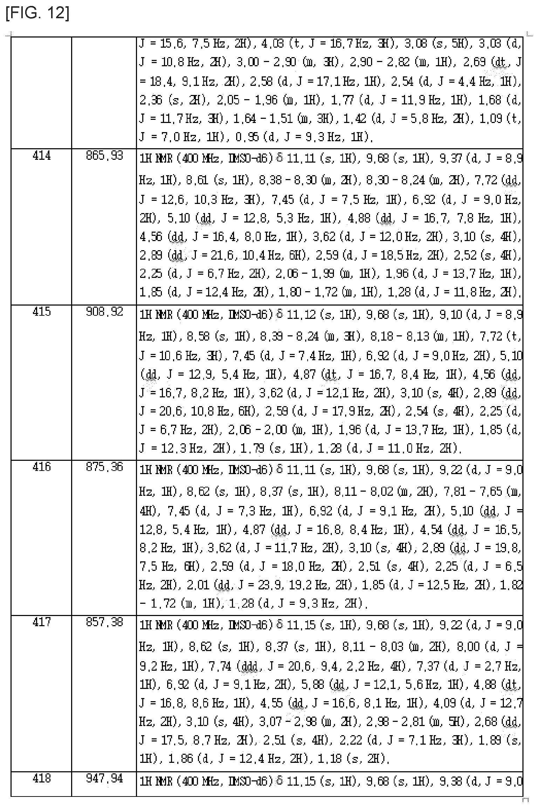

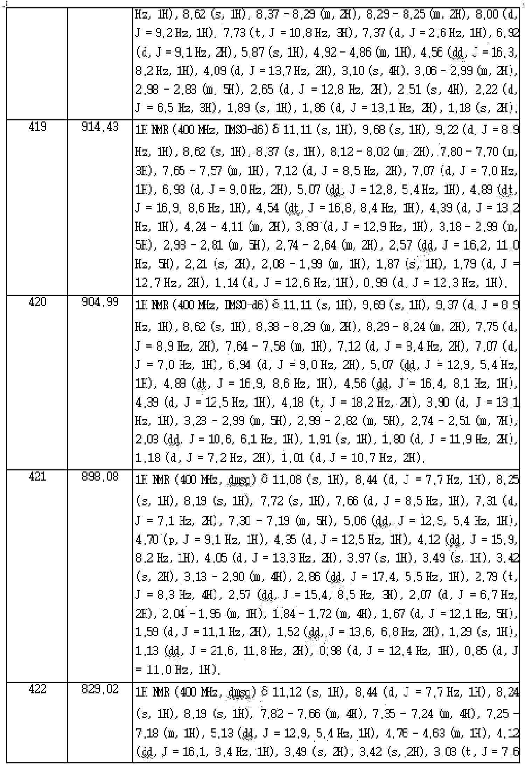

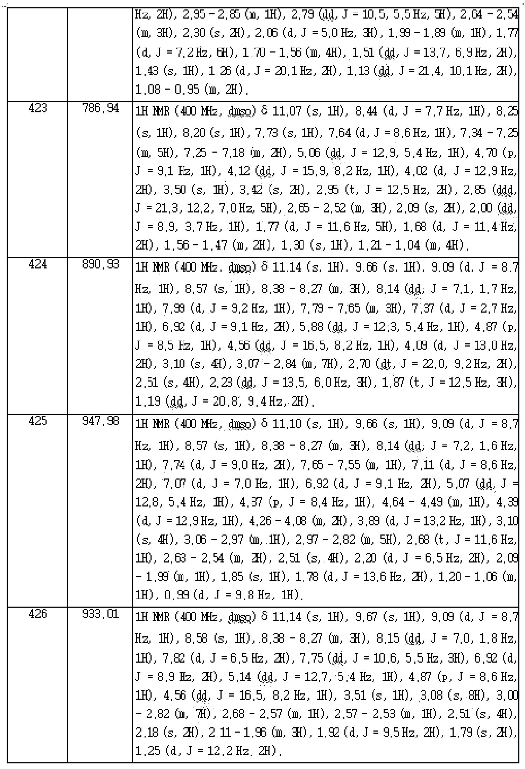

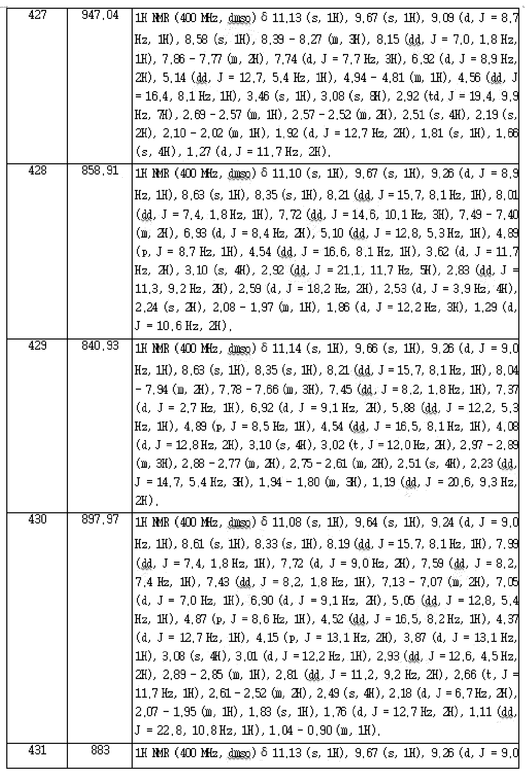

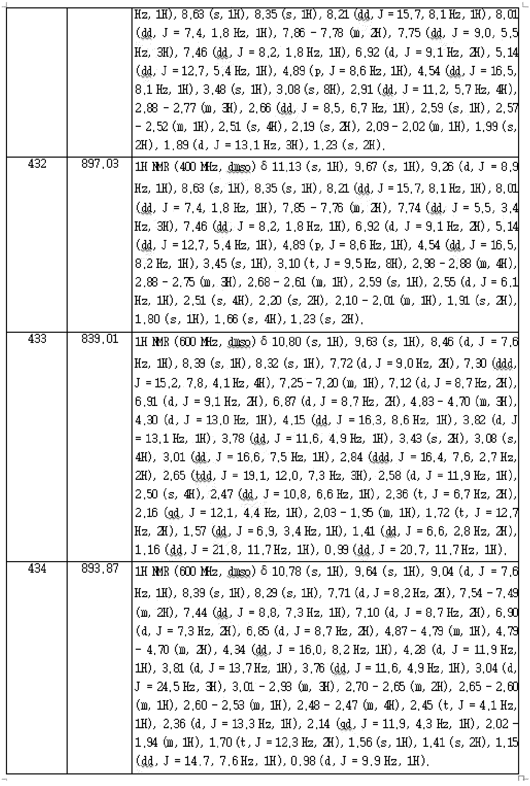

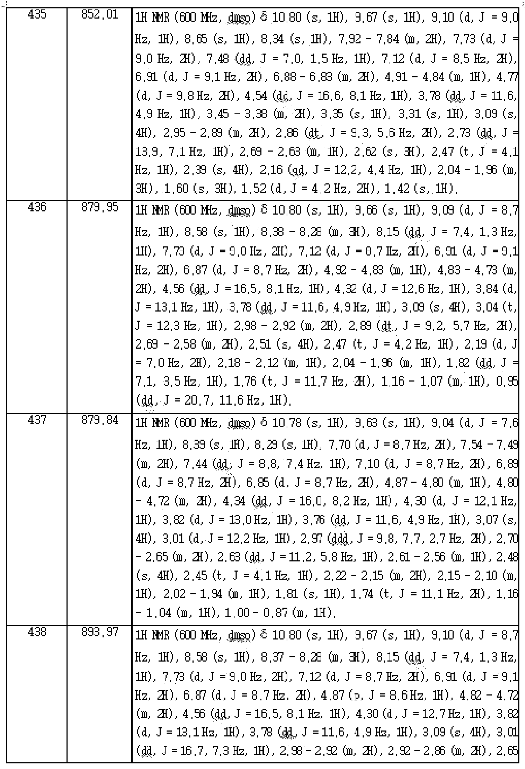

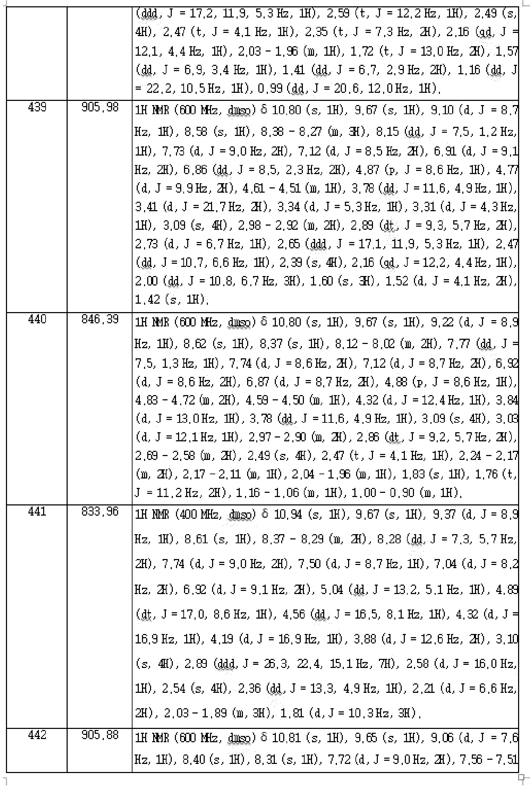

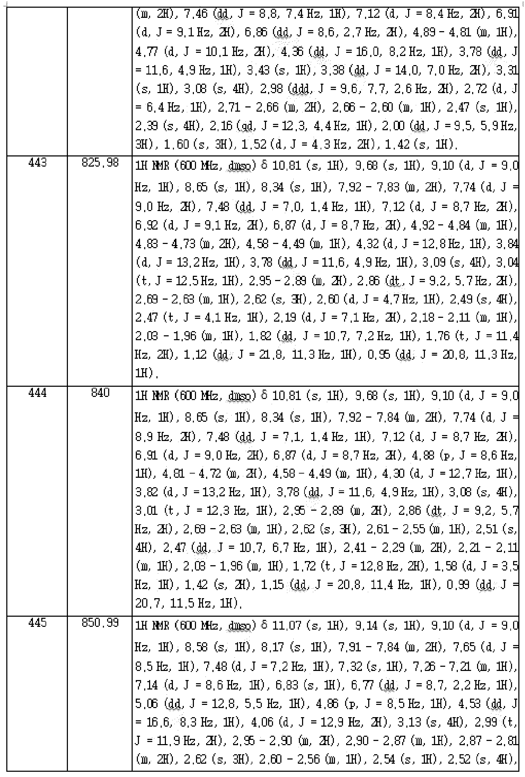

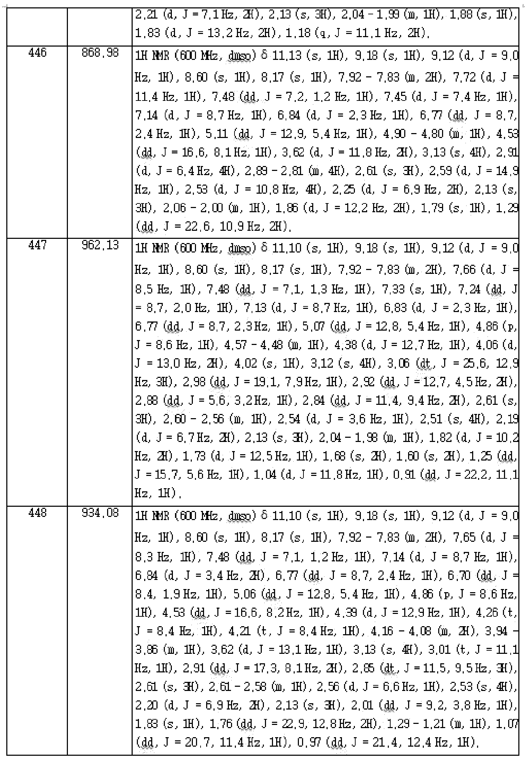

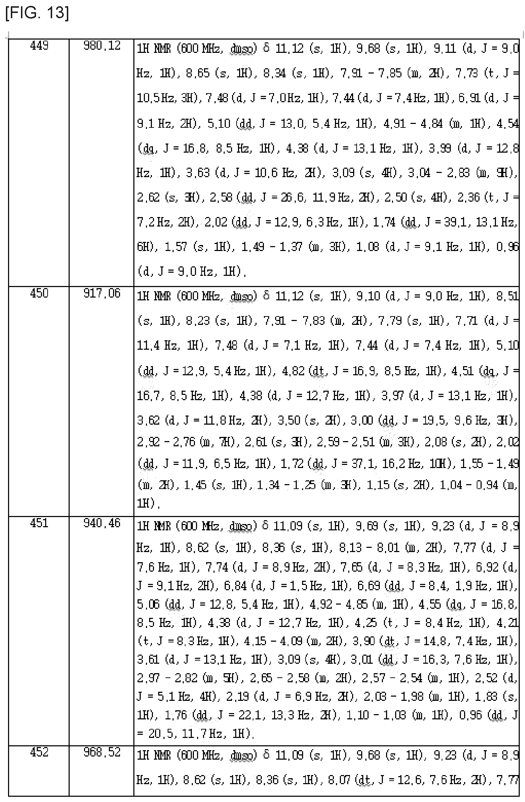

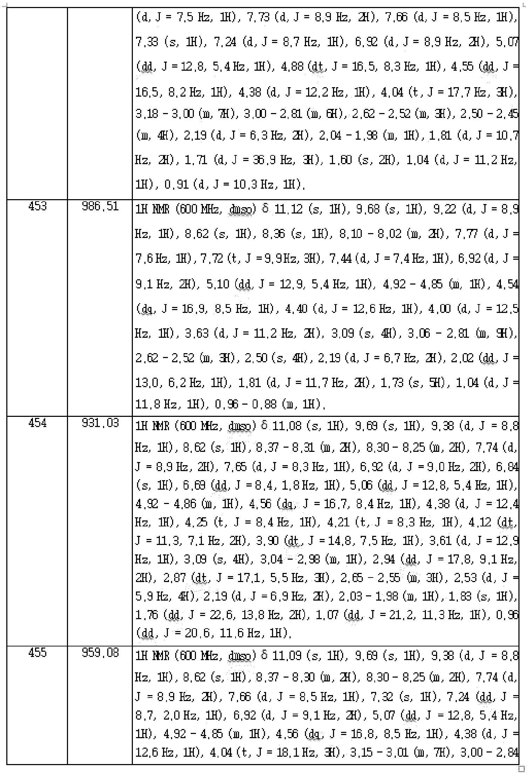

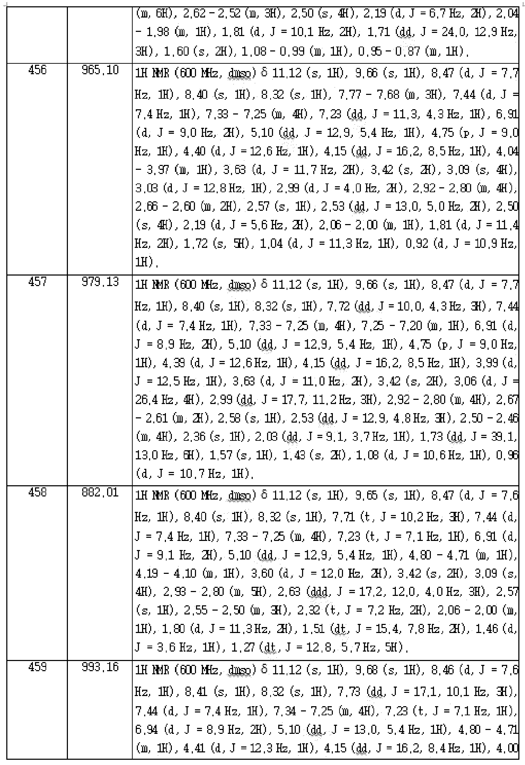

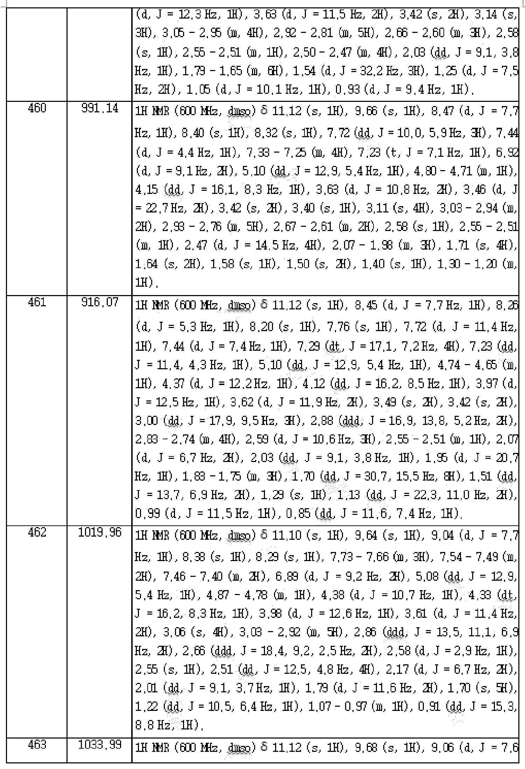

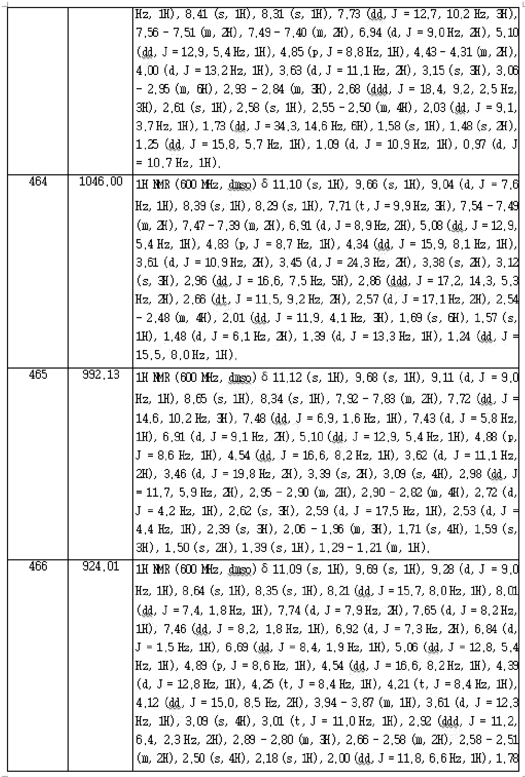

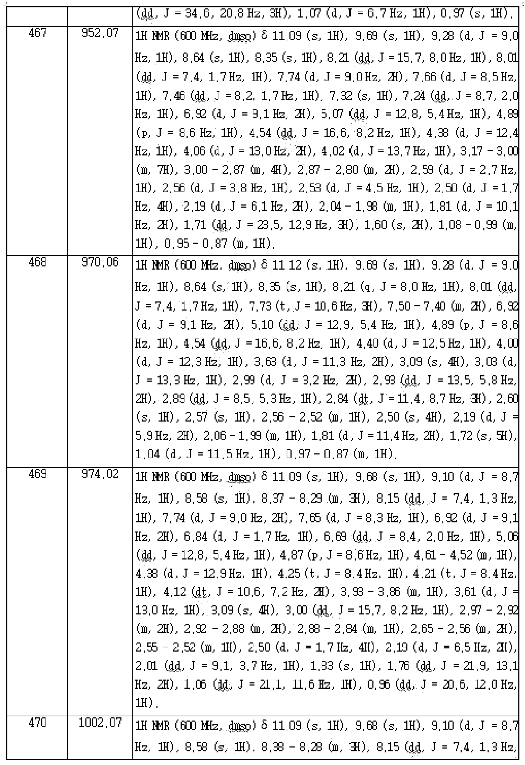

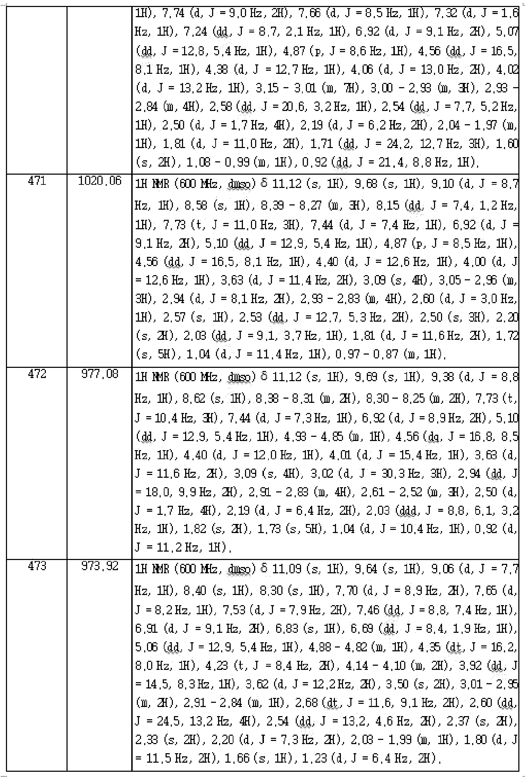

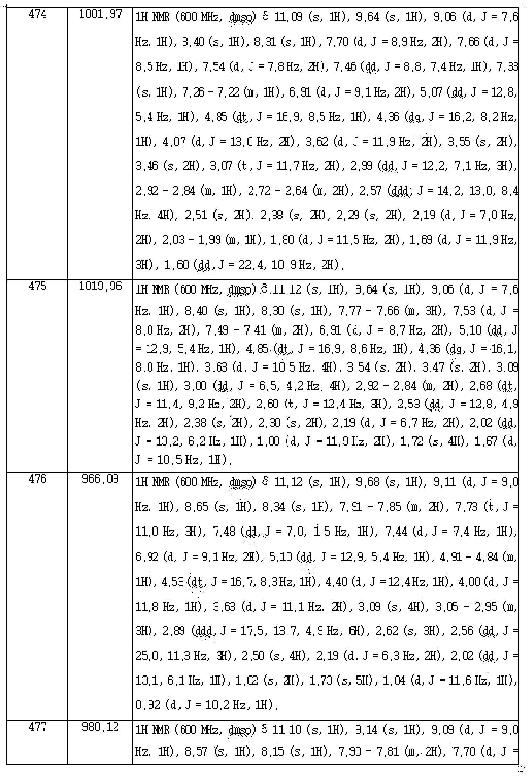

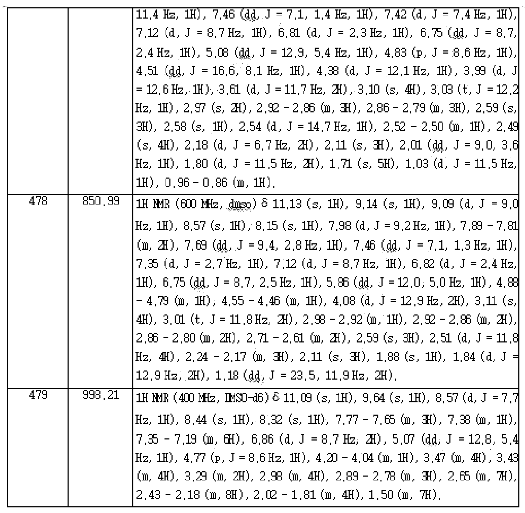

In order to identify the 479 compounds synthesized according to Examples above, the compounds were analyzed using mass spectrometry and NMR spectrometry, and the results are summarized and shown in FIGS. 3 to 13 below.

### Experimental Example 2: Evaluation of CDK Protein Degradation Activity

In order to evaluate the CDK protein degradation activity of the compounds according to the present invention, the following experiment was conducted.

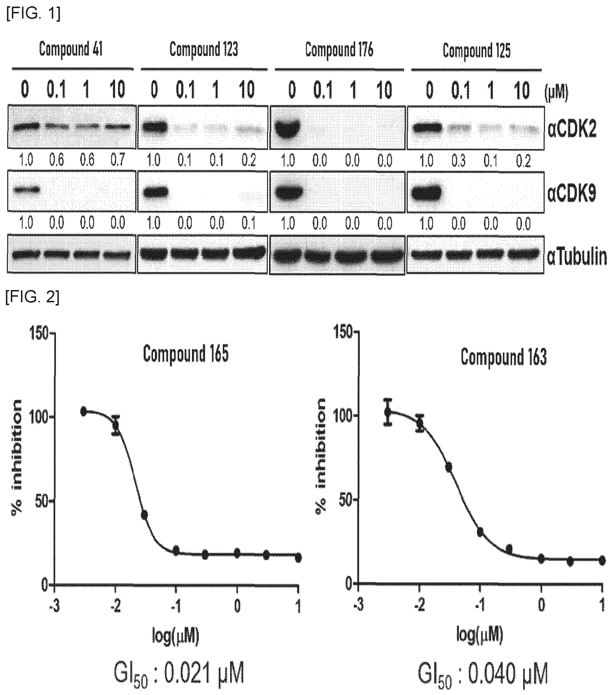

MDA-MB-231 cells were seeded at 5 × 10⁵ cells per well in a 6-well plate. The next day, the compounds were treated in each well to achieve final concentrations of 100 nM, 1 µM, and 10 µM. For the control group, an equal amount of DMSO was treated in the well as the compound. After 16 hours of treatment, cells were collected, and cell lysates were prepared using Triton X-100 lysis buffer (50 mM HEPES, pH 7.5, 40 mM NaCl, 1% Triton X-100, 1 mM EDTA, 1 mM EGTA, 10 mM pyrophosphate, 10 mM β-glycerophosphate, 50 mM NaF, 1 mM NaVO₄, protease inhibitor). Cell lysates were quantified and separated by size using sodium dodecyl sulfate-polyacrylamide gel electrophoresis (SDS-PAGE). Western blotting was then performed using antibodies that recognize CDK2, CDK9, and tubulin, respectively. The results obtained by Western blotting were quantitatively analyzed using the ImageJ program, and representative images according to the experimental results are shown in FIG. 1. Moreover, the residual rates calculated based on the enzymatic degradation ability for CDK2 and CDK9 are shown in Table 15 below.

**[Table 15]**

| Compound No. | Enzyme degradation | |
|---|---|---|
| | CDK2 (%remaining) @ 0.1/1/10 µM | CDK9 (%remaining) @ 0.1/1/10 µM |
| 17 | 90/100/50 | 10/0/0 |
| 25 | 20/20/30 | 0/0/0 |
| 31 | 70/40/30 | 10/0/0 |
| 32 | 60/20/20 | 50/0/0 |
| 33 | 40/30/50 | 0/0/0 |
| 34 | 50/50/100 | 0/0/10 |
| 35 | 100/60/60 | 0/10/10 |
| 36 | 100/100/100 | 30/10/20 |
| 38 | 40/30/20 | 0/0/0 |
| 41 | 60/60/70 | 0/0/0 |
| 42 | 100/60/30 | 30/0/10 |
| 43 | 90/60/70 | 50/0/0 |
| 45 | 80/60/90 | 0/0/0 |
| 46 | 80/90/100 | 20/10/30 |
| 47 | 100/100/100 | 40/0/0 |
| 48 | 100/100/100 | 50/20/20 |
| 50 | 80/40/10 | 90/60/20 |
| 51 | 70/20/20 | 70/0/0 |
| 52 | 40/40/50 | 70/30/10 |
| 53 | 90/80/70 | 90/90/10 |
| 61 | 100/100/40 | 80/50/10 |
| 65 | 100/50/20 | 30/10/0 |
| 74 | 30/40/50 | 0/0/0 |
| 75 | 20/10/30 | 0/0/10 |
| 76 | 100/100/100 | 30/20/10 |
| 84 | 50/30/20 | 70/30/10 |
| 85 | 70/60/50 | 60/40/30 |
| 87 | 90/70/50 | 50/50/40 |
| 88 | 20/20/20 | 0/0/0 |
| 89 | 70/50/90 | 10/0/0 |
| 90 | 40/20/10 | 0/0/0 |
| 91 | 0/0/0 | 0/0/0 |
| 92 | 40/20/20 | 10/0/0 |
| 93 | 0/10/10 | 0/0/0 |
| 94 | 80/60/90 | 50/10/20 |
| 96 | 60/40/50 | 10/0/0 |
| 101 | 70/50/40 | 80/30/30 |
| 102 | 100/70/70 | 90/20/10 |
| 103 | 40/40/60 | 30/10/10 |
| 104 | 90/40/70 | 80/30/10 |
| 106 | 70/50/50 | 70/0/0 |
| 107 | 60/50/100 | 90/20/0 |
| 108 | 30/30/60 | 0/0/0 |
| 109 | 40/60/70 | 0/0/0 |
| 110 | 100/60/80 | 90/20/30 |
| 112 | 100/50/40 | 100/0/0 |
| 113 | 30/30/30 | 100/10/0 |
| 114 | 30/40/50 | 0/0/0 |
| 115 | 30/40/50 | 0/0/0 |
| 116 | 30/40/50 | 10/0/0 |
| 117 | 80/80/80 | 10/10/0 |
| 122 | 30/10/0 | 10/0/0 |
| 123 | 10/10/20 | 0/0/0 |
| 124 | 40/20/10 | 0/0/0 |
| 125 | 30/10/20 | 0/0/0 |
| 126 | 80/70/70 | 80/70/70 |
| 128 | 90/50/100 | 90/60/100 |
| 130 | 60/40/10 | 100/40/20 |
| 131 | 80/100/100 | 60/60/60 |
| 132 | 60/40/40 | 70/10/0 |
| 133 | 90/70/80 | 80/0/0 |
| 134 | 60/50/50 | 0/0/0 |
| 135 | 70/60/40 | 0/0/0 |
| 136 | 100/100/60 | 10/70/40 |
| 137 | 30/40/40 | 0/0/0 |
| 139 | 80/90/80 | 80/90/70 |
| 141 | 70/50/60 | 90/80/50 |
| 142 | 80/70/50 | 70/0/0 |
| 143 | 80/50/100 | 0/0/0 |
| 144 | 100/70/70 | 80/10/0 |
| 145 | 30/50/50 | 0/0/0 |
| 148 | 40/50/60 | 10/0/0 |
| 149 | 10/20/20 | 0/0/0 |
| 150 | 70/80/80 | 20/10/0 |
| 151 | 30/10/10 | 0/0/0 |
| 152 | 10/10/10 | 0/0/0 |
| 153 | 10/10/10 | 0/0/0 |
| 154 | 100/70/70 | 90/70/30 |
| 155 | 100/80/10 | 90/40/0 |
| 156 | 100/80/70 | 80/50/40 |
| 159 | 100/80/50 | 90/70/30 |
| 161 | 90/80/60 | 30/10/10 |
| 162 | 50/50/50 | 0/0/0 |
| 163 | 20/10/10 | 0/0/0 |
| 164 | 30/30/30 | 0/0/0 |
| 165 | 10/10/10 | 0/0/0 |
| 166 | 10/20/20 | 0/0/0 |
| 167 | 30/40/30 | 0/0/0 |
| 168 | 50/10/10 | 80/40/10 |
| 169 | 80/70/70 | 10/0/0 |
| 170 | 0/10/20 | 10/0/10 |
| 171 | 20/10/10 | 0/0/0 |
| 172 | 10/20/20 | 50/0/0 |
| 173 | 30/30/50 | 50/20/0 |
| 174 | 0/0/0 | 0/0/0 |
| 175 | 100/80/30 | 80/30/0 |
| 176 | 0/0/0 | 0/0/0 |
| 177 | 50/10/10 | 30/0/0 |
| 178 | 30/20/20 | 10/0/0 |
| 179 | 10/0/0 | 10/0/0 |
| 180 | 30/30/30 | 20/80/0 |
| 181 | 40/30/50 | 50/40/0 |
| 182 | 70/50/50 | 20/10/0 |
| 184 | 70/70/50 | 0/0/0 |
| 185 | 100/100/100 | 0/0/0 |
| 187 | 30/10/20 | 10/0/0 |
| 188 | 80/70/60 | 20/20/10 |
| 189 | 30/20/40 | 0/0/0 |
| 190 | 90/90/90 | 90/40/40 |
| 191 | 0/0/0 | 0/0/0 |
| 192 | 70/70/50 | 20/20/10 |
| 193 | 20/20/10 | 0/10/10 |
| 194 | 50/10/10 | 70/50/40 |
| 195 | 80/50/50 | 20/10/10 |
| 196 | 80/100/100 | 30/40/40 |
| 197 | 30/30/30 | 10/10/0 |
| 198 | 0/0/0 | 50/30/10 |
| 199 | 50/60/50 | 20/30/30 |
| 200 | 100/100/100 | 20/20/20 |
| 201 | 50/70/50 | 10/10/0 |
| 202 | 30/20/40 | 10/0/0 |
| 203 | 70/40/70 | 20/0/0 |
| 204 | 20/20/20 | 0/0/0 |
| 205 | 30/30/30 | 0/0/0 |
| 206 | 20/20/10 | 0/0/0 |
| 207 | 50/70/50 | 0/0/0 |
| 208 | 80/70/70 | 10/0/0 |
| 209 | 70/60/30 | 30/20/10 |
| 210 | 30/30/30 | 10/0/0 |
| 211 | 50/50/40 | 30/30/30 |
| 212 | 30/20/10 | 70/30/20 |
| 213 | 50/70/50 | 70/30/10 |
| 216 | 40/40/50 | 50/50/50 |
| 217 | 80/100/50 | 0/0/0 |
| 218 | 60/70/80 | 30/30/10 |
| 219 | 80/50/50 | 100/10/0 |
| 220 | 80/80/60 | 40/0/0 |
| 221 | 100/100/100 | 100/60/0 |
| 222 | 100/100/100 | 70/70/80 |
| 223 | 80/100/100 | 20/0/0 |
| 225 | 10/20/40 | 30/0/0 |
| 226 | 40/30/40 | 0/0/0 |
| 227 | 50/20/70 | 60/70/10 |
| 229 | 70/90/100 | 20/10/0 |
| 230 | 50/50/80 | 30/10/0 |
| 231 | 50/60/40 | 20/0/0 |
| 232 | 60/20/20 | 90/10/0 |
| 233 | 80/80/70 | 40/0/0 |
| 234 | 100/80/70 | 60/40/0 |
| 236 | 50/50/50 | 20/0/0 |
| 237 | 10/0/0 | 20/0/0 |
| 238 | 100/80/50 | 70/20/0 |
| 239 | 100/20/20 | 70/0/0 |
| 244 | 70/50/40 | 60/0/0 |
| 246 | 60/40/40 | 80/30/0 |
| 248 | 50/50/50 | 50/20/0 |
| 250 | 60/40/30 | 40/10/0 |
| 251 | 0/0/0 | 30/0/0 |
| 252 | 30/30/50 | 60/60/60 |
| 253 | 20/-/20 | 0/0/0 |
| 254 | 50/30/20 | 50/20/0 |
| 255 | 30/30/30 | 0/0/0 |
| 256 | 40/30/30 | 60/10/0 |
| 257 | 0/20/20 | 80/50/50 |
| 258 | 20/20/20 | 20/0/0 |
| 259 | 0/0/0 | 0/0/0 |
| 260 | 20/20/20 | 60/60/60 |
| 261 | 70/90/100 | 60/30/20 |
| 262 | 70/90/100 | 40/30/10 |
| 263 | 50/40/40 | 20/0/0 |
| 264 | 50/20/10 | 90/40/0 |
| 265 | 90/70/70 | 10/0/0 |
| 268 | 70/50/20 | 70/0/0 |
| 269 | 10/10/10 | 30/10/10 |
| 270 | 40/30/30 | 40/30/30 |
| 271 | 30/40/40 | 20/10/10 |
| 272 | 40/40/40 | 30/20/20 |
| 273 | 100/70/50 | 20/10/10 |
| 274 | 30/20/20 | 30/30/30 |
| 275 | 80/60/60 | 70/30/0 |
| 276 | 30/20/20 | 10/0/0 |
| 277 | 30/30/30 | 0/0/0 |
| 278 | 50/50/50 | 10/0/0 |
| 279 | 50/40/40 | 40/10/10 |
| 280 | 50/30/30 | 50/10/10 |
| 281 | 30/50/50 | 0/0/0 |
| 282 | 10/20/20 | 0/0/0 |
| 284 | 40/50/60 | 0/0/0 |
| 285 | 10/20/20 | 0/0/0 |
| 286 | 40/60/70 | 40/30/30 |
| 287 | 0/0/0 | 0/0/0 |
| 288 | 10/20/20 | 0/0/0 |
| 289 | 40/60/70 | 30/0/0 |
| 290 | 20/20/30 | 10/0/0 |
| 291 | 20/20/20 | 20/0/0 |
| 292 | 10/10/10 | 10/0/0 |
| 293 | 0/0/0 | 0/0/0 |
| 294 | 10/10/10 | 0/0/0 |
| 295 | 20/20/20 | 0/0/0 |
| 296 | 20/0/0 | 0/0/0 |
| 297 | 20/40/40 | 10/0/0 |
| 298 | 10/10/10 | 10/0/0 |
| 299 | 30/30/30 | 0/0/0 |
| 300 | 10/10/10 | 10/0/0 |
| 301 | 30/20/10 | 70/0/0 |
| 304 | 20/30/50 | 20/0/0 |
| 306 | 100/100/100 | 30/0/10 |
| 307 | 0/0/0 | 0/0/0 |
| 308 | 40/30/30 | 20/0/20 |
| 309 | 90/80/100 | 0/0/0 |
| 310 | 100/70/100 | 40/0/0 |
| 311 | 10/20/20 | 10/0/0 |
| 312 | 10/10/10 | 0/0/0 |
| 314 | 10/10/10 | 10/0/0 |

### Experimental Example 3: Cytotoxicity Evaluation

In order to evaluate the cytotoxicity of the compounds according to the present invention, the following experiment was conducted.

MDA-MB-231 cells were seeded at 3 × 10³ cells per well in a 96-well plate. The next day, the compounds were treated in each well to achieve final concentrations of 3nM, 10nM, 30nM, 100nM, 300nM, 1µM, 3µM, and 10µM. For the control group, an equal amount of DMSO was treated in the well as the compound. After 72 hours of compound treatment, the number of surviving cells was analyzed using the CellTiter-Glo Luminescent Cell Viability Assay (Promega). Luminescence was measured using a VICTOR X3 Multilabel Plate Reader (PerkinElmer). The GI₅₀ value of the compounds was implemented using a Prism software (GraphPad Prism 5.01), and the image of a representative experimental result is presented in FIG. 2.

### Experimental Example 4: Evaluation of CDK Protein Inhibition

In order to evaluate CDK2 and CDK9 protein activity inhibition of the compounds according to the present invention, the compounds were tested by using KINOMEscan Technology provided by Eurofins Scientific, and the results for certain selected compounds are shown in Tables 16 and 17 below.

**[Table 16]**

| Compound No. | CDK2/cyclin A inhibition (%) (at concentration of 1 µM) |
|---|---|
| 1 | 77 |
| 2 | 84 |
| 3 | 84 |
| 4 | 84 |
| 5 | 91 |
| 6 | 88 |
| 7 | 94 |
| 8 | 89 |
| 9 | 92 |
| 10 | 90 |
| 11 | 78 |
| 12 | 86 |
| 61 | 84 |
| 62 | 89 |
| 63 | 83 |
| 64 | 85 |
| 65 | 87 |
| 66 | 78 |

**[Table 17]**

| Compound No. | CDK2/cyclin A inhibition | CDK9/cyclin T1 inhibition |
|---|---|---|
| 74 | > 10000 | 25 |
| 91 | 613 | 17 |
| 125 | 1295 | 28 |
| 176 | 439 | 57 |

Upon examining the tables above, it can be found that most of the compounds of the present invention demonstrate high degrading activity against CDK2 and/or CDK9. Specifically, especially in cases of certain compounds, relatively higher degrading activity was demonstrated. This indicates that a pharmaceutical composition for treating CDK2 and/or CDK9-mediated disorders, comprising a compound according to the examples of the present invention as an active ingredient, can be provided.

From the above description, a person of ordinary skill in the art to which the present invention pertains would understand that the present invention can be implemented in other specific forms without changing its technical concept or essential features. In this regard, the above-described examples should be understood as illustrative and not limited in any way. The scope of the present invention should be interpreted to include all modifications or modified forms derived from the meaning and scope of the claims as set forth below, as well as equivalent concepts, rather than from the detailed description above.

## Claims

1. A compound represented by Formula 1 below or a pharmaceutically acceptable salt thereof: 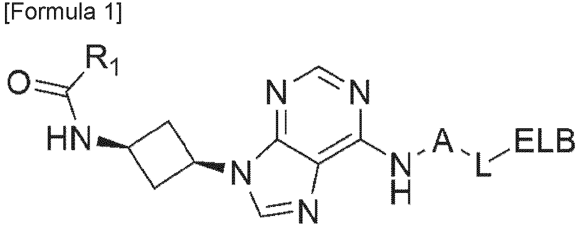 wherein in Formula 1 above:
R₁ is C₁₋₁₀ alkyl, aryl, -C₁₋₃ alkyl-C₆₋₁₀ aryl, 5-10 membered heteroaryl, -C₁₋₃ alkyl-5-10 membered heteroaryl, C₃₋₁₀ cycloalkyl, -C₁₋₃ alkyl-C₃₋₁₀ cycloalkyl, 3-10 membered heterocycloalkyl, or -C₁₋₃ alkyl-3-10 membered heterocycloalkyl, wherein the aryl, heteroaryl, cycloalkyl, or heterocycloalkyl is unsubstituted or substituted with one or more selected from the group consisting of halogen, cyano, tert-butoxycarbonyl (-Boc), amino, nitro, hydroxy, C₁₋₆ alkyl, halo-C₁₋₆ alkyl, -ORₐ, -C(=O)Rₐ, -C(=O)ORₐ, - C(O)NRₐR_{b}, -SO₂Rₐ, -S(O)Rₐ, -SO(N)Rₐ, C₁₋₂ alkyl-C₆₋₁₀ aryl, and C₆₋₁₀ aryl, wherein Rₐ and R_{b} are identical or different and are independently hydrogen, halogen, amino, C₁₋₆ alkyl, halo-C₁₋₆ alkyl, C₆₋₁₀ aryl, 5-10 membered heteroaryl, C₃₋₁₀ cycloalkyl, or 3-10 membered heterocycloalkyl;
A is a moiety functioning as an exit vector;
L is a linker; and
ELB is an E3 ubiquitin ligase binder.

2. The compound according to claim 1 or a pharmaceutically acceptable salt thereof, wherein ELB has a structure represented by Formula 2 below: 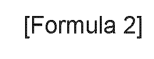 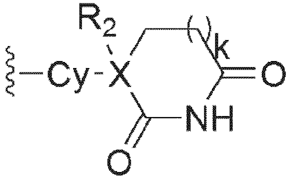 wherein in Formula 2 above:
Cy is C₆₋₁₀ aryl, 5-10 membered heteroaryl, or 5-10 membered heterocycloalkyl;
k is an integer selected from 0 to 2;
X is N or C;
R₂ is absent, hydrogen, deuterium (D), or C₁-C₃ alkyl; and
the aryl, heteroaryl, or heterocycloalkyl is unsubstituted or substituted with one or more selected from the group consisting of oxo, halogen, nitro, amino, hydroxy, carboxy, C₁₋₆ alkyl, and C₁₋₆ alkoxy.

3. The compound according to claim 2 or a pharmaceutically acceptable salt thereof, wherein Formula 2 is represented by any one of the structures below: 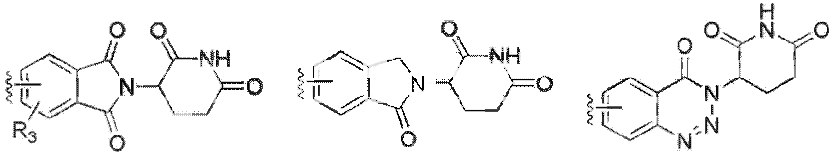 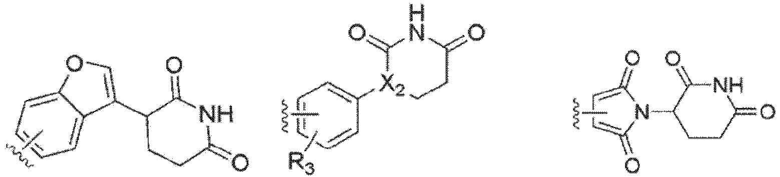 (R₃ is hydrogen or halogen, and X₂ is CH or N).

4. The compound according to claim 2 or a pharmaceutically acceptable salt thereof, wherein Formula 2 is represented by any one of the structures below:

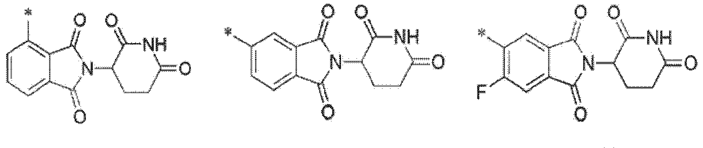

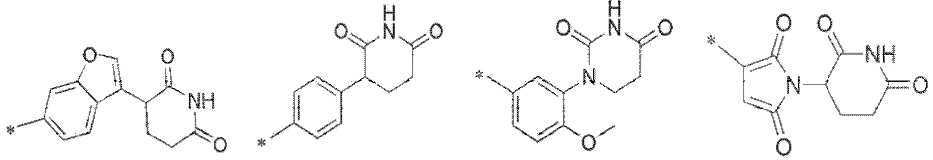

5. The compound according to claim 1 or a pharmaceutically acceptable salt thereof, wherein the R₁ is C₁₋₆ alkyl, -C₁₋₃ alkyl-phenyl, phenyl, -C₁₋₃ alkyl-pyridyl, or pyridyl, wherein the phenyl or pyridyl is unsubstituted or substituted with one or more substituents selected from the group consisting of C₁₋₃ alkyl, halogen, cyano, and halo-C₁₋₃ alkyl.

6. The compound according to claim 1 or a pharmaceutically acceptable salt thereof, wherein the R₁ is represented by any one of the structures below:

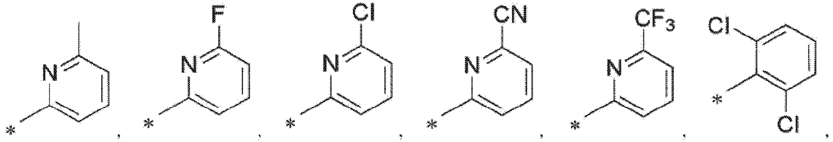

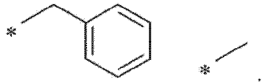

7. The compound according to claim 1 or a pharmaceutically acceptable salt thereof, wherein the A has a structure represented by Formula 3 below: 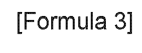 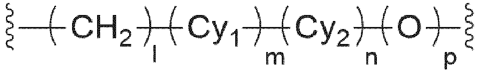 wherein in Formula 3 above:
Cy₁ is C₆₋₁₀ aryl or 5-10 membered heteroaryl, unsubstituted or substituted with C₁₋₃ alkyl;
Cy₂ is 3-10 membered heterocycloalkyl; and
I is an integer selected from 0 to 4, and m, n, and p are independently 0 or 1, wherein all of l, m, n, and p are not simultaneously 0.

8. The compound according to claim 7 or a pharmaceutically acceptable salt thereof, wherein the A is represented by any one of the structures below:

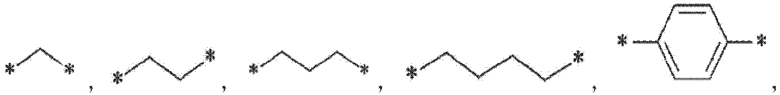

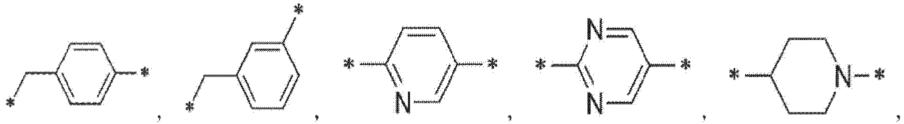

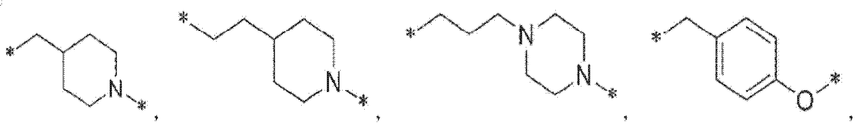

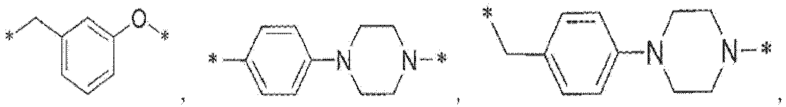

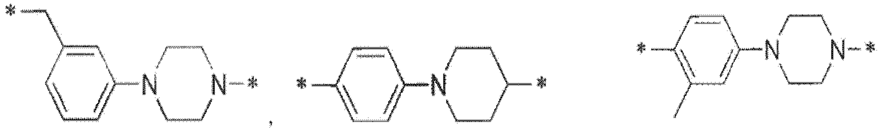

9. The compound according to claim 1 or a pharmaceutically acceptable salt thereof, wherein the linker is -L1-L2-;
L2 is a direct linker, 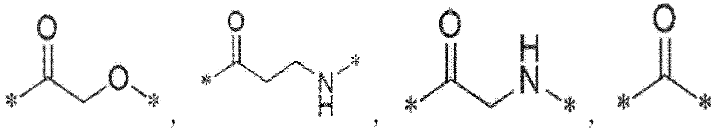 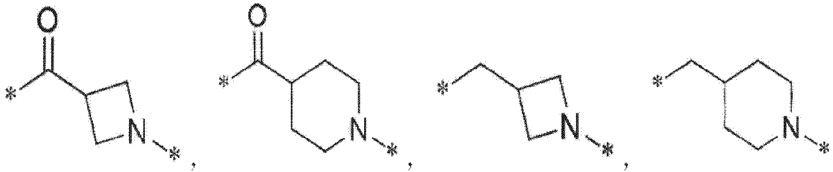 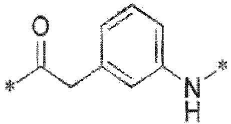 and
L1 is 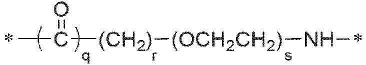 or 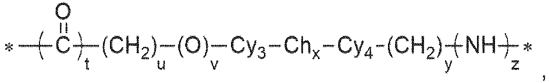
wherein:
q, t, v, x, and z are independently 0 or 1;
r, s, u, and y are independently an integer selected from 0 to 6;
Ch is C₁₋₄ alkenyl or C₁₋₄ alkynyl; and
Cy₃ and Cy₄ are independently absent, 3-10 membered heterocycloalkyl, or C₃₋₁₀ cycloalkyl.

10. The compound according to claim 9 or a pharmaceutically acceptable salt thereof, wherein L₁ is represented by any one of the structures below:

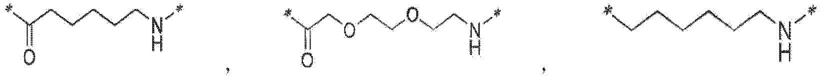

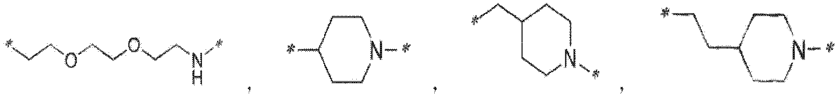

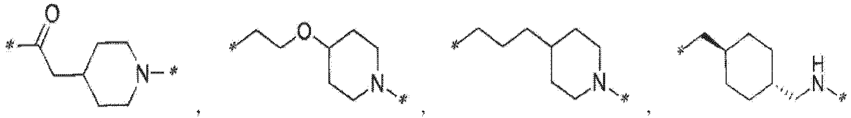

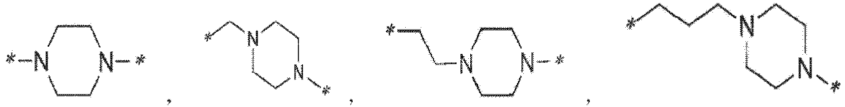

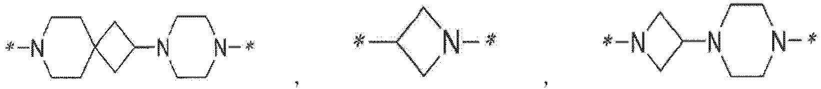

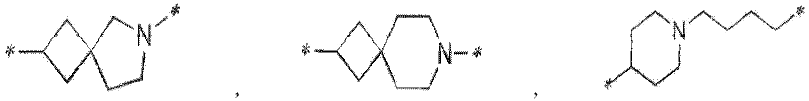

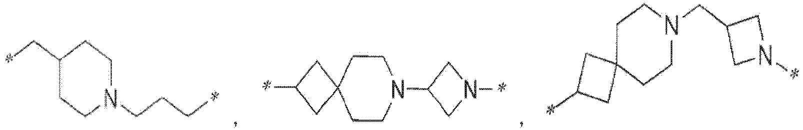

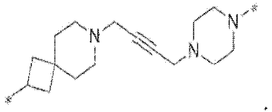

11. The compound according to claim 1 or a pharmaceutically acceptable salt thereof, wherein the compound is:
N-((1s,3s)-3-(6-((1-(6-(2-((2-(2,6-dioxopiperidin-3-yl)-1,3-dioxoisoindolin-5-yl)amino)acetamido)hexanoyl)piperidin-4-yl)amino)-9H-purin-9-yl)cyclobutyl)-6-methylpicolinamide,
N-((1s,3s)-3-(6-((1-(6-(2-((2-(2,6-dioxopiperidin-3-yl)-1,3-dioxoisoindolin-4-yl)amino)acetamido)hexanoyl)piperidin-4-yl)amino)-9H-purin-9-yl)cyclobutyl)-6-methylpicolinamide,
N-((1s,3s)-3-(6-((1-(6-(2-((2-(2,6-dioxopiperidin-3-yl)-1,3-dioxoisoindolin-5-yl)oxy)acetamido)hexanoyl)piperidin-4-yl)amino)-9H-purin-9-yl)cyclobutyl)-6-methylpicolinamide,
N-((1s,3s)-3-(6-((1-(6-(2-((2-(2,6-dioxopiperidin-3-yl)-1,3-dioxoisoindolin-4-yl)oxy)acetamido)hexanoyl)piperidin-4-yl)amino)-9H-purin-9-yl)cyclobutyl)-6-methylpicolinamide,
N-((1s,3s)-3-(6-((1-(6-((2-(2,6-dioxopiperidin-3-yl)-1,3-dioxoisoindolin-5-yl)amino)hexanoyl)piperidin-4-yl)amino)-9H-purin-9-yl)cyclobutyl)-6-methylpicolinamide,
N-((1s,3s)-3-(6-((1-(6-((2-(2,6-dioxopiperidin-3-yl)-1,3-dioxoisoindolin-4-yl)amino)hexanoyl)piperidin-4-yl)amino)-9H-purin-9-yl)cyclobutyl)-6-methylpicolinamide,
N-((1s,3s)-3-(6-((1-(2-(2-(2-(2-((2-(2,6-dioxopiperidin-3-yl)-1,3-dioxoisoindolin-5-yl)oxy)acetamido)ethoxy)ethoxy)acetyl)piperidin-4-yl)amino)-9H-purin-9-yl)cyclobutyl)-6-methylpicolinamide,
N-((1s,3s)-3-(6-((1-(2-(2-(2-(2-((2-(2,6-dioxopiperidin-3-yl)-1,3-dioxoisoindolin-4-yl)oxy)acetamido)ethoxy)ethoxy)acetyl)piperidin-4-yl)amino)-9H-purin-9-yl)cyclobutyl)-6-methylpicolinamide,
N-((1s,3s)-3-(6-((1-(2-(2-(2-(2-((2-(2,6-dioxopiperidin-3-yl)-1,3-dioxoisoindolin-5-yl)amino)acetamido)ethoxy)ethoxy)acetyl)piperidin-4-yl)amino)-9H-purin-9-yl)cyclobutyl)-6-methylpicolinamide,
N-((1s,3s)-3-(6-((1-(2-(2-(2-(2-((2-(2,6-dioxopiperidin-3-yl)-1,3-dioxoisoindolin-4-yl)amino)acetamido)ethoxy)ethoxy)acetyl)piperidin-4-yl)amino)-9H-purin-9-yl)cyclobutyl)-6-methylpicolinamide,
N-((1s,3s)-3-(6-((1-(2-(2-(2-((2-(2,6-dioxopiperidin-3-yl)-1,3-dioxoisoindolin-5-yl)amino)ethoxy)ethoxy)acetyl)piperidin-4-yl)amino)-9H-purin-9-yl)cyclobutyl)-6-methylpicolinamide,
N-((1s,3s)-3-(6-((1-(2-(2-(2-((2-(2,6-dioxopiperidin-3-yl)-1,3-dioxoisoindolin-4-yl)amino)ethoxy)ethoxy)acetyl)piperidin-4-yl)amino)-9H-purin-9-yl)cyclobutyl)-6-methylpicolinamide,
N-((1s,3s)-3-(6-((1-(6-(2-((2-(2,6-dioxopiperidin-3-yl)-1,3-dioxoisoindolin-5-yl)amino)acetamido)hexyl)piperidin-4-yl)amino)-9H-purin-9-yl)cyclobutyl)-6-methylpicolinamide,
N-((1s,3s)-3-(6-((1-(6-(2-((2-(2,6-dioxopiperidin-3-yl)-1,3-dioxoisoindolin-4-yl)amino)acetamido)hexyl)piperidin-4-yl)amino)-9H-purin-9-yl)cyclobutyl)-6-methylpicolinamide,
N-((1s,3s)-3-(6-((1-(6-(2-((2-(2,6-dioxopiperidin-3-yl)-1,3-dioxoisoindolin-5-yl)oxy)acetamido)hexyl)piperidin-4-yl)amino)-9H-purin-9-yl)cyclobutyl)-6-methylpicolinamide,
N-((1s,3s)-3-(6-((1-(6-(2-((2-(2,6-dioxopiperidin-3-yl)-1,3-dioxoisoindolin-4-yl)oxy)acetamido)hexyl)piperidin-4-yl)amino)-9H-purin-9-yl)cyclobutyl)-6-methylpicolinamide,
N-((1s,3s)-3-(6-((1-(6-((2-(2,6-dioxopiperidin-3-yl)-1,3-dioxoisoindolin-5-yl)amino)hexyl)piperidin-4-yl)amino)-9H-purin-9-yl)cyclobutyl)-6-methylpicolinamide,
N-((1s,3s)-3-(6-((1-(6-((2-(2,6-dioxopiperidin-3-yl)-1,3-dioxoisoindolin-4-yl)amino)hexyl)piperidin-4-yl)amino)-9H-purin-9-yl)cyclobutyl)-6-methylpicolinamide,
N-((1s,3s)-3-(6-((1-(2-(2-(2-(2-((2-(2,6-dioxopiperidin-3-yl)-1,3-dioxoisoindolin-5-yl)amino)acetamido)ethoxy)ethoxy)ethyl)piperidin-4-yl)amino)-9H-purin-9-yl)cyclobutyl)-6-methylpicolinamide,
N-((1s,3s)-3-(6-((1-(2-(2-(2-(2-((2-(2,6-dioxopiperidin-3-yl)-1,3-dioxoisoindolin-4-yl)amino)acetamido)ethoxy)ethoxy)ethyl)piperidin-4-yl)amino)-9H-purin-9-yl)cyclobutyl)-6-methylpicolinamide,
N-((1s,3s)-3-(6-((1-(2-(2-(2-(2-((2-(2,6-dioxopiperidin-3-yl)-1,3-dioxoisoindolin-5-yl)oxy)acetamido)ethoxy)ethoxy)ethyl)piperidin-4-yl)amino)-9H-purin-9-yl)cyclobutyl)-6-methylpicolinamide,
N-((1s,3s)-3-(6-((1-(2-(2-(2-(2-((2-(2,6-dioxopiperidin-3-yl)-1,3-dioxoisoindolin-4-yl)oxy)acetamido)ethoxy)ethoxy)ethyl)piperidin-4-yl)amino)-9H-purin-9-yl)cyclobutyl)-6-methylpicolinamide,
N-((1s,3s)-3-(6-((1-(2-(2-(2-((2-(2,6-dioxopiperidin-3-yl)-1,3-dioxoisoindolin-5-yl)amino)ethoxy)ethoxy)ethyl)piperidin-4-yl)amino)-9H-purin-9-yl)cyclobutyl)-6-methylpicolinamide,
N-((1s,3s)-3-(6-((1-(2-(2-(2-((2-(2,6-dioxopiperidin-3-yl)-1,3-dioxoisoindolin-4-yl)amino)ethoxy)ethoxy)ethyl)piperidin-4-yl)amino)-9H-purin-9-yl)cyclobutyl)-6-methylpicolinamide,
N-((1s,3s)-3-(6-((1-((1-((2-(2,6-dioxopiperidin-3-yl)-1,3-dioxoisoindolin-5-yl)glycyl)piperidin-4-yl)methyl)piperidin-4-yl)amino)-9H-purin-9-yl)cyclobutyl)-6-methylpicolinamide,
N-((1s,3s)-3-(6-((1-((1-((2-(2,6-dioxopiperidin-3-yl)-1,3-dioxoisoindolin-4-yl)glycyl)piperidin-4-yl)methyl)piperidin-4-yl)amino)-9H-purin-9-yl)cyclobutyl)-6-methylpicolinamide,
N-((1s,3s)-3-(6-((1-((1-(2-((2-(2,6-dioxopiperidin-3-yl)-1,3-dioxoisoindolin-5-yl)oxy)acetyl)piperidin-4-yl)methyl)piperidin-4-yl)amino)-9H-purin-9-yl)cyclobutyl)-6-methylpicolinamide,
N-((1s,3s)-3-(6-((1-((1-(2-((2-(2,6-dioxopiperidin-3-yl)-1,3-dioxoisoindolin-4-yl)oxy)acetyl)piperidin-4-yl)methyl)piperidin-4-yl)amino)-9H-purin-9-yl)cyclobutyl)-6-methylpicolinamide,
N-((1s,3s)-3-(6-((1-((1-(2-(2,6-dioxopiperidin-3-yl)-1,3-dioxoisoindolin-5-yl)piperidin-4-yl)methyl)piperidin-4-yl)amino)-9H-purin-9-yl)cyclobutyl)-6-methylpicolinamide,
N-((1s,3s)-3-(6-((1-((1-(2-(2,6-dioxopiperidin-3-yl)-1,3-dioxoisoindolin-4-yl)piperidin-4-yl)methyl)piperidin-4-yl)amino)-9H-purin-9-yl)cyclobutyl)-6-methylpicolinamide,
N-((1s,3s)-3-(6-((1-(2-(1-(2-((2-(2,6-dioxopiperidin-3-yl)-1,3-dioxoisoindolin-5-yl)oxy)acetyl)piperidin-4-yl)ethyl)piperidin-4-yl)amino)-9H-purin-9-yl)cyclobutyl)-6-methylpicolinamide,
N-((1s,3s)-3-(6-((1-(2-(1-(2-((2-(2,6-dioxopiperidin-3-yl)-1,3-dioxoisoindolin-4-yl)oxy)acetyl)piperidin-4-yl)ethyl)piperidin-4-yl)amino)-9H-purin-9-yl)cyclobutyl)-6-methylpicolinamide,
N-((1s,3s)-3-(6-((1-(2-(1-((2-(2,6-dioxopiperidin-3-yl)-1,3-dioxoisoindolin-5-yl)glycyl)piperidin-4-yl)ethyl)piperidin-4-yl)amino)-9H-purin-9-yl)cyclobutyl)-6-methylpicolinamide,
N-((1s,3s)-3-(6-((1-(2-(1-((2-(2,6-dioxopiperidin-3-yl)-1,3-dioxoisoindolin-4-yl)glycyl)piperidin-4-yl)ethyl)piperidin-4-yl)amino)-9H-purin-9-yl)cyclobutyl)-6-methylpicolinamide,
N-((1s,3s)-3-(6-((1-(2-(1-(2-(2,6-dioxopiperidin-3-yl)-1,3-dioxoisoindolin-5-yl)piperidin-4-yl)ethyl)piperidin-4-yl)amino)-9H-purin-9-yl)cyclobutyl)-6-methylpicolinamide,
N-((1s,3s)-3-(6-((1-(2-(1-(2-(2,4-dioxocyclohexyl)-1,3-dioxoisoindolin-4-yl)piperidin-4-yl)ethyl)piperidin-4-yl)amino)-9H-purin-9-yl)cyclobutyl)-6-methylpicolinamide,
N-((1s,3s)-3-(6-((1-(3-(4-((2-(2,6-dioxopiperidin-3-yl)-1,3-dioxoisoindolin-5-yl)glycyl)piperazin-1-yl)propyl)piperidin-4-yl)amino)-9H-purin-9-yl)cyclobutyl)-6-methylpicolinamide,
N-((1s,3s)-3-(6-((1-(3-(4-((2-(2,6-dioxopiperidin-3-yl)-1,3-dioxoisoindolin-4-yl)glycyl)piperazin-1-yl)propyl)piperidin-4-yl)amino)-9H-purin-9-yl)cyclobutyl)-6-methylpicolinamide,
N-((1s,3s)-3-(6-((1-(3-(4-(2-((2-(2,6-dioxopiperidin-3-yl)-1,3-dioxoisoindolin-5-yl)oxy)acetyl)piperazin-1-yl)propyl)piperidin-4-yl)amino)-9H-purin-9-yl)cyclobutyl)-6-methylpicolinamide,
N-((1s,3s)-3-(6-((1-(3-(4-(2-((2-(2,6-dioxopiperidin-3-yl)-1,3-dioxoisoindolin-4-yl)oxy)acetyl)piperazin-1-yl)propyl)piperidin-4-yl)amino)-9H-purin-9-yl)cyclobutyl)-6-methylpicolinamide,
N-((1s,3s)-3-(6-((1-(3-(4-(2-(2,6-dioxopiperidin-3-yl)-1,3-dioxoisoindolin-5-yl)piperazin-1-yl)propyl)piperidin-4-yl)amino)-9H-purin-9-yl)cyclobutyl)-6-methylpicolinamide,
N-((1s,3s)-3-(6-((1-(3-(4-(2-(2,6-dioxopiperidin-3-yl)-1,3-dioxoisoindolin-4-yl)piperazin-1-yl)propyl)piperidin-4-yl)amino)-9H-purin-9-yl)cyclobutyl)-6-methylpicolinamide,
N-((1s,3s)-3-(6-((1-(2-((1-(2-((2-(2,6-dioxopiperidin-3-yl)-1,3-dioxoisoindolin-5-yl)oxy)acetyl)piperidin-4-yl)oxy)ethyl)piperidin-4-yl)amino)-9H-purin-9-yl)cyclobutyl)-6-methylpicolinamide,
N-((1s,3s)-3-(6-((1-(2-((1-(2-((2-(2,6-dioxopiperidin-3-yl)-1,3-dioxoisoindolin-4-yl)oxy)acetyl)piperidin-4-yl)oxy)ethyl)piperidin-4-yl)amino)-9H-purin-9-yl)cyclobutyl)-6-methylpicolinamide,
N-((1s,3s)-3-(6-((1-(2-((1-((2-(2,6-dioxopiperidin-3-yl)-1,3-dioxoisoindolin-5-yl)glycyl)piperidin-4-yl)oxy)ethyl)piperidin-4-yl)amino)-9H-purin-9-yl)cyclobutyl)-6-methylpicolinamide,
N-((1s,3s)-3-(6-((1-(2-((1-((2-(2,6-dioxopiperidin-3-yl)-1,3-dioxoisoindolin-4-yl)glycyl)piperidin-4-yl)oxy)ethyl)piperidin-4-yl)amino)-9H-purin-9-yl)cyclobutyl)-6-methylpicolinamide,
N-((1s,3s)-3-(6-((1-(2-((1-(2-(2,6-dioxopiperidin-3-yl)-1,3-dioxoisoindolin-5-yl)piperidin-4-yl)oxy)ethyl)piperidin-4-yl)amino)-9H-purin-9-yl)cyclobutyl)-6-methylpicolinamide,
N-((1s,3s)-3-(6-((1-(2-((1-(2-(2,6-dioxopiperidin-3-yl)-1,3-dioxoisoindolin-4-yl)piperidin-4-yl)oxy)ethyl)piperidin-4-yl)amino)-9H-purin-9-yl)cyclobutyl)-6-methylpicolinamide,
N-((1s,3s)-3-(6-((1-(2-(1-(2-((2-(2,6-dioxopiperidin-3-yl)-1,3-dioxoisoindolin-5-yl)oxy)acetyl)piperidin-4-yl)acetyl)piperidin-4-yl)amino)-9H-purin-9-yl)cyclobutyl)-6-methylpicolinamide,
N-((1s,3s)-3-(6-((1-(2-(1-(2-((2-(2,6-dioxopiperidin-3-yl)-1,3-dioxoisoindolin-4-yl)oxy)acetyl)piperidin-4-yl)acetyl)piperidin-4-yl)amino)-9H-purin-9-yl)cyclobutyl)-6-methylpicolinamide,
N-((1s,3s)-3-(6-((1-(2-(1-((2-(2,6-dioxopiperidin-3-yl)-1,3-dioxoisoindolin-5-yl)glycyl)piperidin-4-yl)acetyl)piperidin-4-yl)amino)-9H-purin-9-yl)cyclobutyl)-6-methylpicolinamide,
N-((1s,3s)-3-(6-((1-(2-(1-((2-(2,6-dioxopiperidin-3-yl)-1,3-dioxoisoindolin-4-yl)glycyl)piperidin-4-yl)acetyl)piperidin-4-yl)amino)-9H-purin-9-yl)cyclobutyl)-6-methylpicolinamide,
N-((1s,3s)-3-(6-((1-(2-(1-(2-(2,6-dioxopiperidin-3-yl)-1,3-dioxoisoindolin-5-yl)piperidin-4-yl)acetyl)piperidin-4-yl)amino)-9H-purin-9-yl)cyclobutyl)-6-methylpicolinamide,
N-((1s,3s)-3-(6-((1-(2-(1-(2-(2,6-dioxopiperidin-3-yl)-1,3-dioxoisoindolin-4-yl)piperidin-4-yl)acetyl)piperidin-4-yl)amino)-9H-purin-9-yl)cyclobutyl)-6-methylpicolinamide,
N-((1s,3s)-3-(6-(((1-((2-(2,6-dioxopiperidin-3-yl)-1, 3-dioxoisoindolin-5-yl)glycyl)piperidin-4-yl)methyl)amino)-9H-purin-9-yl)cyclobutyl)-6-methylpicolinamide,
N-((1s,3s)-3-(6-(((1-((2-(2,6-dioxopiperidin-3-yl)-1,3-dioxoisoindolin-4-yl)glycyl)piperidin-4-yl)methyl)amino)-9H-purin-9-yl)cyclobutyl)-6-methylpicolinamide,
N-((1s,3s)-3-(6-(((1-(2-((2-(2,6-dioxopiperidin-3-yl)-1,3-dioxoisoindolin-5-yl)oxy)acetyl)piperidin-4-yl)methyl)amino)-9H-purin-9-yl)cyclobutyl)-6-methylpicolinamide,
N-((1s,3s)-3-(6-(((1-(2-((2-(2,6-dioxopiperidin-3-yl)-1,3-dioxoisoindolin-4-yl)oxy)acetyl)piperidin-4-yl)methyl)amino)-9H-purin-9-yl)cyclobutyl)-6-methylpicolinamide,
N-((1s,3s)-3-(6-(((1-(2-(2,6-dioxopiperidin-3-yl)-1,3-dioxoisoindolin-5-yl)piperidin-4-yl)methyl)amino)-9H-purin-9-yl)cyclobutyl)-6-methylpicolinamide,
N-((1s,3s)-3-(6-(((1-(2-(2,6-dioxopiperidin-3-yl)-1,3-dioxoisoindolin-4-yl)piperidin-4-yl)methyl)amino)-9H-purin-9-yl)cyclobutyl)-6-methylpicolinamide,
N-((1r,3r)-3-(6-(((1-(6-(2-((2-(2,6-dioxopiperidin-3-yl)-1,3-dioxoisoindolin-5-yl)amino)acetamido)hexanoyl)piperidin-4-yl)methyl)amino)-9H-purin-9-yl)cyclobutyl)-6-methylpicolinamide,
N-((1r,3r)-3-(6-(((1-(6-(2-((2-(2,6-dioxopiperidin-3-yl)-1,3-dioxoisoindolin-4-yl)amino)acetamido)hexanoyl)piperidin-4-yl)methyl)amino)-9H-purin-9-yl)cyclobutyl)-6-methylpicolinamide,
N-((1r,3r)-3-(6-(((1-(6-(2-((2-(2,6-dioxopiperidin-3-yl)-1,3-dioxoisoindolin-5-yl)oxy)acetamido)hexanoyl)piperidin-4-yl)methyl)amino)-9H-purin-9-yl)cyclobutyl)-6-methylpicolinamide,
N-((1r,3r)-3-(6-(((1-(6-(2-((2-(2,6-dioxopiperidin-3-yl)-1,3-dioxoisoindolin-4-yl)oxy)acetamido)hexanoyl)piperidin-4-yl)methyl)amino)-9H-purin-9-yl)cyclobutyl)-6-methylpicolinamide,
N-((1r,3r)-3-(6-(((1-(6-((2-(2,6-dioxopiperidin-3-yl)-1,3-dioxoisoindolin-5-yl)amino)hexanoyl)piperidin-4-yl)methyl)amino)-9H-purin-9-yl)cyclobutyl)-6-methylpicolinamide,
N-((1r,3r)-3-(6-(((1-(6-((2-(2,6-dioxopiperidin-3-yl)-1,3-dioxoisoindolin-4-yl)amino)hexanoyl)piperidin-4-yl)methyl)amino)-9H-purin-9-yl)cyclobutyl)-6-methylpicolinamide,
N-((1s,3s)-3-(6-(((1-(2-(2-(2-(2-((2-(2,6-dioxopiperidin-3-yl)-1,3-dioxoisoindolin-5-yl)oxy)acetamido)ethoxy)ethoxy)acetyl)piperidin-4-yl)methyl)amino)-9H-purin-9-yl)cyclobutyl)-6-methylpicolinamide,
N-((1s,3s)-3-(6-(((1-(2-(2-(2-(2-((2-(2,6-dioxopiperidin-3-yl)-1,3-dioxoisoindolin-4-yl)oxy)acetamido)ethoxy)ethoxy)acetyl)piperidin-4-yl)methyl)amino)-9H-purin-9-yl)cyclobutyl)-6-methylpicolinamide,
N-((1s,3s)-3-(6-(((1-(2-(2-(2-(2-((2-(2,6-dioxopiperidin-3-yl)-1,3-dioxoisoindolin-5-yl)amino)acetamido)ethoxy)ethoxy)acetyl)piperidin-4-yl)methyl)amino)-9H-purin-9-yl)cyclobutyl)-6-methylpicolinamide,
N-((1s,3s)-3-(6-(((1-(2-(2-(2-(2-((2-(2,6-dioxopiperidin-3-yl)-1,3-dioxoisoindolin-4-yl)amino)acetamido)ethoxy)ethoxy)acetyl)piperidin-4-yl)methyl)amino)-9H-purin-9-yl)cyclobutyl)-6-methylpicolinamide,
N-((1s,3s)-3-(6-(((1-(2-(2-(2-((2-(2,6-dioxopiperidin-3-yl)-1,3-dioxoisoindolin-4-yl)amino)ethoxy)ethoxy)acetyl)piperidin-4-yl)methyl)amino)-9H-purin-9-yl)cyclobutyl)-6-methylpicolinamide,
N-((1r,3r)-3-(6-(((1-((1-(2-((2-(2,6-dioxopiperidin-3-yl)-1,3-dioxoisoindolin-5-yl)oxy)acetyl)piperidin-4-yl)methyl)piperidin-4-yl)methyl)amino)-9H-purin-9-yl)cyclobutyl)-6-methylpicolinamide,
N-((1r,3r)-3-(6-(((1-((1-(2-((2-(2,6-dioxopiperidin-3-yl)-1,3-dioxoisoindolin-4-yl)oxy)acetyl)piperidin-4-yl)methyl)piperidin-4-yl)methyl)amino)-9H-purin-9-yl)cyclobutyl)-6-methylpicolinamide,
N-((1r,3r)-3-(6-(((1-((1-((2-(2,6-dioxopiperidin-3-yl)-1,3-dioxoisoindolin-5-yl)glycyl)piperidin-4-yl)methyl)piperidin-4-yl)methyl)amino)-9H-purin-9-yl)cyclobutyl)-6-methylpicolinamide,
N-((1r,3r)-3-(6-(((1-((1-((2-(2,6-dioxopiperidin-3-yl)-1,3-dioxoisoindolin-4-yl)glycyl)piperidin-4-yl)methyl)piperidin-4-yl)methyl)amino)-9H-purin-9-yl)cyclobutyl)-6-methylpicolinamide,
N-((1r,3r)-3-(6-(((1-((1-(2-(2,6-dioxopiperidin-3-yl)-1,3-dioxoisoindolin-5-yl)piperidin-4-yl)methyl)piperidin-4-yl)methyl)amino)-9H-purin-9-yl)cyclobutyl)-6-methylpicolinamide,
N-((1r,3r)-3-(6-(((1-((1-(2-(2,6-dioxopiperidin-3-yl)-1,3-dioxoisoindolin-4-yl)piperidin-4-yl)methyl)piperidin-4-yl)methyl)amino)-9H-purin-9-yl)cyclobutyl)-6-methylpicolinamide,
N-((1r,3r)-3-(6-(((1-(2-(2-(2-(2-((2-(2,6-dioxopiperidin-3-yl)-1,3-dioxoisoindolin-4-yl)oxy)acetamido)ethoxy)ethoxy)ethyl)piperidin-4-yl)methyl)amino)-9H-purin-9-yl)cyclobutyl)-6-methylpicolinamide,
N-((1r,3r)-3-(6-(((1-(2-(2-(2-(2-((2-(2,6-dioxopiperidin-3-yl)-1,3-dioxoisoindolin-4-yl)amino)acetamido)ethoxy)ethoxy)ethyl)piperidin-4-yl)methyl)amino)-9H-purin-9-yl)cyclobutyl)-6-methylpicolinamide,
N-((1r,3r)-3-(6-(((1-(2-(2-(2-(2-((2-(2,6-dioxopiperidin-3-yl)-1,3-dioxoisoindolin-5-yl)oxy)acetamido)ethoxy)ethoxy)ethyl)piperidin-4-yl)methyl)amino)-9H-purin-9-yl)cyclobutyl)-6-methylpicolinamide,
N-((1r,3r)-3-(6-(((1-(2-(2-(2-(2-((2-(2,6-dioxopiperidin-3-yl)-1,3-dioxoisoindolin-5-yl)amino)acetamido)ethoxy)ethoxy)ethyl)piperidin-4-yl)methyl)amino)-9H-purin-9-yl)cyclobutyl)-6-methylpicolinamide,
N-((1s,3s)-3-(6-((4-(4-(2-(2,6-dioxopiperidin-3-yl)-1,3-dioxoisoindolin-5-yl)piperazin-1-yl)phenyl)amino)-9H-purin-9-yl)cyclobutyl)-6-methylpicolinamide,
N-((1s,3s)-3-(6-((4-(4-(2-(2,6-dioxopiperidin-3-yl)-1,3-dioxoisoindolin-4-yl)piperazin-1-yl)phenyl)amino)-9H-purin-9-yl)cyclobutyl)-6-methylpicolinamide,
N-((1s,3s)-3-(6-((4-(4-(2-((2-(2,6-dioxopiperidin-3-yl)-1,3-dioxoisoindolin-5-yl)oxy)acetyl)piperazin-1-yl)phenyl)amino)-9H-purin-9-yl)cyclobutyl)-6-methylpicolinamide,
N-((1s,3s)-3-(6-((4-(4-(2-((2-(2,6-dioxopiperidin-3-yl)-1,3-dioxoisoindolin-4-yl)oxy)acetyl)piperazin-1-yl)phenyl)amino)-9H-purin-9-yl)cyclobutyl)-6-methylpicolinamide,
N-((1s,3s)-3-(6-((4-(4-((2-(2,6-dioxopiperidin-3-yl)-1,3-dioxoisoindolin-5-yl)glycyl)piperazin-1-yl)phenyl)amino)-9H-purin-9-yl)cyclobutyl)-6-methylpicolinamide,
N-((1s,3s)-3-(6-((4-(4-((2-(2,6-dioxopiperidin-3-yl)-1,3-dioxoisoindolin-4-yl)glycyl)piperazin-1-yl)phenyl)amino)-9H-purin-9-yl)cyclobutyl)-6-methylpicolinamide,
N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)phenyl)amino)-9H-purin-9-yl)cyclobutyl)-6-methylpicolinamide,
N-((1s,3s)-3-(6-((4-(4-((1-(2-(2,6-dioxopiperidin-3-yl)-1,3-dioxoisoindolin-4-yl)piperidin-4-yl)methyl)piperazin-1-yl)phenyl)amino)-9H-purin-9-yl)cyclobutyl)-6-methylpicolinamide,
N-((1s,3s)-3-(6-((4-(4-((1-(2-((2-(2,6-dioxopiperidin-3-yl)-1,3-dioxoisoindolin-5-yl)oxy)acetyl)piperidin-4-yl)methyl)piperazin-1-yl)phenyl)amino)-9H-purin-9-yl)cyclobutyl)-6-methylpicolinamide,
N-((1s,3s)-3-(6-((4-(4-((1-(2-((2-(2,6-dioxopiperidin-3-yl)-1,3-dioxoisoindolin-4-yl)oxy)acetyl)piperidin-4-yl)methyl)piperazin-1-yl)phenyl)amino)-9H-purin-9-yl)cyclobutyl)-6-methylpicolinamide,
N-((1s,3s)-3-(6-((4-(4-((1-((2-(2,6-dioxopiperidin-3-yl)-1,3-dioxoisoindolin-5-yl)glycyl)piperidin-4-yl)methyl)piperazin-1-yl)phenyl)amino)-9H-purin-9-yl)cyclobutyl)-6-methylpicolinamide,
N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)phenyl)amino)-9H-purin-9-yl)cyclobutyl)-6-methylpicolinamide,
N-((1s,3s)-3-(6-((3-((1-(2-((2-(2,6-dioxopiperidin-3-yl)-1,3-dioxoisoindolin-5-yl)oxy)acetyl)piperidin-4-yl)methoxy)benzyl)amino)-9H-purin-9-yl)cyclobutyl)-6-methylpicolinamide,
N-((1s,3s)-3-(6-((3-((1-(2-((2-(2,6-dioxopiperidin-3-yl)-1,3-dioxoisoindolin-4-yl)oxy)acetyl)piperidin-4-yl)methoxy)benzyl)amino)-9H-purin-9-yl)cyclobutyl)-6-methylpicolinamide,
N-((1s,3s)-3-(6-((3-((1-((2-(2,6-dioxopiperidin-3-yl)-1,3-dioxoisoindolin-5-yl)glycyl)piperidin-4-yl)methoxy)benzyl)amino)-9H-purin-9-yl)cyclobutyl)-6-methylpicolinamide,
N-((1s,3s)-3-(6-((3-((1-((2-(2,6-dioxopiperidin-3-yl)-1,3-dioxoisoindolin-4-yl)glycyl)piperidin-4-yl)methoxy)benzyl)amino)-9H-purin-9-yl)cyclobutyl)-6-methylpicolinamide,
N-((1s,3s)-3-(6-((3-((1-(2-(2,6-dioxopiperidin-3-yl)-1,3-dioxoisoindolin-5-yl)piperidin-4-yl)methoxy)benzyl)amino)-9H-purin-9-yl)cyclobutyl)-6-methylpicolinamide,
N-((1s,3s)-3-(6-((3-((1-(2-(2,6-dioxopiperidin-3-yl)-1,3-dioxoisoindolin-4-yl)piperidin-4-yl)methoxy)benzyl)amino)-9H-purin-9-yl)cyclobutyl)-6-methylpicolinamide,
N-((1s,3s)-3-(6-((3-(3-(4-(2-((2-(2,6-dioxopiperidin-3-yl)-1,3-dioxoisoindolin-5-yl)oxy)acetyl)piperazin-1-yl)propoxy)benzyl)amino)-9H-purin-9-yl)cyclobutyl)-6-methylpicolinamide,
N-((1s,3s)-3-(6-((3-(3-(4-(2-((2-(2,6-dioxopiperidin-3-yl)-1,3-dioxoisoindolin-4-yl)oxy)acetyl)piperazin-1-yl)propoxy)benzyl)amino)-9H-purin-9-yl)cyclobutyl)-6-methylpicolinamide,
N-((1s,3s)-3-(6-((3-(3-(4-((2-(2,6-dioxopiperidin-3-yl)-1,3-dioxoisoindolin-5-yl)glycyl)piperazin-1-yl)propoxy)benzyl)amino)-9H-purin-9-yl)cyclobutyl)-6-methylpicolinamide,
N-((1s,3s)-3-(6-((3-(3-(4-((2-(2,6-dioxopiperidin-3-yl)-1,3-dioxoisoindolin-4-yl)glycyl)piperazin-1-yl)propoxy)benzyl)amino)-9H-purin-9-yl)cyclobutyl)-6-methylpicolinamide,
N-((1s,3s)-3-(6-((3-(3-(4-(2-(2,6-dioxopiperidin-3-yl)-1,3-dioxoisoindolin-5-yl)piperazin-1-yl)propoxy)benzyl)amino)-9H-purin-9-yl)cyclobutyl)-6-methylpicolinamide,
N-((1s,3s)-3-(6-((3-(3-(4-(2-(2,6-dioxopiperidin-3-yl)-1,3-dioxoisoindolin-4-yl)piperazin-1-yl)propoxy)benzyl)amino)-9H-purin-9-yl)cyclobutyl)-6-methylpicolinamide,
N-((1s,3s)-3-(6-((4-((1-(2-((2-(2,6-dioxopiperidin-3-yl)-1,3-dioxoisoindolin-5-yl)oxy)acetyl)piperidin-4-yl)methoxy)benzyl)amino)-9H-purin-9-yl)cyclobutyl)-6-methylpicolinamide,
N-((1s,3s)-3-(6-((4-((1-(2-((2-(2,6-dioxopiperidin-3-yl)-1,3-dioxoisoindolin-4-yl)oxy)acetyl)piperidin-4-yl)methoxy)benzyl)amino)-9H-purin-9-yl)cyclobutyl)-6-methylpicolinamide,
N-((1s,3s)-3-(6-((4-((1-((2-(2,6-dioxopiperidin-3-yl)-1,3-dioxoisoindolin-5-yl)glycyl)piperidin-4-yl)methoxy)benzyl)amino)-9H-purin-9-yl)cyclobutyl)-6-methylpicolinamide,
N-((1s,3s)-3-(6-((4-((1-((2-(2,6-dioxopiperidin-3-yl)-1,3-dioxoisoindolin-4-yl)glycyl)piperidin-4-yl)methoxy)benzyl)amino)-9H-purin-9-yl)cyclobutyl)-6-methylpicolinamide,
N-((1s,3s)-3-(6-((4-((1-(2-(2,6-dioxopiperidin-3-yl)-1,3-dioxoisoindolin-5-yl)piperidin-4-yl)methoxy)benzyl)amino)-9H-purin-9-yl)cyclobutyl)-6-methylpicolinamide,
N-((1s,3s)-3-(6-((4-((1-(2-(2,6-dioxopiperidin-3-yl)-1,3-dioxoisoindolin-4-yl)piperidin-4-yl)methoxy)benzyl)amino)-9H-purin-9-yl)cyclobutyl)-6-methylpicolinamide,
N-((1s,3s)-3-(6-((4-(3-(4-(2-((2-(2,6-dioxopiperidin-3-yl)-1,3-dioxoisoindolin-5-yl)oxy)acetyl)piperazin-1-yl)propoxy)benzyl)amino)-9H-purin-9-yl)cyclobutyl)-6-methylpicolinamide,
N-((1s,3s)-3-(6-((4-(3-(4-(2-((2-(2,6-dioxopiperidin-3-yl)-1,3-dioxoisoindolin-4-yl)oxy)acetyl)piperazin-1-yl)propoxy)benzyl)amino)-9H-purin-9-yl)cyclobutyl)-6-methylpicolinamide,
N-((1s,3s)-3-(6-((4-(3-(4-((2-(2,6-dioxopiperidin-3-yl)-1,3-dioxoisoindolin-5-yl)glycyl)piperazin-1-yl)propoxy)benzyl)amino)-9H-purin-9-yl)cyclobutyl)-6-methylpicolinamide,
N-((1s,3s)-3-(6-((4-(3-(4-((2-(2,6-dioxopiperidin-3-yl)-1,3-dioxoisoindolin-4-yl)glycyl)piperazin-1-yl)propoxy)benzyl)amino)-9H-purin-9-yl)cyclobutyl)-6-methylpicolinamide,
N-((1s,3s)-3-(6-((4-(3-(4-(2-(2,6-dioxopiperidin-3-yl)-1,3-dioxoisoindolin-5-yl)piperazin-1-yl)propoxy)benzyl)amino)-9H-purin-9-yl)cyclobutyl)-6-methylpicolinamide,
N-((1s,3s)-3-(6-((4-(3-(4-(2-(2,6-dioxopiperidin-3-yl)-1,3-dioxoisoindolin-4-yl)piperazin-1-yl)propoxy)benzyl)amino)-9H-purin-9-yl)cyclobutyl)-6-methylpicolinamide,
N-((1s,3s)-3-(6-((2-(1-(2-((2-(2,6-dioxopiperidin-3-yl)-1,3-dioxoisoindolin-4-yl)oxy)acetyl)piperidin-4-yl)ethyl)amino)-9H-purin-9-yl)cyclobutyl)-6-methylpicolinamide,
N-((1s,3s)-3-(6-((2-(1-((2-(2,6-dioxopiperidin-3-yl)-1,3-dioxoisoindolin-4-yl)glycyl)piperidin-4-yl)ethyl)amino)-9H-purin-9-yl)cyclobutyl)-6-methylpicolinamide,
N-((1s,3s)-3-(6-((2-(1-(2-((2-(2,6-dioxopiperidin-3-yl)-1,3-dioxoisoindolin-5-yl)oxy)acetyl)piperidin-4-yl)ethyl)amino)-9H-purin-9-yl)cyclobutyl)-6-methylpicolinamide,
N-((1s,3s)-3-(6-((2-(1-((2-(2,6-dioxopiperidin-3-yl)-1,3-dioxoisoindolin-5-yl)glycyl)piperidin-4-yl)ethyl)amino)-9H-purin-9-yl)cyclobutyl)-6-methylpicolinamide,
N-((1s,3s)-3-(6-((2-(1-((1-(2-((2-(2,6-dioxopiperidin-3-yl)-1,3-dioxoisoindolin-4-yl)oxy)acetyl)piperidin-4-yl)methyl)piperidin-4-yl)ethyl)amino)-9H-purin-9-yl)cyclobutyl)-6-methylpicolinamide,
N-((1s,3s)-3-(6-((2-(1-((1-((2-(2,6-dioxopiperidin-3-yl)-1,3-dioxoisoindolin-4-yl)glycyl)piperidin-4-yl)methyl)piperidin-4-yl)ethyl)amino)-9H-purin-9-yl)cyclobutyl)-6-methylpicolinamide,
N-((1s,3s)-3-(6-((2-(1-((1-(2-((2-(2,6-dioxopiperidin-3-yl)-1,3-dioxoisoindolin-5-yl)oxy)acetyl)piperidin-4-yl)methyl)piperidin-4-yl)ethyl)amino)-9H-purin-9-yl)cyclobutyl)-6-methylpicolinamide,
N-((1s,3s)-3-(6-((2-(1-((1-((2-(2,6-dioxopiperidin-3-yl)-1,3-dioxoisoindolin-5-yl)glycyl)piperidin-4-yl)methyl)piperidin-4-yl)ethyl)amino)-9H-purin-9-yl)cyclobutyl)-6-methylpicolinamide,
N-((1s,3s)-3-(6-((3-(4-(2-(2,6-dioxopiperidin-3-yl)-1,3-dioxoisoindolin-4-yl)piperazin-1-yl)benzyl)amino)-9H-purin-9-yl)cyclobutyl)-6-methylpicolinamide,
N-((1s,3s)-3-(6-((3-(4-(2-(2,6-dioxopiperidin-3-yl)-1,3-dioxoisoindolin-5-yl)piperazin-1-yl)benzyl)amino)-9H-purin-9-yl)cyclobutyl)-6-methylpicolinamide,
N-((1s,3s)-3-(6-((3-(4-(2-((2-(2,6-dioxopiperidin-3-yl)-1,3-dioxoisoindolin-4-yl)oxy)acetyl)piperazin-1-yl)benzyl)amino)-9H-purin-9-yl)cyclobutyl)-6-methylpicolinamide,
N-((1s,3s)-3-(6-((3-(4-(2-((2-(2,6-dioxopiperidin-3-yl)-1,3-dioxoisoindolin-5-yl)oxy)acetyl)piperazin-1-yl)benzyl)amino)-9H-purin-9-yl)cyclobutyl)-6-methylpicolinamide,
N-((1s,3s)-3-(6-((3-(4-((2-(2,6-dioxopiperidin-3-yl)-1,3-dioxoisoindolin-4-yl)glycyl)piperazin-1-yl)benzyl)amino)-9H-purin-9-yl)cyclobutyl)-6-methylpicolinamide,
N-((1s,3s)-3-(6-((3-(4-((2-(2,6-dioxopiperidin-3-yl)-1,3-dioxoisoindolin-5-yl)glycyl)piperazin-1-yl)benzyl)amino)-9H-purin-9-yl)cyclobutyl)-6-methylpicolinamide,
N-((1s,3s)-3-(6-((3-(4-((1-(2-((2-(2,6-dioxopiperidin-3-yl)-1,3-dioxoisoindolin-4-yl)oxy)acetyl)piperidin-4-yl)methyl)piperazin-1-yl)benzyl)amino)-9H-purin-9-yl)cyclobutyl)-6-methylpicolinamide,
N-((1s,3s)-3-(6-((3-(4-((1-(2-((2-(2,6-dioxopiperidin-3-yl)-1,3-dioxoisoindolin-5-yl)oxy)acetyl)piperidin-4-yl)methyl)piperazin-1-yl)benzyl)amino)-9H-purin-9-yl)cyclobutyl)-6-methylpicolinamide,
N-((1s,3s)-3-(6-((3-(4-((1-((2-(2,6-dioxopiperidin-3-yl)-1,3-dioxoisoindolin-4-yl)glycyl)piperidin-4-yl)methyl)piperazin-1-yl)benzyl)amino)-9H-purin-9-yl)cyclobutyl)-6-methylpicolinamide,
N-((1s,3s)-3-(6-((3-(4-((1-((2-(2,6-dioxopiperidin-3-yl)-1,3-dioxoisoindolin-5-yl)glycyl)piperidin-4-yl)methyl)piperazin-1-yl)benzyl)amino)-9H-purin-9-yl)cyclobutyl)-6-methylpicolinamide,
N-((1s,3s)-3-(6-((3-(4-((1-(2-(2,6-dioxopiperidin-3-yl)-1,3-dioxoisoindolin-4-yl)piperidin-4-yl)methyl)piperazin-1-yl)benzyl)amino)-9H-purin-9-yl)cyclobutyl)-6-methylpicolinamide,
N-((1s,3s)-3-(6-((3-(4-((1-(2-(2,6-dioxopiperidin-3-yl)-1,3-dioxoisoindolin-5-yl)piperidin-4-yl)methyl)piperazin-1-yl)benzyl)amino)-9H-purin-9-yl)cyclobutyl)-6-methylpicolinamide,
N-((1r,3r)-3-(6-((3-(4-(2-((2-(2,6-dioxopiperidin-3-yl)-1,3-dioxoisoindolin-4-yl)oxy)acetyl)piperazin-1-yl)propyl)amino)-9H-purin-9-yl)cyclobutyl)-6-methylpicolinamide,
N-((1r,3r)-3-(6-((3-(4-((2-(2,6-dioxopiperidin-3-yl)-1,3-dioxoisoindolin-4-yl)glycyl)piperazin-1-yl)propyl)amino)-9H-purin-9-yl)cyclobutyl)-6-methylpicolinamide,
N-((1r,3r)-3-(6-((3-(4-(2-((2-(2,6-dioxopiperidin-3-yl)-1,3-dioxoisoindolin-5-yl)oxy)acetyl)piperazin-1-yl)propyl)amino)-9H-purin-9-yl)cyclobutyl)-6-methylpicolinamide,
N-((1r,3r)-3-(6-((3-(4-((2-(2,6-dioxopiperidin-3-yl)-1,3-dioxoisoindolin-5-yl)glycyl)piperazin-1-yl)propyl)amino)-9H-purin-9-yl)cyclobutyl)-6-methylpicolinamide,
N-((1r,3r)-3-(6-((3-(4-((1-(2-((2-(2,6-dioxopiperidin-3-yl)-1,3-dioxoisoindolin-4-yl)oxy)acetyl)piperidin-4-yl)methyl)piperazin-1-yl)propyl)amino)-9H-purin-9-yl)cyclobutyl)-6-methylpicolinamide,
N-((1r,3r)-3-(6-((3-(4-((1-((2-(2,6-dioxopiperidin-3-yl)-1,3-dioxoisoindolin-4-yl)glycyl)piperidin-4-yl)methyl)piperazin-1-yl)propyl)amino)-9H-purin-9-yl)cyclobutyl)-6-methylpicolinamide,
N-((1r,3r)-3-(6-((3-(4-((1-(2-((2-(2,6-dioxopiperidin-3-yl)-1,3-dioxoisoindolin-5-yl)oxy)acetyl)piperidin-4-yl)methyl)piperazin-1-yl)propyl)amino)-9H-purin-9-yl)cyclobutyl)-6-methylpicolinamide,
N-((1r,3r)-3-(6-((3-(4-((1-((2-(2,6-dioxopiperidin-3-yl)-1,3-dioxoisoindolin-5-yl)glycyl)piperidin-4-yl)methyl)piperazin-1-yl)propyl)amino)-9H-purin-9-yl)cyclobutyl)-6-methylpicolinamide,
N-((1s,3s)-3-(6-((4-(4-(2-(2,6-dioxopiperidin-3-yl)-1,3-dioxoisoindolin-4-yl)piperazin-1-yl)benzyl)amino)-9H-purin-9-yl)cyclobutyl)-6-methylpicolinamide,
N-((1s,3s)-3-(6-((4-(4-(2-(2,6-dioxopiperidin-3-yl)-1,3-dioxoisoindolin-5-yl)piperazin-1-yl)benzyl)amino)-9H-purin-9-yl)cyclobutyl)-6-methylpicolinamide,
N-((1s,3s)-3-(6-((4-(4-((2-(2,6-dioxopiperidin-3-yl)-1,3-dioxoisoindolin-4-yl)glycyl)piperazin-1-yl)benzyl)amino)-9H-purin-9-yl)cyclobutyl)-6-methylpicolinamide,
N-((1s,3s)-3-(6-((4-(4-((2-(2,6-dioxopiperidin-3-yl)-1,3-dioxoisoindolin-5-yl)glycyl)piperazin-1-yl)benzyl)amino)-9H-purin-9-yl)cyclobutyl)-6-methylpicolinamide,
N-((1s,3s)-3-(6-((4-(4-((1-(2-(2,6-dioxopiperidin-3-yl)-1,3-dioxoisoindolin-4-yl)piperidin-4-yl)methyl)piperazin-1-yl)benzyl)amino)-9H-purin-9-yl)cyclobutyl)-6-methylpicolinamide,
N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)benzyl)amino)-9H-purin-9-yl)cyclobutyl)-6-methylpicolinamide,
N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)benzyl)amino)-9H-purin-9-yl)cyclobutyl)-6-methylpicolinamide,
N-((1s,3s)-3-(6-((4-(4-((1-((2-(2,6-dioxopiperidin-3-yl)-1,3-dioxoisoindolin-5-yl)glycyl)piperidin-4-yl)methyl)piperazin-1-yl)benzyl)amino)-9H-purin-9-yl)cyclobutyl)-6-methylpicolinamide,
N-((1s,3s)-3-(6-(((1-((1-(2-((2-(2,6-dioxopiperidin-3-yl)-1,3-dioxoisoindolin-5-yl)oxy)acetyl)piperidin-4-yl)methyl)piperidin-4-yl)methyl)amino)-9H-purin-9-yl)cyclobutyl)acetamide,
N-((1s,3s)-3-(6-(((1-((1-(2-((2-(2,6-dioxopiperidin-3-yl)-1,3-dioxoisoindolin-4-yl)oxy)acetyl)piperidin-4-yl)methyl)piperidin-4-yl)methyl)amino)-9H-purin-9-yl)cyclobutyl)acetamide,
N-((1s,3s)-3-(6-(((1-((1-((2-(2,6-dioxopiperidin-3-yl)-1,3-dioxoisoindolin-5-yl)glycyl)piperidin-4-yl)methyl)piperidin-4-yl)methyl)amino)-9H-purin-9-yl)cyclobutyl)acetamide,
N-((1s,3s)-3-(6-(((1-((1-((2-(2,6-dioxopiperidin-3-yl)-1,3-dioxoisoindolin-4-yl)glycyl)piperidin-4-yl)methyl)piperidin-4-yl)methyl)amino)-9H-purin-9-yl)cyclobutyl)acetamide,
N-((1s,3s)-3-(6-(((1-((1-(2-(2,6-dioxopiperidin-3-yl)-1,3-dioxoisoindolin-5-yl)piperidin-4-yl)methyl)piperidin-4-yl)methyl)amino)-9H-purin-9-yl)cyclobutyl)acetamide,
N-((1s,3s)-3-(6-(((1-((1-(2-(2,6-dioxopiperidin-3-yl)-1,3-dioxoisoindolin-4-yl)piperidin-4-yl)methyl)piperidin-4-yl)methyl)amino)-9H-purin-9-yl)cyclobutyl)acetamide,
N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)benzyl)amino)-9H-purin-9-yl)cyclobutyl)acetamide,
N-((1s,3s)-3-(6-((4-(4-((1-((2-(2,6-dioxopiperidin-3-yl)-1,3-dioxoisoindolin-5-yl)glycyl)piperidin-4-yl)methyl)piperazin-1-yl)benzyl)amino)-9H-purin-9-yl)cyclobutyl)acetamide,
2,6-dichloro-N-((1s,3s)-3-(6-((4-(4-((1-(2-((2-(2,6-dioxopiperidin-3-yl)-1,3-dioxoisoindolin-5-yl)oxy)acetyl)piperidin-4-yl)methyl)piperazin-1-yl)phenyl)amino)-9H-purin-9-yl)cyclobutyl)benzamide,
2,6-dichloro-N-((1s,3s)-3-(6-((4-(4-((1-(2-((2-(2,6-dioxopiperidin-3-yl)-1,3-dioxoisoindolin-4-yl)oxy)acetyl)piperidin-4-yl)methyl)piperazin-1-yl)phenyl)amino)-9H-purin-9-yl)cyclobutyl)benzamide,
2,6-dichloro-N-((1s,3s)-3-(6-((4-(4-((1-((2-(2,6-dioxopiperidin-3-yl)-1,3-dioxoisoindolin-5-yl)glycyl)piperidin-4-yl)methyl)piperazin-1-yl)phenyl)amino)-9H-purin-9-yl)cyclobutyl)benzamide,
2,6-dichloro-N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)phenyl)amino)-9H-purin-9-yl)cyclobutyl)benzamide,
2,6-dichloro-N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)phenyl)amino)-9H-purin-9-yl)cyclobutyl)benzamide,
2,6-dichloro-N-((1s,3s)-3-(6-((4-(4-((1-(2-(2,6-dioxopiperidin-3-yl)-1,3-dioxoisoindolin-4-yl)piperidin-4-yl)methyl)piperazin-1-yl)phenyl)amino)-9H-purin-9-yl)cyclobutyl)benzamide,
2,6-dichloro-N-((1s,3s)-3-(6-(((1-((1-(2-((2-(2,6-dioxopiperidin-3-yl)-1,3-dioxoisoindolin-4-yl)oxy)acetyl)piperidin-4-yl)methyl)piperidin-4-yl)methyl)amino)-9H-purin-9-yl)cyclobutyl)benzamide,
2,6-dichloro-N-((1s,3s)-3-(6-(((1-((1-(2-((2-(2,6-dioxopiperidin-3-yl)-1,3-dioxoisoindolin-5-yl)oxy)acetyl)piperidin-4-yl)methyl)piperidin-4-yl)methyl)amino)-9H-purin-9-yl)cyclobutyl)benzamide,
2,6-dichloro-N-((1s,3s)-3-(6-(((1-((1-((2-(2,6-dioxopiperidin-3-yl)-1,3-dioxoisoindolin-4-yl)glycyl)piperidin-4-yl)methyl)piperidin-4-yl)methyl)amino)-9H-purin-9-yl)cyclobutyl)benzamide,
2,6-dichloro-N-((1s,3s)-3-(6-(((1-((1-((2-(2,6-dioxopiperidin-3-yl)-1,3-dioxoisoindolin-5-yl)glycyl)piperidin-4-yl)methyl)piperidin-4-yl)methyl)amino)-9H-purin-9-yl)cyclobutyl)benzamide,
2,6-dichloro-N-((1s,3s)-3-(6-(((1-((1-(2-(2,6-dioxopiperidin-3-yl)-1,3-dioxoisoindolin-4-yl)piperidin-4-yl)methyl)piperidin-4-yl)methyl)amino)-9H-purin-9-yl)cyclobutyl)benzamide,
2,6-dichloro-N-((1s,3s)-3-(6-(((1-((1-(2-(2,6-dioxopiperidin-3-yl)-1,3-dioxoisoindolin-5-yl)piperidin-4-yl)methyl)piperidin-4-yl)methyl)amino)-9H-purin-9-yl)cyclobutyl)benzamide,
N-((1s,3s)-3-(6-((4-(4-((1-(2-((2-(2,6-dioxopiperidin-3-yl)-1,3-dioxoisoindolin-4-yl)oxy)acetyl)piperidin-4-yl)methyl)piperazin-1-yl)phenyl)amino)-9H-purin-9-yl)cyclobutyl)-2-phenylacetamide,
N-((1s,3s)-3-(6-((4-(4-((1-(2-((2-(2,6-dioxopiperidin-3-yl)-1,3-dioxoisoindolin-5-yl)oxy)acetyl)piperidin-4-yl)methyl)piperazin-1-yl)phenyl)amino)-9H-purin-9-yl)cyclobutyl)-2-phenylacetamide,
N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)phenyl)amino)-9H-purin-9-yl)cyclobutyl)-2-phenylacetamide,
N-((1s,3s)-3-(6-((4-(4-((1-((2-(2,6-dioxopiperidin-3-yl)-1,3-dioxoisoindolin-5-yl)glycyl)piperidin-4-yl)methyl)piperazin-1-yl)phenyl)amino)-9H-purin-9-yl)cyclobutyl)-2-phenylacetamide,
N-((1s,3s)-3-(6-((4-(4-((1-(2-(2,6-dioxopiperidin-3-yl)-1,3-dioxoisoindolin-4-yl)piperidin-4-yl)methyl)piperazin-1-yl)phenyl)amino)-9H-purin-9-yl)cyclobutyl)-2-phenylacetamide,
N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)phenyl)amino)-9H-purin-9-yl)cyclobutyl)-2-phenylacetamide,
N-((1s,3s)-3-(6-(((1-((1-(2-(2,6-dioxopiperidin-3-yl)-1,3-dioxoisoindolin-4-yl)piperidin-4-yl)methyl)piperidin-4-yl)methyl)amino)-9H-purin-9-yl)cyclobutyl)-2-phenylacetamide,
N-((1s,3s)-3-(6-(((1-((1-(2-(2,6-dioxopiperidin-3-yl)-1,3-dioxoisoindolin-5-yl)piperidin-4-yl)methyl)piperidin-4-yl)methyl)amino)-9H-purin-9-yl)cyclobutyl)-2-phenylacetamide,
N-((1s,3s)-3-(6-(((1-((1-(2-((2-(2,6-dioxopiperidin-3-yl)-1,3-dioxoisoindolin-4-yl)oxy)acetyl)piperidin-4-yl)methyl)piperidin-4-yl)methyl)amino)-9H-purin-9-yl)cyclobutyl)-2-phenylacetamide,
N-((1s,3s)-3-(6-(((1-((1-(2-((2-(2,6-dioxopiperidin-3-yl)-1,3-dioxoisoindolin-5-yl)oxy)acetyl)piperidin-4-yl)methyl)piperidin-4-yl)methyl)amino)-9H-purin-9-yl)cyclobutyl)-2-phenylacetamide,
N-((1s,3s)-3-(6-(((1-((1-((2-(2,6-dioxopiperidin-3-yl)-1,3-dioxoisoindolin-4-yl)glycyl)piperidin-4-yl)methyl)piperidin-4-yl)methyl)amino)-9H-purin-9-yl)cyclobutyl)-2-phenylacetamide,
N-((1s,3s)-3-(6-(((1-((1-((2-(2,6-dioxopiperidin-3-yl)-1,3-dioxoisoindolin-5-yl)glycyl)piperidin-4-yl)methyl)piperidin-4-yl)methyl)amino)-9H-purin-9-yl)cyclobutyl)-2-phenylacetamide,
N-((1s,3s)-3-(6-((4-(4-((1-(3-(2,4-dioxotetrahydropyrimidin-1(2H)-yl)-4-methoxybenzoyl)piperidin-4-yl)methyl)piperazin-1-yl)phenyl)amino)-9H-purin-9-yl)cyclobutyl)-2-phenylacetamide,
N-((1s,3s)-3-(6-((4-(4-((1-(3-(2,4-dioxotetrahydropyrimidin-1(2H)-yl)benzofuran-6-carbonyl)piperidin-4-yl)methyl)piperazin-1-yl)phenyl)amino)-9H-purin-9-yl)cyclobutyl)-2-phenylacetamide,
N-((1s,3s)-3-(6-((4-(4-((1-(2-(3-((1-(2,6-dioxopiperidin-3-yl)-2,5-dioxo-2,5-dihydro-1H-pyrrol-3-yl)amino)phenyl)acetyl)piperidin-4-yl)methyl)piperazin-1-yl)phenyl)amino)-9H-purin-9-yl)cyclobutyl)-2-phenylacetamide,
N-((1s,3s)-3-(6-((4-(4-((1-(1-(1-(2,6-dioxopiperidin-3-yl)-2,5-dioxo-2,5-dihydro-1H-pyrrol-3-yl)piperidin-4-carbonyl)piperidin-4-yl)methyl)piperazin-1-yl)phenyl)amino)-9H-purin-9-yl)cyclobutyl)-2-phenylacetamide,
N-((1s,3s)-3-(6-((4-(4-(7-(3-(2,4-dioxotetrahydropyrimidin-1(2H)-yl)-4-methoxybenzoyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-(3-(2,4-dioxotetrahydropyrimidin-1(2H)-yl)benzofuran-6-carbonyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-(2-(3-((1-(2,6-dioxopiperidin-3-yl)-2,5-dioxo-2,5-dihydro-1H-pyrrol-3-yl)amino)phenyl)acetyl)-7-azaspiro[3.5]nonan-2-yl)piperazin-1-yl)phenyl)amino)-9H-purin-9-yl)cyclobutyl)-2-phenylacetamide,
N-((1 s,3s)-3-(6-((4-(4-(7-(1-(1-(2,6-dioxopiperidin-3-yl)-2,5-dioxo-2,5-dihydro-1H-pyrrol-3-yl)piperidin-4-carbonyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(2-(1-(3-(2,4-dioxotetrahydropyrimidin-1(2H)-yl)-4-methoxybenzoyl)piperidin-4-yl)acetyl)piperazin-1-yl)phenyl)amino)-9H-purin-9-yl)cyclobutyl)-2-phenylacetamide,
N-((1s,3s)-3-(6-((4-(4-(2-(1-(3-(2,4-dioxotetrahydropyrimidin-1(2H)-yl)benzofuran-6-carbonyl)piperidin-4-yl)acetyl)piperazin-1-yl)phenyl)amino)-9H-purin-9-yl)cyclobutyl)-2-phenylacetamide,
N-((1s,3s)-3-(6-((4-(4-(2-(1-(2-(3-((1-(2,6-dioxopiperidin-3-yl)-2,5-dioxo-2,5-dihydro-1H-pyrrol-3-yl)amino)phenyl)acetyl)piperidin-4-yl)acetyl)piperazin-1-yl)phenyl)amino)-9H-purin-9-yl)cyclobutyl)-2-phenylacetamide,
N-((1s,3s)-3-(6-((4-(4-(2-(1-(1-(1-(2,6-dioxopiperidin-3-yl)-2,5-dioxo-2,5-dihydro-1H-pyrrol-3-yl)piperidin-4-carbonyl)piperidin-4-yl)acetyl)piperazin-1-yl)phenyl)amino)-9H-purin-9-yl)cyclobutyl)-2-phenylacetamide,
3-(2,4-dioxotetrahydropyrimidin-1(2H)-yl)-4-methoxy-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)benzamide,
3-(2,4-dioxotetrahydropyrimidin-1(2H)-yl)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)benzofuran-6-carboxamide,
2-(3-((1-(2,6-dioxopiperidin-3-yl)-2,5-dioxo-2,5-dihydro-1H-pyrrol-3-yl)amino)phenyl)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)acetamide,
1-(1-(2,6-dioxopiperidin-3-yl)-2,5-dioxo-2,5-dihydro-1H-pyrrol-3-yl)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)piperidin-4-carboxamide,
N-((1s,3s)-3-(6-((4-(4-((1-(2-(4-(2,6-dioxopiperidin-3-yl)phenoxy)acetyl)piperidin-4-yl)methyl)piperazin-1-yl)phenyl)amino)-9H-purin-9-yl)cyclobutyl)-2-phenylacetamide,
N-((1s,3s)-3-(6-((4-(4-(2-(1-(2-(4-(2,6-dioxopiperidin-3-yl)phenoxy)acetyl)piperidin-4-yl)acetyl)piperazin-1-yl)phenyl)amino)-9H-purin-9-yl)cyclobutyl)-2-phenylacetamide,
N-((1s,3s)-3-(6-((4-(4-(7-((2-(2,6-dioxopiperidin-3-yl)-1,3-dioxoisoindolin-4-yl)glycyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-(2-(4-(2,6-dioxopiperidin-3-yl)phenoxy)acetyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-(2-((2-(2,6-dioxopiperidin-3-yl)-1,3-dioxoisoindolin-4-yl)oxy)acetyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-((2-(2,6-dioxopiperidin-3-yl)-1,3-dioxoisoindolin-5-yl)glycyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-(2-((2-(2,6-dioxopiperidin-3-yl)-1,3-dioxoisoindolin-5-yl)oxy)acetyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-(2-(2,6-dioxopiperidin-3-yl)-1,3-dioxoisoindolin-4-yl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-(2-(2,6-dioxopiperidin-3-yl)-1,3-dioxoisoindolin-5-yl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(2-(1-((2-(2,6-dioxopiperidin-3-yl)-1,3-dioxoisoindolin-4-yl)glycyl)piperidin-4-yl)acetyl)piperazin-1-yl)phenyl)amino)-9H-purin-9-yl)cyclobutyl)-2-phenylacetamide,
N-((1s,3s)-3-(6-((4-(4-(2-(1-(2-((2-(2,6-dioxopiperidin-3-yl)-1,3-dioxoisoindolin-4-yl)oxy)acetyl)piperidin-4-yl)acetyl)piperazin-1-yl)phenyl)amino)-9H-purin-9-yl)cyclobutyl)-2-phenylacetamide,
N-((1s,3s)-3-(6-((4-(4-(2-(1-((2-(2,6-dioxopiperidin-3-yl)-1,3-dioxoisoindolin-5-yl)glycyl)piperidin-4-yl)acetyl)piperazin-1-yl)phenyl)amino)-9H-purin-9-yl)cyclobutyl)-2-phenylacetamide,
N-((1s,3s)-3-(6-((4-(4-(2-(1-(2-((2-(2,6-dioxopiperidin-3-yl)-1,3-dioxoisoindolin-5-yl)oxy)acetyl)piperidin-4-yl)acetyl)piperazin-1-yl)phenyl)amino)-9H-purin-9-yl)cyclobutyl)-2-phenylacetamide,
N-((1s,3s)-3-(6-((4-(4-(2-(1-(2-(2,6-dioxopiperidin-3-yl)-1,3-dioxoisoindolin-4-yl)piperidin-4-yl)acetyl)piperazin-1-yl)phenyl)amino)-9H-purin-9-yl)cyclobutyl)-2-phenylacetamide,
N-((1s,3s)-3-(6-((4-(4-(2-(1-(2-(2,6-dioxopiperidin-3-yl)-1,3-dioxoisoindolin-5-yl)piperidin-4-yl)acetyl)piperazin-1-yl)phenyl)amino)-9H-purin-9-yl)cyclobutyl)-2-phenylacetamide,
2-(4-(2,6-dioxopiperidin-3-yl)phenoxy)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)acetamide,
2-((2-(2,6-dioxopiperidin-3-yl)-1,3-dioxoisoindolin-4-yl)amino)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)acetamide,
2-((2-(2,6-dioxopiperidin-3-yl)-1,3-dioxoisoindolin-4-yl)oxy)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)acetamide,
2-((2-(2,6-dioxopiperidin-3-yl)-1,3-dioxoisoindolin-5-yl)amino)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)acetamide,
2-((2-(2,6-dioxopiperidin-3-yl)-1,3-dioxoisoindolin-5-yl)oxy)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)acetamide,
N-((1s,3s)-3-(6-((4-(4-(((1r,4r)-4-(((2-(2,6-dioxopiperidin-3-yl)-1,3-dioxoisoindolin-4-yl)amino)methyl)cyclohexyl)methyl)piperazin-1-yl)phenyl)amino)-9H-purin-9-yl)cyclobutyl)-2-phenylacetamide,
2-(3-(2,6-dioxopiperidin-3-yl)phenoxy)-N-(((1r,4r)-4-((4-(4-((9-((1s,3s)-3-(2-phenylacetamido)cyclobutyl)-9H-purin-6-yl)amino)phenyl)piperazin-1-yl)methyl)cyclohexyl)methyl)acetamide,
N-((1s,3s)-3-(6-((3-(4-((1-(2-(2,6-dioxopiperidin-3-yl)-1,3-dioxoisoindolin-4-yl)piperidin-4-yl)methyl)piperazin-1-yl)propyl)amino)-9H-purin-9-yl)cyclobutyl)-6-methylpicolinamide,
N-((1s,3s)-3-(6-((4-(4-((1-(2-(3-(2,6-dioxopiperidin-3-yl)phenoxy)acetyl)piperidin-4-yl)methyl)piperazin-1-yl)phenyl)amino)-9H-purin-9-yl)cyclobutyl)-2-phenylacetamide,
N-((1s,3s)-3-(6-((4-(4-(7-(2-(3-(2,6-dioxopiperidin-3-yl)phenoxy)acetyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(2-(1-(2-(3-(2,6-dioxopiperidin-3-yl)phenoxy)acetyl)piperidin-4-yl)acetyl)piperazin-1-yl)phenyl)amino)-9H-purin-9-yl)cyclobutyl)-2-phenylacetamide,
N-((1s,3s)-3-(6-((3-(4-((1-(3-(2,4-dioxotetrahydropyrimidin-1(2H)-yl)-4-methoxybenzoyl)piperidin-4-yl)methyl)piperazin-1-yl)propyl)amino)-9H-purin-9-yl)cyclobutyl)-6-methylpicolinamide,
N-((1s,3s)-3-(6-((3-(4-((1-(2-(4-(2,6-dioxopiperidin-3-yl)phenoxy)acetyl)piperidin-4-yl)methyl)piperazin-1-yl)propyl)amino)-9H-purin-9-yl)cyclobutyl)-6-methylpicolinamide,
N-((1s,3s)-3-(6-((3-(4-((1-(2-(3-(2,6-dioxopiperidin-3-yl)phenoxy)acetyl)piperidin-4-yl)methyl)piperazin-1-yl)propyl)amino)-9H-purin-9-yl)cyclobutyl)-6-methylpicolinamide,
N-((1s,3s)-3-(6-((4-(4-(3-(4-((2-(2,6-dioxopiperidin-3-yl)-1,3-dioxoisoindolin-4-yl)glycyl)piperazin-1-yl)propyl)piperazin-1-yl)phenyl)amino)-9H-purin-9-yl)cyclobutyl)-2-phenylacetamide,
N-((1s,3s)-3-(6-((4-(4-(3-(4-(2-((2-(2,6-dioxopiperidin-3-yl)-1,3-dioxoisoindolin-4-yl)oxy)acetyl)piperazin-1-yl)propyl)piperazin-1-yl)phenyl)amino)-9H-purin-9-yl)cyclobutyl)-2-phenylacetamide,
N-((1s,3s)-3-(6-((4-(4-(3-(4-((2-(2,6-dioxopiperidin-3-yl)-1,3-dioxoisoindolin-5-yl)glycyl)piperazin-1-yl)propyl)piperazin-1-yl)phenyl)amino)-9H-purin-9-yl)cyclobutyl)-2-phenylacetamide,
N-((1s,3s)-3-(6-((4-(4-(3-(4-(2-((2-(2,6-dioxopiperidin-3-yl)-1,3-dioxoisoindolin-5-yl)oxy)acetyl)piperazin-1-yl)propyl)piperazin-1-yl)phenyl)amino)-9H-purin-9-yl)cyclobutyl)-2-phenylacetamide,
N-((1s,3s)-3-(6-((4-(4-(3-(4-(3-(2,4-dioxotetrahydropyrimidin-1(2H)-yl)-4-methoxybenzoyl)piperazin-1-yl)propyl)piperazin-1-yl)phenyl)amino)-9H-purin-9-yl)cyclobutyl)-2-phenylacetamide,
N-((1s,3s)-3-(6-((4-(4-(3-(4-(2-(4-(2,6-dioxopiperidin-3-yl)phenoxy)acetyl)piperazin-1-yl)propyl)piperazin-1-yl)phenyl)amino)-9H-purin-9-yl)cyclobutyl)-2-phenylacetamide,
N-((1s,3s)-3-(6-((4-(4-(3-(4-(2-(3-(2,6-dioxopiperidin-3-yl)phenoxy)acetyl)piperazin-1-yl)propyl)piperazin-1-yl)phenyl)amino)-9H-purin-9-yl)cyclobutyl)-2-phenylacetamide,
N-((1s,3s)-3-(6-((4-(4-(3-(4-(2-(2,6-dioxopiperidin-3-yl)-1,3-dioxoisoindolin-4-yl)piperazin-1-yl)propyl)piperazin-1-yl)phenyl)amino)-9H-purin-9-yl)cyclobutyl)-2-phenylacetamide,
N-((1s,3s)-3-(6-((4-(4-(3-(4-(2-(2,6-dioxopiperidin-3-yl)-1,3-dioxoisoindolin-5-yl)piperazin-1-yl)propyl)piperazin-1-yl)phenyl)amino)-9H-purin-9-yl)cyclobutyl)-2-phenylacetamide,
N-((1s,3s)-3-(6-((4-(4-(3-(4-(2-(2,6-dioxopiperidin-3-yl)-6-fluoro-1,3-dioxoisoindolin-5-yl)piperazin-1-yl)propyl)piperazin-1-yl)phenyl)amino)-9H-purin-9-yl)cyclobutyl)-2-phenylacetamide,
N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)-9H-purin-9-yl)cyclobutyl)-2-phenylacetamide,
N-((1s,3s)-3-(6-((4-(4-(2-(1-(2-(2,6-dioxopiperidin-3-yl)-6-fluoro-1,3-dioxoisoindolin-5-yl)piperidin-4-yl)acetyl)piperazin-1-yl)phenyl)amino)-9H-purin-9-yl)cyclobutyl)-2-phenylacetamide,
N-((1s,3s)-3-(6-((4-(4-(((1r,4r)-4-(((2-(2,6-dioxopiperidin-3-yl)-6-fluoro-1,3-dioxoisoindolin-5-yl)amino)methyl)cyclohexyl)methyl)piperazin-1-yl)phenyl)amino)-9H-purin-9-yl)cyclobutyl)-2-phenylacetamide,
N-((1s,3s)-3-(6-((4-(4-(7-(2-(2,6-dioxopiperidin-3-yl)-6-fluoro-1,3-dioxoisoindolin-5-yl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(2-((1-((2-(2,6-dioxopiperidin-3-yl)-1,3-dioxoisoindolin-4-yl)glycyl)piperidin-4-yl)oxy)ethyl)piperazin-1-yl)phenyl)amino)-9H-purin-9-yl)cyclobutyl)-2-phenylacetamide,
N-((1s,3s)-3-(6-((4-(4-(2-((1-(2-((2-(2,6-dioxopiperidin-3-yl)-1,3-dioxoisoindolin-4-yl)oxy)acetyl)piperidin-4-yl)oxy)ethyl)piperazin-1-yl)phenyl)amino)-9H-purin-9-yl)cyclobutyl)-2-phenylacetamide,
N-((1s,3s)-3-(6-((4-(4-(2-((1-(2-((2-(2,6-dioxopiperidin-3-yl)-1,3-dioxoisoindolin-5-yl)oxy)acetyl)piperidin-4-yl)oxy)ethyl)piperazin-1-yl)phenyl)amino)-9H-purin-9-yl)cyclobutyl)-2-phenylacetamide,
N-((1s,3s)-3-(6-((4-(4-(2-((1-((2-(2,6-dioxopiperidin-3-yl)-1,3-dioxoisoindolin-5-yl)glycyl)piperidin-4-yl)oxy)ethyl)piperazin-1-yl)phenyl)amino)-9H-purin-9-yl)cyclobutyl)-2-phenylacetamide,
N-((1s,3s)-3-(6-((4-(4-(2-((1-(3-(2,4-dioxotetrahydropyrimidin-1(2H)-yl)-4-methoxybenzoyl)piperidin-4-yl)oxy)ethyl)piperazin-1-yl)phenyl)amino)-9H-purin-9-yl)cyclobutyl)-2-phenylacetamide,
N-((1s,3s)-3-(6-((4-(4-(2-((1-(2-(4-(2,6-dioxopiperidin-3-yl)phenoxy)acetyl)piperidin-4-yl)oxy)ethyl)piperazin-1-yl)phenyl)amino)-9H-purin-9-yl)cyclobutyl)-2-phenylacetamide,
N-((1s,3s)-3-(6-((4-(4-(2-((1-(2-(3-(2,6-dioxopiperidin-3-yl)phenoxy)acetyl)piperidin-4-yl)oxy)ethyl)piperazin-1-yl)phenyl)amino)-9H-purin-9-yl)cyclobutyl)-2-phenylacetamide,
N-((1s,3s)-3-(6-((4-(4-(2-((1-(2-(2,6-dioxopiperidin-3-yl)-1,3-dioxoisoindolin-5-yl)piperidin-4-yl)oxy)ethyl)piperazin-1-yl)phenyl)amino)-9H-purin-9-yl)cyclobutyl)-2-phenylacetamide,
N-((1s,3s)-3-(6-((4-(4-(3-(1-((2-(2,6-dioxopiperidin-3-yl)-1,3-dioxoisoindolin-4-yl)glycyl)piperidin-4-yl)propyl)piperazin-1-yl)phenyl)amino)-9H-purin-9-yl)cyclobutyl)-2-phenylacetamide,
N-((1s,3s)-3-(6-((4-(4-(3-(1-(2-((2-(2,6-dioxopiperidin-3-yl)-1,3-dioxoisoindolin-4-yl)oxy)acetyl)piperidin-4-yl)propyl)piperazin-1-yl)phenyl)amino)-9H-purin-9-yl)cyclobutyl)-2-phenylacetamide,
N-((1s,3s)-3-(6-((4-(4-(3-(1-((2-(2,6-dioxopiperidin-3-yl)-1,3-dioxoisoindolin-5-yl)glycyl)piperidin-4-yl)propyl)piperazin-1-yl)phenyl)amino)-9H-purin-9-yl)cyclobutyl)-2-phenylacetamide,
N-((1s,3s)-3-(6-((4-(4-(3-(1-(2-((2-(2,6-dioxopiperidin-3-yl)-1,3-dioxoisoindolin-5-yl)oxy)acetyl)piperidin-4-yl)propyl)piperazin-1-yl)phenyl)amino)-9H-purin-9-yl)cyclobutyl)-2-phenylacetamide,
N-((1s,3s)-3-(6-((4-(4-(3-(1-(3-(2,4-dioxotetrahydropyrimidin-1(2H)-yl)-4-methoxybenzoyl)piperidin-4-yl)propyl)piperazin-1-yl)phenyl)amino)-9H-purin-9-yl)cyclobutyl)-2-phenylacetamide,
N-((1s,3s)-3-(6-((4-(4-(3-(1-(2-(4-(2,6-dioxopiperidin-3-yl)phenoxy)acetyl)piperidin-4-yl)propyl)piperazin-1-yl)phenyl)amino)-9H-purin-9-yl)cyclobutyl)-2-phenylacetamide,
N-((1s,3s)-3-(6-((4-(4-(3-(1-(2-(3-(2,6-dioxopiperidin-3-yl)phenoxy)acetyl)piperidin-4-yl)propyl)piperazin-1-yl)phenyl)amino)-9H-purin-9-yl)cyclobutyl)-2-phenylacetamide,
N-((1s,3s)-3-(6-((4-(4-(2-((1-(2-(2,6-dioxopiperidin-3-yl)-1,3-dioxoisoindolin-4-yl)piperidin-4-yl)oxy)ethyl)piperazin-1-yl)phenyl)amino)-9H-purin-9-yl)cyclobutyl)-2-phenylacetamide,
N-((1s,3s)-3-(6-((4-(4-(3-(1-(2-(2,6-dioxopiperidin-3-yl)-1,3-dioxoisoindolin-4-yl)piperidin-4-yl)propyl)piperazin-1-yl)phenyl)amino)-9H-purin-9-yl)cyclobutyl)-2-phenylacetamide,
N-((1s,3s)-3-(6-((4-(4-(3-(1-(2-(2,6-dioxopiperidin-3-yl)-1,3-dioxoisoindolin-5-yl)piperidin-4-yl)propyl)piperazin-1-yl)phenyl)amino)-9H-purin-9-yl)cyclobutyl)-2-phenylacetamide,
N-((1s,3s)-3-(6-((4-(4-(6-((2-(2,6-dioxopiperidin-3-yl)-1,3-dioxoisoindolin-4-yl)glycyl)-6-azaspiro[3.4]octan-2-yl)piperazin-1-yl)phenyl)amino)-9H-purin-9-yl)cyclobutyl)-2-phenylacetamide,
N-((1s,3s)-3-(6-((4-(4-(6-(2-((2-(2,6-dioxopiperidin-3-yl)-1,3-dioxoisoindolin-4-yl)oxy)acetyl)-6-azaspiro[3.4]octan-2-yl)piperazin-1-yl)phenyl)amino)-9H-purin-9-yl)cyclobutyl)-2-phenylacetamide,
N-((1s,3s)-3-(6-((4-(4-(6-((2-(2,6-dioxopiperidin-3-yl)-1,3-dioxoisoindolin-5-yl)glycyl)-6-azaspiro[3.4]octan-2-yl)piperazin-1-yl)phenyl)amino)-9H-purin-9-yl)cyclobutyl)-2-phenylacetamide,
N-((1s,3s)-3-(6-((4-(4-(6-(2-((2-(2,6-dioxopiperidin-3-yl)-1,3-dioxoisoindolin-5-yl)oxy)acetyl)-6-azaspiro[3.4]octan-2-yl)piperazin-1-yl)phenyl)amino)-9H-purin-9-yl)cyclobutyl)-2-phenylacetamide,
N-((1s,3s)-3-(6-((4-(4-(6-(3-(2,4-dioxotetrahydropyrimidin-1(2H)-yl)-4-methoxybenzoyl)-6-azaspiro[3.4]octan-2-yl)piperazin-1-yl)phenyl)amino)-9H-purin-9-yl)cyclobutyl)-2-phenylacetamide,
N-((1s,3s)-3-(6-((4-(4-(6-(2-(4-(2,6-dioxopiperidin-3-yl)phenoxy)acetyl)-6-azaspiro[3.4]octan-2-yl)piperazin-1-yl)phenyl)amino)-9H-purin-9-yl)cyclobutyl)-2-phenylacetamide,
N-((1s,3s)-3-(6-((4-(4-(6-(2-(3-(2,6-dioxopiperidin-3-yl)phenoxy)acetyl)-6-azaspiro[3.4]octan-2-yl)piperazin-1-yl)phenyl)amino)-9H-purin-9-yl)cyclobutyl)-2-phenylacetamide,
N-((1s,3s)-3-(6-((4-(4-(7-(1-(2-(2,6-dioxopiperidin-3-yl)-1,3-dioxoisoindolin-4-yl)azetidin-3-yl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-(1-(2-(2,6-dioxopiperidin-3-yl)-1,3-dioxoisoindolin-5-yl)azetidin-3-yl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-(1-(3-(2,4-dioxotetrahydropyrimidin-1(2H)-yl)-4-methoxybenzoyl)azetidin-3-yl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-(1-(2-(4-(2,6-dioxopiperidin-3-yl)phenoxy)acetyl)azetidin-3-yl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-(1-(2-(3-(2,6-dioxopiperidin-3-yl)phenoxy)acetyl)azetidin-3-yl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-((1-(2-(4-(2,6-dioxopiperidin-3-yl)phenoxy)acetyl)azetidin-3-yl)methyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-((1-(2-(3-(2,6-dioxopiperidin-3-yl)phenoxy)acetyl)azetidin-3-yl)methyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-(1-((2-(2,6-dioxopiperidin-3-yl)-1,3-dioxoisoindolin-4-yl)glycyl)azetidin-3-yl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-(1-((2-(2,6-dioxopiperidin-3-yl)-1,3-dioxoisoindolin-5-yl)glycyl)azetidin-3-yl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-((1-(2-(2,6-dioxopiperidin-3-yl)-1,3-dioxoisoindolin-4-yl)azetidin-3-yl)methyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-((1-(3-(2,4-dioxotetrahydropyrimidin-1(2H)-yl)-4-methoxybenzoyl)azetidin-3-yl)methyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(1-((1-(2-(4-(2,6-dioxopiperidin-3-yl)phenoxy)acetyl)piperidin-4-yl)methyl)piperidin-4-yl)phenyl)amino)-9H-purin-9-yl)cyclobutyl)-2-phenylacetamide,
N-((1s,3s)-3-(6-((4-(1-((1-(2-(3-(2,6-dioxopiperidin-3-yl)phenoxy)acetyl)piperidin-4-yl)methyl)piperidin-4-yl)phenyl)amino)-9H-purin-9-yl)cyclobutyl)-2-phenylacetamide,
N-((1s,3s)-3-(6-((4-(1-((1-(3-(2,4-dioxotetrahydropyrimidin-1(2H)-yl)-4-methoxybenzoyl)piperidin-4-yl)methyl)piperidin-4-yl)phenyl)amino)-9H-purin-9-yl)cyclobutyl)-2-phenylacetamide,
N-((1s,3s)-3-(6-((4-(1-(7-(2-(4-(2,6-dioxopiperidin-3-yl)phenoxy)acetyl)-7-azaspiro[3.5]nonan-2-yl)piperidin-4-yl)phenyl)amino)-9H-purin-9-yl)cyclobutyl)-2-phenylacetamide,
N-((1s,3s)-3-(6-((4-(1-(7-(2-(3-(2,6-dioxopiperidin-3-yl)phenoxy)acetyl)-7-azaspiro[3.5]nonan-2-yl)piperidin-4-yl)phenyl)amino)-9H-purin-9-yl)cyclobutyl)-2-phenylacetamide,
N-((1s,3s)-3-(6-((4-(1-(7-(3-(2,4-dioxotetrahydropyrimidin-1(2H)-yl)-4-methoxybenzoyl)-7-azaspiro[3.5]nonan-2-yl)piperidin-4-yl)phenyl)amino)-9H-purin-9-yl)cyclobutyl)-2-phenylacetamide,
N-((1s,3s)-3-(6-((4-(1-((1-(2-((2-(2,6-dioxopiperidin-3-yl)-1,3-dioxoisoindolin-4-yl)oxy)acetyl)piperidin-4-yl)methyl)piperidin-4-yl)phenyl)amino)-9H-purin-9-yl)cyclobutyl)-2-phenylacetamide,
N-((1s,3s)-3-(6-((4-(1-((1-((2-(2,6-dioxopiperidin-3-yl)-1,3-dioxoisoindolin-4-yl)glycyl)piperidin-4-yl)methyl)piperidin-4-yl)phenyl)amino)-9H-purin-9-yl)cyclobutyl)-2-phenylacetamide,
N-((1s,3s)-3-(6-((4-(1-((1-(2-((2-(2,6-dioxopiperidin-3-yl)-1,3-dioxoisoindolin-5-yl)oxy)acetyl)piperidin-4-yl)methyl)piperidin-4-yl)phenyl)amino)-9H-purin-9-yl)cyclobutyl)-2-phenylacetamide,
N-((1s,3s)-3-(6-((4-(1-((1-((2-(2,6-dioxopiperidin-3-yl)-1,3-dioxoisoindolin-5-yl)glycyl)piperidin-4-yl)methyl)piperidin-4-yl)phenyl)amino)-9H-purin-9-yl)cyclobutyl)-2-phenylacetamide,
N-((1s,3s)-3-(6-((4-(1-((1-(2-(2,6-dioxopiperidin-3-yl)-1,3-dioxoisoindolin-4-yl)piperidin-4-yl)methyl)piperidin-4-yl)phenyl)amino)-9H-purin-9-yl)cyclobutyl)-2-phenylacetamide,
N-((1s,3s)-3-(6-((4-(1-((1-(2-(2,6-dioxopiperidin-3-yl)-1,3-dioxoisoindolin-5-yl)piperidin-4-yl)methyl)piperidin-4-yl)phenyl)amino)-9H-purin-9-yl)cyclobutyl)-2-phenylacetamide,
N-((1s,3s)-3-(6-((4-(1-(7-(2-((2-(2,6-dioxopiperidin-3-yl)-1,3-dioxoisoindolin-4-yl)oxy)acetyl)-7-azaspiro[3.5]nonan-2-yl)piperidin-4-yl)phenyl)amino)-9H-purin-9-yl)cyclobutyl)-2-phenylacetamide,
N-((1s,3s)-3-(6-((4-(1-(7-((2-(2,6-dioxopiperidin-3-yl)-1,3-dioxoisoindolin-4-yl)glycyl)-7-azaspiro[3.5]nonan-2-yl)piperidin-4-yl)phenyl)amino)-9H-purin-9-yl)cyclobutyl)-2-phenylacetamide,
N-((1s,3s)-3-(6-((4-(1-(7-(2-((2-(2,6-dioxopiperidin-3-yl)-1,3-dioxoisoindolin-5-yl)oxy)acetyl)-7-azaspiro[3.5]nonan-2-yl)piperidin-4-yl)phenyl)amino)-9H-purin-9-yl)cyclobutyl)-2-phenylacetamide,
N-((1s,3s)-3-(6-((4-(1-(7-((2-(2,6-dioxopiperidin-3-yl)-1,3-dioxoisoindolin-5-yl)glycyl)-7-azaspiro[3.5]nonan-2-yl)piperidin-4-yl)phenyl)amino)-9H-purin-9-yl)cyclobutyl)-2-phenylacetamide,
N-((1s,3s)-3-(6-((4-(1-(7-(2-(2,6-dioxopiperidin-3-yl)-1,3-dioxoisoindolin-4-yl)-7-azaspiro[3.5]nonan-2-yl)piperidin-4-yl)phenyl)amino)-9H-purin-9-yl)cyclobutyl)-2-phenylacetamide,
N-((1s,3s)-3-(6-((4-(1-(7-(2-(2,6-dioxopiperidin-3-yl)-1,3-dioxoisoindolin-5-yl)-7-azaspiro[3.5]nonan-2-yl)piperidin-4-yl)phenyl)amino)-9H-purin-9-yl)cyclobutyl)-2-phenylacetamide,
2,6-dichloro-N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)-9H-purin-9-yl)cyclobutyl)benzamide,
2,6-dichloro-N-((1s,3s)-3-(6-((4-(4-((4-(2-(2,6-dioxopiperidin-3-yl)-1,3-dioxoisoindolin-5-yl)piperazin-1-yl)methyl)piperidin-1-yl)phenyl)amino)-9H-purin-9-yl)cyclobutyl)benzamide,
2,6-dichloro-N-((1s,3s)-3-(6-((4-(4-((4-(2-(2,6-dioxopiperidin-3-yl)-6-fluoro-1,3-dioxoisoindolin-5-yl)piperazin-1-yl)methyl)piperidin-1-yl)phenyl)amino)-9H-purin-9-yl)cyclobutyl)benzamide,
N-((1s,3s)-3-(6-((2-(1-((1-(2-(2,6-dioxopiperidin-3-yl)-6-fluoro-1,3-dioxoisoindolin-5-yl)piperidin-4-yl)methyl)piperidin-4-yl)ethyl)amino)-9H-purin-9-yl)cyclobutyl)-6-methylpicolinamide,
N-((1s,3s)-3-(6-((2-(1-((1-(3-(2,6-dioxopiperidin-3-yl)-4-oxo-3,4-dihydrobenzo[d][1,2,3]triazin-5-yl)piperidin-4-yl)methyl)piperidin-4-yl)ethyl)amino)-9H-purin-9-yl)cyclobutyl)-6-methylpicolinamide,
N-((1s,3s)-3-(6-((2-(1-((1-(3-(2,6-dioxopiperidin-3-yl)-4-oxo-3,4-dihydrobenzo[d][1,2,3]triazin-6-yl)piperidin-4-yl)methyl)piperidin-4-yl)ethyl)amino)-9H-purin-9-yl)cyclobutyl)-6-methylpicolinamide,
2,6-dichloro-N-((1s,3s)-3-(6-((4-(4-((4-(2-(2,6-dioxopiperidin-3-yl)-1,3-dioxoisoindolin-4-yl)piperazin-1-yl)methyl)piperidin-1-yl)phenyl)amino)-9H-purin-9-yl)cyclobutyl)benzamide,
N-((1s,3s)-3-(6-((2-(1-((1-(2-(2,6-dioxopiperidin-3-yl)-1,3-dioxoisoindolin-4-yl)piperidin-4-yl)methyl)piperidin-4-yl)ethyl)amino)-9H-purin-9-yl)cyclobutyl)-6-methylpicolinamide,
N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)phenyl)amino)-9H-purin-9-yl)cyclobutyl)-2-phenylacetamide,
N-((1s,3s)-3-(6-((2-(1-((1-(2-(2,6-dioxopiperidin-3-yl)-1,3-dioxoisoindolin-5-yl)piperidin-4-yl)methyl)piperidin-4-yl)ethyl)amino)-9H-purin-9-yl)cyclobutyl)-6-methylpicolinamide,
N-((1s,3s)-3-(6-((2-(1-((1-(1-(2-(2,6-dioxopiperidin-3-yl)-1,3-dioxoisoindolin-5-yl)azetidin-3-carbonyl)piperidin-4-yl)methyl)piperidin-4-yl)ethyl)amino)-9H-purin-9-yl)cyclobutyl)-6-methylpicolinamide,
N-((1s,3s)-3-(6-((2-(1-((1-(3-((2-(2,6-dioxopiperidin-3-yl)-1,3-dioxoisoindolin-5-yl)amino)propanoyl)piperidin-4-yl)methyl)piperidin-4-yl)ethyl)amino)-9H-purin-9-yl)cyclobutyl)-6-methylpicolinamide,
2,6-dichloro-N-((1s,3s)-3-(6-((4-(4-((4-((2-(2,6-dioxopiperidin-3-yl)-1,3-dioxoisoindolin-4-yl)glycyl)piperazin-1-yl)methyl)piperidin-1-yl)phenyl)amino)-9H-purin-9-yl)cyclobutyl)benzamide,
2,6-dichloro-N-((1s,3s)-3-(6-((4-(4-((4-((2-(2,6-dioxopiperidin-3-yl)-1,3-dioxoisoindolin-5-yl)glycyl)piperazin-1-yl)methyl)piperidin-1-yl)phenyl)amino)-9H-purin-9-yl)cyclobutyl)benzamide,
N-((1s,3s)-3-(6-((4-(4-((1-(3-(2,6-dioxopiperidin-3-yl)-4-oxo-3,4-dihydrobenzo[d][1,2,3]triazin-5-yl)piperidin-4-yl)methyl)piperazin-1-yl)phenyl)amino)-9H-purin-9-yl)cyclobutyl)-2-phenylacetamide,
N-((1s,3s)-3-(6-((4-(4-(7-(3-(2,6-dioxopiperidin-3-yl)-4-oxo-3,4-dihydrobenzo[d][1,2,3]triazin-6-yl)-7-azaspiro[3.5]nonan-2-yl)piperazin-1-yl)phenyl)amino)-9H-purin-9-yl)cyclobutyl)-2-phenylacetamide,
2,6-dichloro-N-((1s,3s)-3-(6-((4-(4-(7-(2-(2,6-dioxopiperidin-3-yl)-1,3-dioxoisoindolin-5-yl)-7-azaspiro[3.5]nonan-2-yl)piperazin-1-yl)phenyl)amino)-9H-purin-9-yl)cyclobutyl)benzamide,
2,6-dichloro-N-((1s,3s)-3-(6-((4-(4-(7-(2-(2,6-dioxopiperidin-3-yl)-6-fluoro-1,3-dioxoisoindolin-5-yl)-7-azaspiro[3.5]nonan-2-yl)piperazin-1-yl)phenyl)amino)-9H-purin-9-yl)cyclobutyl)benzamide,
2,6-dichloro-N-((1s,3s)-3-(6-((4-(4-(7-(3-(2,6-dioxopiperidin-3-yl)-4-oxo-3,4-dihydrobenzo[d][1,2,3]triazin-6-yl)-7-azaspiro[3.5]nonan-2-yl)piperazin-1-yl)phenyl)amino)-9H-purin-9-yl)cyclobutyl)benzamide,
2,6-dichloro-N-((1s,3s)-3-(6-((4-(4-(7-((2-(2,6-dioxopiperidin-3-yl)-1,3-dioxoisoindolin-5-yl)glycyl)-7-azaspiro[3.5]nonan-2-yl)piperazin-1-yl)phenyl)amino)-9H-purin-9-yl)cyclobutyl)benzamide,
N-((1s,3s)-3-(6-((4-(4-((4-(2-(2,6-dioxopiperidin-3-yl)-1,3-dioxoisoindolin-5-yl)piperazin-1-yl)methyl)piperidin-1-yl)phenyl)amino)-9H-purin-9-yl)cyclobutyl)-2-phenylacetamide,
N-((1s,3s)-3-(6-((4-(4-((4-(3-(2,6-dioxopiperidin-3-yl)-4-oxo-3,4-dihydrobenzo[d][1,2,3]triazin-6-yl)piperazin-1-yl)methyl)piperidin-1-yl)phenyl)amino)-9H-purin-9-yl)cyclobutyl)-2-phenylacetamide,
N-((1s,3s)-3-(6-((4-(4-((4-(3-(2,6-dioxopiperidin-3-yl)-4-oxo-3,4-dihydrobenzo[d][1,2,3]triazin-5-yl)piperazin-1-yl)methyl)piperidin-1-yl)phenyl)amino)-9H-purin-9-yl)cyclobutyl)-2-phenylacetamide,
N-((1s,3s)-3-(6-((4-(4-((4-((2-(2,6-dioxopiperidin-3-yl)-1,3-dioxoisoindolin-4-yl)glycyl)piperazin-1-yl)methyl)piperidin-1-yl)phenyl)amino)-9H-purin-9-yl)cyclobutyl)-2-phenylacetamide,
N-((1s,3s)-3-(6-((4-(4-(2-(1-(2-(2,6-dioxopiperidin-3-yl)-6-fluoro-1,3-dioxoisoindolin-5-yl)piperidin-4-yl)ethyl)piperazin-1-yl)phenyl)amino)-9H-purin-9-yl)cyclobutyl)-2-phenylacetamide,
N-((1s,3s)-3-(6-((4-(4-(2-(1-(3-(2,6-dioxopiperidin-3-yl)-4-oxo-3,4-dihydrobenzo[d][1,2,3]triazin-6-yl)piperidin-4-yl)ethyl)piperazin-1-yl)phenyl)amino)-9H-purin-9-yl)cyclobutyl)-2-phenylacetamide,
2,6-dichloro-N-((1s,3s)-3-(6-((4-(4-((4-(2-((2-(2,6-dioxopiperidin-3-yl)-1,3-dioxoisoindolin-5-yl)oxy)acetyl)piperazin-1-yl)methyl)piperidin-1-yl)phenyl)amino)-9H-purin-9-yl)cyclobutyl)benzamide,
N-((1s,3s)-3-(6-((4-(4-((4-(2-((2-(2,6-dioxopiperidin-3-yl)-1,3-dioxoisoindolin-4-yl)oxy)acetyl)piperazin-1-yl)methyl)piperidin-1-yl)phenyl)amino)-9H-purin-9-yl)cyclobutyl)-2-phenylacetamide,
N-((1s,3s)-3-(6-((4-(4-(2-(1-(2-(2,6-dioxopiperidin-3-yl)-1,3-dioxoisoindolin-5-yl)piperidin-4-yl)ethyl)piperazin-1-yl)phenyl)amino)-9H-purin-9-yl)cyclobutyl)-2-phenylacetamide,
N-((1s,3s)-3-(6-((4-(4-(2-(1-(1-(2-(2,6-dioxopiperidin-3-yl)-1,3-dioxoisoindolin-5-yl)azetidin-3-carbonyl)piperidin-4-yl)ethyl)piperazin-1-yl)phenyl)amino)-9H-purin-9-yl)cyclobutyl)-2-phenylacetamide,
N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)phenyl)amino)-9H-purin-9-yl)cyclobutyl)-2-phenylacetamide,
N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)phenyl)amino)-9H-purin-9-yl)cyclobutyl)-2-phenylacetamide,
N-((1s,3s)-3-(6-((4-(4-((4-(1-(2-(2,6-dioxopiperidin-3-yl)-1,3-dioxoisoindolin-5-yl)azetidin-3-carbonyl)piperazin-1-yl)methyl)piperidin-1-yl)phenyl)amino)-9H-purin-9-yl)cyclobutyl)-2-phenylacetamide,
N-((1s,3s)-3-(6-((4-(4-(1-(2-(2,6-dioxopiperidin-3-yl)-1,3-dioxoisoindolin-5-yl)piperidin-4-yl)piperazin-1-yl)phenyl)amino)-9H-purin-9-yl)cyclobutyl)-2-phenylacetamide,
N-((1s,3s)-3-(6-((4-(4-(1-(2-(2,6-dioxopiperidin-3-yl)-6-fluoro-1,3-dioxoisoindolin-5-yl)piperidin-4-yl)piperazin-1-yl)phenyl)amino)-9H-purin-9-yl)cyclobutyl)-2-phenylacetamide,
N-((1s,3s)-3-(6-((4-(4-(1-(3-(2,6-dioxopiperidin-3-yl)-4-oxo-3,4-dihydrobenzo[d][1,2,3]triazin-6-yl)piperidin-4-yl)piperazin-1-yl)phenyl)amino)-9H-purin-9-yl)cyclobutyl)-2-phenylacetamide,
N-((1s,3s)-3-(6-((4-(2-(4-(2-(2,6-dioxopiperidin-3-yl)-1,3-dioxoisoindolin-5-yl)piperazin-1-yl)-7-azaspiro[3.5]nonan-7-yl)phenyl)amino)-9H-purin-9-yl)cyclobutyl)-2-phenylacetamide,
N-((1s,3s)-3-(6-((4-(2-(4-(2-(2,6-dioxopiperidin-3-yl)-6-fluoro-1,3-dioxoisoindolin-5-yl)piperazin-1-yl)-7-azaspiro[3.5]nonan-7-yl)phenyl)amino)-9H-purin-9-yl)cyclobutyl)-2-phenylacetamide,
N-((1s,3s)-3-(6-((4-(2-(4-(3-(2,6-dioxopiperidin-3-yl)-4-oxo-3,4-dihydrobenzo[d][1,2,3]triazin-6-yl)piperazin-1-yl)-7-azaspiro[3.5]nonan-7-yl)phenyl)amino)-9H-purin-9-yl)cyclobutyl)-2-phenylacetamide,
N-((1s,3s)-3-(6-((4-(4-(2-(4-(2-(2,6-dioxopiperidin-3-yl)-1,3-dioxoisoindoIin-5-yl)piperazin-1-yl)ethyl)piperidin-1-yl)phenyl)amino)-9H-purin-9-yl)cyclobutyl)-2-phenylacetamide,
N-((1s,3s)-3-(6-((4-(4-(2-(4-(2-(2,6-dioxopiperidin-3-yl)-6-fluoro-1,3-dioxoisoindolin-5-yl)piperazin-1-yl)ethyl)piperidin-1-yl)phenyl)amino)-9H-purin-9-yl)cyclobutyl)-2-phenylacetamide,
N-((1s,3s)-3-(6-((4-(4-(2-(4-(3-(2,6-dioxopiperidin-3-yl)-4-oxo-3,4-dihydrobenzo[d][1,2,3]triazin-6-yl)piperazin-1-yl)ethyl)piperidin-1-yl)phenyl)amino)-9H-purin-9-yl)cyclobutyl)-2-phenylacetamide,
N-((1s,3s)-3-(6-((4-(4-((4-(2-(2,6-dioxopiperidin-3-yl)-6-fluoro-1,3-dioxoisoindolin-5-yl)piperazin-1-yl)methyl)piperidin-1-yl)phenyl)amino)-9H-purin-9-yl)cyclobutyl)-2-phenylacetamide,
N-((1s,3s)-3-(6-((4-(4-(1-(1-(2-(2,6-dioxopiperidin-3-yl)-1,3-dioxoisoindolin-5-yl)azetidin-3-carbonyl)piperidin-4-yl)piperazin-1-yl)phenyl)amino)-9H-purin-9-yl)cyclobutyl)-2-phenylacetamide,
N-((1s,3s)-3-(6-((4-(4-(1-(1-(2-(2,6-dioxopiperidin-3-yl)-1,3-dioxoisoindolin-5-yl)piperidin-4-carbonyl)piperidin-4-yl)piperazin-1-yl)phenyl)amino)-9H-purin-9-yl)cyclobutyl)-2-phenylacetamide,
N-((1s,3s)-3-(6-((4-(4-(1-(2-(2,6-dioxopiperidin-3-yl)-1,3-dioxoisoindolin-5-yl)azetidin-3-yl)piperazin-1-yl)phenyl)amino)-9H-purin-9-yl)cyclobutyl)-2-phenylacetamide,
N-((1s,3s)-3-(6-((4-(4-(1-(2-(2,6-dioxopiperidin-3-yl)-6-fluoro-1,3-dioxoisoindolin-5-yl)azetidin-3-yl)piperazin-1-yl)phenyl)amino)-9H-purin-9-yl)cyclobutyl)-2-phenylacetamide,
N-((1s,3s)-3-(6-((4-(4-(1-(3-(2,6-dioxopiperidin-3-yl)-4-oxo-3,4-dihydrobenzo[d][1,2,3]triazin-6-yl)azetidin-3-yl)piperazin-1-yl)phenyl)amino)-9H-purin-9-yl)cyclobutyl)-2-phenylacetamide,
N-((1s,3s)-3-(6-((4-(4-(3-(4-(2-(2,6-dioxopiperidin-3-yl)-1,3-dioxoisoindolin-5-yl)piperazin-1-yl)propyl)piperidin-1-yl)phenyl)amino)-9H-purin-9-yl)cyclobutyl)-2-phenylacetamide,
N-((1s,3s)-3-(6-((4-(4-(3-(4-(2-(2,6-dioxopiperidin-3-yl)-6-fluoro-1,3-dioxoisoindolin-5-yl)piperazin-1-yl)propyl)piperidin-1-yl)phenyl)amino)-9H-purin-9-yl)cyclobutyl)-2-phenylacetamide,
N-((1s,3s)-3-(6-((4-(4-(3-(4-(3-(2,6-dioxopiperidin-3-yl)-4-oxo-3,4-dihydrobenzo[d][1,2,3]triazin-6-yl)piperazin-1-yl)propyl)piperidin-1-yl)phenyl)amino)-9H-purin-9-yl)cyclobutyl)-2-phenylacetamide,
N-((1s,3s)-3-(6-((4-(4-(1-(1-(2-(2,6-dioxopiperidin-3-yl)-1,3-dioxoisoindolin-5-yl)piperidin-4-carbonyl)azetidin-3-yl)piperazin-1-yl)phenyl)amino)-9H-purin-9-yl)cyclobutyl)-2-phenylacetamide,
N-((1s,3s)-3-(6-((4-(4-(3-(1-(3-(2,6-dioxopiperidin-3-yl)-4-oxo-3,4-dihydrobenzo[d][1,2,3]triazin-6-yl)piperidin-4-yl)propyl)piperazin-1-yl)phenyl)amino)-9H-purin-9-yl)cyclobutyl)-2-phenylacetamide,
N-((1s,3s)-3-(6-((4-(4-(7-(1-(2-(2,6-dioxopiperidin-3-yl)-1,3-dioxoisoindolin-5-yl)azetidin-3-carbonyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(7-(1-(2-(2,6-dioxopiperidin-3-yl)-1,3-dioxoisoindolin-5-yl)piperidin-4-carbonyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((4-(4-(3-(1-(1-(2-(2,6-dioxopiperidin-3-yl)-1,3-dioxoisoindolin-5-yl)piperidin-4-carbonyl)piperidin-4-yl)propyl)piperazin-1-yl)phenyl)amino)-9H-purin-9-yl)cyclobutyl)-2-phenylacetamide,
N-((1s,3s)-3-(6-((2-(1-((1-(1-(2-(2,6-dioxopiperidin-3-yl)-1,3-dioxoisoindolin-5-yl)piperidin-4-carbonyl)piperidin-4-yl)methyl)piperidin-4-yl)ethyl)amino)-9H-purin-9-yl)cyclobutyl)-6-methylpicolinamide,
N-((1s,3s)-3-(6-((4-(4-(4-(2-(2,6-dioxopiperidin-3-yl)-1,3-dioxoisoindolin-5-yl)piperazin-1-yl)piperidin-1-yl)phenyl)amino)-9H-purin-9-yl)cyclobutyl)-2-phenylacetamide,
N-((1s,3s)-3-(6-((4-(4-(4-(3-(2,6-dioxopiperidin-3-yl)-4-oxo-3,4-dihydrobenzo[d][1,2,3]triazin-6-yl)piperazin-1-yl)piperidin-1-yl)phenyl)amino)-9H-purin-9-yl)cyclobutyl)-2-phenylacetamide,
N-((1s,3s)-3-(6-((4-(3-(4-(2-(2,6-dioxopiperidin-3-yl)-1,3-dioxoisoindolin-5-yl)piperazin-1-yl)azetidin-1-yl)phenyl)amino)-9H-purin-9-yl)cyclobutyl)-2-phenylacetamide,
N-((1s,3s)-3-(6-((4-(3-(4-(2-(2,6-dioxopiperidin-3-yl)-6-fluoro-1,3-dioxoisoindolin-5-yl)piperazin-1-yl)azetidin-1-yl)phenyl)amino)-9H-purin-9-yl)cyclobutyl)-2-phenylacetamide,
N-((1s,3s)-3-(6-((4-(2-(4-(1-(2-(2,6-dioxopiperidin-3-yl)-1,3-dioxoisoindolin-5-yl)azetidin-3-carbonyl)piperazin-1-yl)-7-azaspiro[3.5]nonan-7-yl)phenyl)amino)-9H-purin-9-yl)cyclobutyl)-2-phenylacetamide,
N-((1s,3s)-3-(6-((4-(2-(4-(1-(2-(2,6-dioxopiperidin-3-yl)-1,3-dioxoisoindolin-5-yl)piperidin-4-carbonyl)piperazin-1-yl)-7-azaspiro[3.5]nonan-7-yl)phenyl)amino)-9H-purin-9-yl)cyclobutyl)-2-phenylacetamide,
N-((1s,3s)-3-(6-((4-(4-(2-(4-(1-(2-(2,6-dioxopiperidin-3-yl)-1,3-dioxoisoindolin-5-yl)azetidin-3-carbonyl)piperazin-1-yl)ethyl)piperidin-1-yl)phenyl)amino)-9H-purin-9-yl)cyclobutyl)-2-phenylacetamide,
N-((1s,3s)-3-(6-((4-(4-(2-(4-(1-(2-(2,6-dioxopiperidin-3-yl)-1,3-dioxoisoindolin-5-yl)piperidin-4-carbonyl)piperazin-1-yl)ethyl)piperidin-1-yl)phenyl)amino)-9H-purin-9-yl)cyclobutyl)-2-phenylacetamide,
N-((1s,3s)-3-(6-((4-(4-(4-(1-(2-(2,6-dioxopiperidin-3-yl)-1,3-dioxoisoindolin-5-yl)azetidin-3-carbonyl)piperazin-1-yl)piperidin-1-yl)phenyl)amino)-9H-purin-9-yl)cyclobutyl)-2-phenylacetamide,
N-((1s,3s)-3-(6-((4-(4-(4-(1-(2-(2,6-dioxopiperidin-3-yl)-1,3-dioxoisoindolin-5-yl)piperidin-4-carbonyl)piperazin-1-yl)piperidin-1-yl)phenyl)amino)-9H-purin-9-yl)cyclobutyl)-2-phenylacetamide,
N-((1s,3s)-3-(6-((4-(4-(3-(4-(1-(2-(2,6-dioxopiperidin-3-yl)-1,3-dioxoisoindolin-5-yl)azetidin-3-carbonyl)piperazin-1-yl)propyl)piperidin-1-yl)phenyl)amino)-9H-purin-9-yl)cyclobutyl)-2-phenylacetamide,
N-((1s,3s)-3-(6-((4-(4-(3-(4-(1-(2-(2,6-dioxopiperidin-3-yl)-1,3-dioxoisoindolin-5-yl)piperidin-4-carbonyl)piperazin-1-yl)propyl)piperidin-1-yl)phenyl)amino)-9H-purin-9-yl)cyclobutyl)-2-phenylacetamide,
N-((1s,3s)-3-(6-((4-(3-(4-(3-(2,6-dioxopiperidin-3-yl)-4-oxo-3,4-dihydrobenzo[d][1,2,3]triazin-6-yl)piperazin-1-yl)azetidin-1-yl)phenyl)amino)-9H-purin-9-yl)cyclobutyl)-2-phenylacetamide,
N-((1s,3s)-3-(6-((4-(4-(4-(2-(2,6-dioxopiperidin-3-yl)-6-fluoro-1,3-dioxoisoindolin-5-yl)piperazin-1-yl)piperidin-1-yl)phenyl)amino)-9H-purin-9-yl)cyclobutyl)-2-phenylacetamide,
2,6-dichloro-N-((1s,3s)-3-(6-((4-(4-((4-(3-(2,6-dioxopiperidin-3-yl)-4-oxo-3,4-dihydrobenzo[d][1,2,3]triazin-6-yl)piperazin-1-yl)methyl)piperidin-1-yl)phenyl)amino)-9H-purin-9-yl)cyclobutyl)benzamide,
2,6-dichloro-N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)phenyl)amino)-9H-purin-9-yl)cyclobutyl)benzamide,
N-((1s,3s)-3-(6-((4-(4-(3-(1-(1-(2-(2,6-dioxopiperidin-3-yl)-1,3-dioxoisoindolin-5-yl)azetidin-3-carbonyl)piperidin-4-yl)propyl)piperazin-1-yl)phenyl)amino)-9H-purin-9-yl)cyclobutyl)-2-phenylacetamide,
N-((1s,3s)-3-(6-((4-(3-(4-(1-(2-(2,6-dioxopiperidin-3-yl)-1,3-dioxoisoindolin-5-yl)azetidin-3-carbonyl)piperazin-1-yl)azetidin-1-yl)phenyl)amino)-9H-purin-9-yl)cyclobutyl)-2-phenylacetamide,
N-((1s,3s)-3-(6-((2-(1-((1-((1-(2-(2,6-dioxopiperidin-3-yl)-1,3-dioxoisoindolin-5-yl)piperidin-4-yl)methyl)piperidin-4-yl)methyl)piperidin-4-yl)ethyl)amino)-9H-purin-9-yl)cyclobutyl)-6-methylpicolinamide,
N-((1s,3s)-3-(6-((4-(4-(2-(1-((1-(2-(2,6-dioxopiperidin-3-yl)-1,3-dioxoisoindolin-5-yl)azetidin-3-yl)methyl)piperidin-4-yl)ethyl)piperazin-1-yl)phenyl)amino)-9H-purin-9-yl)cyclobutyl)-2-phenylacetamide,
2,6-dichloro-N-((1s,3s)-3-(6-((4-(4-(7-(1-(2-(2,6-dioxopiperidin-3-yl)-1,3-dioxoisoindolin-5-yl)piperidin-4-carbonyl)-7-azaspiro[3.5]nonan-2-yl)piperazin-1-yl)phenyl)amino)-9H-purin-9-yl)cyclobutyl)benzamide,
N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)-9H-purin-9-yl)cyclobutyl)-6-methylpicolinamide,
N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)phenyl)amino)-9H-purin-9-yl)cyclobutyl)-6-methylpicolinamide,
N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)phenyl)amino)-9H-purin-9-yl)cyclobutyl)-6-methylpicolinamide,
N-((1s,3s)-3-(6-((4-(4-(2-(1-(2-(2,6-dioxopiperidin-3-yl)-1,3-dioxoisoindolin-5-yl)piperidin-4-yl)ethyl)piperazin-1-yl)phenyl)amino)-9H-purin-9-yl)cyclobutyl)-6-methylpicolinamide,
N-((1s,3s)-3-(6-((4-(4-(2-(1-(2-(2,6-dioxopiperidin-3-yl)-6-fluoro-1,3-dioxoisoindolin-5-yl)piperidin-4-yl)ethyl)piperazin-1-yl)phenyl)amino)-9H-purin-9-yl)cyclobutyl)-6-methylpicolinamide,
N-((1s,3s)-3-(6-((4-(4-(2-(1-(3-(2,6-dioxopiperidin-3-yl)-4-oxo-3,4-dihydrobenzo[d][1,2,3]triazin-6-yl)piperidin-4-yl)ethyl)piperazin-1-yl)phenyl)amino)-9H-purin-9-yl)cyclobutyl)-6-methylpicolinamide,
2,6-dichloro-N-((1s,3s)-3-(6-((4-(4-(7-(1-(2-(2,6-dioxopiperidin-3-yl)-1,3-dioxoisoindolin-5-yl)azetidin-3-carbonyl)-7-azaspiro[3.5]nonan-2-yl)piperazin-1-yl)phenyl)amino)-9H-purin-9-yl)cyclobutyl)benzamide,
N-((1s,3s)-3-(6-((4-(4-(7-(2-(2,6-dioxopiperidin-3-yl)-1,3-dioxoisoindolin-5-yl)-7-azaspiro[3.5]nonan-2-yl)piperazin-1-yl)phenyl)amino)-9H-purin-9-yl)cyclobutyl)-6-methylpicolinamide,
N-((1s,3s)-3-(6-((4-(4-(7-(2-(2,6-dioxopiperidin-3-yl)-6-fluoro-1,3-dioxoisoindolin-5-yl)-7-azaspiro[3.5]nonan-2-yl)piperazin-1-yl)phenyl)amino)-9H-purin-9-yl)cyclobutyl)-6-methylpicolinamide,
N-((1s,3s)-3-(6-((4-(4-(7-(3-(2,6-dioxopiperidin-3-yl)-4-oxo-3,4-dihydrobenzo[d][1,2,3]triazin-6-yl)-7-azaspiro[3.5]nonan-2-yl)piperazin-1-yl)phenyl)amino)-9H-purin-9-yl)cyclobutyl)-6-methylpicolinamide,
N-((1s,3s)-3-(6-((4-(4-(7-(1-(2-(2,6-dioxopiperidin-3-yl)-1,3-dioxoisoindolin-5-yl)azetidin-3-carbonyl)-7-azaspiro[3.5]nonan-2-yl)piperazin-1-yl)phenyl)amino)-9H-purin-9-yl)cyclobutyl)-6-methylpicolinamide,
N-((1s,3s)-3-(6-((4-(4-(2-(1-(1-(2-(2,6-dioxopiperidin-3-yl)-1,3-dioxoisoindolin-5-yl)azetidin-3-carbonyl)piperidin-4-yl)ethyl)piperazin-1-yl)phenyl)amino)-9H-purin-9-yl)cyclobutyl)-6-methylpicolinamide,
N-((1s,3s)-3-(6-((4-(4-(2-(1-(1-(2-(2,6-dioxopiperidin-3-yl)-1,3-dioxoisoindolin-5-yl)piperidin-4-carbonyl)piperidin-4-yl)ethyl)piperazin-1-yl)phenyl)amino)-9H-purin-9-yl)cyclobutyl)-6-methylpicolinamide,
N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)phenyl)amino)-9H-purin-9-yl)cyclobutyl)-6-fluoropicolinamide,
6-chloro-N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)phenyl)amino)-9H-purin-9-yl)cyclobutyl)picolinamide,
6-cyano-N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)phenyl)amino)-9H-purin-9-yl)cyclobutyl)picolinamide,
N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)phenyl)amino)-9H-purin-9-yl)cyclobutyl)-6-(trifluoromethyl)picolinamide,
2,6-dichloro-N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)phenyl)amino)-9H-purin-9-yl)cyclobutyl)benzamide,
2,6-dichloro-N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)phenyl)amino)-9H-purin-9-yl)cyclobutyl)benzamide,
2,6-dichloro-N-((1s,3s)-3-(6-((2-(1-((1-(2-(2,6-dioxopiperidin-3-yl)-1,3-dioxoisoindolin-5-yl)piperidin-4-yl)methyl)piperidin-4-yl)ethyl)amino)-9H-purin-9-yl)cyclobutyl)benzamide,
2,6-dichloro-N-((1s,3s)-3-(6-((2-(1-((1-(2-(2,6-dioxopiperidin-3-yl)-6-fluoro-1,3-dioxoisoindolin-5-yl)piperidin-4-yl)methyl)piperidin-4-yl)ethyl)amino)-9H-purin-9-yl)cyclobutyl)benzamide,
2,6-dichloro-N-((1s,3s)-3-(6-((2-(1-((1-(3-(2,6-dioxopiperidin-3-yl)-4-oxo-3,4-dihydrobenzo[d][1,2,3]triazin-6-yl)piperidin-4-yl)methyl)piperidin-4-yl)ethyl)amino)-9H-purin-9-yl)cyclobutyl)benzamide,
2,6-dichloro-N-((1s,3s)-3-(6-((2-(1-((1-(1-(2-(2,6-dioxopiperidin-3-yl)-1,3-dioxoisoindolin-5-yl)azetidin-3-carbonyl)piperidin-4-yl)methyl)piperidin-4-yl)ethyl)amino)-9H-purin-9-yl)cyclobutyl)benzamide,
2,6-dichloro-N-((1s,3s)-3-(6-((2-(1-((1-(1-(2-(2,6-dioxopiperidin-3-yl)-1,3-dioxoisoindolin-5-yl)piperidin-4-carbonyl)piperidin-4-yl)methyl)piperidin-4-yl)ethyl)amino)-9H-purin-9-yl)cyclobutyl)benzamide,
N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)phenyl)amino)-9H-purin-9-yl)cyclobutyl)-6-methylpicolinamide,
N-((1s,3s)-3-(6-((2-(1-((1-((1-(2-(2,6-dioxopiperidin-3-yl)-1,3-dioxoisoindolin-5-yl)azetidin-3-yl)methyl)piperidin-4-yl)methyl)piperidin-4-yl)ethyl)amino)-9H-purin-9-yl)cyclobutyl)-6-methylpicolinamide,
N-((1s,3s)-3-(6-((4-(4-(7-(1-(2-(2,6-dioxopiperidin-3-yl)-1,3-dioxoisoindolin-5-yl)piperidin-4-carbonyl)-7-azaspiro[3.5]nonan-2-yl)piperazin-1-yl)phenyl)amino)-9H-purin-9-yl)cyclobutyl)-6-methylpicolinamide,
N-((1s,3s)-3-(6-((4-(4-(2-(1-(1-(2-(2,6-dioxopiperidin-3-yl)-1,3-dioxoisoindolin-5-yl)piperidin-4-carbonyl)piperidin-4-yl)ethyl)piperazin-1-yl)phenyl)amino)-9H-purin-9-yl)cyclobutyl)-2-phenylacetamide,
2,6-dichloro-N-((1s,3s)-3-(6-((4-(4-(2-(1-(2-(2,6-dioxopiperidin-3-yl)-1,3-dioxoisoindolin-5-yl)piperidin-4-yl)ethyl)piperazin-1-yl)phenyl)amino)-9H-purin-9-yl)cyclobutyl)benzamide,
2,6-dichloro-N-((1s,3s)-3-(6-((4-(4-(2-(1-(2-(2,6-dioxopiperidin-3-yl)-6-fluoro-1,3-dioxoisoindolin-5-yl)piperidin-4-yl)ethyl)piperazin-1-yl)phenyl)amino)-9H-purin-9-yl)cyclobutyl)benzamide,
2,6-dichloro-N-((1s,3s)-3-(6-((4-(4-(2-(1-(3-(2,6-dioxopiperidin-3-yl)-4-oxo-3,4-dihydrobenzo[d][1,2,3]triazin-6-yl)piperidin-4-yl)ethyl)piperazin-1-yl)phenyl)amino)-9H-purin-9-yl)cyclobutyl)benzamide,
N-((1s,3s)-3-(6-((4-(4-(1-(4-(2-(2,6-dioxopiperidin-3-yl)-1,3-dioxoisoindolin-5-yl)butyl)piperidin-4-yl)piperazin-1-yl)phenyl)amino)-9H-purin-9-yl)cyclobutyl)-2-phenylacetamide,
N-((1s,3s)-3-(6-((2-(1-((1-(2-(2,6-dioxopiperidin-3-yl)-6-fluoro-1,3-dioxoisoindolin-5-yl)piperidin-4-yl)methyl)piperidin-4-yl)ethyl)amino)-9H-purin-9-yl)cyclobutyl)-2-phenylacetamide,
N-((1s,3s)-3-(6-((2-(1-((1-(3-(2,6-dioxopiperidin-3-yl)-4-oxo-3,4-dihydrobenzo[d][1,2,3]triazin-6-yl)piperidin-4-yl)methyl)piperidin-4-yl)ethyl)amino)-9H-purin-9-yl)cyclobutyl)-2-phenylacetamide,
N-((1s,3s)-3-(6-((2-(1-((1-(1-(2-(2,6-dioxopiperidin-3-yl)-1,3-dioxoisoindolin-5-yl)azetidin-3-carbonyl)piperidin-4-yl)methyl)piperidin-4-yl)ethyl)amino)-9H-purin-9-yl)cyclobutyl)-2-phenylacetamide,
2,6-dichloro-N-((1s,3s)-3-(6-((4-(4-(2-(1-(1-(2-(2,6-dioxopiperidin-3-yl)-1,3-dioxoisoindolin-5-yl)azetidin-3-carbonyl)piperidin-4-yl)ethyl)piperazin-1-yl)phenyl)amino)-9H-purin-9-yl)cyclobutyl)benzamide,
2,6-dichloro-N-((1s,3s)-3-(6-((4-(4-(2-(1-(1-(2-(2,6-dioxopiperidin-3-yl)-1,3-dioxoisoindolin-5-yl)piperidin-4-carbonyl)piperidin-4-yl)ethyl)piperazin-1-yl)phenyl)amino)-9H-purin-9-yl)cyclobutyl)benzamide,
6-cyano-N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)-9H-purin-9-yl)cyclobutyl)picolinamide,
N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)-9H-purin-9-yl)cyclobutyl)-6-(trifluoromethyl)picolinamide,
6-chloro-N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)-9H-purin-9-yl)cyclobutyl)picolinamide,
6-chloro-N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)phenyl)amino)-9H-purin-9-yl)cyclobutyl)picolinamide,
6-cyano-N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)phenyl)amino)-9H-purin-9-yl)cyclobutyl)picolinamide,
6-chloro-N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)phenyl)amino)-9H-purin-9-yl)cyclobutyl)picolinamide,
6-cyano-N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)phenyl)amino)-9H-purin-9-yl)cyclobutyl)picolinamide,
N-((1s,3s)-3-(6-((2-(1-((1-(1-(2-(2,6-dioxopiperidin-3-yl)-1,3-dioxoisoindolin-5-yl)piperidin-4-carbonyl)piperidin-4-yl)methyl)piperidin-4-yl)ethyl)amino)-9H-purin-9-yl)cyclobutyl)-2-phenylacetamide,
N-((1s,3s)-3-(6-((2-(1-((1-(3-(2-(2,6-dioxopiperidin-3-yl)-1,3-dioxoisoindolin-4-yl)propyl)piperidin-4-yl)methyl)piperidin-4-yl)ethyl)amino)-9H-purin-9-yl)cyclobutyl)-2-phenylacetamide,
N-((1s,3s)-3-(6-((2-(1-((1-(2-(2,6-dioxopiperidin-3-yl)-1,3-dioxoisoindolin-5-yl)piperidin-4-yl)methyl)piperidin-4-yl)ethyl)amino)-9H-purin-9-yl)cyclobutyl)-2-phenylacetamide,
N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)phenyl)amino)-9H-purin-9-yl)cyclobutyl)-6-(trifluoromethyl)picolinamide,
N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)phenyl)amino)-9H-purin-9-yl)cyclobutyl)-6-(trifluoromethyl)picolinamide,
N-((1s,3s)-3-(6-((4-(4-((1-(3-(2-(2,6-dioxopiperidin-3-yl)-1,3-dioxoisoindolin-4-yl)propyl)piperidin-4-yl)methyl)piperazin-1-yl)phenyl)amino)-9H-purin-9-yl)cyclobutyl)-6-(trifluoromethyl)picolinamide,
N-((1s,3s)-3-(6-((4-(4-((1-(4-(2-(2,6-dioxopiperidin-3-yl)-1,3-dioxoisoindolin-4-yl)butyl)piperidin-4-yl)methyl)piperazin-1-yl)phenyl)amino)-9H-purin-9-yl)cyclobutyl)-6-(trifluoromethyl)picolinamide,
N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)-9H-purin-9-yl)cyclobutyl)-6-fluoropicolinamide,
N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)phenyl)amino)-9H-purin-9-yl)cyclobutyl)-6-fluoropicolinamide,
N-((1s,3s)-3-(6-((4-(4-((1-((2-(2,6-dioxopiperidin-3-yl)-1,3-dioxoisoindolin-4-yl)glycyl)piperidin-4-yl)methyl)piperazin-1-yl)phenyl)amino)-9H-purin-9-yl)cyclobutyl)-6-fluoropicolinamide,
N-((1s,3s)-3-(6-((4-(4-((1-(3-(2-(2,6-dioxopiperidin-3-yl)-1,3-dioxoisoindolin-4-yl)propyl)piperidin-4-yl)methyl)piperazin-1-yl)phenyl)amino)-9H-purin-9-yl)cyclobutyl)-6-fluoropicolinamide,
N-((1s,3s)-3-(6-((4-(4-((1-(4-(2-(2,6-dioxopiperidin-3-yl)-1,3-dioxoisoindolin-4-yl)butyl)piperidin-4-yl)methyl)piperazin-1-yl)phenyl)amino)-9H-purin-9-yl)cyclobutyl)-6-fluoropicolinamide,
N-((1s,3s)-3-(6-((4-(4-(2-(1-(2-(4-(2,6-dioxopiperidin-3-yl)phenoxy)acetyl)piperidin-4-yl)ethyl)piperazin-1-yl)phenyl)amino)-9H-purin-9-yl)cyclobutyl)-2-phenylacetamide,
2,6-dichloro-N-((1s,3s)-3-(6-((4-(4-(2-(1-(2-(4-(2,6-dioxopiperidin-3-yl)phenoxy)acetyl)piperidin-4-yl)ethyl)piperazin-1-yl)phenyl)amino)-9H-purin-9-yl)cyclobutyl)benzamide,
N-((1s,3s)-3-(6-((4-(4-(7-(2-(4-(2,6-dioxopiperidin-3-yl)phenoxy)acetyl)-7-azaspiro[3.5]nonan-2-yl)piperazin-1-yl)phenyl)amino)-9H-purin-9-yl)cyclobutyl)-6-methylpicolinamide,
N-((1s,3s)-3-(6-((4-(4-((1-(2-(4-(2,6-dioxopiperidin-3-yl)phenoxy)acetyl)piperidin-4-yl)methyl)piperazin-1-yl)phenyl)amino)-9H-purin-9-yl)cyclobutyl)-6-(trifluoromethyl)picolinamide,
2,6-dichloro-N-((1s,3s)-3-(6-((4-(4-((1-(2-(4-(2,6-dioxopiperidin-3-yl)phenoxy)acetyl)piperidin-4-yl)methyl)piperazin-1-yl)phenyl)amino)-9H-purin-9-yl)cyclobutyl)benzamide,
N-((1s,3s)-3-(6-((4-(4-(2-(1-(2-(4-(2,6-dioxopiperidin-3-yl)phenoxy)acetyl)piperidin-4-yl)ethyl)piperazin-1-yl)phenyl)amino)-9H-purin-9-yl)cyclobutyl)-6-(trifluoromethyl)picolinamide,
N-((1s,3s)-3-(6-((4-(4-(7-(2-(4-(2,6-dioxopiperidin-3-yl)phenoxy)acetyl)-7-azaspiro[3.5]nonan-2-yl)piperazin-1-yl)phenyl)amino)-9H-purin-9-yl)cyclobutyl)-6-(trifluoromethyl)picolinamide,
6-chloro-N-((1s,3s)-3-(6-((4-(4-((1-(2-(4-(2,6-dioxopiperidin-3-yl)phenoxy)acetyl)piperidin-4-yl)methyl)piperazin-1-yl)phenyl)amino)-9H-purin-9-yl)cyclobutyl)picolinamide,
6-cyano-N-((1s,3s)-3-(6-((4-(4-((1-(2-(2,6-dioxopiperidin-3-yl)-1-oxoisoindolin-5-yl)piperidin-4-yl)methyl)piperazin-1-yl)phenyl)amino)-9H-purin-9-yl)cyclobutyl)picolinamide,
2,6-dichloro-N-((1s,3s)-3-(6-((4-(4-(7-(2-(4-(2,6-dioxopiperidin-3-yl)phenoxy)acetyl)-7-azaspiro[3.5]nonan-2-yl)piperazin-1-yl)phenyl)amino)-9H-purin-9-yl)cyclobutyl)benzamide,
N-((1s,3s)-3-(6-((4-(4-((1-(2-(4-(2,6-dioxopiperidin-3-yl)phenoxy)acetyl)piperidin-4-yl)methyl)piperazin-1-yl)phenyl)amino)-9H-purin-9-yl)cyclobutyl)-6-methylpicolinamide,
N-((1s,3s)-3-(6-((4-(4-(2-(1-(2-(4-(2,6-dioxopiperidin-3-yl)phenoxy)acetyl)piperidin-4-yl)ethyl)piperazin-1-yl)phenyl)amino)-9H-purin-9-yl)cyclobutyl)-6-methylpicolinamide,
N-((1s,3s)-3-(6-((4-(4-((1-(2-(2,6-dioxopiperidin-3-yl)-1,3-dioxoisoindolin-5-yl)piperidin-4-yl)methyl)piperazin-1-yl)-2-methylphenyl)amino)-9H-purin-9-yl)cyclobutyl)-6-methylpicolinamide,
N-((1s,3s)-3-(6-((4-(4-((1-(2-(2,6-dioxopiperidin-3-yl)-6-fluoro-1,3-dioxoisoindolin-5-yl)piperidin-4-yl)methyl)piperazin-1-yl)-2-methylphenyl)amino)-9H-purin-9-yl)cyclobutyl)-6-methylpicolinamide,
N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)-2-methylphenyl)amino)-9H-purin-9-yl)cyclobutyl)-6-methylpicolinamide,
N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)-2-methylphenyl)amino)-9H-purin-9-yl)cyclobutyl)-6-methylpicolinamide,
N-((1s,3s)-3-(6-((4-(4-(2-(1-(1-(2-(2,6-dioxopiperidin-3-yl)-6-fluoro-1,3-dioxoisoindolin-5-yl)piperidin-4-carbonyl)piperidin-4-yl)ethyl)piperazin-1-yl)phenyl)amino)-9H-purin-9-yl)cyclobutyl)-6-methylpicolinamide,
N-((1s,3s)-3-(6-((2-(1-((1-(1-(2-(2,6-dioxopiperidin-3-yl)-6-fluoro-1,3-dioxoisoindolin-5-yl)piperidin-4-carbonyl)piperidin-4-yl)methyl)piperidin-4-yl)ethyl)amino)-9H-purin-9-yl)cyclobutyl)-6-methylpicolinamide,
6-chloro-N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)phenyl)amino)-9H-purin-9-yl)cyclobutyl)picolinamide,
6-chloro-N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)phenyl)amino)-9H-purin-9-yl)cyclobutyl)picolinamide,
6-chloro-N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)phenyl)amino)-9H-purin-9-yl)cyclobutyl)picolinamide,
6-cyano-N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)phenyl)amino)-9H-purin-9-yl)cyclobutyl)picolinamide,
6-cyano-N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)phenyl)amino)-9H-purin-9-yl)cyclobutyl)picolinamide,
N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)phenyl)amino)-9H-purin-9-yl)cyclobutyl)-2-phenylacetamide,
N-((1s,3s)-3-(6-((4-(4-(2-(1-(1-(2-(2,6-dioxopiperidin-3-yl)-6-fluoro-1,3-dioxoisoindolin-5-yl)piperidin-4-carbonyl)piperidin-4-yl)ethyl)piperazin-1-yl)phenyl)amino)-9H-purin-9-yl)cyclobutyl)-2-phenylacetamide,
N-((1s,3s)-3-(6-((4-(4-(3-(1-(2-(2,6-dioxopiperidin-3-yl)-6-fluoro-1,3-dioxoisoindolin-5-yl)piperidin-4-yl)propyl)piperazin-1-yl)phenyl)amino)-9H-purin-9-yl)cyclobutyl)-2-phenylacetamide,
N-((1s,3s)-3-(6-((4-(4-(3-(1-(1-(2-(2,6-dioxopiperidin-3-yl)-6-fluoro-1,3-dioxoisoindolin-5-yl)piperidin-4-carbonyl)piperidin-4-yl)propyl)piperazin-1-yl)phenyl)amino)-9H-purin-9-yl)cyclobutyl)-2-phenylacetamide,
N-((1s,3s)-3-(6-((4-(4-(7-(1-(2-(2,6-dioxopiperidin-3-yl)-6-fluoro-1,3-dioxoisoindolin-5-yl)piperidin-4-carbonyl)-7-azaspiro[3.5]nonan-2-yl)piperazin-1-yl)phenyl)amino)-9H-purin-9-yl)cyclobutyl)-2-phenylacetamide,
N-((1s,3s)-3-(6-((2-(1-((1-(1-(2-(2,6-dioxopiperidin-3-yl)-6-fluoro-1,3-dioxoisoindolin-5-yl)piperidin-4-carbonyl)piperidin-4-yl)methyl)piperidin-4-yl)ethyl)amino)-9H-purin-9-yl)cyclobutyl)-2-phenylacetamide,
2,6-dichloro-N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)phenyl)amino)-9H-purin-9-yl)cyclobutyl)benzamide,
2,6-dichloro-N-((1s,3s)-3-(6-((4-(4-(2-(1-(1-(2-(2,6-dioxopiperidin-3-yl)-6-fluoro-1,3-dioxoisoindolin-5-yl)piperidin-4-carbonyl)piperidin-4-yl)ethyl)piperazin-1-yl)phenyl)amino)-9H-purin-9-yl)cyclobutyl)benzamide,
2,6-dichloro-N-((1s,3s)-3-(6-((4-(4-(7-(1-(2-(2,6-dioxopiperidin-3-yl)-6-fluoro-1,3-dioxoisoindolin-5-yl)piperidin-4-carbonyl)-7-azaspiro[3.5]nonan-2-yl)piperazin-1-yl)phenyl)amino)-9H-purin-9-yl)cyclobutyl)benzamide,
N-((1s,3s)-3-(6-((4-(4-(7-(1-(2-(2,6-dioxopiperidin-3-yl)-6-fluoro-1,3-dioxoisoindolin-5-yl)piperidin-4-carbonyl)-7-azaspiro[3.5]nonan-2-yl)piperazin-1-yl)phenyl)amino)-9H-purin-9-yl)cyclobutyl)-6-methylpicolinamide,
N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)phenyl)amino)-9H-purin-9-yl)cyclobutyl)-6-fluoropicolinamide,
N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)phenyl)amino)-9H-purin-9-yl)cyclobutyl)-6-fluoropicolinamide,
N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)phenyl)amino)-9H-purin-9-yl)cyclobutyl)-6-fluoropicolinamide,
N-((1s,3s)-3-(6-((4-(4-((1-(1-(2-(2,6-dioxopiperidin-3-yl)-1,3-dioxoisoindolin-5-yl)azetidin-3-carbonyl)piperidin-4-yl)methyl)piperazin-1-yl)phenyl)amino)-9H-purin-9-yl)cyclobutyl)-6-(trifluoromethyl)picolinamide,
N-((1s,3s)-3-(6-((4-(4-((1-(1-(2-(2,6-dioxopiperidin-3-yl)-1,3-dioxoisoindolin-5-yl)piperidin-4-carbonyl)piperidin-4-yl)methyl)piperazin-1-yl)phenyl)amino)-9H-purin-9-yl)cyclobutyl)-6-(trifluoromethyl)picolinamide,
N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)phenyl)amino)-9H-purin-9-yl)cyclobutyl)-6-(trifluoromethyl)picolinamide,
6-cyano-N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)phenyl)amino)-9H-purin-9-yl)cyclobutyl)picolinamide,
2,6-dichloro-N-((1s,3s)-3-(6-((4-(4-((4-(1-(2-(2,6-dioxopiperidin-3-yl)-1,3-dioxoisoindolin-5-yl)azetidin-3-carbonyl)piperazin-1-yl)methyl)piperidin-1-yl)phenyl)amino)-9H-purin-9-yl)cyclobutyl)benzamide,
2,6-dichloro-N-((1s,3s)-3-(6-((4-(4-((4-(1-(2-(2,6-dioxopiperidin-3-yl)-1,3-dioxoisoindolin-5-yl)piperidin-4-carbonyl)piperazin-1-yl)methyl)piperidin-1-yl)phenyl)amino)-9H-purin-9-yl)cyclobutyl)benzamide,
2,6-dichloro-N-((1s,3s)-3-(6-((4-(4-((4-(1-(2-(2,6-dioxopiperidin-3-yl)-6-fluoro-1,3-dioxoisoindolin-5-yl)piperidin-4-carbonyl)piperazin-1-yl)methyl)piperidin-1-yl)phenyl)amino)-9H-purin-9-yl)cyclobutyl)benzamide,
N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)phenyl)amino)-9H-purin-9-yl)cyclobutyl)-6-methylpicolinamide,
N-((1s,3s)-3-(6-((4-(4-((1-(1-(2-(2,6-dioxopiperidin-3-yl)-6-fluoro-1,3-dioxoisoindolin-5-yl)piperidin-4-carbonyl)piperidin-4-yl)methyl)piperazin-1-yl)-2-methylphenyl)amino)-9H-purin-9-yl)cyclobutyl)-6-methylpicolinamide,
N-((1s,3s)-3-(6-((4-(4-((1-(3-(2,6-dioxopiperidin-3-yl)-4-oxo-3,4-dihydrobenzo[d][1,2,3]triazin-6-yl)piperidin-4-yl)methyl)piperazin-1-yl)-2-methylphenyl)amino)-9H-purin-9-yl)cyclobutyl)-6-methylpicolinamide, or
N-((1s,3s)-3-(6-((4-(4-(7-(4-(4-(2-(2,6-dioxopiperidin-3-yl)-1,3-dioxoisoindolin-5-yl)piperazin-1-yl)but-2-en-1-yl)-7-azaspiro[3.5]nonan-2-yl)piperazin-1-yl)phenyl)amino)-9H-purin-9-yl)cyclobutyl)-2-phenylacetamide.

12. A method for preparing a compound according to any one of claims 1 to 11 or a pharmaceutically acceptable salt thereof, comprising:
a first step of reacting a compound of the Formula 4 below with a derivative of moiety A, comprising an amine group at one end and a protecting group at the other end, and deprotecting the compound to prepare a compound of Formula 5;
a second step of reacting the compound of Formula 5 with a derivative of an L1 moiety, comprising a reactive functional group X₂ at one end and a protecting group at the other end, and deprotecting the compound to prepare a compound of Formula 6; and
a third step of reacting the compound of Formula 5 with a CRBN binder, comprising a reactive functional group X₃ at one end. 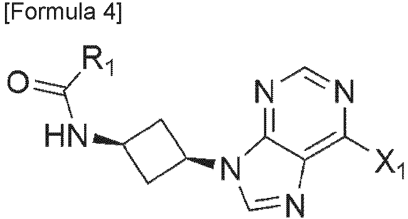 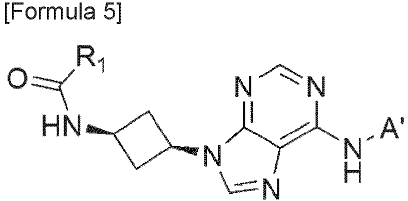 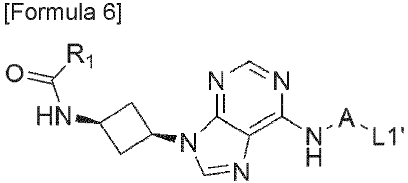
Wherein in Formulas 4 to 6 above:
X₁, X₂, and X₃ are each independently halogen or hydroxy;
the protecting group is tert-butoxycarbonyl; and
A' and L1' are identical to A and L1, respectively, or in the form of a salt thereof.

13. The method of claim 12,
wherein the CRBN binder is any one selected from the group consisting of

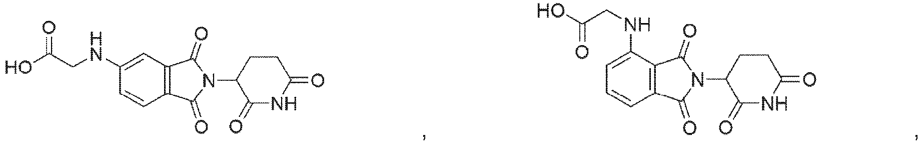

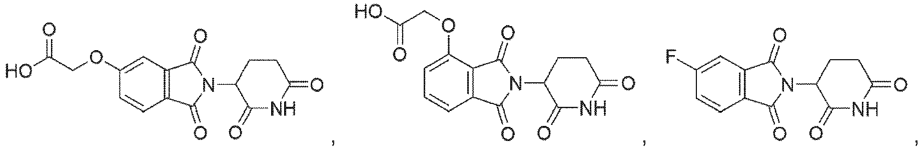

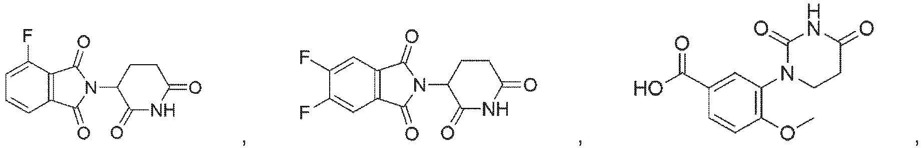

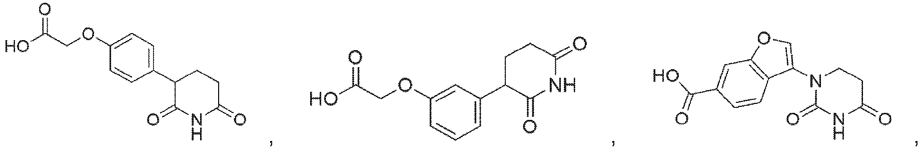

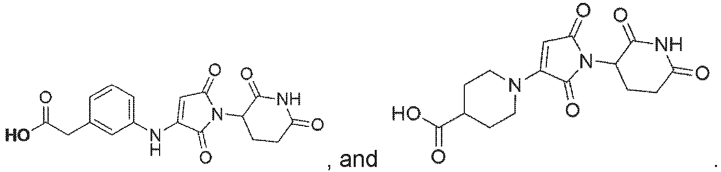

14. The method of claim 12, wherein the first step is performed by reacting a compound, in which X₁ is halogen, by heating at a temperature of 70°C to 100°C in a lower alcohol solvent and deprotecting by treating with a strong acid solution in an organic solvent.

15. The method of claim 12, wherein the second step is performed by:
i) reacting a compound, in which X₂ is hydroxy, with 1-ethyl-3-(3-dimethylaminopropyl)carbodiimide (EDCI), hydroxybenzotriazole (HOBt), and N,N-diisopropylethylamine (DIPEA) in an organic solvent at a temperature of 15°C to 40°C;
ii) reacting a compound, in which X₂ is halogen, in an organic solvent under basic conditions at a temperature of 40°C to 80°C;
iii) reacting a compound, in which X₂ is oxo, with DIPEA and sodium triacetoxyborohydride (NaBH(OAc)₃) at a temperature of 15°C to 40°C; or
iv) reacting a compound, in which X₂ is oxo, with sodium cyanoborohydride (NaBH(OAc)₃) in a lower alcohol solvent in the presence of acetic acid at a temperature of 15°C to 40°C, and
deprotecting by treating with a strong acid solution in an organic solvent.

16. The method of claim 12, wherein the third step is performed by:
i) reacting a compound, in which X₃ is hydroxy, with EDCL, HOBt, and DIPEA in an organic solvent at a temperature of 15°C to 40°C; or
ii) reacting a compound, in which X₃ is halogen, with DIPEA in an organic solvent at a temperature of 70°C to 100°C.

17. A compound comprising a compound according to any one of claims 1 to 11 or pharmaceutically acceptable salt thereof, and a pharmaceutically acceptable carrier.

18. A pharmaceutical composition for inhibiting or degrading cyclin-dependent kinase 2 (CDK2), cyclin-dependent kinase 9 (CDK9), or both, comprising a compound according to any one of claims 1 to 11 or a pharmaceutically acceptable salt thereof as an active ingredient.

19. A pharmaceutical composition for preventing or treating cancer or human immunodeficiency virus (HIV) infection, comprising a compound according to any one of claims 1 to 11 or a pharmaceutically acceptable salt thereof as an active ingredient.

20. The pharmaceutical composition of claim 19, wherein the cancer is prostate cancer, lymphoma, glioblastoma, neuroblastoma, ovarian cancer, hepatocellular carcinoma, liver cancer, breast cancer, leukemia, pancreatic cancer, colon cancer, lung cancer, colorectal cancer, or multiple myeloma.
